# EUROPEAN PATENT APPLICATION

(11) **EP 1 810 965 A1**
(43) Date of publication of application: **25.07.2007**
(21) Application number: 05793650.2
(22) Date of filing: 13.10.2005
(51) Int. Cl.: C07C 257/18, A61K 31/16, A61K 31/196, A61K 31/275, A61K 31/341, A61K 31/381, A61K 31/4035, A61K 31/404, A61K 31/417, A61K 31/4178, A61K 31/4192, A61K 31/44, A61K 31/4402, A61K 31/4406, A61K 31/4409, A61K 31/455, A61K 31/47, A61K 31/4965, A61K 31/50, A61K 31/505

(54) **HYDRAZIDE DERIVATIVES**

(30) Priority: 13.10.2004 JP 2004298379
(71) Applicant: Eisai R&D Management Co., Ltd., Tokyo 112-8088 (JP)
(72) Inventor: CLARK, Richard, Ibaraki 3002635 (JP); HIROTA, Shinsuke, Ibaraki 302635 (JP); AZUMA, Hiroshi, Ibaraki 3002635 (JP); KIRA, Kazunobu, Ibaraki 3002635 (JP); WATANABE, Nobuhisa, Ibaraki, 3002635 (JP); NAGAKURA, Tadashi, Ibaraki 3002635 (JP); HORIZOE, Tatsuo, Ibaraki 3002635 (JP)
(74) Representative: HOFFMANN EITLE
(86) International application number: PCT/JP2005/018853
(87) International publication number: WO 2006/041119

(57) **Abstract**

A compound represented by the following general formula (1) or a salt thereof or a hydrate of the foregoing is safe while exhibiting suitable physicochemical stability, and is useful as therapeutic or prophylactic agents for diseases associated with thrombus formation. 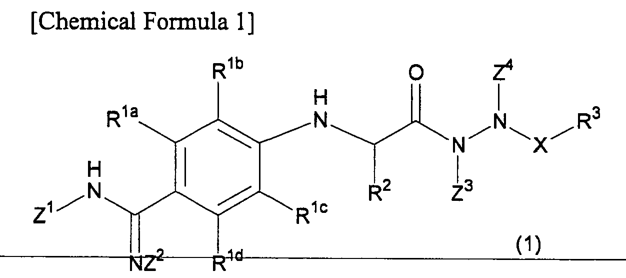 wherein R^{1a}, R^{1b}, R^{1c} and R^{1d} each independently represent hydrogen, etc., R² represents optionally substituted phenyl, etc., R³ represents optionally substituted C6-10 aryl, etc., Z¹, Z² and Z³ each independently represent hydrogen, etc., Z⁴ represents hydrogen, etc. and X represents a single bond or -CO-, etc.

## Description

### Technical Field

The present invention relates to novel hydrazide derivatives which are useful as medicaments, to their pharmacologically acceptable salts, or hydrates thereof, and to therapeutic or prophylactic agents for diseases associated with thrombus formation comprising the same as active ingredients.

### Background Art

Living organisms with damaged blood vessels avoid hemorrhage death by rapid production of thrombin. However, excess production of thrombin due to inflammatory reaction in damaged blood vessels causes thrombosis, which impairs the function of essential organs. Thrombin inhibitors such as heparin and warfarin, which inhibit thrombin production or directly block thrombin activity, have long been used as anticoagulants to treat or prevent thrombosis. Still, it cannot be said that such medicaments are very satisfactory from a medical standpoint, and efforts continue throughout the world toward research and development of new orally administrable anticoagulants with excellent dose response and low risk of bleeding.

The blood clotting mechanism has been classified into two pathways, the "intrinsic clotting pathway" which begins with activation of factor XII (FXII) upon contact with negative charged substances, and the "extrinsic clotting pathway" which is activated by tissue factor (TF) and factor VII (FVII). Since the pathology of thrombosis onset is associated with specific expression of TF, it has been suggested that extrinsic clotting is of major importance. Compounds that inhibit clotting factor VIIa, which is furthest upstream in the extrinsic clotting pathway of the clotting cascade, are thought to have potential use as therapeutic and/or prophylactic agents for diseases associated with thrombus formation, such as thrombosis, in which the extrinsic clotting mechanism plays a part.

As compounds that inhibit clotting factor VIIa there are known in the prior art amidinonaphthol derivatives (see Non-patent document 1), amidino derivatives (see Patent document 1), N-sulfonyl dipeptide derivatives (see Patent document 2), 6-[[(allyl)oxy]methyl]naphthalene-2-carboxyimidamide derivatives (see Patent document 3) and phenylglycine derivatives (Patent documents 4 and 5).

However, these known compounds are inadequate from the standpoint of inhibition activity against clotting factor VIIa, blood clotting effects and thrombosis-treating effects.

Also, no therapeutic agents with hydrazide structures are known for diseases associated with thrombus formation.

[Non-patent document 1] Tetrahedron, 55, p.6219, 1999
[Patent document 1] EP 1078917
[Patent document 2] WO 00/58346
[Patent document 3] WO 00/66545
[Patent document 4] WO 00/35858
[Patent document 5] WO 00/41531

### Disclosure of the Invention

### Problems to be Solved by the Invention

It is an object of the present invention, which has been accomplished in light of the aforementioned problems of the prior art, to provide novel hydrazide derivatives having serine protease inhibitory activity, and particularly excellent inhibitory activity against clotting factor VIIa, as well as their pharmacologically acceptable salts and hydrates thereof, and therapeutic and/or prophylactic agents for diseases associated with thrombus formation, that employ the foregoing.

### Means for Solving the Problems

As a result of much diligent research in light of the circumstances described above, the present inventors have succeeded in synthesizing novel hydrazide derivatives having a specific chemical structure, and have completed this invention upon discovering that these compounds have excellent inhibitory activity against clotting factor VIIa, and particularly that they are useful as therapeutic and/or prophylactic agents for diseases associated with thrombus formation. In other words, the present invention provides the following [1]-[35].
[1] A compound represented by the following general formula (1) or a salt thereof or a hydrate of the foregoing: 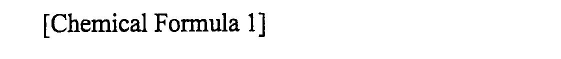 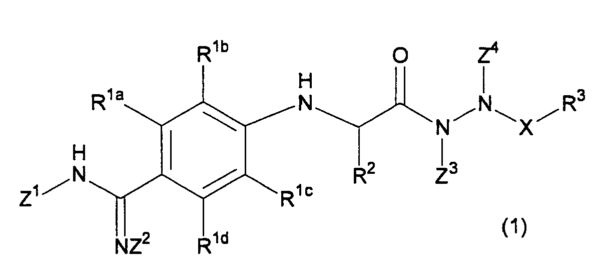 wherein R^{1a}, R^{1b}, R^{1c} and R^{1d} each independently represent hydrogen, C1-6 alkyl or halogen;
   R² represents phenyl optionally having 1-5 substituents selected from Group A1 below;
   R³ represents hydrogen, C1-6 alkyl, C3-8 cycloalkyl optionally having 1-5 substituents selected from Group A1 below, 5- or 6-membered non-aromatic heterocyclyl optionally having 1-5 substituents selected from Group A1 below, C6-10 aryl optionally having 1-5 substituents selected from Group A1 below, 5- to 10-membered heteroaryl optionally having 1-5 substituents selected from Group A1 below, C6-10 arylmethyl optionally having 1-5 substituents selected from Group A1 below, C6-10 arylamino optionally having 1-5 substituents selected from Group A1 below, 5- to 10-membered heteroarylmethyl optionally having 1-5 substituents selected from Group A1 below or 5- to 10-membered heteroarylamino optionally having 1-5 substituents selected from Group A1 below;
   Z¹, Z² and Z³ each independently represent hydrogen or C1-6 alkyl;
   Z⁴ represents hydrogen or C1-6 alkyl;
   X represents a single bond or -SO₂-, -CO- or -CS-;
   Group A1 consists of hydroxyl, halogen, cyano, carboxyl, carbamoyl, nitro, C1-6 alkyl optionally having 1-3 substituents selected from Group B1 below, C3-8 cycloalkyl optionally having 1-5 substituents selected from Group C1 below, C2-6 alkenyl, C2-6 alkynyl, C1-6 alkoxy optionally having 1-3 substituents selected from Group B1 below, C3-8 cycloalkyloxy optionally having 1-5 substituents selected from Group C1 below, C2-6 alkenyloxy, C2-6 alkynyloxy, C1-6 alkylthio, C1-6 alkylsulfinyl, C1-6 alkylsulfonyl, C2-7 alkylcarbonyl, C6-10 aryl optionally having 1-5 substituents selected from Group C1 below, C6-10 aryloxy optionally having 1-5 substituents selected from Group C1 below, 5- to 10-membered heteroaryl optionally having 1-5 substituents selected from Group C1 below, 5- to 10-membered heteroaryloxy optionally having 1-5 substituents selected from Group C1 below, 5- or 6-membered non-aromatic heterocyclyl optionally having 1-5 substituents selected from Group C1 below, 5- or 6-membered non-aromatic heterocyclooxy optionally having 1-5 substituents selected from Group C1 below, and -NR^{1t}-R^{2t} wherein R^{1t} and R^{2t} each independently represent hydrogen, C1-6 alkyl, C2-7 alkylcarbonyl, C6-10 aryl optionally having 1-5 substituents selected from Group C1 below or 5- to 10-membered heteroaryl optionally having 1-5 substituents selected from Group C1 below;
   Group B1 consists of halogen, C1-6 alkoxy, C3-8 cycloalkyl, amino, mono(C1-6 alkyl)amino, di(C1-6 alkyl)amino, carbamoyl, mono(C1-6 alkyl)aminocarbonyl, di(C1-6 alkyl)aminocarbonyl, C6-10 aryl optionally having 1-5 substituents selected from Group C1 below and 5- to 10-membered heteroaryl optionally having 1-5 substituents selected from Group C1 below; and
   Group C1 consists of halogen, C1-6 alkyl and C1-6 alkoxy.
[2] A compound according to [1] above or a salt thereof or a hydrate of the foregoing, wherein R^{1a}, R^{1b}, R^{1c} and R^{1d} are hydrogen.
[3] A compound according to [1] or [2] above or a salt thereof or a hydrate of the foregoing, wherein Z¹ is hydrogen.
[4] A compound according to any one of [1] to [3] above or a salt thereof or a hydrate of the foregoing, wherein Z² is hydrogen.
[5] A compound according to any one of claims 1 to 4 or a salt thereof or a hydrate of the foregoing, wherein Z³ is hydrogen.
[6] A compound according to any one of [1] to [5] above or a salt thereof or a hydrate of the foregoing, wherein Z⁴ is hydrogen.
[7] A compound according to any one of [1] to [6] above or a salt thereof or a hydrate of the foregoing, wherein X is a single bond, -SO₂- or -CO-.
[8] A compound according to any one of [1] to [6] above or a salt thereof or a hydrate of the foregoing, wherein X is a single bond or -CO-.
[9] A compound according to any one of [1] to [8] above or a salt thereof or a hydrate of the foregoing, wherein R² is phenyl optionally having 1-3 substituents selected from Group A2 below;
   Group A2 consists of C1-6 alkoxy optionally having a group selected from Group B2 below, C1-6 alkyl, C1-6 alkoxy-C1-6 alkyl, C3-8 cycloalkyloxy, C2-6 alkenyloxy, C2-6 alkynyloxy, benzyloxy, pyridylmethoxy, hydroxyl and halogen; and
   Group B2 consists of fluorine, C1-6 alkoxy, amino, mono(C1-6 alkyl)amino, di(C1-6 alkyl)amino, carbamoyl, mono(C1-6 alkyl)aminocarbonyl and di(C1-6 alkyl)aminocarbonyl.
[10] A compound according to any one of [1] to [8] above or a salt thereof or a hydrate of the foregoing, wherein R² is phenyl having 2 or 3 substituents selected from Group A3 below;
   Group A3 consists of C1-6 alkoxy optionally having a group selected from Group B3 below, C2-6 alkenyloxy, hydroxyl, and halogen; and
   Group B3 consists of fluorine, C1-6 alkoxy, amino, mono(C1-6 alkyl)amino, di(C1-6 alkyl)amino and di(C1-6 alkyl)aminocarbonyl.
[11] A compound according to any one of [1] to [8] above or a salt thereof or a hydrate of the foregoing, wherein R² is phenyl having 2 or 3 substituents selected from Group A4 below; and
   Group A4 consists of methoxy, ethoxy, isopropoxy, n-propoxy, dimethylaminocarbonylmethoxy, 2-dimethylaminoethoxy, 2-amino-2-methylpropoxy, 2-dimethylamino-1-methylethoxy, 2-methoxyethoxy, allyloxy, hydroxyl, fluorine and 2-fluoroethoxy.
[12] A compound according to any one of [1] to [8] above or a salt thereof or a hydrate of the foregoing, wherein R² is phenyl having 2 or 3 substituents represented by the following formula: 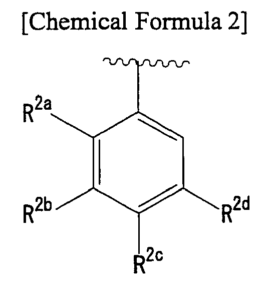 wherein R^{2a} represents hydrogen or fluorine;
   R^{2b} represents hydrogen, methoxy, isopropoxy, n-propoxy, allyloxy or 2-fluoroethoxy; and
   R^{2c} and R^{2d} each independently represent hydrogen, methoxy, ethoxy, isopropoxy, n-propoxy, dimethylaminocarbonylmethoxy, 2-dimethylaminoethoxy, 2-amino-2-methylpropoxy, 2-dimethylamino-1-methylethoxy, 2-methoxyethoxy, allyloxy, hydroxyl, fluorine or 2-fluoroethoxy.
[13] A compound according to any one of [1] to [8] above or a salt thereof or a hydrate of the foregoing, wherein R² is 3-ethoxy-4-dimethylaminocarbonylmethoxyphenyl, 3,4-diallyloxyphenyl, 3-ethoxy-4-(2-dimethylaminoethoxy)phenyl, 3-ethoxy-4-(2-methoxyethoxy)phenyl, 3,4-diethoxyphenyl, 3,5-dimethoxy-4-hydroxyphenyl, 3-ethoxy-6-fluoro-4-isopropoxyphenyl, 3-ethoxy-4-isopropoxyphenyl, 3-methoxy-4-isopropoxyphenyl, 3,4-dimethoxy-6-fluorophenyl, 3,4,5-trimethoxyphenyl, 3-methoxy-5-n-propoxy-6-fluorophenyl, 3-methoxy-5-allyloxy-6-fluorophenyl, 3-methoxy-5-isopropoxy-6-fluorophenyl, 3-methoxy-5-(2-fluoroethoxy)-6-fluorophenyl, 4-(2-amino-2-methylpropoxy)-3-ethoxyphenyl or 4-(2-dimethylamino-1-methylethoxy)-3-ethoxyphenyl.
[14] A compound according to any one of [1] to [13] above or a salt thereof or a hydrate of the foregoing, wherein R³ is 5- or 6-membered non-aromatic heterocyclyl optionally having 1-3 substituents selected from Group D1 below, phenyl optionally having 1-3 substituents selected from Group D1 below, 5- to 10-membered heteroaryl optionally having 1-3 substituents selected from Group D1 below, C6-10 arylmethyl optionally having 1-3 substituents selected from Group D1 below or 5- to 10-membered heteroarylmethyl optionally having 1-3 substituents selected from Group D 1 below; and
   Group D1 consists of hydroxyl, halogen, cyano, nitro, carboxyl, carbamoyl, C2-7 alkylcarbonyl, C1-6 alkyl, C1-6 alkyl having 1-3 halogen, C1-6 alkoxy, C1-6 alkylthio, C1-6 alkylsulfonyl, amino, mono(C1-6 alkyl)amino, di(C1-6 alkyl)amino, mono(C2-7 alkylcarbonyl)amino, di(C2-7 alkylcarbonyl)amino, phenyl, phenoxy, benzyloxy, 5-tetrazolyl, pyrrolyl and morpholino.
[15] A compound according to any one of [1] to [13] above or a salt thereof or a hydrate of the foregoing, wherein R³ is phenyl, indazolyl, furyl, pyridyl, N-oxypyridyl, pyrimidinyl, thienyl, pyrazinyl, benzotriazolyl, pyridonyl, benzyl, pyridylmethyl or thienylmethyl and R³ optionally has 1-3 substituents selected from Group D2 below; and
   Group D2 consists of hydroxyl, fluorine, chlorine, bromine, cyano, nitro, carboxyl, carbamoyl, acetyl, methyl, ethyl, trifluoromethyl, methoxy, ethoxy, n-propoxy, isopropoxy, benzyloxy, phenoxy, methylthio, methylsulfonyl, amino, methylamino, dimethylamino, acetylamino, phenyl, 5-tetrazolyl, pyrrolyl and morpholino.
[16] A compound according to any one of [1] to [13] above or a salt thereof or a hydrate of the foregoing, wherein R³ is (1) phenyl optionally having a group selected from the group consisting of hydroxyl, fluorine, chlorine, bromine, cyano, nitro, carboxyl, acetylamino, methylsulfonyl and methoxy, (2) pyridyl optionally having a group selected from the group consisting of fluorine, chlorine, bromine, methyl, dimethylamino and carboxyl, (3) N-oxypyridyl, (4) pyrimidinyl, (5) N-methylpyridonyl, (6) pyridylmethyl or (7) thienylmethyl.
[17] A compound according to any one of [1] to [13] above or a salt thereof or a hydrate of the foregoing, wherein R³ is 2-pyridyl, 3-bromopyridin-2-yl, 3-carboxypyridin-2-yl, 3-methylpyridin-2-yl, 3-pyridyl, 2-chloropyridin-3-yl, 2-fluoropyridin-3-yl, 2-methylpyridin-3-yl, 4-chloropyridin-3-yl, 4-methylpyridin-3-yl, 2-(dimethylamino)pyridin-3-yl, 4-pyridyl, 3-bromopyridin-4-yl, 3-chloropyridin-4-yl, 3-fluoropyridin-4-yl, 3-methylpyridin-4-yl, 2-pyrimidinyl, (pyridine-N-oxide)-3-yl, (pyridine-N-oxide)-2-yl, 3-pyridylmethyl, 3-thienylmethyl, N-methyl-2-pyridon-5-yl, N-methyl-4-pyridon-3-yl, phenyl, 2-bromophenyl, 2-chlorophenyl, 2-fluorophenyl, 2-cyanophenyl, 2-carboxyphenyl, 2-nitrophenyl, 2-methoxyphenyl, 2-methylsulfonylphenyl, 4-acetylaminophenyl or 4-hydroxyphenyl.
[18] A medicament comprising a compound according to any one of [1] to [17] above or a salt thereof or a hydrate of the foregoing.
[19] A therapeutic or prophylactic agent for a disease associated with thrombus formation, comprising a compound according to any one of [1] to [17] above or a salt thereof or a hydrate of the foregoing.
[20] A therapeutic or prophylactic agent for a disease selected from Group E1 below, comprising a compound according to any one of [1] to [17] above or a salt thereof or a hydrate of the foregoing,
   wherein Group E1 consists of thrombosis, deep vein thrombosis, pulmonary embolism, cerebral infarction, myocardial infarction, vascular restenosis, disseminated intravascular coagulation syndrome and malignant tumor.
[21] A therapeutic or prophylactic agent for a disease selected from Group E2 below, comprising a compound according to any one of [1] to [17] above or a salt thereof or a hydrate of the foregoing,
   wherein Group E2 consists of thrombosis, deep vein thrombosis, pulmonary embolism, cerebral infarction, myocardial infarction, vascular restenosis and disseminated intravascular coagulation syndrome.
[22] A compound represented by the following general formula (1-2) or a salt thereof or a hydrate of the foregoing: 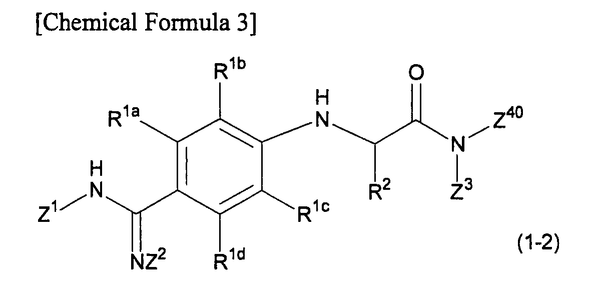 wherein Z⁴⁰ is morpholino or a group represented by the formula: 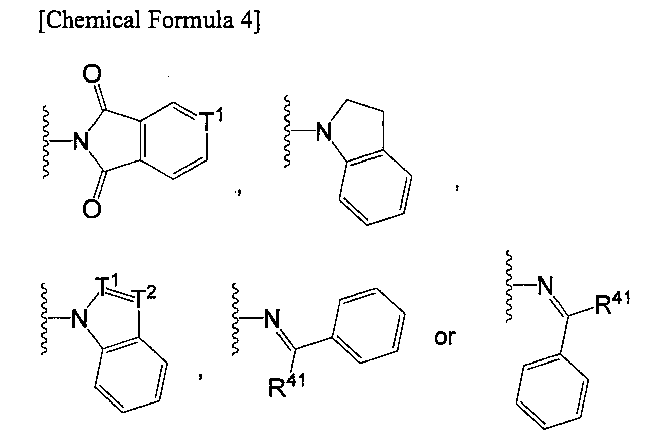 wherein R⁴¹ represents hydrogen or C1-6 alkyl and T¹ and T² each independently represent methine or nitrogen and Z⁴⁰ optionally has 1-3 substituents selected from Group A1 recited in [1] above; and
   R^{1a}, R^{1b} R^{1c}, R^{1d}, R², Z¹, Z² and Z³ have the same definitions as R^{1a}, R^{1b}, R^{1c}, R^{1d}, R², Z¹, Z² and Z³ recited in [1] above.
[23] A compound according to [22] above or a salt thereof or a hydrate of the foregoing, wherein R^{1a}, R^{1b}, R^{1c} and R^{1d} are hydrogen.
[24] A compound according to [22] or [23] above or a salt thereof or a hydrate of the foregoing, wherein Z¹ is hydrogen.
[25] A compound according to any one of [22] to [24] above or a salt thereof or a hydrate of the foregoing, wherein Z² is hydrogen.
[26] A compound according to any one of [22] to [25] above or a salt thereof or a hydrate of the foregoing, wherein Z³ is hydrogen.
[27] A compound according to any one of [22] to [26] above or a salt thereof or a hydrate of the foregoing, wherein R² is phenyl optionally having 1-3 substituents selected from Group A2 below;
   Group A2 consists of C1-6 alkoxy optionally having a group selected from Group B2 below, C1-6 alkyl, C1-6 alkoxy-C1-6 alkyl, C3-8 cycloalkyloxy, C2-6 alkenyloxy, C2-6 alkynyloxy, benzyloxy, pyridylmethoxy, hydroxyl and halogen; and
   Group B2 consists of fluorine, C1-6 alkoxy, amino, mono(C1-6 alkyl)amino, di(C1-6 alkyl)amino, carbamoyl, mono(C1-6 alkyl)aminocarbonyl and di(C1-6 alkyl)aminocarbonyl.
[28] A compound according to any one of [22] to [26] above or a salt thereof or a hydrate of the foregoing, wherein R² is phenyl having 2 or 3 substituents selected from Group A3 below;
   Group A3 consists of C1-6 alkoxy optionally having a group selected from Group B3 below, C2-6 alkenyloxy, hydroxyl, and halogen; and
   Group B3 consists of fluorine, C1-6 alkoxy, amino, mono(C1-6 alkyl)amino, di(C1-6 alkyl)amino and di(C1-6 alkyl)aminocarbonyl.
[29] A compound according to any one of [22] to [26] above or a salt thereof or a hydrate of the foregoing wherein R² is phenyl having 2 or 3 substituents selected from Group A4 below; and
   Group A4 consists of methoxy, ethoxy, isopropoxy, n-propoxy, dimethylaminocarbonylmethoxy, 2-dimethylaminoethoxy, 2-amino-2-methylpropoxy, 2-dimethylamino-1-methylethoxy, 2-methoxyethoxy, allyloxy, hydroxyl, fluorine and 2-fluoroethoxy.
[30] A compound according to any one of [22] to [26] above or a salt thereof or a hydrate of the foregoing wherein R² is phenyl having 2 or 3 substituents represented by the following formula: 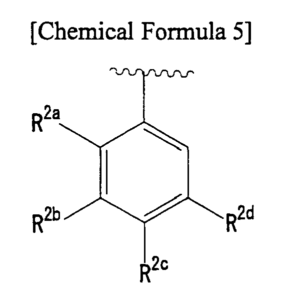 wherein R^{2a} represents hydrogen or fluorine;
   R^{2b} represents hydrogen, methoxy, isopropoxy, n-propoxy, allyloxy or 2-fluoroethoxy; and
   R^{2c} and R^{2d} each independently represent hydrogen, methoxy, ethoxy, isopropoxy, n-propoxy, dimethylaminocarbonylmethoxy, 2-dimethylaminoethoxy, 2-amino-2-methylpropoxy, 2-dimethylamino-1-methylethoxy, 2-methoxyethoxy, allyloxy, hydroxyl, fluorine or 2-fluoroethoxy.
[31] A compound according to any one of [22] to [26] above or a salt thereof or a hydrate of the foregoing, wherein R² is 3-ethoxy-4-dimethylaminocarbonylmethoxyphenyl, 3,4-diallyloxyphenyl, 3-ethoxy-4-(2-dimethylaminoethoxy)phenyl, 3-ethoxy-4-(2-methoxyethoxy)phenyl, 3,4-diethoxyphenyl, 3,5-dimethoxy-4-hydroxyphenyl, 3-ethoxy-6-fluoro-4-isopropoxyphenyl, 3-ethoxy-4-isopropoxyphenyl, 3-methoxy-4-isopropoxyphenyl, 3,4-dimethoxy-6-fluorophenyl, 3,4,5-trimethoxyphenyl, 3-methoxy-5-n-propoxy-6-fluorophenyl, 3-methoxy-5-allyloxy-6-fluorophenyl, 3-methoxy-5-isopropoxy-6-fluorophenyl, 3-methoxy-5-(2-fluoroethoxy)-6-fluorophenyl, 4-(2-amino-2-methylpropoxy)-3-ethoxyphenyl or 4-(2-dimethylamino-1-methylethoxy)-3-ethoxyphenyl.
[32] A medicament comprising a compound according to any one of [22] to [31] above or a salt thereof or a hydrate of the foregoing.
[33] A therapeutic or prophylactic agent for a disease associated with thrombus formation, comprising a compound according to any one of [22] to [31] above or a salt thereof or a hydrate of the foregoing.
[34] A therapeutic or prophylactic agent for a disease selected from Group E1 below, comprising a compound according to any one of [22] to [31] above or a salt thereof or a hydrate of the foregoing,
   wherein Group E1 consists of thrombosis, deep vein thrombosis, pulmonary embolism, cerebral infarction, myocardial infarction, vascular restenosis, disseminated intravascular coagulation syndrome and malignant tumor.
[35] A therapeutic or prophylactic agent for a disease selected from Group E2 below, comprising a compound according to any one of [22] to [31] above or a salt thereof or a hydrate of the foregoing,
wherein Group E2 consists of thrombosis, deep vein thrombosis, pulmonary embolism, cerebral infarction, myocardial infarction, vascular restenosis and disseminated intravascular coagulation syndrome.

The present invention will now be explained in detail.

Throughout the present specification, the structural formulas for the compounds will show only one specific isomer for convenience, but the invention includes all isomers such as geometric isomers, optical isomers, stereoisomers and tautomers implied by the compound structures, as well as their isomer mixtures, and the compounds may therefore be any of the isomers or their mixtures, without being limited to the formulas shown for convenience. Thus, the compounds of the invention may exist as optically active forms or racemic mixtures, all of which are included without limitations according to the invention. Polymorphic crystals may also exist, and there may be used any of the possible crystal forms or a mixture thereof without any restrictions, while the compounds of the invention may include both anhydrous and hydrated forms.

The definitions of the terms and symbols used throughout the present specification will now be explained, prior to a more detailed description of the invention.

The term "disease associated with thrombus formation" is not particularly restricted so long as it is a disease with onset directly or indirectly caused by thrombus formation, and as specific examples there may be mentioned thrombosis, deep vein thrombosis, pulmonary embolism, cerebral infarction, myocardial infarction, vascular restenosis, disseminated intravascular coagulation syndrome and malignant tumor, and preferably thrombosis, deep vein thrombosis, pulmonary embolism, cerebral infarction, myocardial infarction, vascular restenosis and disseminated intravascular coagulation syndrome.

The term "halogen" refers to fluorine, chlorine, bromine and iodine. As preferred examples of "halogen" there may be mentioned fluorine and chlorine.

The term "C1-6 alkyl" refers to a straight-chain or branched C1-6 alkyl group, and as specific examples there may be mentioned methyl, ethyl, 1-propyl (n-propyl), 2-propyl (i-propyl), 2-methyl-1-propyl (i-butyl), 2-methyl-2-propyl (t-butyl), 1-butyl (n-butyl), 2-butyl (s-butyl), 1-pentyl, 2-pentyl, 3-pentyl, 2-methyl-1-butyl, 3-methyl-1-butyl, 2-methyl-2-butyl, 3-methyl-2-butyl, 2,2-dimethyl-1-propyl, 1-hexyl, 2-hexyl, 3-hexyl, 2-methyl-1-pentyl, 3-methyl-1-pentyl, 4-methyl-1-pentyl, 2-methyl-2-pentyl, 3-methyl-2-pentyl, 4-methyl-2-pentyl, 2-methyl-3-pentyl, 3-methyl-3-pentyl, 2,3-dimethyl-1-butyl, 3,3-dimethyl-1-butyl, 2,2-dimethyl-1-butyl, 2-ethyl-1-butyl, 3,3-dimethyl-2-butyl and 2,3-dimethyl-2-butyl.

The term "C2-6 alkenyl" refers to a straight-chain or branched C2-6 alkenyl group containing one double bond, and as specific examples there may be mentioned vinyl (ethenyl), allyl (2-propenyl), 1-propenyl, isopropenyl (1-methylvinyl), 1-butenyl, 2-butenyl, 3-butenyl, pentenyl and hexenyl.

The term "C2-6 alkynyl" refers to a straight-chain or branched C2-6 alkynyl group containing one triple bond, and as specific examples there may be mentioned ethynyl, 1-propynyl, 2-propynyl, butynyl, pentynyl and hexynyl.

The term "C3-8 cycloalkyl" refers to a C3-8 monocyclic saturated aliphatic hydrocarbon group, and as specific examples there may be mentioned cyclopropyl, cyclobutyl, cyclopentyl, cyclohexyl, cycloheptyl and cyclooctyl.

The term "C6-10 aryl" refers to a C6-10 aromatic hydrocarbon cyclic group, and as specific examples there may be mentioned phenyl and naphthyl.

The term "5- to 10-membered heteroaryl" refers to an aromatic cyclic group having 5-10 atoms composing the ring and containing 1-5 heteroatoms among the atoms composing the ring, and as specific examples there may be mentioned furyl, thienyl, pyrrolyl, imidazolyl, triazolyl, tetrazolyl, thiazolyl, pyrazolyl, oxazolyl, isoxazolyl, isothiazolyl, furazanyl, thiadiazolyl, oxadiazolyl, pyridyl, pyrazinyl, pyridazinyl, pyrimidinyl, triazinyl, purinyl, pteridinyl, quinolyl, isoquinolyl, naphthylidinyl, quinoxalinyl, cinnolinyl, quinazolinyl, phthalazinyl, imidazopyridyl, imidazothiazolyl, imidazooxazolyl, benzothiazolyl, benzoxazolyl, benzimidazolyl, indolyl, isoindolyl, indazolyl, pyrrolopyridyl, thienopyridyl, furopyridyl, benzothiadiazolyl, benzoxadiazolyl, pyridopyrimidinyl, benzofuryl, benzothienyl, benzo[1,3]dioxole and thienofuryl.

The term "5- to 6-membered non-aromatic heterocyclyl" refers to (5) a non-aromatic cyclic group (1) having 5 or 6 atoms composing the ring, (2) containing 1 or 2 heteroatoms among the atoms composing the ring, (3) optionally having 1 or 2 double bonds in the ring and (4) optionally containing 1 or 2 carbonyl in the ring, and as specific examples there may be mentioned pyrrolidinyl, piperidinyl, morpholinyl, thiomorpholinyl, tetrahydrofuryl, tetrahydropyranyl and pyridonyl.

The term "C1-6 alkoxy" refers to an oxy group bonded to "C1-6 alkyl" as defined above, and as specific examples there may be mentioned methoxy, ethoxy, 1-propyloxy, 2-propyloxy, 2-methyl-1-propyloxy, 2-methyl-2-propyloxy, 1-butyloxy, 2-butyloxy, 1-pentyloxy, 2-pentyloxy, 3-pentyloxy, 2-methyl-1-butyloxy, 3-methyl-1-butyloxy, 2-methyl-2-butyloxy, 3-methyl-2-butyloxy, 2,2-dimethyl-1-propyloxy, 1-hexyloxy, 2-hexyloxy, 3-hexyloxy, 2-methyl-1-pentyloxy, 3-methyl-1-pentyloxy, 4-methyl-1-pentyloxy, 2-methyl-2-pentyloxy, 3-methyl-2-pentyloxy, 4-methyl-2-pentyloxy, 2-methyl-3-pentyloxy, 3-methyl-3-pentyloxy, 2,3-dimethyl-1-butyloxy, 3,3-dimethyl-1-butyloxy, 2,2-dimethyl-1-butyloxy, 2-ethyl-1-butyloxy, 3,3-dimethyl-2-butyloxy and 2,3-dimethyl-2-butyloxy.

The term "C3-8 cycloalkyloxy" refers to an oxy group bonded to "C3-8 cycloalkyl" as defined above, and as specific examples there may be mentioned cyclopropyloxy, cyclobutyloxy, cyclopentyloxy, cyclohexyloxy, cycloheptyloxy and cyclooctyloxy.

The term "C2-6 alkenyloxy" refers to an oxy group bonded to "C2-6 alkenyl" as defined above, and as specific examples there may be mentioned vinyloxy (ethenyloxy), allyloxy (2-propenyloxy), 1-propenyloxy, isopropenyloxy (1-methylvinyloxy), 1-butenyloxy, 2-butenyloxy, 3-butenyloxy, pentenyloxy and hexenyloxy.

The term "C2-6 alkynyloxy" refers to an oxy group bonded to "C2-6 alkynyl" as defined above, and as specific examples there may be mentioned ethynyloxy, 1-propynyloxy, 2-propynyloxy, butynyloxy, pentynyloxy and hexynyloxy.

The term "C1-6 alkylthio" refers to a thio group bonded to "C1-6 alkyl" as defined above, and as specific examples there may be mentioned methylthio, ethylthio, 1-propylthio, 2-propylthio, butylthio and pentylthio.

The term "C1-6 alkylsulfinyl" refers to a sulfinyl group bonded to "C1-6 alkyl" as defined above, and as specific examples there may be mentioned methylsulfinyl, ethylsulfinyl, 1-propylsulfinyl, 2-propylsulfinyl, butylsulfinyl and pentylsulfinyl.

The term "C1-6 alkylsulfonyl" refers to a sulfonyl group bonded to "C1-6 alkyl" as defined above, and as specific examples there may be mentioned methylsulfonyl, ethylsulfonyl, 1-propylsulfonyl, 2-propylsulfonyl, butylsulfonyl and pentylsulfonyl.

The term "C2-7 alkylcarbonyl" refers to a carbonyl group bonded to "C1-6 alkyl" as defined above, and as specific examples there may be mentioned acetyl, propionyl, isopropionyl, butyryl, isobutyryl, valeryl, isovaleryl and pivaloyl.

The term "C6-10 aryloxy" refers to an oxy group bonded to "C6-10 aryl" as defined above, and as specific examples there may be mentioned phenyloxy, 1-naphthyloxy and 2-naphthyloxy.

The term "5- to 10-membered heteroaryloxy" refers to an oxy group bonded to a "5- to 10-membered heteroaryl" as defined above, and as specific examples there may be mentioned furyloxy, thienyloxy, pyrolyloxy, imidazolyloxy, pyridyloxy and pyrazinyloxy.

The term "5- or 6-membered non-aromatic heterocyclooxy" refers to an oxy group bonded to a "5- or 6-membered non-aromatic heterocyclyl" as defined above, and as specific examples there may be mentioned pyrrolidinyloxy, piperidinyloxy, morpholinyloxy, thiomorpholinyloxy, tetrahydrofuryloxy and tetrahydropyranyloxy.

The term "C6-10 arylmethyl" refers to a methyl group bonded to "C6-10 aryl" as defined above, and as specific examples there may be mentioned benzyl, 1-naphthylmethyl and 2-naphthylmethyl.

The term "C6-10 arylamino" refers to an amino group bonded to "C6-10 aryl" as defined above, and as specific examples there may be mentioned phenylamino, 1-naphthylamino and 2-naphthylamino.

The term "5- to 10-membered heteroarylmethyl" refers to a methyl group bonded to a "5- to 10-membered heteroaryl" as defined above, and as specific examples there may be mentioned furylmethyl, thienylmethyl, pyrolylmethyl, imidazolylmethyl, pyridylmethyl and pyrazinylmethyl.

The term "5- to 10-membered heteroarylamino" refers to an amino group bonded to a "5- to 10-membered heteroaryl" as defined above, and as specific examples there may be mentioned furylamino, thienylamino, pyrolylamino, imidazolylamino, pyridylamino and pyrazinylamino.

The term "mono(C1-6 alkyl)amino" refers to an amino group bonded to one "C1-6 alkyl" as defined above, and as specific examples there may be mentioned methylamino and ethylamino.

The term "di(C1-6 alkyl)amino" refers to an amino group bonded to two "C1-6 alkyl" as defined above, and as specific examples there may be mentioned dimethylamino and methylethylamino.

The term "mono(C1-6 alkyl)aminocarbonyl" refers to a carbonyl group bonded to "mono(C1-6 alkyl)amino" as defined above, and as specific examples there may be mentioned methylaminocarbonyl and ethylaminocarbonyl.

The term "di(C1-6 alkyl)aminocarbonyl" refers to a carbonyl group bonded to "di(C1-6 alkyl)amino" as defined above, and as specific examples there may be mentioned dimethylaminocarbonyl and methylethylaminocarbonyl.

The term "mono(C2-7 alkyl)amino group" refers to an amino group bonded to one "C2-7 alkylcarbonyl" as defined above, and as specific examples there may be mentioned acetylamino and ethylcarbonylamino.

The term "di(C2-7 alkylcarbonyl)amino group" refers to an amino group bonded to two "C2-7 alkylcarbonyl" as defined above, and as specific examples there may be mentioned diacetylamino and di(ethylcarbonyl)amino.

The term "dimethylamino-C1-6 alkoxy" refers to "C1-6 alkoxy" bonded to "dimethylamino" as defined above, and specifically there may be mentioned 2-dimethylamino-ethoxy and 3-dimethylamino-propoxy.

The term "C1-6 alkoxy-C1-6 alkyl" refers to "C1-6 alkyl" bonded to "C1-6 alkoxy" as defined above, and specifically there may be mentioned methoxymethyl and ethoxymethyl.

The term "pyridylmethoxy" refers to a methoxy group bonded to a pyridyl group, and specifically there may be mentioned 2-pyridylmethoxy, 3-pyridylmethoxy and 4-pyridylmethoxy.

The term "indazolyl" refers to a monovalent group derived by removing one hydrogen from any position of an indazole ring, and specifically there may be mentioned 1-indazolyl and 3-indazolyl.

The term "furyl" refers to a monovalent group derived by removing one hydrogen from any position of a furan ring, and specifically there may be mentioned 2-furyl and 3-furyl.

The term "pyridyl" refers to a monovalent group derived by removing one hydrogen from any position of a pyridine ring, and specifically there may be mentioned 2-pyridyl, 3-pyridyl and 4-pyridyl.

The term "N-oxypyridyl" refers to the aforementioned "pyridyl" having nitrogen of the ring oxidized, and specifically there may be mentioned N-oxy-2-pyridyl, N-oxy-3-pyridyl and N-oxy-4-pyridyl.

The term "pyrimidinyl" refers to a monovalent group derived by removing one hydrogen from any position of a pyrimidine ring, and specifically there may be mentioned 2-pyrimidinyl, 4-pyrimidinyl and 5-pyrimidinyl.

The term "thienyl" refers to a monovalent group derived by removing one hydrogen from any position of a thiophene ring, and specifically there may be mentioned 2-thienyl and 3-thienyl.

The term "pyrazinyl" refers to a monovalent group derived by removing one hydrogen from any position of a pyrazine ring.

The term "benzotriazolyl" refers to a monovalent group derived by removing one hydrogen from any position of a benzotriazole ring, and specifically there may be mentioned 4-benzotriazolyl.

The term "pyridonyl" refers to a monovalent group derived by removing one hydrogen from any position of a pyridone ring, and specifically there may be mentioned groups represented by any of the following formulas.

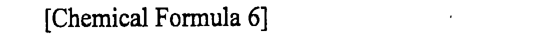

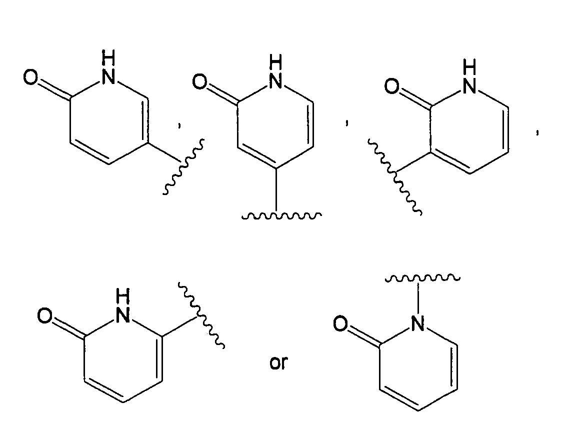

The term "N-methylpyridonyl" refers to the aforementioned "pyridonyl" having a methyl group bonded to nitrogen on the ring, and specifically there may be mentioned groups represented by any of the following formulas.

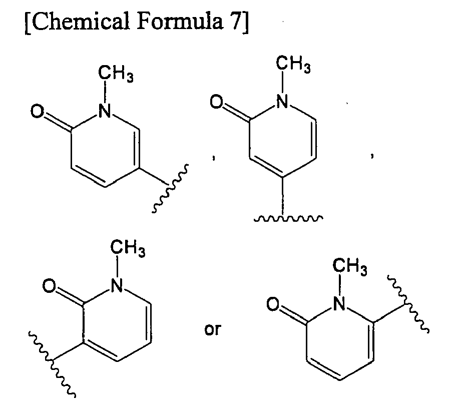

The term "pyridylmethyl" refers to a methyl group bonded to "pyridyl" as defined above, and specifically there may be mentioned 2-pyridylmethyl, 3-pyridylmethyl and 4-pyridylmethyl.

The term "thienylmethyl" refers to a methyl group bonded to "thienyl" as defined above, and specifically there may be mentioned 2-thienylmethyl and 3-thienylmethyl.

The term "optionally having a substituent(s)" means that the compound may have one or more substituents in any desired combination at substitutable positions.

[Definition of Z⁴⁰]
Z⁴⁰ is morpholino or a group represented by the formula: 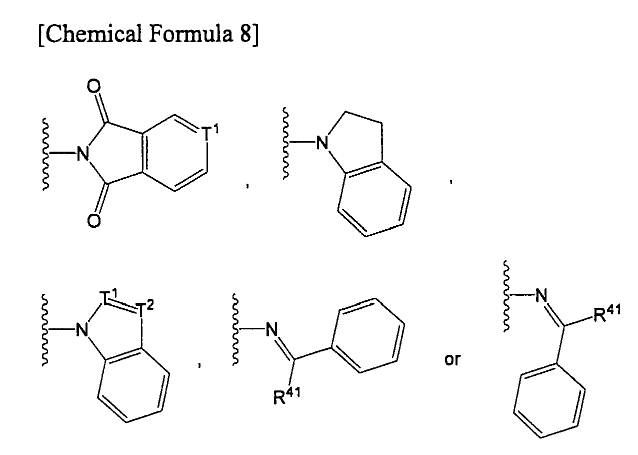 wherein R⁴¹ represents hydrogen or C1-6 alkyl, and T¹ and T² each independently represent methine or nitrogen and Z⁴⁰ optionally has 1-3 substituents selected from Group A1 mentioned in [1] above.
As a preferred example of Z⁴⁰ there may be mentioned the group represented by the following formula.

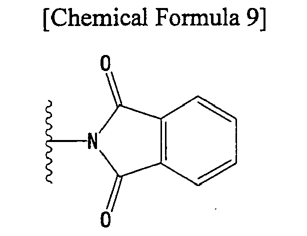

A "salt" as referred to throughout the present specification is not particularly restricted so long as it is formed with the compound of the invention and is pharmacologically acceptable, and as examples there may be mentioned inorganic acid salts, organic acid salts, inorganic base salts, organic base salts and acidic or basic amino acid salts.

As preferred examples of inorganic acid salts there may be mentioned hydrochloride, hydrobromide, sulfate, nitrate and phosphate, and as preferred examples of organic acid salts there may be mentioned acetate, succinate, fumarate, maleate, tartarate, citrate, lactate, stearate, benzoate, methanesulfonate, ethanesulfonate, p-toluenesulfonate and benzenesulfonate.

As preferred examples of inorganic base salts there may be mentioned alkali metal salts such as sodium and potassium salts, alkaline earth metal salts such as calcium and magnesium salts, aluminum salts and ammonium salts, and as preferred examples of organic base salts there may be mentioned diethylamine salts, diethanolamine salts, meglumine salts and N,N'-dibenzylethylenediamine salts.

As preferred examples of acidic amino acid salts there may be mentioned aspartic acid salts and glutamic acid salts, and as examples of basic amino acid salts there may be mentioned arginine salts, lysine salts and ornithine salts.

### Effect of the Invention

The compounds of the invention have excellent inhibiting effects against clotting factor VIIa and excellent anticoagulant effects, and are therefore useful as therapeutic and/or prophylactic agents for diseases associated with thrombus formation (for example, deep vein thrombosis, pulmonary embolism, cerebral infarction, myocardial infarction, vascular restenosis or disseminated intravascular coagulation syndrome) (Johannes Ruef & Hugo A Katus, New antithrombotic drugs on the horizon, Expert Opin. Investig. Drugs (2003)12(5): 781-797).

Substances with inhibiting effects against clotting factor VIIa have also been reported to exhibit malignant tumor metastasis suppression and reduction. Thus, the compounds of the present invention that have excellent inhibiting effects against clotting factor VIIa are also useful as therapeutic and/or prophylactic agents for malignant tumors and the like (Mattias Belting et al., Regulation of angiogenesis by tissue factor cytoplasmic domain signaling, Nature Medicine (2004) 10(5): 502-509; X Jiang et al., Formation of tissue factor-factor VIIa-factor Xa complex promotes cellular signaling and migration of human breast cancer cells, J Thromb Haemost, (2004) 2: 93-101; Hembrough TA. Swartz GM. Papathanassiu A. Vlasuk GP. Rote WE. Green SJ. Pribluda VS., Tissue factor VIIa inhibitors block angiogenesis and tumor growth through a nonhemostatic mechanism. Cancer Research (2003) 63(11): 2997-3000).

Since the compounds of the invention have excellent suppressing effects against blood clotting, and are safe with suitable physicochemical stability, they are useful as medicaments, and especially as therapeutic and/or prophylactic agents for diseases associated with thrombus formation.

### Best Mode for Carrying Out the Invention

[General production processes for compounds of the invention]
The compounds of the invention may be produced by the processes described below. However, the processes for production of the compounds of the invention are not restricted to these alone.
Compound (1) of the invention may be produced by the following Process A, Process B or Process C. In particular, among compounds (1) of the invention, compound (1a) wherein Z¹ and Z² are hydrogen, may be produced by Process C below and compound (1b) wherein X is -CO-, -CS- or -SO₂- may be produced by Process B below.
The processes will now be explained.

[Process A] 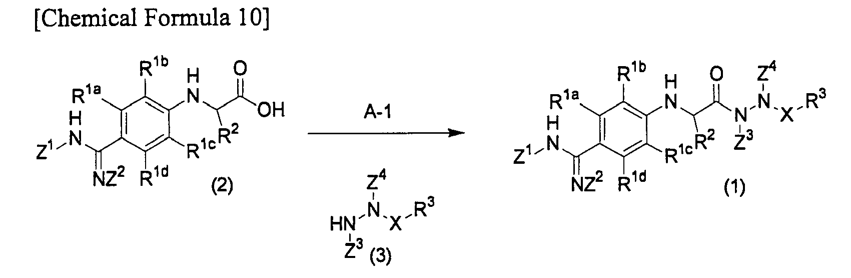 In these formulas, R^{1a}, R^{1b}, R^{1c}, R^{1d}, R², R³, X, Z¹, Z², Z³ and Z⁴ have the same definitions as above.
Process A will now be explained.

(Step A-1) Condensation
This step is a step of reacting compound (2) and compound (3) in a solvent, in the presence or in the absence of an active esterifying agent, in the presence of a condensing agent, in the presence or in the absence of a base, to produce compound (1) of the invention.
Compound (2) may be used as either a free form or a salt. The salt of compound (2) is not particularly restricted, and specifically there may be mentioned hydrochloride (amidine portion), trifluoroacetate (amidine portion), lithium salt (carboxylic acid portion) and sodium salt (carboxylic acid portion).
Compound (3) may be used as either a free form or a salt. The salt of compound (3) is not particularly restricted, and specifically there may be mentioned hydrochloride. When hydrochloride of compound (3) is used, the reaction is carried out in the presence of a base such as triethylamine.
In the presence of an active esterifying agent, compound (2), compound (3) and the active esterifying agent may be reacted together, alternatively compound (2) and the active esterifying agent may be reacted first and then compound (3) added to the reaction mixture.

There are no particular restrictions on the solvent to be used so long as it does not inhibit the reaction and can dissolve the starting compounds and reagents to some extent, and as examples there may be mentioned halogenated hydrocarbons (dichloromethane, chloroform and the like), ethers (diethyl ether, tetrahydrofuran and the like), amides (dimethylformamide, dimethylacetamide and the like) and the like, among which dichloromethane, tetrahydrofuran or dimethylformamide is preferred.
As the condensation agent to be used there may be mentioned dicyclohexylcarbodiimide (DCC), 1,1'-oxalyldiimidazole, 2,2'-dipyridyl disulfide, N,N'-disuccinimidylcarbonate, diphenylazide phosphate (DPPA), diethylcyanophosphoric acid (DEPC), N,N'-bis(2-oxo-3-oxazolidinyl)phosphinic chloride (BOP-Cl), N,N'-carbonyldiimidazole, N,N'-disuccinimidyl oxalate (DSO), N,N'-diphthalimide oxalate (DPO), N,N'-bis(norbornenylsuccinimidyl)oxalate (BNO), 1,1'-bis(benzotriazolyl)oxalate (BBTO), 1,1'-bis(6-chlorobenzotriazolyl)oxalate (BCTO), 1,1'-bis(6-trifluoromethylbenzotriazolyl)oxalate (BTBO), bromo-tris-pyrrolidino-phosphonium-hexafluoro-phosphate (PyBrOP), 1-ethyl-3-(3-dimethylaminopropyl)carbodiimide (WSCD), 1-ethyl-3-(3-dimethylaminopropyl)carbodiimide hydrochloride (WSCD/HCl), benzotriazol-1-yloxytris (dimethylamino)phosphonium hexafluorophosphate (BOP) and the like, among which 1-ethyl-3-(3-dimethylaminopropyl)carbodiimide hydrochloride (WSCD/HCl) is preferred.
As bases there may be used tertiary organic amines such as triethylamine and N-methylmorpholine, and preferably triethylamine.
As active esterifying agents there may be mentioned N-hydroxy compounds such as N-hydroxysuccinimide, 1-hydroxybenzotriazole and N-hydroxy-5-norbornene-2,3-dicarboximide, among which 1-hydroxybenzotriazole is preferred.
The reaction temperature will differ depending on the starting compounds, solvent, condensation agent, active esterifying agent and the like, but for active esterification step it will usually be from -10 to 25°C and preferably from -5 to 10°C. In the subsequent amidation step, the temperature will usually be 0-40°C and preferably 10-30°C.
The reaction time will also differ depending on the starting compounds, solvent, condensation agent, active esterifying agent and reaction temperature, but will usually be 0.5-72 hours and preferably 1-24 hours.

[Process B] 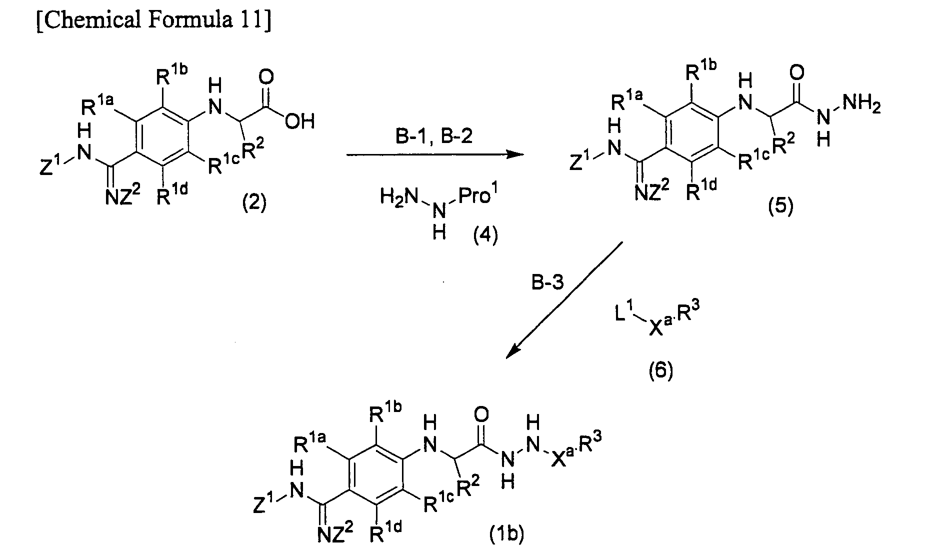 In these formulas, R^{1a}, R^{1b}, R^{1c}, R^{1d}, R², R³, Z¹ and Z² have the same definitions as above. Pro¹ represents a protecting group for amino (preferably t-butyloxycarbonyl), X^{a} represents -CO-, -CS- or -SO₂-, and L¹ represents hydroxyl, chlorine, bromine, iodine or -S-CH₂-CO₂H.
The steps of Process B will now be explained.

(Step B-1)
This step is a condensation reaction step wherein compound (2) and compound (4) are reacted in a solvent in the presence or in the absence of an active esterifying agent, in the presence of a condensation agent and in the presence or in the absence of a base.
Compound (2) may be either a free form or a salt. The salt of compound (2) is not particularly restricted, and specifically there may be mentioned hydrochlorides (amidine portion), trifluoroacetate (amidine portion), lithium salt (carboxylic acid portion) and sodium salt (carboxylic acid portion).
This step may be carried out similar to step A-1 described above.

(Step B-2)
This is a step of deprotecting the protecting group in the compound obtained in step B-1 above in a solvent to produce compound (5).
The method of removing the protecting group will differ depending on the type of the protecting group, but generally it may be accomplished in the following manner by a process known in the field of organic synthetic chemistry, and for example, the process described in T.W. Greene, (Protective Groups in Organic Synthesis), John Wiley & Sons: J.F.W. McOmis, (Protective Groups in Organic Chemistry), Plenum Press.
The protecting group for amino group may be, for example, optionally substituted aliphatic acyl, optionally substituted aromatic acyl, optionally substituted alkoxycarbonyl or a substituted methylene group forming a Schiff base, and it is preferably t-butyloxycarbonyl.
When the protecting group in the compound obtained from step B-1 is a protecting group for amino, compound (5) may be produced by reaction with the compound obtained from step B-1 in a solvent in the presence of an acid or base.
There are no particular restrictions on the solvent to be used so long as it does not inhibit the reaction and can dissolve the starting compounds and reagents to some extent, and as examples there may be mentioned alcohols (methanol, ethanol, n-propanol, isopropanol, n-butanol, isobutanol, t-butanol, isoamyl alcohol, diethylene glycol, glycerin, octanol, cyclohexanol, methyl cellosolve and the like), ethers (diethyl ether, diisopropyl ether, tetrahydrofuran, dioxane, dimethoxyethane, diethyleneglycol dimethyl ether and the like), halogenated hydrocarbons (dichloromethane, 1,2-dichloroethane, chloroform and the like), water or mixtures of water with the aforementioned solvents, among which ethers (especially dioxane) or alcohols (especially ethanol) are preferred.
The acid used may be, for example, an inorganic acid (hydrobromic acid, hydrogen bromide, hydrochloric acid, hydrogen chloride, hydrogen fluoride, sulfuric acid, perchloric acid, phosphoric acid or nitric acid) or an organic acid (trifluoroacetic acid, trifluoromethanesulfonic acid or the like), among which hydrochloric acid or trifluoroacetic acid is preferred.
As examples of the base to be used there may be mentioned alkali metal carbonic acid salt (lithium carbonate, sodium carbonate, potassium carbonate and the like), alkali metal hydroxide (lithium hydroxide, sodium hydroxide, potassium hydroxide and the like), metal alkoxides (lithium methoxide, sodium methoxide, sodium ethoxide, potassium t-butoxide and the like) or ammonia (aqueous ammonia, concentrated ammonia-methanol and the like).
The reaction temperature will differ depending on the starting compounds, solvent, deprotecting agent and the like, but will usually be 0-150°C and preferably 10-60°C.
The reaction time will also differ depending on the starting compounds, solvent, deprotecting agent and reaction temperature, but will usually be 1-72 hours and preferably 1-24 hours.

(Step B-3)
The method for this step will differ depending on the type of L¹.
Compound (5) may be either a free form or a salt. There are no particular restrictions on the salt of compound (5), and specifically there may be mentioned hydrochloride and trifluoroacetate.
When compound (6) is a carboxylic acid, it may be either a free form or a salt. There are no particular restrictions on the salt of compound (6), and specifically there may be mentioned lithium salt and sodium salt.

### [In the case where L¹ is hydroxyl]

This is a step of reacting compound (5) with compound (6) in a solvent, in the presence of a base and condensation agent to produce compound (1b).
This step may be carried out similar to step A-1 described above.

### [In the case where L¹ is leaving group (preferably halogen such as chlorine or bromine)]

This is a step of reacting compound (5) with compound (6) in a solvent, in the presence of a base to produce compound (1b).
There are no particular restrictions on the solvent to be used so long as it does not inhibit the reaction and can dissolve the starting compounds and reagents to some extent, and as examples there may be mentioned organic solvents such as halogenated hydrocarbons (chloroform, dichloromethane, 1,2-dichloroethane, carbon tetrachloride and the like), aromatic hydrocarbons (benzene, toluene, chlorobenzene and the like), ethers (diethyl ether, diisopropyl ether, tetrahydrofuran, dioxane, dimethoxyethane, diethyleneglycol dimethyl ether and the like), nitriles (acetonitrile, propionitrile and the like), sulfoxides (dimethylsulfoxide and the like), or mixtures of these solvents, and a mixed solvent of 1,2-dichloroethane and dimethylsulfoxide is preferred.
The base to be used is not particularly restricted so long as it can yield the target compound and does not produce unseparable by-products, and specifically there may be mentioned organic bases such as N-methylmorpholine, triethylamine, tripropylamine, tributylamine, diisopropylethylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU), with triethylamine or pyridine being preferred.
The reaction temperature will differ depending on the starting compounds, solvent and base, but will usually be from -10 to 50°C and preferably 0-40°C.
The reaction time will also differ depending on the starting compounds, solvent, base and reaction temperature, but will usually be 0.5-72 hours and preferably 1-36 hours.

[Process C] 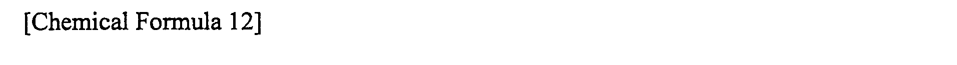 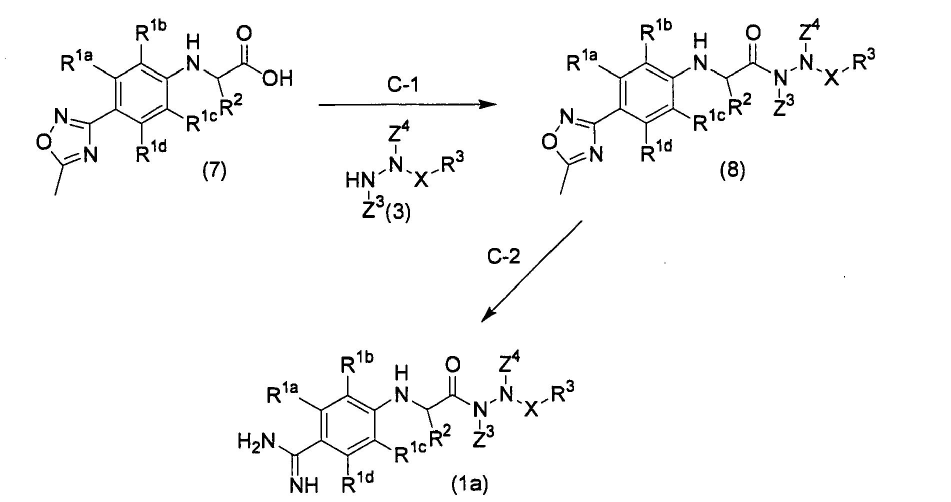 In these formulas, R^{1a}, R^{1b}, R^{1c}, R^{1d}, R², R³, X, Z³ and Z⁴ have the same definitions as above.
The steps of Process C will now be explained.

(Step C-1)
This step is a condensation reaction step wherein compound (7) and compound (3) are reacted in a solvent in the presence or in the absence of an active esterifying agent, in the presence of a condensation agent and in the presence or in the absence of a base, to produce compound (8).
This step may be carried out similar to step A-1 described above.

(Step C-2)
This is a step of producing compound (1a) of the invention from compound (8), and it may be carried out (1) in a solvent in the presence of an acid and a metal reagent, or (2) in a solvent in the presence of a reduction catalyst.

### [Reaction in the presence of acid and metal reagent]

There are no particular restrictions on the solvent used so long as it does not inhibit the reaction and can dissolve the starting compounds and reagents to some extent, and as examples there may be mentioned alcohols (methanol, ethanol, n-propanol, isopropanol, n-butanol, isobutanol, t-butanol, isoamyl alcohol, diethylene glycol, glycerin, octanol, cyclohexanol, methyl cellosolve and the like), ethers (diethyl ether, diisopropyl ether, tetrahydrofuran, dioxane, dimethoxyethane, diethyleneglycol dimethyl ether and the like), water or mixtures of water with these organic solvents, and preferred are mixtures of alcohols and water (most preferably methanol and water).
As examples of the acid to be used there may be mentioned inorganic acids (aqueous hydrobromic acid, aqueous hydrochloric acid, sulfuric acid, perchloric acid, phosphoric acid or nitric acid) or organic acids (acetic acid, trifluoroacetic acid, trifluoromethanesulfonic acid and the like), among which aqueous hydrochloric acid and acetic acid are preferred.
Examples of the metal reagent to be used include iron, zinc, tin and the like, with iron being preferred.
The reaction temperature will differ depending on the starting compounds, solvent, acid and metal reagent, but will normally be 10-100°C and preferably 30-60°C.
The reaction time will also differ depending on the starting compounds, solvent, acid and reaction temperature, but will usually be 1-48 hours and preferably 1-12 hours.

### [Reaction in the presence of reduction catalyst]

The solvent used is not particularly restricted so long as it is inert to the reaction, and as examples there may be mentioned esters (methyl acetate, ethyl acetate, propyl acetate, butyl acetate, diethyl carbonate), ethers (diethyl ether, diisopropyl ether, tetrahydrofuran, dioxane, dimethoxyethane, diethyleneglycol dimethyl ether and the like), alcohols (methanol, ethanol, n-propanol, isopropanol, n-butanol, isobutanol, t-butanol, isoamyl alcohol, diethylene glycol, glycerin, octanol, cyclohexanol, methyl cellosolve and the like), organic acids (acetic acid and the like), water or mixtures of these solvents with water, among which alcohols, ethers, organic acids or water (most preferably alcohols or organic acids) are preferred.
The catalyst used is preferably palladium-carbon, Raney nickel, platinum oxide, platinum black, rhodium-aluminum oxide, triphenylphosphine-rhodium chloride or palladium-barium sulfate.
The pressure is not particularly restricted but will usually be 1-10 atmospheric pressures.
The reaction temperature will differ depending on the starting compounds, catalyst, pressure and solvent, but will usually be 0°C-100°C. The reaction time will differ depending on the starting compounds, catalyst, solvent and reaction temperature, but will usually be 1-72 hours.

Compound (2), compound (3), compound (4), compound (6) and compound (7) used as intermediates in Processes A, B and C may be commercially available products, or they may be easily produced from commercially available products by ordinary methods employed by those skilled in the art. In particular, compound (2) and compound (7) may be produced by Process D (steps D1-1 and D2-1) or Process E (steps E1-1 and E2-1) described below.

[Process D] 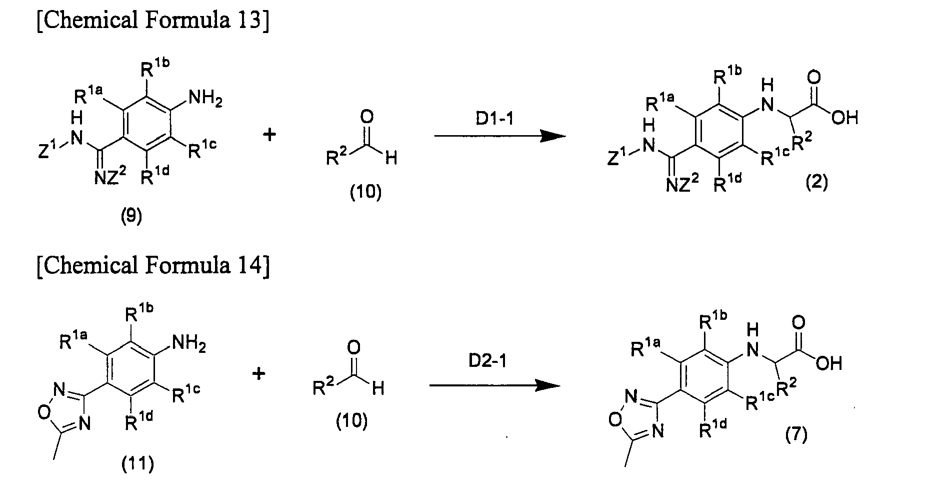 In these formulas, R^{1a}, R^{1b}, R^{1c}, R^{1d}, R², Z¹ and Z² have the same definitions as above.
Compound (9), compound (10) and compound (11) may be commercially available products or they may be easily produced from commercially available products by ordinary methods employed by those skilled in the art.
Compound (9) may be either a free form or a salt. There are no particular restrictions on the salt of compound (9), and specifically there may be mentioned hydrochloride and trifluoroacetate.
The steps of Process D will now be explained.

(Step D1-1)
This is step of reacting compound (9), compound (10) and an isocyanide compound in an appropriate solvent in the presence of a Lewis acid to produce an ester (first part of step), and then hydrolyzing the ester to produce compound (2) (second part of step).
The solvent used in the first part may be, for example, alcohols (methanol, ethanol, n-propanol, isopropanol, n-butanol, isobutanol, t-butanol, isoamyl alcohol, diethylene glycol, glycerin, octanol, cyclohexanol, methyl cellosolve or the like), and methanol is preferred.
The Lewis acid used may be boron trifluoride/diethyl ether complex, aluminum chloride, zinc chloride, tin chloride, titanium tetrachloride or the like, and boron trifluoride/diethyl ether complex is preferred.
The isocyanide compound used may be (4-tolylsulfonyl)methyl isocyanide (para-toluenesulfonylmethyl isocyanide) or 2-(4-morpholinyl)ethyl isocyanide, and (4-tolylsulfonyl)methyl isocyanide is preferred. The reaction temperature will differ depending on the starting compounds, solvent and isocyanide compound, but will normally be from -10°C to 100°C and preferably 0°C-60°C. The reaction time will also differ depending on the starting compounds, solvent, isocyanide compound and reaction temperature, but will usually be 1-72 hours and preferably 1-12 hours.
There are no particular restrictions on the solvent to be used in the second part of the step so long as it does not inhibit the reaction and can dissolve the starting compounds and reagents to some extent, and as examples there may be mentioned alcohols (methanol, ethanol, n-propanol, isopropanol, n-butanol, isobutanol, t-butanol, isoamyl alcohol, diethylene glycol, glycerin, octanol, cyclohexanol, methyl cellosolve and the like), amides (formamide, dimethylformamide, dimethylacetamide, hexamethylphosphoric triamide, N-methylpyrrolidone), ethers (diethyl ether, diisopropyl ether, tetrahydrofuran, dioxane, dimethoxyethane, diethyleneglycol dimethyl ether and the like), sulfoxides (dimethylsulfoxide and the like), or mixtures of these solvents, among which alcohols (especially methanol) are preferred.
The base used in the second part of the step may be lithium hydroxide, sodium hydroxide, potassium hydroxide or calcium hydroxide, and is preferably sodium hydroxide.
The reaction temperature in the second part will differ depending on the starting compounds, solvent and base, but will usually be from -10 to 150°C and preferably 0-60°C.
The reaction time in the second part will also differ depending on the starting compounds, solvent, base and reaction temperature, but will usually be 1-72 hours and preferably 1-24 hours.

(Step D2-1)
This is a step of reacting compound (11), compound (10) and an isocyanide compound in an appropriate solvent in the presence of a Lewis acid, and then hydrolyzing the obtained ester to produce compound (7).
This step may be carried out similar to step D1-1 described above.

[Process E] 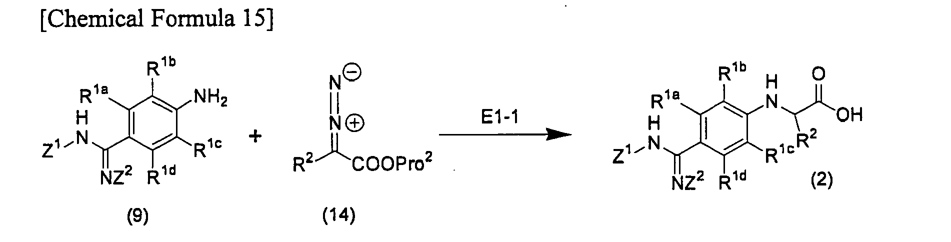 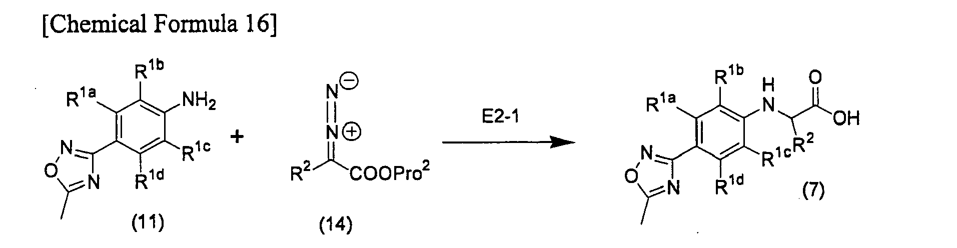 In steps E1-1 and E2-1 above, R^{1a}, R^{1b}, R^{1c}, R^{1d}, R², Z¹ and Z² have the same definitions as above.
Pro² represents a carboxyl-protecting group (preferably C1-6 alkyl, and especially methyl, ethyl, isopropyl or the like).
Compound (14) may be a commercially available product or it may be easily produced from a commercially available product by ordinary methods employed by those skilled in the art.
The steps of Process E will now be explained.

(Step E1-1)
This is a step of reacting compound (9) and compound (14) in a solvent in the presence of a metal catalyst (first part of step), and then removing the protecting group of the obtained compound by hydrolysis to produce compound (2) (second part of step).
There are no particular restrictions on the solvent used in the first part of the step so long it does not inhibit the reaction and can dissolve the starting compounds and reagents to some extent, and as examples there may be mentioned aliphatic hydrocarbons (pentane, hexane, heptane and the like), aromatic hydrocarbons (toluene, benzene, xylene, mesitylene, nitrobenzene and the like), nitriles (acetonitrile and the like), halogenated hydrocarbons (dichloromethane, chloroform, carbon tetrachloride and the like) or mixtures thereof, with toluene being preferred.
The metal catalyst used in the first part may be rhodium acetate, rhodium trifluoroacetate, rhodium chloride, copper sulfate, copper chloride, diacetylacetone copper or the like, with rhodium acetate being preferred.
The reaction temperature in the first part will differ depending on the starting compounds, solvent and metal catalyst, but will normally be from -10 to 150°C and preferably 30-120°C.
The reaction time in the first part will also differ depending on the starting compounds, solvent, metal catalyst and reaction temperature, but will usually be 1-72 hours and preferably 1-12 hours.
There are no particular restrictions on the solvent used in the second part of the step so long as it does not inhibit the reaction and can dissolve the starting compounds and reagents to some extent, and as examples there may be mentioned alcohols (methanol, ethanol, n-propanol, isopropanol, n-butanol, isobutanol, t-butanol, isoamyl alcohol, diethylene glycol, glycerin, octanol, cyclohexanol, methyl cellosolve and the like), amides (formamide, dimethylformamide, dimethylacetamide, hexamethylphosphoric triamide, N-methylpyrrolidone and the like), ethers (diethyl ether, diisopropyl ether, tetrahydrofuran, dioxane, dimethoxyethane, diethyleneglycol dimethyl ether and the like), nitriles (acetonitrile and the like), sulfoxides (dimethylsulfoxide and the like) or mixtures thereof, with alcohols (especially ethanol) being preferred.
The base used in the second part of the step may be lithium hydroxide, sodium hydroxide, potassium hydroxide or calcium hydroxide, and sodium hydroxide is preferred.
The reaction temperature in the second part will differ depending on the starting compounds, solvent and base, but will usually be from -10 to 150°C and preferably 0-60°C.
The reaction time in the second part will also differ depending on the starting compounds, solvent, base and reaction temperature, but will normally be 1-72 hours and preferably 6-24 hours.

(Step E2-1)
This is a step of reacting compound (11) and compound (14) in a solvent in the presence of a metal catalyst, and further removing the protecting group, to produce compound (7).
This step may be carried out similar to step E1-1 described above.
Compounds (11) and (14) in Processes D and E described above may be produced by the following process.

[Process F] 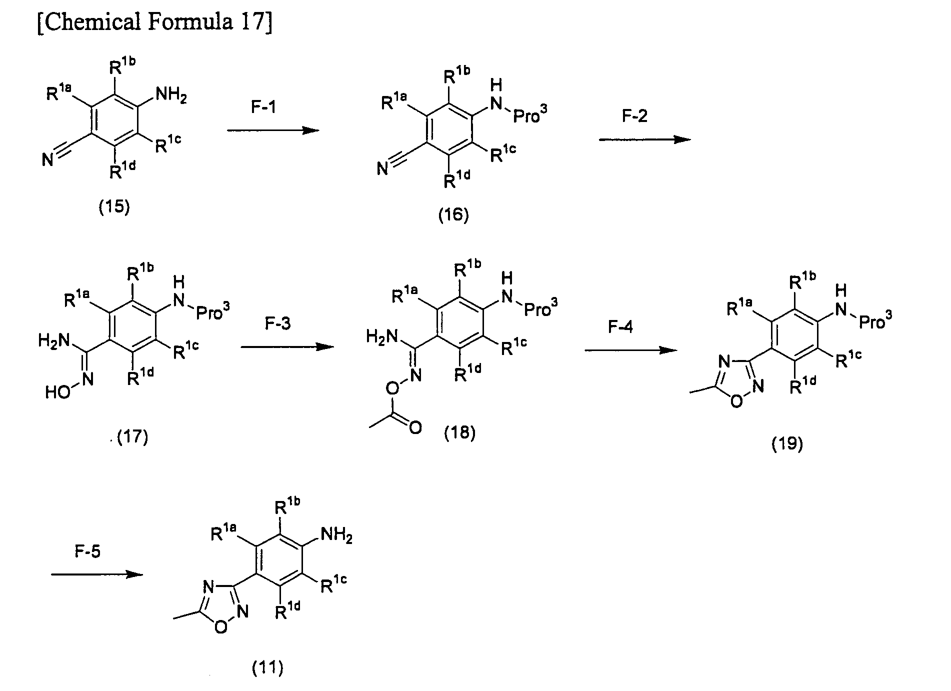 This is a production process for compound (11).
In these formulas, R^{1a}, R^{1b}, R^{1c} and R^{1d} have the same definitions as above, and Pro³ represents an amino-protecting group (preferably t-butyloxycarbonyl).
The steps of Process F will now be explained.

(Step F-1)
This is a step of protecting the amino group of compound (15) in a solvent to produce compound (16).
Introduction of the protecting group will differ depending on its type, but generally it may be accomplished in the following manner by a process known in the field of organic synthetic chemistry, and for example, the process described in T.W. Greene, (Protective Groups in Organic Synthesis), John Wiley & Sons: J.F.W. McOmis, (Protective Groups in Organic Chemistry), Plenum Press.
When the protecting group is t-butyloxycarbonyl, for example, compound (16) may be produced by reacting compound (15) with di-t-butyldicarbonate or the like in the presence of a base such as N,N-dimethylaminopyridine.

(Step F-2)
This is a step of reacting compound (16) with hydroxylamine hydrochloride in a solvent in the presence of a base to produce compound (17).
There are no particular restrictions on the solvent used so long as it does not inhibit the reaction and can dissolve the starting compounds and reagents to some extent, and as examples there may be mentioned alcohols (methanol, ethanol, n-propanol, isopropanol, n-butanol, isobutanol, t-butanol, isoamyl alcohol, diethylene glycol, glycerin, octanol, cyclohexanol, methyl cellosolve and the like), amides (formamide, dimethylformamide, dimethylacetamide, hexamethylphosphoric triamide, N-methylpyrrolidone and the like), ethers (diethyl ether, diisopropyl ether, tetrahydrofuran, dioxane, dimethoxyethane, diethyleneglycol dimethyl ether and the like), sulfoxides (dimethylsulfoxide and the like), or mixtures of these solvents, with alcohols (especially ethanol) being preferred.
As the base there may be used tertiary organic amines such as triethylamine and N-methylmorpholine, and preferably triethylamine.
The reaction temperature will differ depending on the starting compounds, solvent and base, but will usually be 0-150°C and preferably 50-100°C.
The reaction time will also differ depending on the starting compounds, solvent, base and reaction temperature, but will usually be 1-96 hours and preferably 1-24 hours.

(Step F-3)
This is a step of reacting compound (17) with an acetylating agent in a solvent in the presence of a base to produce compound (18).
There are no particular restrictions on the solvent used so long as it does not inhibit the reaction and can dissolve the starting compounds and reagents to some extent, and as examples there may be mentioned aliphatic hydrocarbons (pentane, hexane, heptane and the like), aromatic hydrocarbons (toluene, benzene, xylene, mesitylene, nitrobenzene and the like), esters (methyl acetate, ethyl acetate and the like), amides (formamide, dimethylformamide, dimethylacetamide, hexamethylphosphoric triamide, N-methylpyrrolidone and the like), ethers (diethyl ether, diisopropyl ether, tetrahydrofuran, dioxane, dimethoxyethane, diethyleneglycol dimethyl ether and the like), ketones (acetone, methyl ethyl ketone and the like), nitriles (acetonitrile and the like), halogens (dichloromethane, 1,2-dichloroethane, chloroform and the like), or mixtures of these solvents, among which a mixed solvent of dichloromethane and tetrahydrofuran is preferred.
The acetylating agent used may be acetyl chloride or acetic anhydride, and acetyl chloride is preferred.
As the base there may be used tertiary organic amines such as triethylamine and N-methylmorpholine, and preferably triethylamine.
The reaction temperature will differ depending on the starting compounds, solvent and acetylating agent, but will normally be from -10 to 100°C and preferably 25-60°C.
The reaction time will also differ depending on the starting compounds, solvent, acetylating agent and reaction temperature, but will usually be 1-72 hours and preferably 1-12 hours.

(Step F-4)
This is a step of reacting compound (18) with a base in a solvent to produce compound (19).
There are no particular restrictions on the solvent used so long as it does not inhibit the reaction and can dissolve the starting compounds and reagents to some extent, and as examples there may be mentioned ethers (diethyl ether, diisopropyl ether, tetrahydrofuran, dioxane, dimethoxyethane, diethyleneglycol dimethyl ether), ketones (acetone, methyl ethyl ketone), nitriles (acetonitrile and the like), halogens (dichloromethane, 1,2-dichloroethane, chloroform and the like), sulfoxides (dimethylsulfoxide and the like) or mixtures of these solvents, with tetrahydrofuran being preferred.
The base used may be an organic base such as tetrabutylammonium fluoride or triethylamine, and tetrabutylammonium fluoride is preferred.
The reaction temperature will differ depending on the starting compounds, solvent and base, but will usually be from -10 to 150°C and preferably 0-100°C.
The reaction time will also differ depending on the starting compounds, solvent, base and reaction temperature, but will usually be 1-72 hours and preferably 6-24 hours.

(Step F-5)
This is a step of removing the protecting group of compound (19) in a solvent to produce compound (11).
This step may be carried out similar to step B-2 described above.

Compound (14) can be produced by the following process.
[Process G] 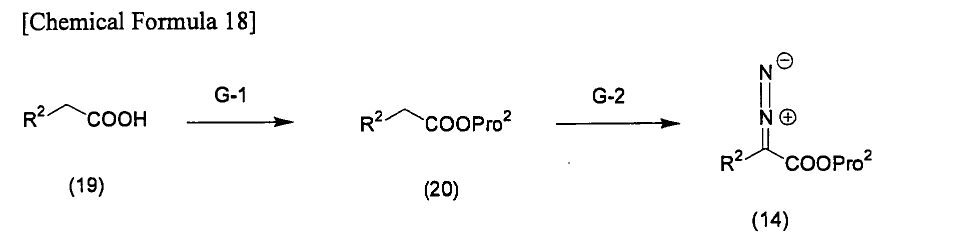 This is a production process for compound (14).
In these formulas, R² has the same definition as above, and Pro² is a carboxyl-protecting group (preferably C1-6 alkyl, and more preferably methyl, ethyl, isopropyl or the like).
The steps of Process G will now be explained.

(Step G-1)
This is a step of protecting the carboxyl group of compound (19) in a solvent to produce compound (20).
Introduction of the protecting group will differ depending on its type, but generally it may be accomplished in the following manner by a process known in the field of organic synthetic chemistry, and for example, the process described in T.W. Greene, (Protective Groups in Organic Synthesis), John Wiley & Sons: J.F.W. McOmis, (Protective Groups in Organic Chemistry), Plenum Press.
When the protecting group is methyl, ethyl or isopropyl, for example, compound (19) may be reacted with a halogenated alkyl group (preferably iodomethane, iodoethane, 2-iodopropane or the like) in the presence of a base to produce compound (20).

(Step G-2)
This is a step of reacting compound (20) with a diazotizing reagent in a solvent in the presence of a base to produce compound (14).
There are no particular restrictions on the solvent used so long as it does not inhibit the reaction and can dissolve the starting compounds and reagents to some extent, and as examples there may be mentioned aliphatic hydrocarbons (pentane, hexane, heptane), aromatic hydrocarbons (toluene, benzene, xylene, mesitylene, nitrobenzene and the like), esters (methyl acetate, ethyl acetate and the like), amides (formamide, dimethylformamide, dimethylacetamide, hexamethylphosphoric triamide, N-methylpyrrolidone and the like), ethers (diethyl ether, diisopropyl ether, tetrahydrofuran, dioxane, dimethoxyethane, diethyleneglycol dimethyl ether and the like), ketones (acetone, methyl ethyl ketone and the like), nitriles (acetonitrile and the like), halogenated hydrocarbons (dichloromethane, 1,2-dichloroethane, chloroform and the like), sulfoxides (dimethylsulfoxide and the like) or mixtures of these solvents, with acetonitrile being preferred.
As diazotizing reagents there may be used 4-acetylaminobenzenesulfonyl azide, 4-methylbenzenesulfonyl azide, 2-naphthalenesulfonyl azide, benzenesulfonyl azide and trifluoromethylsulfonyl azide, among which 4-acetylaminobenzenesulfonyl azide is preferred.
The base to be used is not particularly restricted so long as it can yield the target compound and does not produce unseparable by-products, and specifically there may be mentioned organic bases such as N-methylmorpholine, triethylamine, tripropylamine, tributylamine, diisopropylethylamine, N-methylpiperidine, pyridine, 4-pyrrolidinopyridine, picoline, 4-(N,N-dimethylamino)pyridine, 2,6-di(t-butyl)-4-methylpyridine, quinoline, N,N-dimethylaniline, N,N-diethylaniline, 1,5-diazabicyclo[4.3.0]non-5-ene (DBN), 1,4-diazabicyclo[2.2.2]octane (DABCO) and 1,8-diazabicyclo[5.4.0]undec-7-ene (DBU), among which 1,5-diazabicyclo[4.3.0]non-5-ene (DBN), 1,4-diazabicyclo[2.2.2]octane (DABCO) or 1,8-diazabicyclo[5.4.0]undec-7-ene (DBU) is preferred.
The reaction temperature will differ depending on the starting compounds, solvent and diazotizing range, but will normally be from -10 to 150°C and preferably 0-40°C.
The reaction time will also differ depending on the starting compounds, solvent, diazotizing reagent and reaction temperature, but will usually be 1-72 hours and preferably 1-16 hours.

Compounds (1-2) of the invention may be produced by the following Processes Y and Z. Among compounds (1-2) of the invention, compound (1-2a) wherein Z¹ and Z² are hydrogen may be produced by the following Process Z.
These processes will now be explained.

[Process Y] 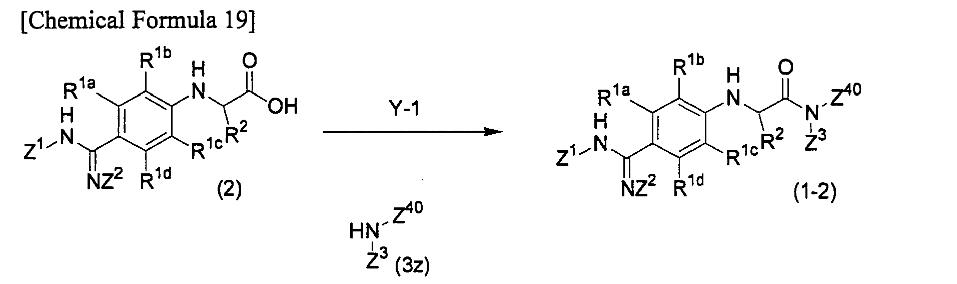 In these formulas, R^{1a}, R^{1b}, R^{1c}, R^{1d}, R², Z¹, Z², Z³ and Z⁴⁰ have the same definitions as above.
The steps of Process Y will now be explained.

(Step Y-1)
This step is a condensation reaction step wherein compound (2) and compound (3z) are reacted in a solvent in the presence or in the absence of an active esterifying agent, in the presence of a condensation agent and in the presence or in the absence of a base, to produce compound (1-2) of the invention.
This step may be carried out similar to step A-1 described above.

[Process Z] 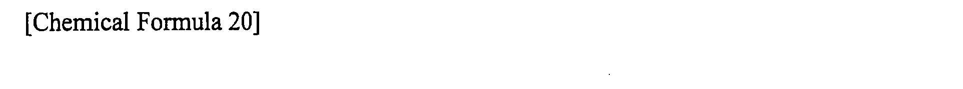 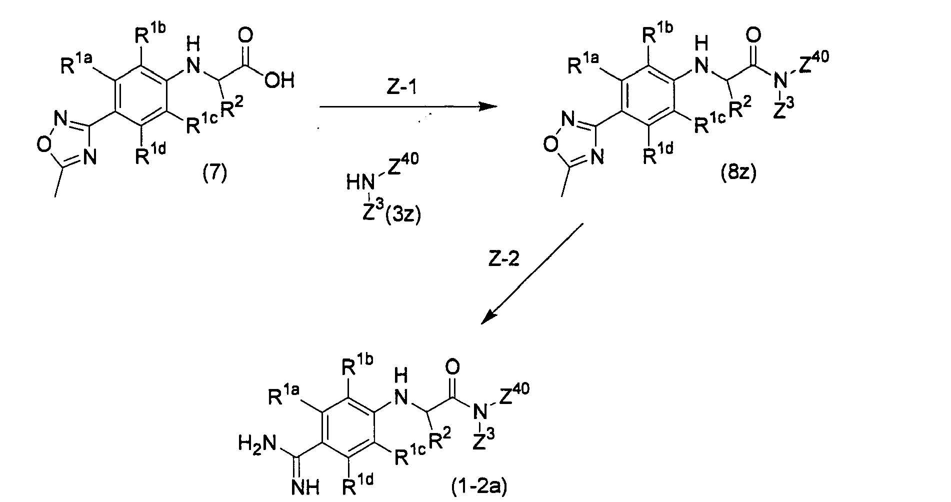 In these formulas, R^{1a}, R^{1b}, R^{1c}, R^{1d} R² Z³ and Z⁴⁰ have the same definitions as above.
The steps of Process Z will now be explained.

(Step Z-1)
This step is a condensation reaction step wherein compound (7) and compound (3z) are reacted in a solvent in the presence or in the absence of an active esterifying agent, in the presence of a condensation agent and in the presence or in the absence of a base, to produce compound (8z).
This step may be carried out similar to step A-1 described above.

(Step Z-2)
This is a step of producing compound (1-2a) of the invention from compound (8z), and it may be carried out (1) in a solvent in the presence of an acid and a metal reagent, or (2) in a solvent in the presence of a metal catalyst and a reduction catalyst.
This step may be carried out similar to step C-2 described above.

Upon completion of the reaction in each step of the processes described above, the target compound of each step may be recovered from the reaction mixture by an ordinary method.
For example, when the entire reaction mixture is liquid, the reaction mixture may be brought to room temperature or cooled on ice as desired, and neutralized with an appropriate acid, alkali, oxidizing agent or reducing agent, prior to addition of water and an organic solvent that is immiscible therewith and does not react with the target compound, such as ethyl acetate, and separation of the layer containing the target compound. Next, a solvent that is immiscible with the obtained layer and does not react with the target compound may be added, and then the layer containing the target compound washed and separated. When the layer is an organic layer, it may be dried using a desiccant such as anhydrous magnesium sulfate or anhydrous sodium sulfate, and the solvent distilled off to recover the target compound. When the layer is an aqueous layer, it may be electrically desalted and then freeze-dried to recover the target compound.
When the entire reaction mixture is liquid, it may be possible to recover the target compound simply by distilling off the components other than the target compound (for example, solvent, reagents and the like) at ordinary pressure or under reduced pressure.
When the target compound precipitates alone as a solid, or when the entire reaction mixture is liquid and the target compound precipitates alone as a solid during the recovery process, the target compound may be obtained by first collected the target compound by filtration, washing the collected target compound with a suitable organic or inorganic solvent and drying and the target compound may be obtained further by treating the mother liquor in the same manner as describe above when the entire reaction mixture is liquid.
On the other hand, when the reagents or catalyst are the only solids present, or when the entire reaction mixture is liquid and the reagents or catalyst alone precipitate as solid during the recovery process with the target compound remaining dissolved in the solution, the target compound may be obtained by first filtering the reagents or catalyst, washing the filtered reagents or catalyst with a suitable organic or inorganic solvent, combining the resultant wash with the mother liquor and treating the obtained mixture in the same manner as described above when the entire reaction mixture is liquid.
The reaction mixture may be used directly for subsequent steps without isolation of the target compound in cases where components other than the target compound in the reaction mixture will not inhibit reaction in the subsequent steps.

Purity of the target compound recovered by such methods can be increased by appropriately carrying out recrystallization, various chromatography methods or distillation.
When the recovered target compound is a solid, purity of the target compound can usually be improved by recrystallization. For recrystallization there may be used a simple solvent or a multiple solvent mixture that does not react with the target compound. Specifically, the target compound may first be dissolved at room temperature or with heating in the simple solvent or solvent mixture that does not react with the target compound. The obtained mixture may then be cooled with ice water or the like or allowed to stand at room temperature to cause precipitation of the target compound from the mixture.
When the recovered target compound is a liquid, purity of the target compound can be improved by a chromatography method. In most cases a weakly acidic silica gel such as silica gel 60 (70-230 mesh or 340-400 mesh) by Merck, Ltd. or BW-300 (300 mesh) by Fuji Silysia Chemical, Ltd. may be used. If the target compound is basic and adsorption onto the aforementioned silica gel types is too strong, there may be used propylamine-coated silica gel (200-350 mesh) by Fuji Silysia Chemical, Ltd. If the target compound is dipolar or requires elution with a highly polar solvent such as methanol, there may be used NAM-200H or NAM-300H by Nam Research Institute. Using these silica gels, the target compound may be eluted in a simple solvent or solvent mixture that does not react with the target compound, and then the solvent distilled off to obtain the target compound with enhanced purity.
When the recovered target compound is a liquid, purity of the target compound can also be improved by distillation. For distillation, the target compound may be placed under reduced pressure at room temperature or with heating to achieve distillation of the target compound.

Representative examples of production processes for compounds (1) and (1-2) according to the invention were described above, but the starting compounds and reagents for production of the invention compounds may form salts, hydrates or solvates, will differ depending on the starting compounds and solvents used, and are not particularly restricted so long as they do not inhibit the reaction. The solvent used will also differ depending on the starting compounds and reagents, and of course is not particularly restricted so long as it can dissolve the starting compounds to some degree and does not inhibit the reaction. When compounds (1) and (1-2) of the invention are obtained in free form, they may be converted to their acceptable salts, or hydrates of either, by an ordinary method.
When compounds (1) and (1-2) are obtained as salts of compounds (1) and (1-2) or hydrates of compounds (1) and (1-2), they may be converted to free forms of compounds (1) and (1-2) by an ordinary method.
Various isomers (for example, geometric isomers, optical isomers, rotational isomers, stereoisomers, tautomers and the like) obtained for compounds (1) and (1-2) of the invention may be purified and isolated using ordinary separation means such as, for example, recrystallization, diastereomer salt methods, enzymatic resolution or chromatography methods (for example, thin-layer chromatography, column chromatography, gas chromatography and the like).

When a compound of the invention is to be used as a medicament, the compound of the invention will usually be used after mixture and formulation with appropriate additives. However, this does not negate the use of the compounds of the invention in simple forms as medicament.

As additives there may be mentioned diluents, binders, lubricants, disintegrants, coloring agents, taste correctives, emulsifiers, surfactants, dissolving aids, suspending agents, isotonizing agents, buffering agents, antiseptic agents, antioxidants, stabilizers, absorption accelerators and the like which are commonly used in drugs, and these may also be used in appropriate combinations as desired.
As examples of diluents there may be mentioned lactose, sucrose, glucose, corn starch, mannitol, sorbitol, starch, alpha starch, dextrin, crystalline cellulose, light anhydrous silicic acid, aluminum silicate, calcium silicate, magnesium aluminometasilicate, calcium hydrogenphosphate and the like.
As examples of binders there may be mentioned polyvinyl alcohol, methylcellulose, ethylcellulose, gum arabic, tragacanth, gelatin, shellac, hydroxypropylmethylcellulose, hydroxypropylcellulose, carboxymethylcellulose sodium, polyvinylpyrrolidone, macrogol and the like.
As examples of lubricants there may be mentioned magnesium stearate, calcium stearate, sodium stearyl fumarate, talc, polyethylene glycol, colloidal silica and the like.
As examples of disintegrants there may be mentioned crystalline cellulose, agar, gelatin, calcium carbonate, sodium hydrogencarbonate, calcium citrate, dextrin, pectin, low-substituted hydroxypropylcellulose, carboxymethylcellulose, carboxymethylcellulose calcium, croscarmellose sodium, carboxymethyl starch, carboxymethyl starch sodium and the like.
As examples of coloring agents there may be mentioned those approved for addition to pharmaceuticals, such as iron sesquioxide, yellow iron sesquioxide, calamine, caramel, β-carotene, titanium oxide, talc, riboflavin sodium phosphate, yellow aluminum lake and the like.
As taste correctives there may be mentioned cocoa powder, menthol, aromatic powders, peppermint oil, camphor, cinnamon powder and the like.
As emulsifiers or surfactants there may be mentioned stearyl triethanolamine, sodium lauryl sulfate, lauryl aminopropionic acid, lecithin, glycerin monostearate, sucrose fatty acid esters, glycerin fatty acid esters and the like.
As dissolving aids there may be mentioned polyethylene glycol, propylene glycol, benzyl benzoate, ethanol, cholesterol, triethanolamine, sodium carbonate, sodium citrate, polysorbate 80, nicotinic acid amide and the like.
As suspending agents there may be mentioned the aforementioned surfactants, as well as hydrophilic polymers such as polyvinyl alcohol, polyvinylpyrrolidone, methylcellulose, hydroxymethylcellulose, hydroxyethylcellulose and hydroxypropylcellulose.
As isotonizing agents there may be mentioned glucose, sodium chloride, mannitol, sorbitol and the like.
As buffering agents there may be mentioned buffering solutions containing phosphate, acetate, carbonate, citrate and the like.
As antiseptic agents there may be mentioned methylparaben, propylparaben, chlorobutanol, benzyl alcohol, phenethyl alcohol, dehydroacetic acid, sorbic acid and the like.
As antioxidants there may be mentioned sulfurous acid salts, ascorbic acid, α-tocopherol and the like.
As stabilizers there may be mentioned those commonly used in medicament.
As absorption accelerators there may also be mentioned those commonly used in medicament.

As formulations there may be mentioned oral forms such as tablets, powders, granules, capsules, syrups, lozenges and inhalants; external forms such as suppositories, ointments, eye salves, tapes, eye drops, nose drops, ear drops, poultices, lotions, and the like; and injections.
The aforementioned oral forms may be formulated with appropriate combinations of the additives mentioned above. Their surfaces may also be coated if necessary.
The aforementioned external forms may be formulated with appropriate combinations of the additives mentioned above, and especially diluents, binders, taste correctives, emulsifiers, surfactants, dissolving aids, suspending agents, isotonizing agents, antiseptic agents, antioxidants, stabilizers and absorption accelerators.
Injections may also be formulated with appropriate combinations of the additives mentioned above, and especially emulsifiers, surfactants, dissolving aids, suspending agents, isotonizing agents, buffering agents, antiseptic agents, antioxidants, stabilizers and absorption accelerators.

The dosage of the medicament according to the invention will differ depending on the severity of symptoms, patient age, gender and body weight, type of dosage form/salt, patient drug sensitivity and specific nature of the disease, but the dosage per day for adults will generally be about 1 mg to about 1000 mg (preferably about 10 mg to about 300 mg) for oral administration, about 1 mg to about 1000 mg (preferably about 10 to about 300 mg) for external application, and in the case of an injection, about 1 µg to about 3000 µg (preferably about 3 µg to about 3000 µg) per kilogram of body weight, either administered at a single time or divided into 2 to 6 times per day.
These values are the actual administered amounts in the case of oral formulations and injections, and are the amounts actually absorbed by the body in the case of external formulations.

### Examples

Compound (1) or (1-2) of the invention may be produced by the methods described in the following examples, and the effects of the compounds can be confirmed by the methods described in the test examples that follow. However, these specific examples are merely illustrative and not intended to restrict the invention in any way, while various modifications may be implemented such as are within the scope of the invention.
Compounds mentioned with reference to published documents were produced in the manner described in those documents.

In the following examples, purification by reversed-phase high performance liquid chromatography was conducted in the following manner unless otherwise specified.

### [Column used]

One of the following columns or a combination thereof may be selected for use.
Manufacturer: SHISEIDO
Name: CAPCELL PAK C18
Size: 50 mm x 20 mmI.D.
Type: ACR 5 µm
Catalog: 91702

Manufacturer: YMC
Name: YMC CombiPrep ODS-A
Size: 50 mm x 20 mmI.D.
Type: S-5 µm
Catalog: CCAAS05-0520WT A-340-CC

Manufacturer: WAKO
Name: WAKOpak Combi ODS-A
Size: 50 mm x 20 mmI.D.

### [Mobile phase]

As the mobile phase for liquid chromatography, the following (1) and (2) were used by a gradient with a range of 100:0 - 0:100.
(1) 1% CH₃CN-H₂O (0.1 % trifluoroacetic acid)
(2) 99.9% CH₃CN (0.1% trifluoroacetic acid)

The "room temperature" referred to in the Reference Examples and Examples is usually from about 10°C to 35°C. The percentage values are weight percentages, unless otherwise specified. The other symbols used in the examples stand for the following.
s: singlet
d: doublet
t: triplet
q: quartet
m: multiplet
br:broad
sept: septet
J: coupling constant
Hz: Hertz
CDCl₃: deutero chloroform
D₆-DMSO: deutero dimethylsulfoxide
CD₃OD: deutero methanol
¹H-NMR: Proton nuclear magnetic resonance

(Example 1) 4-(((3-Ethoxy-4-isopropoxyphenyl)-(N'-phenylhydrazinocarbonyl)methyl)amino)benzamidine trifluoroacetate

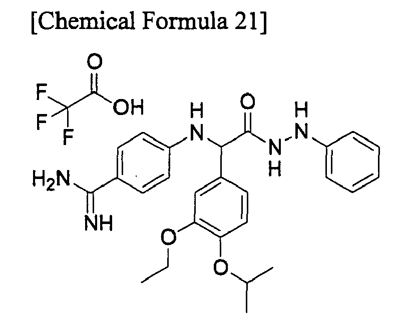

(1a) (4-Carbamimidoylphenylamino)-(3-ethoxy-4-isopropoxyphenyl)acetic acid methyl ester 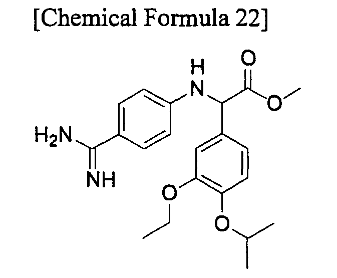 A mixture of 3-ethoxy-4-isopropoxybenzaldehyde (2.0 g, 9.6 mmol), 4-aminobenzamidine dihydrochloride (2.0 g, 9.61 mmol) and methanol (40 ml) was stirred at 60°C for 30 minutes. After cooling the reaction mixture to room temperature, para-toluenesulfonylmethyl isocyanide (2.25 g, 11.5 mmol) was added. The reaction mixture was then cooled to 0°C, and boron trifluoride/diethyl ether complex (3.65 ml, 28.8 mmol) was added. The reaction mixture was stirred overnight at room temperature, and then heptane was added, the methanol layer was separated, and concentration was performed under reduced pressure. Ethyl acetate and aqueous ammonia were added to the obtained residue and the insoluble portion was filtered. The filtrate was concentrated under reduced pressure, and the obtained residue was crudely purified by silica gel column chromatography (NH silica gel (Fuji Silysia Chemical, Ltd.), ethyl acetate-methanol-aqueous ammonia) to give the title compound as a yellow solid (3.7 g, yield: 116%).
¹H-NMR (400 MHz, CD₃OD) δ: 1.30 (d, J = 6.4 Hz, 6H), 1.38 (t, J = 7.2 Hz, 3H), 3.73 (s, 3H), 4.04 (q, J = 5.2 Hz, 2H), 4.51 (sept, J = 6.0 Hz, 1H), 5.22 (s, 1H), 6.77 (d, J = 8.8 Hz, 2H), 6.93 (d, J = 8.4 Hz, 1H), 7.00 (dd, J = 2.0, 8.0 Hz, 1H),7.07 (d, J = 2.0 Hz, 1H), 7.58 (d, J = 8.8 Hz, 2H)

(1b) (4-Carbamimidoylphenylamino)-(3-ethoxy-4-isopropoxyphenyl)acetic acid hydrochloride 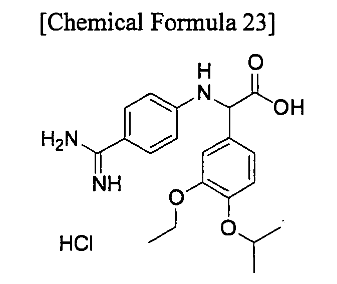 (4-Carbamimidoylphenylamino)-(3-ethoxy-4-isopropoxyphenyl)acetic acid methyl ester (3.7 g, 9.64 mmol) prepared in Example 1a was dissolved in a mixed solvent (50 ml) of tetrahydrofuran and methanol, and a 5N sodium hydroxide aqueous solution (2.5 ml) was added. The reaction mixture was stirred at 30°C for 2 hours and then concentrated under reduced pressure. The obtained concentrate was suspended in tetrahydrofuran, a 1N hydrochloric acid aqueous solution (20 ml) was added and the suspension was concentrated under reduced pressure. The obtained residue was washed with a mixed solvent of ethyl acetate and methanol to give the title compound as a pale yellow solid (2.55 g, yield: 65%).
¹H-NMR (400 MHz, CD₃OD) δ: 1.29 (d, J = 6.0 Hz, 6H), 1.37 (t, J = 6.8 Hz, 3H), 4.01-4.07 (m, 2H), 4.51 (sept, J = 6.0 Hz, 1H), 5.13 (s, 1H), 6.77 (d, J = 9.2 Hz, 2H), 6.93 (d, J = 8.4 Hz, 1H), 7.03 (dd, J = 2.4, 8.8Hz, 1H), 7.10 (d, J = 2.4 Hz, 1H), 7.58 (d, J = 8.8 Hz, 2H)

(1c) 4-(((3-Ethoxy-4-isopropoxyphenyl)-(N'-phenylhydrazinocarbonyl)methyl)amino)benzamidine trifluoroacetate 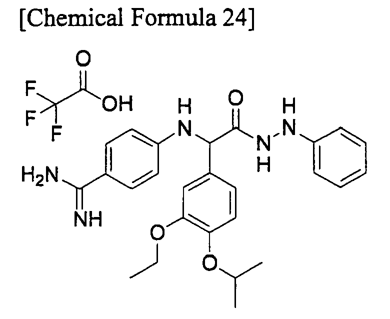 (4-Carbamimidoylphenylamino)-(3-ethoxy-4-isopropoxyphenyl)acetic acid hydrochloride (68 mg, 0.167 mmol) prepared in Example 1b was dissolved in N,N-dimethylformamide (2.5 ml) and cooled to -5°C. To the reaction mixture were added 1-hydroxybenzotriazole monohydrate (30 mg, 0.196 mmol) and 1-ethyl-3-(3-dimethylaminopropyl)carbodiimide hydrochloride (39 mg, 0.202 mmol), followed by stirring for 30 minutes and addition of a solution of phenylhydrazine (0.02 ml, 0.203 mmol) in N,N-dimethylformamide (1 ml). The reaction mixture was stirred overnight at room temperature, and then ethyl acetate (50 ml) and water (20 ml) were added and the organic layer was separated. The organic layer was dried over anhydrous magnesium sulfate. The desiccant was filtered and the filtrate was concentrated under reduced pressure. The obtained residue was purified by reversed-phase high performance liquid chromatography to give the title compound as a pale brown solid (29.9 mg, yield: 39%).
¹H-NMR (400 MHz, CD₃OD) δ: 1.31 (d, J = 6.0 Hz, 6H), 1.38 (t, J = 7.2 Hz, 3H), 4.06 (q, J = 7.2 Hz, 2H), 4.55 (sept, J = 6.0 Hz, 1H), 5.12 (s, 1H), 6.56 (d, J = 9.6 Hz, 2H), 6.71-6.78 (m, 2H), 6.83 (d, J = 8.4 Hz, 1H), 6.99 (d, J = 8.8Hz, 1H), 7.01-7.13 (m, 3H), 7.18 (d, J = 2.0 Hz, 1H), 7.63 (d, J = 8.8 Hz, 2H); Mass spectrum (ESI) *m*/*z:* 462 (M+H)⁺

(Example 2) 4-(((3-Ethoxy-4-isopropoxyphenyl)-(N'-pylidin-2-ylhydrazinocarbonyl)methyl)amino)benzamidine trifluoroacetate 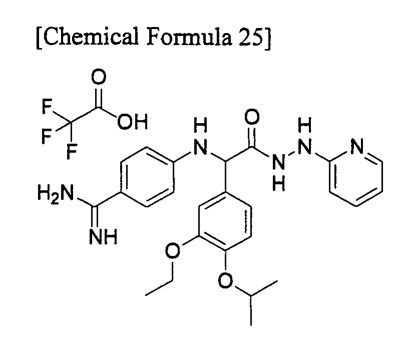 (4-Carbamimidoylphenylamino)-(3-ethoxy-4-isopropoxyphenyl)acetic acid hydrochloride (50 mg, 0.135 mmol) prepared in Example 1b was dissolved in N,N-dimethylformamide (1 ml) and cooled to -5°C. To the reaction mixture were added 1-hydroxybenzotriazole monohydrate (55 mg, 0.405 mmol) and 1-ethyl-3-(3-dimethylaminopropyl)carbodiimide hydrochloride (78 mg, 0.405 mmol), followed by stirring for 30 minutes and addition of 2-hydrazinopyridine (44 mg, 0.405 mmol). The reaction mixture was stirred overnight at room temperature, and then ethyl acetate and water were added and the organic layer was separated. The organic layer was dried over anhydrous magnesium sulfate. The desiccant was filtered and the filtrate was concentrated under reduced pressure. The obtained residue was purified by reversed-phase high performance liquid chromatography to give the title compound as a pale brown solid (78 mg, yield: 21 %).
¹H-NMR (400 MHz, CD₃OD) δ: 1.30 (d, J = 5.2 Hz, 6H), 1.39 (t, J = 6.8 Hz, 3H), 4.07 (q, J = 6.8 Hz, 2H), 4.52 (sept, J = 5.2 Hz, 1H), 5.33 (s, 1H), 6.86 (d, J = 8.4 Hz, 2H), 6.97 (d, J = 8.0 Hz, 2H), 7.09-7.19 (m, 3H), 7.61 (d, J = 8.4 Hz, 2H), 8.01 (br, 2H); Mass spectrum (ESI) *m*/*z:* 463 (M+H)⁺

(Example 3) 2-(N'-(2-(4-Carbamimidoylphenylamino)-2-(3-ethoxy-4-isopropoxyphenyl)acetyl)hydrazino)benzoic acid trifluoroacetate 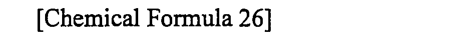 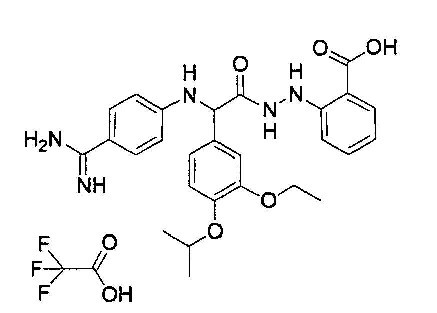 (4-Carbamimidoylphenylamino)-(3-ethoxy-4-isopropoxyphenyl)acetic acid hydrochloride (40 mg, 0.098 mmol) prepared in Example 1b was dissolved in N,N-dimethylformamide (0.5 ml) and cooled to -5°C. To the reaction mixture were added 1-hydroxybenzotriazole monohydrate (45 mg, 0.294 mmol) and 1-ethyl-3-(3-dimethylaminopropyl)carbodiimide hydrochloride (56 mg, 0.294 mmol), followed by stirring for 30 minutes and addition of 2-hydrazinobenzoic acid (55 mg, 0.294 mmol). The reaction mixture was stirred overnight at room temperature, and then ethyl acetate and water were added and the organic layer was separated. The organic layer was dried over anhydrous magnesium sulfate. The desiccant was filtered and the filtrate was concentrated under reduced pressure. The obtained residue was purified by reversed-phase high performance liquid chromatography to give the title compound as a pale brown solid (27 mg, yield: 45%).
¹H-NMR (400 MHz, CD₃OD) δ: 1.32 (d, J = 6.4 Hz, 6H), 1.38 (t, J = 6.8 Hz, 3H), 4.06 (q, J = 7.6 Hz, 2H), 4.55 (sept, J = 6.4 Hz, 1H), 5.13 (s, 1H), 6.44 (d, J = 8.4 Hz, 1H), 6.74-6.77 (m, 2H), 6.83 (d, J = 6.8 Hz, 2H), 7.00 (d, J = 8.4 Hz, 1H), 7.12 (dd, J = 2.0, 10.0 Hz, 1H), 7.16 (d, J = 2.0 Hz, 1H), 7.62 (d, J = 8.8 Hz, 2H), 7.88 (dd, J = 1.2, 7.6 Hz, 1H); Mass spectrum (ESI) *m*/*z:* 506 (M+H)⁺

(Example 4) 4-(1-(3-Ethoxy-4-isopropoxyphenyl)-2-(N'-(1-methyl-6-oxo-1,6-dihydropyridine-3-carbonyl)hydrazino)-2-oxoethylamino)benzamidine trifluoroacetate

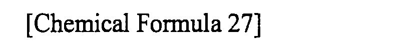

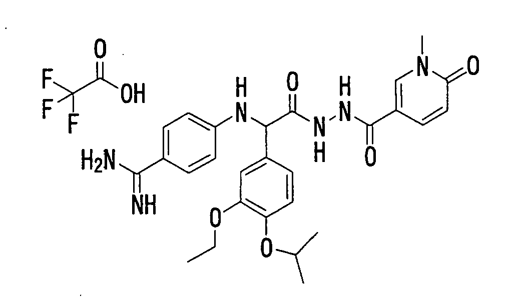

(4a) N'-(2-(4-Carbamimidoylphenylamino)-2-(3-ethoxy-4-isopropoxyphenyl)acetyl)hydrazinecarboxylic acid t-butyl ester 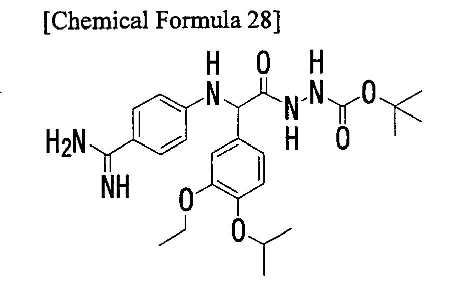 (4-Carbamimidoylphenylamino)-(3-ethoxy-4-isopropoxyphenyl)acetic acid (300 mg, 0.735 mmol) prepared in Example 1b was dissolved in N,N-dimethylformamide (15 ml) and cooled to 0°C. To the reaction mixture were added 1-hydroxybenzotriazole monohydrate (338 mg, 2.21 mmol) and 1-ethyl-3-(3-dimethylaminopropyl)carbodiimide hydrochloride (423 mg, 2.21 mmol), followed by stirring for 1 hour and addition of hydrazinecarboxylic acid t-butyl ester (291 mg, 2.21 mmol). The reaction mixture was stirred at room temperature for 5 days and then crudely purified by silica gel column chromatography (NH silica gel (Fuji Silysia Chemical, Ltd.), dichloromethane-methanol) to give the title compound as a yellow solid (357 mg, yield: 102%).
¹H-NMR (400 MHz, CD₃OD) δ: 1.29 (d, J = 6.0 Hz, 6H), 1.38 (t, J = 6.8 Hz, 3H), 1.46 (brs, 9H), 4.03-4.11 (m, 2H), 4.49 (sept, J = 6.0 Hz, 1H), 5.00 (s, 1H), 6.79 (d, J = 8.8 Hz, 2H), 6.92 (d, J = 8.4 Hz, 1H), 7.04 (dd, J = 2.0, 8.4Hz, 1H), 7.15 (d, J = 2.0 Hz, 1H), 7.59 (d, J = 8.8 Hz, 2H)

(4b) 4-(((3-Ethoxy-4-isopropoxyphenyl)hydrazinocarbonylmethyl)amino)benzamidine dihydrochloride 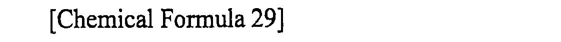 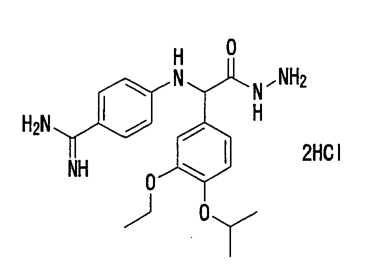 N'-(2-(4-Carbamimidoylphenylamino)-2-(3-ethoxy-4-isopropoxyphenyl)acetyl)hydrazinecarboxylic acid t-butyl ester (365 mg, 0.752 mmol) prepared in Example 4a was dissolved in ethanol (3 ml), and then 40% hydrogen chloride in ethanol (3 ml) was added and the mixture was stirred at room temperature for 10 hours. The reaction mixture was concentrated to give the title compound as a crude product (345 mg, yield: 108%).
¹H-NMR (400 MHz, CD₃OD) δ: 1.29 (d, J = 6.0 Hz, 6H), 1.39 (t, J = 7.2 Hz, 3H), 4.07 (q, J = 6.8 Hz, 2H), 4.52 (sept, J = 6.0 Hz, 1H), 5.19 (s, 1H), 6.83 (d, J = 9.2 Hz, 2H), 6.96 (d, J = 8.0 Hz, 1H), 7.07 (dd, J = 2.4, 8.4Hz, 1H), 7.14 (d, J = 2.4 Hz, 1H), 7.63 (d, J = 9.2 Hz, 2H)

(4c) 4-(1-(3-Ethoxy-4-isopropoxyphenyl)-2-(N'-(1-methyl-6-oxo-1,6-dihydropyridine-3-carbonyl)hydrazino)-2-oxoethylamino)benzamidine trifluoroacetate 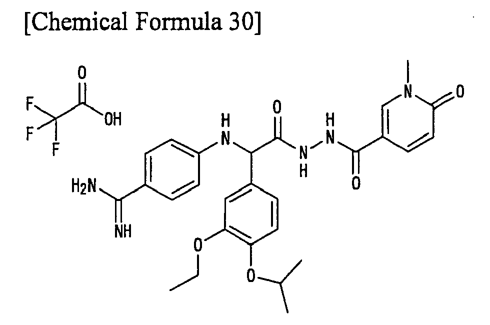 1-Methyl-6-oxo-1,6-dihydropyridine-3-carboxylic acid (4 mg, 0.0872 mmol) was dissolved in N,N-dimethylformamide (0.25 ml) and cooled to 0°C. To the reaction mixture were added 1-ethyl-3-(3-dimethylaminopropyl)carbodiimide hydrochloride (4 mg, 0.0209 mmol) and 1-hydroxybenzotriazole monohydrate (5 mg, 0.0326 mmol), followed by stirring for 1 hour and addition of 4-(((3-ethoxy-4-isopropoxyphenyl)hydrazinocarbonylmethyl)amino)benzamidine dihydrochloride (10 mg, 0.0218 mmol) prepared in Example 4b and triethylamine (0.01 ml). After stirring the reaction mixture at room temperature for 3 days, it was directly purified by reversed-phase high performance liquid chromatography to give the title compound (2.8 mg, yield: 20%).
¹H-NMR (400 MHz, CD₃OD) δ: 1.29 (d, J = 6.0 Hz, 6H), 1.39 (t, J = 6.8 Hz, 3H), 3.59 (s, 3H), 4.09 (q, J = 7.2 Hz, 2H), 4.52 (sept, J = 6.0 Hz, 1H), 5.14 (s, 1H), 6.53 (d, J = 9.6 Hz, 1H), 6.86 (d, J = 8.4 Hz, 2H), 6.96 (d, J = 8.0 Hz, 1H), 7.10 (d, J = 8.0Hz, 1H), 7.21 (s, 1H), 7.63 (d, J = 8.8 Hz, 2H), 7.88 (d, J = 9.6 Hz, 1H), 8.30 (s, 1H); Mass spectrum (ESI) *m*/*z:* 521 (M+H)⁺

(Example 5) 4-(1-(3-Ethoxy-4-isopropoxyphenyl)-2-(N'-(1-methyl-4-oxo-1,4-dihydropyridine-3-carbonyl)hydrazino)-2-oxo-ethylamino)-benzamidine trifluoroacetate 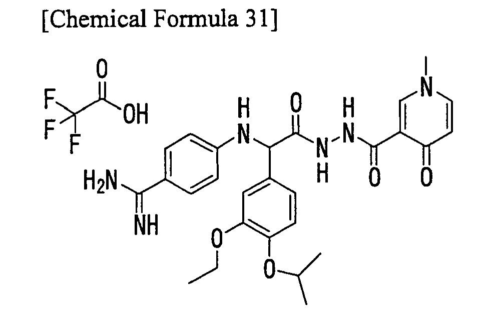 1-Methyl-4-oxo-1,4-dihydropyridine-3-carboxylic acid (5 mg, 0.109 mmol) was dissolved in N,N-dimethylformamide (0.25 ml) and cooled to 0°C. To the reaction mixture were added 1-ethyl-3-(3-dimethylaminopropyl)carbodiimide hydrochloride (5 mg, 0.0261 mmol) and 1-hydroxybenzotriazole monohydrate (6 mg, 0.0407 mmol), followed by stirring for 1 hour and addition of 4-(((3-ethoxy-4-isopropoxyphenyl)hydrazinocarbonylmethyl)amino)benzamidine dihydrochloride (10 mg, 0.0218 mmol) prepared in Example 4b and triethylamine (0.01 ml). The reaction mixture was stirred overnight at room temperature and then directly purified by reversed-phase high performance liquid chromatography to give the title compound (1.6 mg, yield: 9%).
¹H-NMR (400 MHz, CD₃OD) δ: 1.29 (d, J = 6.0 Hz, 6H), 1.39 (t, J = 6.8 Hz, 3H), 3.59 (s, 3H), 4.08 (q, J = 6.8 Hz, 2H), 4.52 (sept, J = 6.0 Hz, 1H), 5.13 (s, 1H), 6.53 (d, J = 9.6 Hz, 1H), 6.86 (d, J = 8.4 Hz, 2H), 6.95 (d, J = 8.4 Hz, 1H), 7.09 (d, J = 8.4Hz, 1H), 7.20 (s, 1H), 7.63 (d, J = 8.8 Hz, 2H), 7.87 (d, J = 8.4 Hz, 1H), 8.30 (s, 1H); Mass spectrum (ESI) *m*/*z*: 521 (M+H)⁺

(Example 6) 2-(4-(1-(4-Carbamimidoylphenylamino)-2-(N'-(2-chlorobenzoyl)hydrazino)-2-oxoethyl)-2-ethoxyphenoxy)-N,N-dimethylacetamide trifluoroacetate

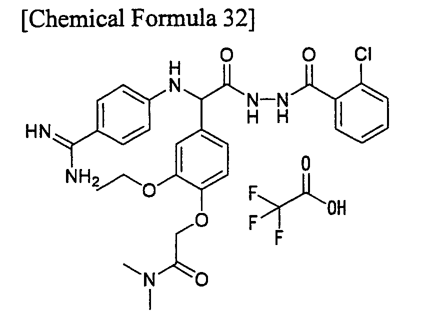

(6a) (4-Carbamimidoylphenylamino)-(4-dimethylcarbamoylmethoxy-3-ethoxyphenyl)acetic acid methyl ester 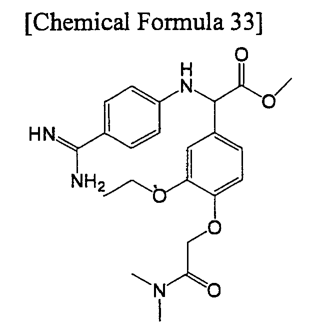 A mixture of 2-(2-ethoxy-4-formylphenoxy)-N,N-dimethylacetamide [CAS: 93475-19-5] (6.471 g, 25.75 mmol), 4-aminobenzamidine dihydrochloride (5.36 g, 25.75 mmol) and methanol (100 ml) was stirred at 60°C for 30 minutes. After then cooling the reaction mixture to room temperature, para-toluenesulfonylmethyl isocyanide (6.00 g, 30.90 mmol) was added. The reaction mixture was then cooled to 0°C, and boron trifluoride/diethyl ether complex (9.8 ml, 77.26 mmol) was added. The reaction mixture was stirred overnight at room temperature, and then heptane was added, the methanol layer was separated, and concentration was performed under reduced pressure. Aqueous sodium hydrogencarbonate was added to the obtained residue, and extraction was performed with ethyl acetate. The organic layer was dried over anhydrous magnesium sulfate and filtered. The filtrate was concentrated under reduced pressure, and the obtained residue was crudely purified by silica gel column chromatography (NH silica gel (Fuji Silysia Chemical, Ltd.), ethyl acetate-methanol-water) to give the title compound as a yellow solid (399 mg, yield: 3.6%).

(6b) (4-Carbamimidoylphenylamino)-(4-dimethylcarbamoylmethoxy-3-ethoxyphenyl)acetic acid hydrochloride 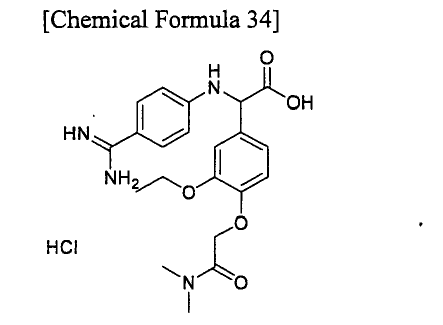 (4-Carbamimidoyl-phenylamino)-(4-dimethylcarbamoylmethoxy-3-ethoxyphenyl)acetic acid methyl ester (379 mg, 0.885 mmol) prepared in Example 6a was dissolved in methanol (5 ml), and 5N sodium hydroxide aqueous solution (0.2 ml) was added. After stirring the reaction mixture overnight at room temperature, 5N hydrochloric acid (0.2 ml) was added and the suspension was concentrated under reduced pressure to give a crude product of the title compound as a pale yellow solid (397 mg, yield: 109%).
¹H-NMR (400 MHz, CD₃OD) δ: 1.38 (t, J = 7.2 Hz, 3H), 2.96 (s, 3H), 3.10 (s, 3H), 4.02-4.13 (m, 2H), 4.78 (s, 2H), 5.14 (s, 1H), 6.75 (d, J = 9.2 Hz, 2H), 6.90 (d, J = 8.4 Hz, 1H), 7.02 (dd, J = 2.0, 8.4 Hz, 1H), 7.12 (d, J = 2.0 Hz, 1H), 7.57 (d, J = 9.2 Hz, 2H)

(6c) 2-(4-(1-(4-Carbamimidoylphenylamino)-2-(N'-(2-chlorobenzoyl)hydrazino)-2-oxoethyl)-2-ethoxyphenoxy)-N,N-dimethylacetamide trifluoroacetate 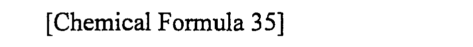 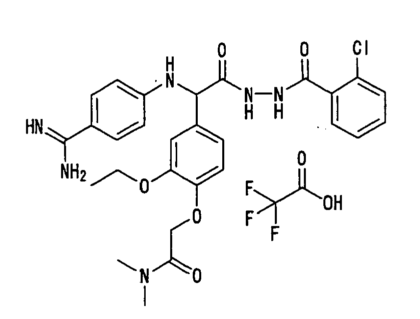 (4-Carbamimidoylphenylamino)-(4-dimethylcarbamoylmethoxy-3-ethoxyphenyl)acetic acid hydrochloride (8.0 mg, 0.015 mmol) prepared in Example 6b was dissolved in N,N-dimethylformamide (0.5 ml) and cooled to -5°C. To the reaction mixture were added 1-hydroxybenzotriazole monohydrate (7.0 mg, 0.045 mmol) and 1-ethyl-3-(3-dimethylaminopropyl)carbodiimide hydrochloride (9.0 mg, 0.045 mmol), followed by stirring for 30 minutes and addition of 2-chloro-benzoic hydrazide (7.7 mg, 0.045 mmol). The reaction mixture was stirred overnight at room temperature and then directly purified by reversed-phase high performance liquid chromatography to give the title compound (2.54 mg, yield: 25%).
¹H-NMR (400 MHz, CD₃OD) δ: 1.40 (t, J = 7.2 Hz, 3H), 2.95 (s, 3H), 3.10 (s, 3H), 4.12 (q, J = 7.2 Hz, 2H), 4.80 (s, 2H), 5.15 (s, 1H), 6.87 (d, J = 8.8 Hz, 2H), 6.93 (d, J = 8.4 Hz, 1H), 7.09 (dd, J = 2.0, 8.4 Hz, 1H), 7.23 (d, J = 2.0 Hz, 1H), 7.36-7.41 (m, 1H), 7.42-7.51 (m, 2H), 7.57-7.60 (m, 1H), 7.63 (d, J = 9.2 Hz, 2H), 8.22 (br, 1H), 8.78 (br, 1H)
Mass spectrum (ESI) *m*/*z:* 567 (M+H)⁺

(Example 7) 2-(4-(1-(4-Carbamimidoyl-phenylamino)-2-oxo-2-(N'-(pyridine-2-carbonyl)hydrazino)-ethyl)-2-ethoxyphenoxy)-N,N-dimethylacetamide trifluoroacetate 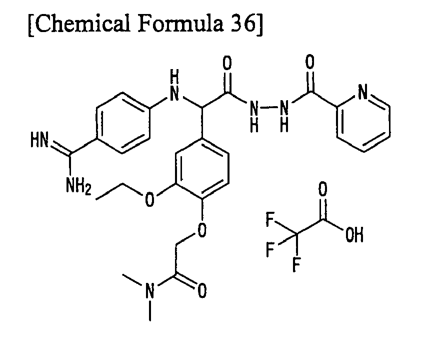 (4-Carbamimidoylphenylamino)-(4-dimethylcarbamoylmethoxy-3-ethoxyphenyl)acetic acid hydrochloride (8.0 mg, 0.015 mmol) prepared in Example 6b was dissolved in N,N-dimethylformamide (0.5 ml) and cooled to -5°C. To the reaction mixture were added 1-hydroxybenzotriazole monohydrate (7.0 mg, 0.045 mmol) and 1-ethyl-3-(3-dimethylaminopropyl)carbodiimide hydrochloride (9.0 mg, 0.045 mmol), followed by stirring for 30 minutes and addition of pyridine-2-carboxylic acid hydrazide (6.2 mg, 0.045 mmol). The reaction mixture was stirred overnight at room temperature and then directly purified by reversed-phase high performance liquid chromatography to give the title compound (3.09 mg, yield: 32%).
¹H-NMR (400 MHz, CD₃OD) δ: 1.40 (t, J = 7.2 Hz, 3H), 2.96 (s, 3H), 3.11 (s, 3H), 4.20-4.38 (m, 2H), 4.80 (s, 2H), 5.18 (s, 1H), 6.87 (d, J = 9.2 Hz, 2H), 6.94 (d, J = 8.0 Hz, 1H), 7.11 (dd, J = 2.0, 8.4 Hz, 1H), 7.25 (d, J = 2.0 Hz, 1H), 7.57 (ddd, J = 1.2, 4.8, 8.0 Hz, 1H), 7.63 (d, J = 8.8 Hz, 2H), 7.96 (dt, J = 2.0, 8.0 Hz, 1H), 8.07 (dt, J = 1.2, 8.0Hz, 1H), 8.23 (br, 1H), 8.63 (ddd, J = 0.8, 1.6, 4.4 Hz, 1H), 8.78 (br, 1H)
Mass spectrum (ESI) *m*/*z*: 534 (M+H)⁺

(Example 8) 2-(4-(1-(4-Carbamimidoylphenylamino)-2-oxo-2-(N'-(pyridine-4-carbonyl)hydrazino)ethyl)-2-ethoxy-phenoxy)-N,N-dimethylacetamide trifluoroacetate 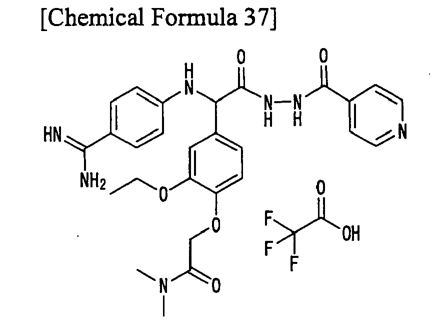 (4-Carbamimidoylphenylamino)-(4-dimethylcarbamoylmethoxy-3-ethoxyphenyl)acetic acid hydrochloride (8.0 mg, 0.015 mmol) prepared in Example 6b was dissolved in N,N-dimethylformamide (0.5 ml) and cooled to -5°C. To the reaction mixture were added 1-hydroxybenzotriazole monohydrate (7.0 mg, 0.045 mmol) and 1-ethyl-3-(3-dimethylaminopropyl)carbodiimide hydrochloride (9.0 mg, 0.045 mmol), followed by stirring for 30 minutes and addition of pyridine-4-carboxylic acid hydrazide (6.2 mg, 0.045 mmol). The reaction mixture was stirred overnight at room temperature and then directly purified by reversed-phase high performance liquid chromatography to give the title compound (3.26 mg, yield: 34%).
¹H-NMR (400 MHz, CD₃OD) δ: 1.40 (t, J = 7.2 Hz, 3H), 2.96 (s, 3H), 3.11 (s, 3H), 4.13 (q, J = 7.2 Hz, 2H), 4.80 (s, 2H), 5.17 (s, 1H), 6.88 (d, J = 9.2 Hz, 2H), 6.94 (d, J = 8.4 Hz, 1H), 7.10 (dd, J = 2.4, 8.4 Hz, 1H), 7.25 (d, J = 2.4 Hz, 1H), 7.64 (d, J = 6.8 Hz, 2H), 7.92 (d, J = 6.4 Hz, 2H), 8.77 (d, J = 6.0Hz, 2H)
Mass spectrum (ESI) *m*/*z*: 534 (M+H)⁺

(Example 9) N-(4-(2-(2-(4-Carbamimidoylphenylamino)-2-(3-ethoxy-4-isopropoxyphenyl)acetyl)hydrazinosulfonyl)phenyl)acetamide trifluoroacetate 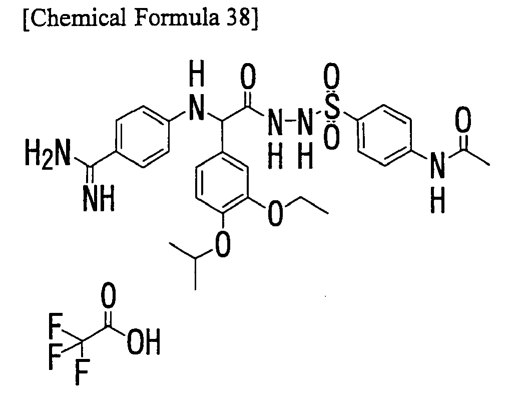 4-(((3-Ethoxy-4-isopropoxyphenyl)hydrazinocarbonylmethyl)amino)benzamidine dihydrochloride (15 mg, 0.0327 mmol) prepared in Example 4b was dissolved in a mixture of acetonitrile (0.8 ml) and dimethylsulfoxide (0.2 ml), and cooled to - 30°C. To the reaction mixture were added triethylamine (7 mg, 0.0654 mmol) and 4-acetylaminobenzenesulfonyl chloride (7.7 mg, 0.0327 mmol) followed by stirring at the same temperature for 1 hour, and it was warmed to room temperature. Stirring was continued for 10 hours, and then the reaction mixture was directly purified by reversed-phase high performance liquid chromatography to give the title compound as a yellow oil (2.99 mg, yield: 16%).
¹H-NMR (400 MHz, CD₃OD) δ: 1.34 (d, J = 6.0 Hz, 6H), 1.40 (t, J = 7.2 Hz, 3H), 2.15 (s, 3H), 4.02 (q, J = 7.2 Hz, 2H), 4.58 (sept, J = 6.0 Hz, 1H), 4.90 (s, 1H), 6.68 (d, J = 8.8 Hz, 2H), 6.88 (dd, J = 8.4, 2.4 Hz, 1H), 6.95 (d, J = 8.4 Hz, 1H), 6.98 (d, J = 2.4 Hz, 1H), 7.42-7.44 (m, 2H), 7.49-7.51 (m, 2H), 7.58 (d, J = 8.8 Hz, 2H); Mass spectrum (ESI) *m*/*z:* 583 (M+H)⁺

(Example 10) 4-(1-(3-Ethoxy-4-isopropoxyphenyl)-2-(2-((2-methanesulfonyl)phenylsulfonyl)hydrazino)-2-oxoethylamino)benzamidine trifluoroacetate 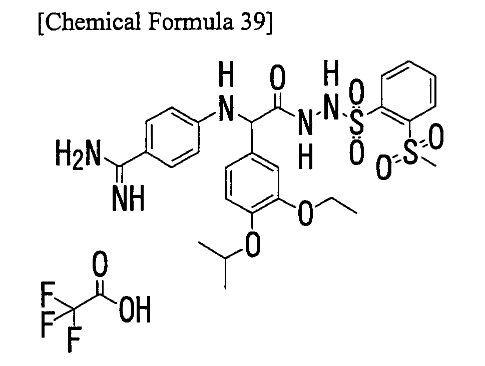 4-(((3-Ethoxy-4-isopropoxyphenyl)hydrazinocarbonylmethyl)amino)benzamidine dihydrochloride (15 mg, 0.0327 mmol) prepared in Example 4b was dissolved in a mixture of acetonitrile (0.8 ml) and dimethylsulfoxide (0.2 ml), and cooled to - 30°C. To the reaction mixture were added triethylamine (7 mg, 0.0654 mmol) and 2-methanesulfonylbenzenesulfonyl chloride (8.4 mg, 0.0327 mmol), followed by stirring at the same temperature for 1 hour, and it was warmed to room temperature. Stirring was continued for 10 hours, and then the reaction mixture was purified by reversed-phase high performance liquid chromatography to give the title compound as a yellow oil (3.98 mg, yield: 20%).
¹H-NMR (400 MHz, CD₃OD) δ: 1.36 (d, J = 6.0 Hz, 6H), 1.42 (t, J = 7.2 Hz, 3H), 3.35 (s, 3H), 3.99 (q, J = 7.2 Hz, 2H), 4.59 (sept, J = 6.0 Hz, 1H), 4.84 (s, 1H), 6.66 (d, J = 8.8 Hz, 2H), 6.85-6.86 (m, 1H), 6.86 (s, 1H), 6.94 (d, J = 8.4 Hz, 1H), 7.33 (t, J = 7.6 Hz, 1H), 7.46 (d, J = 8.0 Hz, 1H), 7.57 (d, J = 8.8 Hz, 2H), 7.74 (t, J = 7.6 Hz, 1H), 8.20 (d, J = 7.6 Hz, 1H); Mass spectrum (ESI) *m*/*z:* 604 (M+H)⁺

(Example 11) 4-(1-(4-Isopropoxy-3-methoxyphenyl)-2-oxo-2-(N'-(pyridine-4-carbonyl)hydrazino)ethylamino)benzamidine trifluoroacetate

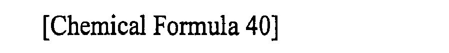

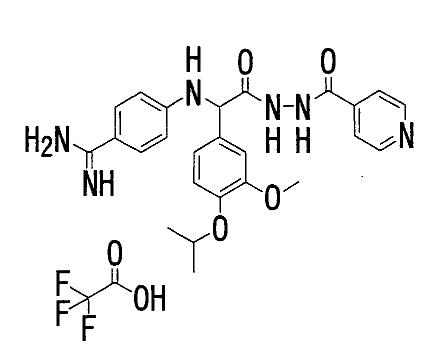

(11a) (4-Carbamimidoylphenylamino)-(4-isopropoxy-3-methoxyphenyl)acetic acid trifluoroacetate 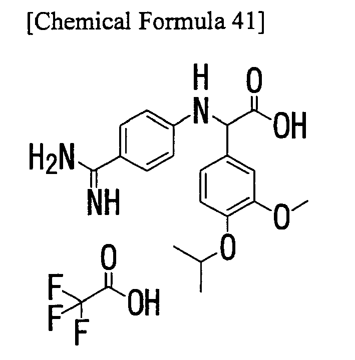 4-Hydroxy-3-methoxybenzaldehyde (70 mg, 0.46 mmol) was dissolved in N,N-dimethylformamide (1.5 ml), and 2-iodopropane (0.070 ml, 0.72 mmol) and potassium carbonate (125 mg, 0.9 mmol) were added. The reaction mixture was stirred overnight at room temperature, and then 1N hydrochloric acid was added to the reaction mixture and extraction was performed with ethyl acetate. The obtained organic layer was concentrated to give 4-isopropoxy-3-methoxybenzaldehyde as a crude product.
This was dissolved in methanol (1.5 ml), 4-aminobenzamidine dihydrochloride, (100 mg, 0.48 mmol) was added, and the mixture was stirred at 60°C for 6 hours. After then cooling the reaction mixture to room temperature, para-toluenesulfonylmethyl isocyanide (120 g, 0.62 mmol) was added. The reaction mixture was cooled to 0°C, and boron trifluoride/diethyl ether complex (0.175 ml, 1.38 mmol) was added. The reaction mixture was stirred overnight at room temperature, and then heptane was added and the methanol layer was separated. Ethyl acetate and saturated aqueous sodium hydrogencarbonate were added to the obtained methanol layer, extraction was performed with ethyl acetate, and the obtained organic layer was concentrated.
The obtained crude product was dissolved in methanol (1.5 ml), and a 5N sodium hydroxide aqueous solution (0.25 ml) was added. After stirring for 3 hours at room temperature, the reaction mixture was neutralized with 5N hydrochloric acid. This was purified by reversed-phase high performance liquid chromatography to give the title compound (39.3 mg, 18%).
¹H-NMR (400 MHz, CD₃OD) δ: 1.30 (d, J = 6.0 Hz, 6H), 3.81 (s, 3H), 4.52 (sept, J = 6.0 Hz, 1H), 5.14 (s, 1H), 6.77 (d, J = 8.8 Hz, 2H), 6.93 (d, J = 8.4Hz, 1H), 7.03 (dd, J = 8.4, 2.4 Hz, 1H), 7.11 (d, J = 2.4 Hz, 1H), 7.58 (d, J = 8.8 Hz, 2H); Mass spectrum (ESI) *m*/*z:* 358 (M+H)⁺

(11b) 4-(1-(4-Isopropoxy-3-methoxyphenyl)-2-oxo-2-(N'-(pyridine-4-carbonyl)hydrazino)ethylamino)benzamidine trifluoroacetate 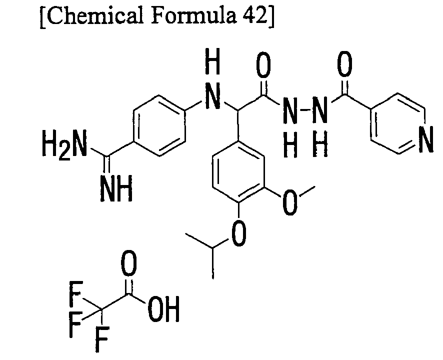 (4-Carbamimidoylphenylamino)-(4-isopropoxy-3-methoxyphenyl)acetic acid trifluoroacetate (10 mg, 0.0212 mmol) prepared in Example 11a was dissolved in N,N-dimethylformamide (1 ml) and cooled to 0°C. To the reaction mixture were added 1-hydroxybenzotriazole monohydrate (10 mg, 0.0635 mmol) and 1-ethyl-3-(3-dimethylaminopropyl)carbodiimide hydrochloride (12 mg, 0.0635 mmol), followed by stirring for 90 minutes and addition of isonicotinic acid hydrazide (9 mg, 0.0635 mmol). The reaction mixture was stirred overnight at room temperature and then directly purified by reversed-phase high performance liquid chromatography to give the title compound as a pale yellow oil (7.29 mg, yield: 58%).
¹H-NMR (400 MHz, CD₃OD) δ: 1.31 (d, J = 6.0 Hz, 6H), 3.87 (s, 3H), 4.55 (sept, J = 6.0 Hz, 1H), 5.17 (s, 1H), 6.87-6.91 (m, 2H), 6.97 (d, J = 8.0 Hz, 1H), 7.12 (dd, J = 8.0, 2.0 Hz, 1H), 7.25 (d, J = 2.0 Hz, 1H), 7.63-7.66 (m, 2H), 7.89-7.90 (m, 2H), 8.75-8.77 (m, 2H); Mass spectrum (ESI) *m*/*z:* 477 (M+H)⁺

(Example 12) 4-(1-(3,4-Bisallyloxyphenyl)-2-(N'-(2-chlorobenzoyl)hydrazino)-2-oxoethylamino)benzamidine trifluoroacetate

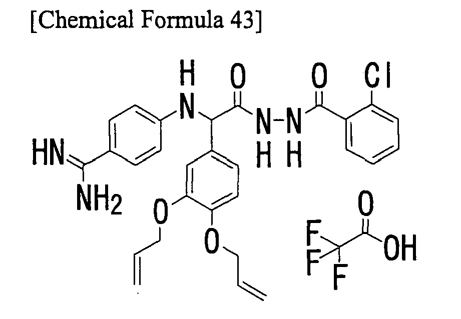

(12a) (3,4-Bisallyloxyphenyl)-(4-carbamimidoylphenylamino)acetic acid methyl ester 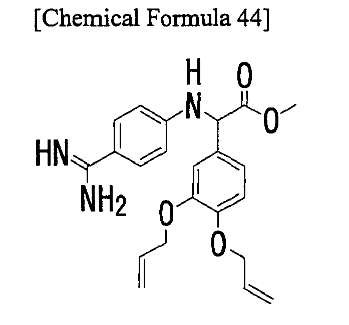 A mixture of 3,4-bisallyloxybenzaldehyde (2.96 g, 13.6 mmol), 4-aminobenzamidine dihydrochloride (2.97 g, 14.3 mmol) and methanol (50 ml) was stirred at 70°C for 2 hours. The reaction mixture was cooled to room temperature, and then para-toluenesulfonylmethyl isocyanide (3.32 g, 17 mmol) was added. After then cooling the reaction mixture to 0°C, boron trifluoride/diethyl ether complex (5.16 ml, 40.8 mmol) was added. The reaction mixture was stirred overnight at room temperature, and then heptane was added and the methanol layer was separated. Saturated aqueous sodium hydrogencarbonate was added to the obtained methanol layer, and extraction was performed with ethyl acetate. The separated organic layer was washed with brine and dried over anhydrous sodium sulfate. The desiccant was removed by filtration and the filtrate was concentrated under reduced pressure. The obtained residue was purified by silica gel column chromatography (NH silica gel (Fuji Silysia Chemical, Ltd.), ethyl acetate-chloroform-methanol) to give the title compound as a pale yellow solid (1.93 g, yield: 36%).
¹H-NMR (400 MHz, CD₃OD) δ: 3.72 (s, 3H), 4.55-4.57 (m, 4H), 5.20-5.25 (m, 3H), 5.35-5.42 (m, 2H), 5.99-6.11 (m, 2H), 6.75 (d, J = 9.2 Hz, 2H), 6.94 (d, J = 8.4 Hz, 1H), 7.02 (dd, J = 8.4, 2.0 Hz, 1H), 7.09 (d, J = 2.0 Hz, 1H), 7.57 (d, J = 9.2 Hz, 2H)

(12b) (3,4-Bisallyloxyphenyl)-(4-carbamimidoylphenylamino)acetic acid hydrochloride 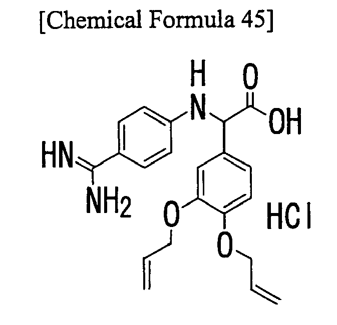 (3,4-Bisallyloxyphenyl)-(4-carbamimidoylphenylamino)acetic acid methyl ester (1.93 g, 4.88 mmol) prepared in Example 12a was dissolved in methanol (50 ml), and a 2N sodium hydroxide aqueous solution (2.56 ml, 5.12 mmol) was added. The reaction mixture was stirred overnight at room temperature and then neutralized with 5N hydrochloric acid. Upon adding diethyl ether and tetrahydrofuran to the mixture, the precipitated solid was collected by filtration. The obtained solid was suspended in tetrahydrofuran, a 2N hydrochloric acid aqueous solution (8 ml) was added, and the mixture was concentrated under reduced pressure. The obtained residue was washed with a mixed solvent of ethyl acetate and dichloromethane to give the title compound as a yellow solid (2.04 g, yield: 78%).
¹H-NMR (400 MHz, CD₃OD) δ: 4.53-4.59 (m, 4H), 5.13 (s, 1H), 5.18-5.26 (m, 2H), 5.34-5.43 (m, 2H), 5.99-6.11 (m, 2H), 6.76 (d, J = 8.8 Hz, 2H), 6.95 (d, J = 8.8 Hz, 1H), 7.05 (dd, J = 8.8, 2.4 Hz, 1H), 7.11 (d, J = 2.4 Hz, 1H), 7.58 (d, J = 8.8 Hz, 2H)

(12c) 4-(1-(3,4-Bisallyloxyphenyl)-2-(N'-(2-chlorobenzoyl)hydrazino)-2-oxoethylamino)benzamidine trifluoroacetate 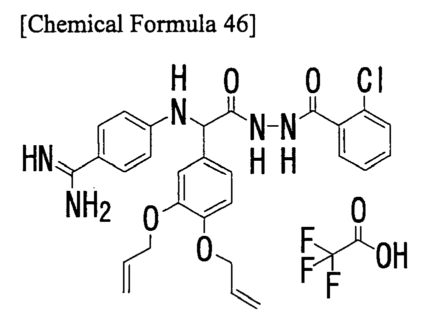 A mixture of (3,4-bisallyloxyphenyl)-(4-carbamimidoylphenylamino)acetic acid hydrochloride (15 mg, 0.036 mmol) prepared in Example 12b, 1-ethyl-3-(3-dimethylaminopropyl)carbodiimide hydrochloride (21 mg, 0.108 mmol), 1-hydroxybenzotriazole monohydrate (16.5 mg, 0.108 mmol) and N,N-dimethylformamide (1 ml) was stirred at 0°C for 1 hour. To the reaction mixture was added 2-chlorobenzoic acid hydrazide (18 mg, 0.106 mmol), followed by stirring overnight at room temperature. The reaction mixture was purified by reversed-phase high performance liquid chromatography to give the title compound as a pale yellow solid (11.84 mg, yield: 51 %).
¹H-NMR (400 MHz, CD₃OD) δ: 4.57 (dt, J = 5.2, 1.6 Hz, 2H), 4.60 (dt, J = 5.2, 1.6 Hz, 2H), 5.13 (s, 1H), 5.20-5.25 (m, 2H), 5.36-5.44 (m, 2H), 6.01-6.12 (m, 2H), 6.87 (d, J = 8.8 Hz, 2H), 6.97 (d, J = 8.4 Hz, 1H), 7.13 (dd, J = 8.4, 2.0 Hz, 1H), 7.23 (d, J = 2.0 Hz, 1H), 7.35-7.61 (m, 4H), 7.63 (d, J = 8.8 Hz, 2H); Mass spectrum (ESI) *m*/*z:* 534 (M+H)⁺

(Example 13) 4-(1-(3,4-Bisallyloxyphenyl)-2-(N'-(4-hydroxybenzoyl)hydrazino)-2-oxoethylamino)benzamidine trifluoroacetate 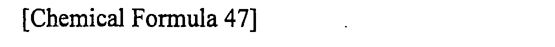 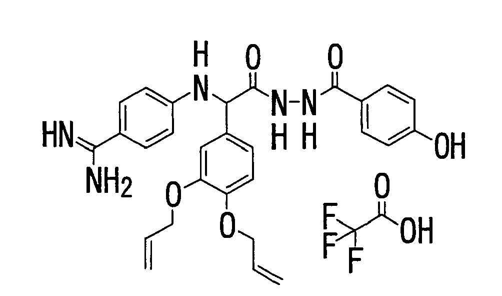 A mixture of (3,4-bisallyloxyphenyl)-(4-carbamimidoylphenylamino)acetic acid hydrochloride (15 mg, 0.036 mmol) prepared in Example 12b, 1-ethyl-3-(3-dimethylaminopropyl)carbodiimide hydrochloride (21 mg, 0.108 mmol), 1-hydroxybenzotriazole monohydrate (16.5 mg, 0.108 mmol) and N,N-dimethylformamide (1 ml) was stirred at 0°C for 1 hour. To the reaction mixture was added 4-hydroxybenzoic acid hydrazide (16 mg, 0.105 mmol), followed by stirring overnight at room temperature. The reaction mixture was purified by reversed-phase high performance liquid chromatography to give the title compound as a pale yellow solid (13.57 mg, yield: 60%).
¹H-NMR (400 MHz, CD₃OD) δ: 4.56 (dt, J = 5.2, 1.6 Hz, 2H), 4.58-4.62 (m, 2H), 5.15 (s, 1H), 5.20-5.26 (m, 2H), 5.36-5.44 (m, 2H), 6.01-6.12 (m, 2H), 6.82 (d, J = 8.8 Hz, 2H), 6.86 (d, J = 8.8 Hz, 2H), 6.96 (d, J = 8.4 Hz, 1H), 7.14 (dd, J = 8.4, 2.4 Hz, 1 H), 7.23 (d, J = 2.4 Hz, 1H), 7.62 (d, J = 8.8 Hz, 2H), 7.73 (d, J = 8.8 Hz, 2H); Mass spectrum (ESI) *m*/*z:* 516 (M+H)⁺

(Example 14) 4-(1-(2-Fluoro-4,5-dimethoxyphenyl)-2-(N'-3-methylpyridine-2-carbonyl)hydrazino)-2-oxoethylamino)benzamidine trifluoroacetate

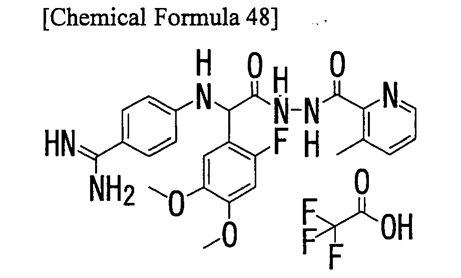

(14a) (4-Carbamimidoylphenylamino)-(2-fluoro-4,5-dimethoxyphenyl)acetic acid methyl ester 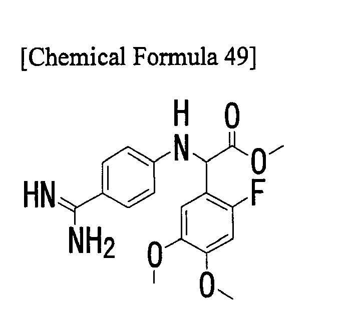 A mixture of 2-fluoro-4,5-dimethoxybenzaldehyde (2.5 g, 13.6 mmol), 4-aminobenzamidine dihydrochloride (2.97 g, 14.3 mmol) and methanol (50 ml) was stirred at 60°C for 4 hours. The reaction mixture was cooled to room temperature, and then para-toluenesulfonylmethyl isocyanide (3.32 g, 17 mmol) was added. After then cooling the reaction mixture to 0°C, boron trifluoride/diethyl ether complex (5.16 ml, 40.8 mmol) was added. The reaction mixture was stirred overnight at room temperature, and then heptane was added and the methanol layer was separated. Saturated aqueous sodium hydrogencarbonate was added to the obtained methanol layer, and extraction was performed with ethyl acetate. The separated organic layer was washed with water and brine in that order and dried over anhydrous sodium sulfate. The desiccant was removed by filtration and the filtrate was concentrated under reduced pressure. The obtained residue was crudely purified by silica gel column chromatography (NH silica gel (Fuji Silysia Chemical, Ltd.), ethyl acetate-chloroform-methanol) to give the title compound as a yellow solid (754 mg).

(14b) (4-Carbamimidoylphenylamino)-(2-fluoro-4,5-dimethoxyphenyl)acetic acid hydrochloride 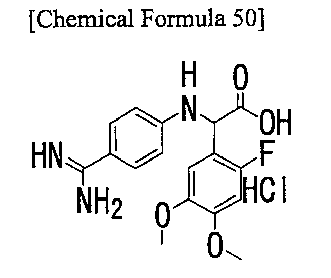 The crude product of (4-carbamimidoylphenylamino)-(2-fluoro-4,5-dimethoxyphenyl)acetic acid methyl ester (754 mg) prepared in Example 14a was dissolved in methanol (2 ml) and a 2N sodium hydroxide aqueous solution (1.1 ml, 2.2 mmol) was added. The reaction mixture was stirred overnight at room temperature and then neutralized with 2N hydrochloric acid. Upon adding diethyl ether and tetrahydrofuran to the mixture, the precipitated solid was collected by filtration. The obtained solid was suspended in tetrahydrofuran, 2N hydrochloric acid (1.5 ml) was added, and the mixture was concentrated under reduced pressure. The obtained residue was washed with a mixed solvent of ethyl acetate and dichloromethane to give the title compound as a yellow solid (560 mg, two-step yield: 11 %).
¹H-NMR (400 MHz, CD₃OD) δ: 3.76 (s, 3H), 3.83 (s, 3H), 5.43 (s, 1H), 6.78 (d, J = 9.2 Hz, 2H), 6.83 (d, J = 11.2 Hz, 1H), 6.97 (d, J = 7.2 Hz, 1H), 7.59 (d, J = 9.2 Hz, 2H); Mass spectrum (ESI) *m*/*z*: 348 (M+H)⁺

(14c) N'-(2-(4-Carbamimidoylphenylamino)-2-(2-fluoro-4,5-dimethoxyphenyl)acetyl)hydrazinecarboxylic acid t-butyl ester 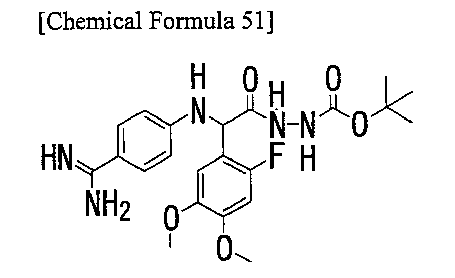 A mixture of (4-carbamimidoylphenylamino)-(2-fluoro-4,5-dimethoxyphenyl)acetic acid hydrochloride (250 mg, 0.651 mmol) prepared in Example 14b, 1-ethyl-3-(3-dimethylaminopropyl)carbodiimide hydrochloride (374 mg, 1.95 mmol), 1-hydroxybenzotriazole monohydrate (299 mg, 1.95 mmol) and N,N-dimethylformamide (5 ml) was stirred at 0°C for 1 hour. To the reaction mixture was added hydrazinecarboxylic acid t-butyl ester (258 mg, 1.95 mmol), followed by stirring overnight at room temperature The reaction mixture was crudely purified by silica gel column chromatography (NH silica gel (Fuji Silysia Chemical, Ltd.), ethyl acetate-dichloromethane-methanol) to give the title compound as a colorless solid (277 mg, yield: 92%).

(14d) 4-(((2-Fluoro-4,5-dimethoxyphenyl)hydrazinocarbonylmethyl)amino)benzamidine dihydrochloride 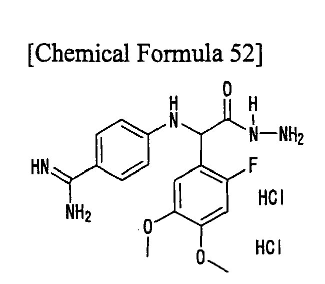 To a mixture of N'-(2-(4-carbamimidoylphenylamino)-2-(2-fluoro-4,5-dimethoxyphenyl)acetyl)hydrazinecarboxylic acid t-butyl ester (277 mg, 0.600 mmol) prepared in Example 14c and ethanol (2 ml) was added a 40% solution of hydrochloric acid in ethanol (2 ml). After stirring overnight at room temperature, the reaction mixture was concentrated under reduced pressure. The residue was washed with dichloromethane to give the title compound as a yellow solid (250 mg, yield: 96%).
Mass spectrum (ESI) *m*/*z:* 362 (M+H)⁺

(14e) 4-(1-(2-Fluoro-4,5-dimethoxyphenyl)-2-(N'-(3-methylpyridine-2-carbonyl)hydrazino)-2-oxoethylamino)benzamidine trifluoroacetate 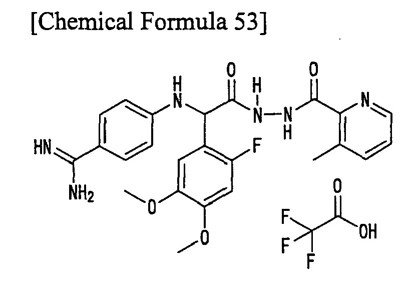 A mixture of 3-methylpyridine-2-carboxylic acid (4.5 mg, 0.0328 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (1 ml) was stirred at 0°C for 1 hour. To the reaction mixture was added 4-(((2-Fluoro-4,5-dimethoxyphenyl)hydrazinocarbonylmethyl)amino)benzamidine dihydrochloride (14 mg, 0.0322 mmol) prepared in Example 14d, followed by stirring overnight at room temperature. The reaction mixture was purified by reversed-phase high performance liquid chromatography to give the title compound as a colorless solid (5.47 mg, yield: 29%).
¹H-NMR (400 MHz, CD₃OD) δ: 2.60 (s, 3H), 3.83 (s, 3H), 3.85 (s, 3H), 5.50 (s, 1H), 6.85 (d, J = 11.6 Hz, 1H), 6.89 (d, J = 9.2 Hz, 2H), 7.26 (d, J = 7.2 Hz, 1H), 7.44 (dd, J = 8.0, 4.4 Hz, 1H), 7.65 (d, J = 9.2 Hz, 2H), 7.72-7.76 (m, 1H), 8.42-8.43 (m, 1H); Mass spectrum (ESI) *m*/*z*: 481 (M+H)⁺

(Example 15) 4-(2-(N'-(3-Bromopyridine-2-carbonyl)hydrazino)-1-(2-fluoro-4,5-dimethoxyphenyl)-2-oxoethylamino)benzamidine trifluoroacetate 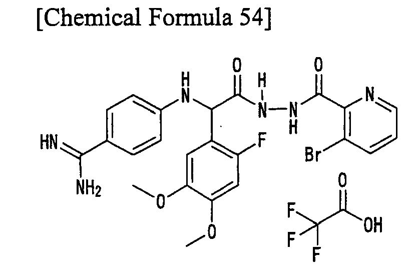 A mixture of3-bromopyridine-2-carboxylic acid (6.5 mg, 0.0322 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (1 ml) was stirred at 0°C for 1 hour. To the reaction mixture was added 4-(((2-Fluoro-4,5-dimethoxyphenyl)hydrazinocarbonylmethyl)amino)benzamidine dihydrochloride (14 mg, 0.0322 mmol) prepared in Example 14d, followed by stirring overnight at room temperature. The reaction mixture was purified by reversed-phase high performance liquid chromatography to give the title compound as a colorless solid (6.66 mg, yield: 31 %).
¹H-NMR (400 MHz, CD₃OD) δ: 3.83 (s, 3H), 3.84 (s, 3H), 5.49 (s, 1H), 6.85 (d, J = 11.6 Hz, 1H), 6.89 (d, J = 9.2 Hz, 2H), 7.24 (d, J = 6.8 Hz, 1H), 7.44 (dd, J = 8.0, 4.8 Hz, 1H), 7.65 (d, J = 9.2 Hz, 2H), 8.16 (dd, J = 8.0, 1.6Hz, 1H), 8.57 (dd, J = 4.8,1.6 Hz, 1H); Mass spectrum (ESI) *m*/*z*: 545 (M+H)⁺

(Example 16) 4-(1-(2-Fluoro-4,5-dimethoxyphenyl)-2-(N'-(2-fluoropyridine-3-carbonyl)hydrazino)-2-oxoethylamino)benzamidine trifluoroacetate 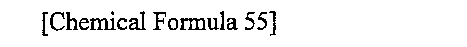 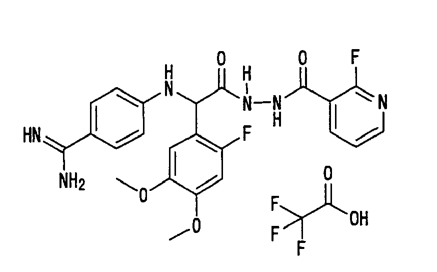 A mixture of 2-fluoronicotinic acid (4.5 mg, 0.0319 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (1 ml) was stirred at 0°C for 1 hour. To the reaction mixture was added 4-(((2-fluoro-4,5-dimethoxyphenyl)hydrazinocarbonylmethyl)amino)benzamidine dihydrochloride (14 mg, 0.0322 mmol) prepared in Example 14d, followed by stirring overnight at room temperature. The reaction mixture was purified by reversed-phase high performance liquid chromatography to give the title compound as a colorless solid (5.24 mg, yield: 27%).
¹H-NMR (400 MHz, CD₃OD) δ: 3.834 (s, 3H), 3.837 (s, 3H), 5.48 (s, 1H), 6.86 (d, J = 11.6 Hz, 1H), 6.89 (d, J = 8.8 Hz, 2H), 7.23 (d, J = 7.2 Hz, 1H), 7.42-7.46 (m, 1H), 7.65 (d, J = 8.8 Hz, 2H), 8.27-8.32 (m, 1H), 8.35-8.37 (m, 1H); Mass spectrum (ESI) *m*/*z:* 485 (M+H)⁺

(Example 17) 4-(1-(2-Fluoro-4,5-dimethoxyphenyl)-2-(N'-(3-fluoropyridine-4-carbonyl)hydrazino)-2-oxoethylamino)benzamidine trifluoroacetate 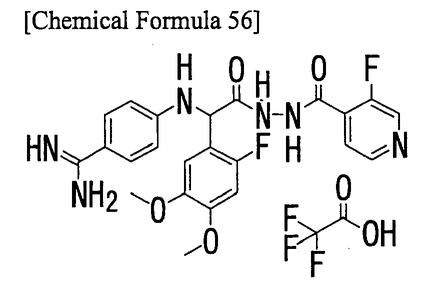 A mixture of 3-fluoroisonicotinic acid (4.5 mg, 0.0319 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (1 ml) was stirred at 0°C for 1 hour. To the reaction mixture was added 4-(((2-fluoro-4,5-dimethoxyphenyl)hydrazinocarbonylmethyl)amino)benzamidine dihydrochloride (14 mg, 0.0322 mmol) prepared in Example 14d, followed by stirring overnight at room temperature. The reaction mixture was purified by reversed-phase high performance liquid chromatography to give the title compound as a yellow solid (6.13 mg, yield: 32%).
¹H-NMR(400 MHz, CD₃OD)δ:3.83(s, 6H), 5.48(s, 1H), 6.86(d, J = 11.6 Hz, 1H), 6.89(d, J = 9.2 Hz, 2H), 7.22(d, J = 6.8 Hz, 1H), 7.65(d, J = 9.2 Hz, 2H), 7.72(t, J = 5.2 Hz, 1H), 8.53(d, J = 5.2 Hz, 1H), 8.62(d, J = 1.6 Hz, 1H); Mass spectrum
(ESI)*m*/*z*: 485(M+H)⁺

(Example 18) 4-(2-(N'-(3-Chloropyridine-4-carbonyl)hydrazino)-1-(2-fluoro-4,5-dimethoxyphenyl)-2-oxoethylamino)benzamidine trifluoroacetate 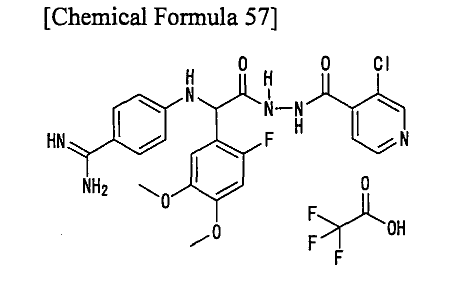 A mixture of 3-chloroisonicotinic acid (5.2 mg, 0.0330 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (1 ml) was stirred at 0°C for 1 hour. To the reaction mixture was added 4-(((2-fluoro-4,5-dimethoxyphenyl)hydrazinocarbonylmethyl)amino)benzamidine dihydrochloride (14 mg, 0.0322 mmol) prepared in Example 14d, followed by stirring overnight at room temperature. The reaction mixture was purified by reversed-phase high performance liquid chromatography to give the title compound as a yellow solid (6.24 mg, yield: 32%).
¹H-NMR (400 MHz, CD₃OD) δ: 3.825 (s, 3H), 3.831 (s, 3H), 5.47 (s, 1H), 6.86 (d, J = 11.6 Hz, 1H), 6.89 (d, J = 8.8 Hz, 2H), 7.21 (d, J = 6.8 Hz, 1H), 7.59 (d, J = 5.2 Hz, 1H), 7.65 (d, J = 8.8 Hz, 2H), 8.58 (d, J = 5.2 Hz, 1H), 8.69 (s, 1H); Mass spectrum (ESI) *m*/*z:* 501 (M+H)⁺

(Example 19) 4-(1-(3-Ethoxy-4-isopropoxyphenyl)-2-(N'-(3-methylpyridine-4-carbonyl)hydrazino)-2-oxoethylamino)benzamidine trifluoroacetate 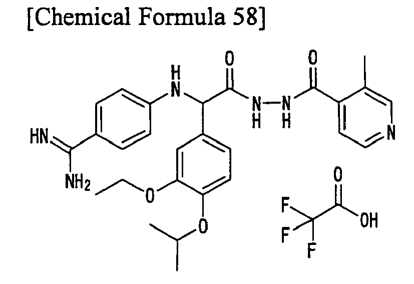 A mixture of 3-methylisonicotinic acid (4.5 mg, 0.0328 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (1 ml) was stirred at 0°C for 1 hour. To the reaction mixture was added 4-(((3-ethoxy-4-isopropoxyphenyl)hydrazinocarbonylmethyl)amino)benzamidine dihydrochloride (15 mg, 0.0327 mmol) prepared in Example 4b, followed by stirring overnight at room temperature. The reaction mixture was purified by reversed-phase high performance liquid chromatography to give the title compound as a colorless solid (2.45 mg, yield: 12%). ¹H-NMR (400 MHz, CD₃OD) δ: 1.29 (d, J = 6.0 Hz, 6H), 1.39 (t, J = 6.4 Hz, 3H), 2.48 (s, 3H), 4.13 (q, J = 7.2 Hz, 2H), 4.52 (sept, 6.4 Hz, 1H), 5.15 (s, 1H), 6.88 (d, J = 9.2 Hz, 2H), 6.98 (d, J = 8.4 Hz, 1H), 7.10 (dd, J = 2.4, 8.4 Hz, 1H), 7.22 (d, J = 2.4 Hz, 1H), 7.57 (d, J = 4.8 Hz, 1H), 7.64 (d, J = 9.2 Hz, 2H), 8.52 (d, J = 5.2 Hz, 1H), 8.57 (s, 1H); Mass spectrum (ESI) *m*/*z:* 505 (M+H)⁺

(Example 20) 4-(1-(3-Ethoxy-4-isopropoxyphenyl)-2-(N'-(4-methoxypyridine-3-carbonyl)hydrazino)-2-oxoethylamino)benzamidine trifluoroacetate 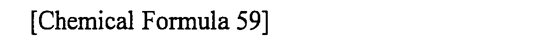 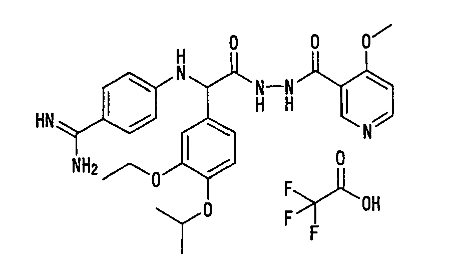 A mixture of 4-methoxynicotinic acid lithium salt (8 mg, 0.0503 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (1 ml) was stirred at 0°C for 1 hour. To the reaction mixture was added 4-(((3-ethoxy-4-isopropoxyphenyl)hydrazinocarbonylmethyl)amino)benzamidine dihydrochloride (15 mg, 0.0327 mmol) prepared in Example 4b, followed by stirring overnight at room temperature. The reaction mixture was purified by reversed-phase high performance liquid chromatography to give the title compound as a colorless solid (8.5 mg, yield: 41%).
¹H-NMR (400 MHz, CD₃OD) δ: 1.29 (d, J = 6.0 Hz, 6H), 1.39 (t, J = 7.2 Hz, 3H), 3.96 (s, 3H), 4.10 (q, J = 7.2 Hz, 2H), 4.52 (sept, 6.4 Hz, 1H), 5.15 (s, 1H), 6.85 (dd, J = 0.8, 8.8 Hz, 1H), 6.88 (d, J = 9.2 Hz, 2H), 6.96 (d, J = 8.4 Hz, 1H), 7.11 (dd, J = 2.4, 8.4 Hz, 1H), 7.22 (d, J = 2.4 Hz, 1H), 7.64 (d, J = 8.8 Hz, 2H), 8.08 (dd, J = 2.4, 8.8 Hz, 1H), 8.65 (d, J = 2.0 Hz, 1H); Mass spectrum (ESI) *m*/*z*: 521 (M+H)⁺

(Example 21) 4-(2-(N'-(3-Bromopyridine-2-carbonyl)hydrazino)-1-(3-ethoxy-4-isopropoxyphenyl)-2-oxoethylamino)benzamidine trifluoroacetate 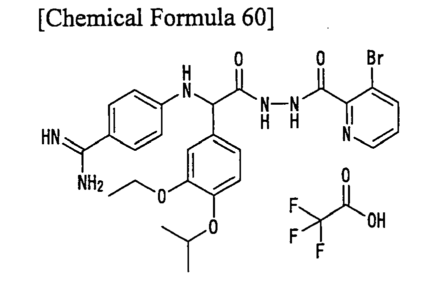 A mixture of 3-bromopyridine-2-carboxylic acid (6.5 mg, 0.0322 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (0.5 ml) was stirred at 0°C for 1 hour. To the reaction mixture was added 4-(((3-ethoxy-4-isopropoxyphenyl)hydrazinocarbonylmethyl)amino)benzamidine dihydrochloride (15 mg, 0.0327 mmol) prepared in Example 4b, followed by stirring overnight at room temperature. The reaction mixture was purified by reversed-phase high performance liquid chromatography to give the title compound as a colorless solid (8.24 mg, yield: 37%). ¹H-NMR (400 MHz, CD₃OD) δ: 1.29 (d, J = 6.0 Hz, 6H), 1.39 (t, J = 6.8 Hz, 3H), 4.07-4.14 (m, 2H), 4.52 (sept, 6.4 Hz, 1H), 5.16 (s, 1H), 6.88 (d, J = 8.8 Hz, 2H), 6.96 (d, J = 8.4 Hz, 1H), 7.10 (dd, J = 2.4, 8.0 Hz, 1H), 7.23 (d, J = 2.4 Hz, 1H), 7.43 (dd, J = 4.4, 8.0 Hz, 1H), 7.64 (d, J = 9.2 Hz, 2H), 8.15 (dd, J = 1.2, 8.0 Hz, 1H), 8.56 (d, J = 1.6,4.4 Hz, 1H); Mass spectrum (ESI) *m*/*z:* 569 (M+H)⁺

(Example 22) 4-(1-(3-Ethoxy-4-isopropoxyphenyl)-2-(N'-(2-fluoropyridine-3-carbonyl)hydrazino)-2-oxoethylamino)benzamidine trifluoroacetate 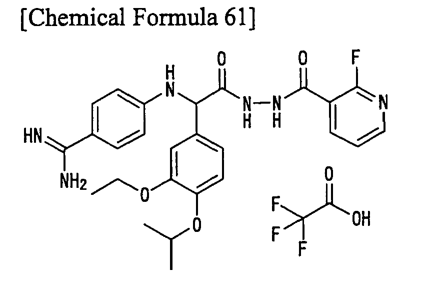 A mixture of 2-fluoronicotinic acid (4.8 mg, 0.0340 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (0.5 ml) was stirred at 0°C for 1 hour. To the reaction mixture was added 4-(((3-ethoxy-4-isopropoxyphenyl)hydrazinocarbonylmethyl)amino)benzamidine dihydrochloride (15 mg, 0.0327 mmol) prepared in Example 4b, followed by stirring overnight at room temperature. The reaction mixture was purified by reversed-phase high performance liquid chromatography to give the title compound as a colorless solid (3.69 mg, yield: 18%).
¹H-NMR (400 MHz, CD₃OD) δ: 1.29 (d, J = 6.0 Hz, 6H), 1.39 (t, J = 6.8 Hz, 3H), 4.10 (q, J = 6.8 Hz, 2H), 4.52 (sept, 6.4 Hz, 1H), 5.15 (s, 1H), 6.88 (d, J = 9.2 Hz, 2H), 6.96 (d, J = 8.4 Hz, 1H), 7.11 (dd, J = 2.4, 8.4 Hz, 1H), 7.22 (d, J = 2.4 Hz, 1H), 7.42-7.45 (m, 1H), 7.64 (d, J = 9.2 Hz, 2H), 8.26-8.32 (m, 1H), 8.34-8.38 (m, 1H); Mass spectrum (ESI) *m*/*z*: 509 (M+H)⁺

(Example 23) 4-(2-(N'-(2-Chlorobenzoyl)hydrazino)-1-(2-fluoro-4,5-dimethoxyphenyl)-2-oxoethylamino)benzamidine trifluoroacetate 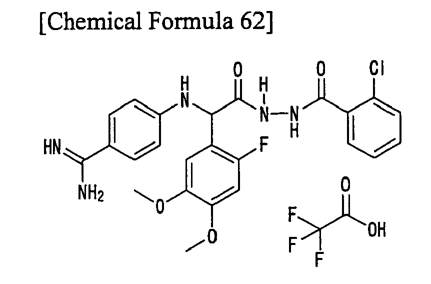 A mixture of (4-carbamimidoylphenylamino)-(2-fluoro-4,5-dimethoxyphenyl)acetic acid hydrochloride (10 mg, 0.0261 mmol) prepared in Example 14b, 1-ethyl-3-(3-dimethylaminopropyl)carbodiimide hydrochloride (15 mg, 0.0783 mmol), 1-hydroxybenzotriazole monohydrate (12 mg, 0.0783 mmol) and N,N-dimethylformamide (1 ml) was stirred at 0°C for 1 hour. To the reaction mixture was added 2-chlorobenzoic acid hydrazide (13 mg, 0.0783 mmol), followed by stirring overnight at room temperature. The reaction mixture was purified by reversed-phase high performance liquid chromatography to give the title compound as a pale yellow solid (9.57 mg, yield: 60%).
¹H-NMR (400 MHz, CD₃OD) δ: 3.82 (s, 3H), 3.83 (s, 3H), 5.48 (s, 1H), 6.85 (d, J = 11.6 Hz, 1H), 6.89 (d, J = 8.8 Hz, 2H), 7.22 (d, J = 7.2 Hz, 1H), 7.37-7.41 (m, 1H), 7.40-7.50 (m, 2H), 7.58-7.60 (m, 1H), 7.65 (d, J = 8.8 Hz, 2H); Mass spectrum (ESI) *m*/*z*: 500 (M+H)⁺

(Example 24) 4-(1-(2-Fluoro-4,5-dimethoxyphenyl)-2-oxo-2-(N'-(pyridine-4-carbonyl)hydrazino)ethylamino)benzamidine trifluoroacetate 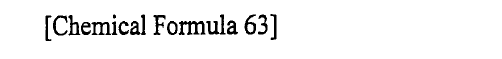 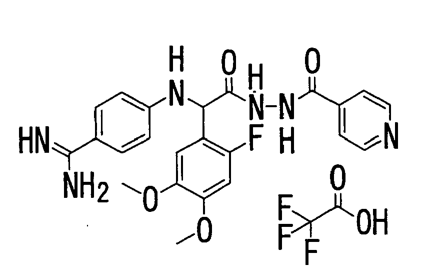 A mixture of (4-carbamimidoylphenylamino)-(2-fluoro-4,5-dimethoxyphenyl)acetic acid hydrochloride (10 mg, 0.0261 mmol) prepared in Example 14b, 1-ethyl-3-(3-dimethylaminopropyl)carbodiimide hydrochloride (15 mg, 0.0783 mmol), 1-hydroxybenzotriazole monohydrate (12 mg, 0.0783 mmol) and N,N-dimethylformamide (1 ml) was stirred at 0°C for 1 hour. To the reaction mixture was added isonicotinic acid hydrazide (11 mg, 0.0783 mmol), followed by stirring overnight at room temperature. The reaction mixture was purified by reversed-phase high performance liquid chromatography to give the title compound as a yellow solid (9.88 mg, yield: 65%).
¹H-NMR (400 MHz, CD₃OD) δ: 3.835 (s, 3H), 3.842 (s, 3H), 5.49 (s, 1H), 6.86 (d, J = 11.2 Hz, 1H), 6.89 (d, J = 9.2 Hz, 2H), 7.24 (d, J = 6.8 Hz, 1H), 7.66 (d, J = 8.8 Hz, 2H), 7.91 (d, J = 6.4 Hz, 2H), 8.77 (d, J = 6.4 Hz, 2H); Mass spectrum (ESI) *m*/*z:* 467 (M+H)⁺

(Example 25) 4-(2-(N'-(4-Chloropyridine-3-carbonyl)hydrazino)-1-(3-ethoxy-4-isopropoxyphenyl)-2-oxoethylamino)benzamidine trifluoroacetate 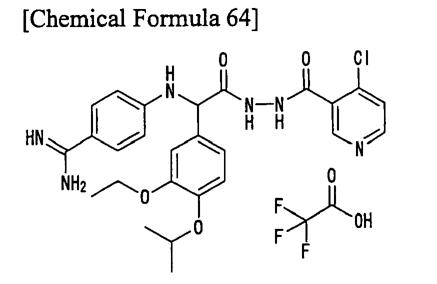 A mixture of 4-chloronicotinic acid (5 mg, 0.0317 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (1 ml) was stirred at 0°C for 1 hour. To the reaction mixture was added 4-(((3-ethoxy-4-isopropoxyphenyl)hydrazinocarbonylmethyl)amino)benzamidine dihydrochloride (15 mg, 0.0327 mmol) prepared in Example 4b, followed by stirring overnight at room temperature. The reaction mixture was purified by reversed-phase high performance liquid chromatography to give the title compound as a yellow solid (2.84 mg, yield: 14%). ¹H-NMR (400 MHz, CD₃OD) δ: 1.30 (d, J = 6.0 Hz, 6H), 1.40 (t, J = 7.2 Hz, 3H), 4.10 (q, J = 7.2 Hz, 2H), 4.53 (sept, J = 6.0 Hz, 1H), 5.15 (s, 1H), 6.88 (d, J = 9.2 Hz, 2H), 6.96 (d, J = 8.4 Hz, 1H), 7.10 (dd, J = 8.4, 2.4 Hz, 1H), 7.21 (d, J = 2.4 Hz, 1H), 7.60 (d, J = 5.6 Hz, 1H), 7.64 (d, J = 9.2 Hz, 2H), 8.57 (d, J = 5.6 Hz, 1H), 8.71 (s, 1H); Mass spectrum (ESI) *m*/*z*: 525 (M+H)⁺

(Example 26) 4-(1-(3-Ethoxy-4-isopropoxyphenyl)-2-(N'-(2-methylpyridine-3-carbonyl)hydrazino)-2-oxoethylamino)benzamidine trifluoroacetate 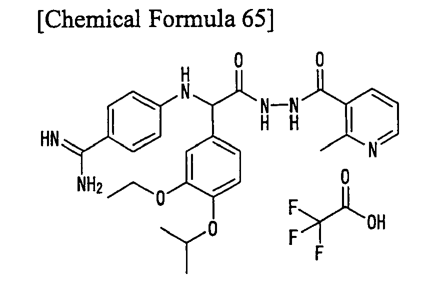 A mixture of 2-methylnicotinic acid (4.6 mg, 0.0335 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (1 ml) was stirred at 0°C for 1 hour. To the reaction mixture was added 4-(((3-ethoxy-4-isopropoxyphenyl)hydrazinocarbonylmethyl)amino)benzamidine dihydrochloride (15 mg, 0.0327 mmol) prepared in Example 4b, followed by stirring overnight at room temperature. The reaction mixture was purified by reversed-phase high performance liquid chromatography to give the title compound as a pale yellow solid (14.82 mg, yield: 73%).
¹H-NMR (400 MHz, CD₃OD) δ: 1.30 (d, J = 6.0 Hz, 6H), 1.40 (t, J = 7.2 Hz, 3H), 2.80 (s, 3H), 4.10 (q, J = 7.2 Hz, 2H), 4.53 (sept, J = 6.0 Hz, 1H), 5.17 (s, 1H), 6.88 (d, J = 9.2 Hz, 2H), 6.96 (d, J = 8.4 Hz, 1H), 7.10 (dd, J = 8.4, 2.0 Hz, 1H), 7.23 (d, J = 2.0 Hz, 1H), 7.64 (d, J = 9.2 Hz, 2H), 7.70 (dd, J = 8.0, 5.6 Hz, 1H), 8.33 (dd, J = 8.0, 1.6 Hz, 1H), 8.69 (dd, J = 5.6, 1.6 Hz, 1H); Mass spectrum (ESI) *m*/*z:* 505 (M+H)⁺

(Example 27) 4-(1-(3-Ethoxy-4-isopropoxyphenyl)-2-(N'-(4-methylpyridine-3-carbonyl)hydrazino)-2-oxoethylamino)benzamidine trifluoroacetate 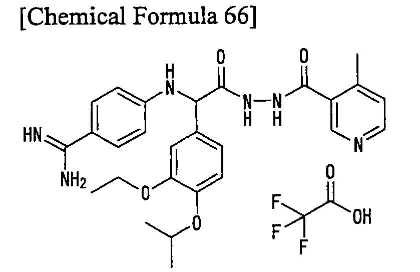 A mixture of 4-methylnicotinic acid hydrochloride (5.7 mg, 0.0328 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (1 ml) was stirred at 0°C for 1 hour. To the reaction mixture was added 4-(((3-ethoxy-4-isopropoxyphenyl)hydrazinocarbonylmethyl)amino)benzamidine dihydrochloride (15 mg, 0.0327 mmol) prepared in Example 4b, followed by stirring overnight at room temperature. The reaction mixture was purified by reversed-phase high performance liquid chromatography to give the title compound as a colorless solid (10.99 mg, yield: 54%). ¹H-NMR (400 MHz, CD₃OD) δ: 1.30 (d, J = 6.0 Hz, 6H), 1.40 (t, J = 7.2 Hz, 3H), 2.61 (s, 3H), 4.10 (q, J = 7.2 Hz, 2H), 4.53 (sept, J = 6.0 Hz, 1H), 5.16 (s, 1H), 6.88 (d, J = 8.8 Hz, 2H), 6.96 (d, J = 8.4 Hz, 1H), 7.10 (dd, J = 8.4, 2.0 Hz, 1H), 7.23 (d, J = 2.0 Hz, 1H), 7.60 (d, J = 5.6 Hz, 1H), 7.64 (d, J = 8.8 Hz, 2H), 8.58 (d, J = 5.6 Hz, 1H), 8.72 (s, 1H); Mass spectrum (ESI) *m*/*z:* 505 (M+H)⁺

(Example 28) 4-(1-(3-Ethoxy-4-isopropoxyphenyl)-2-(N'-(3-fluoropyridine-4-carbonyl)hydrazino)-2-oxoethylainino)benzamidine trifluoroacetate 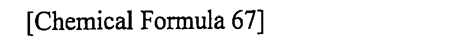 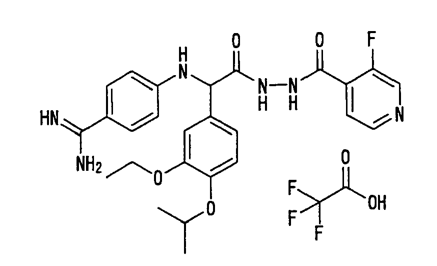 A mixture of 3-fluoroisonicotinic acid (4.8 mg, 0.0340 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (1 ml) was stirred at 0°C for 1 hour. To the reaction mixture was added 4-(((3-ethoxy-4-isopropoxyphenyl)hydrazinocarbonylmethyl)amino)benzamidine dihydrochloride (15 mg, 0.0327 mmol) prepared in Example 4b, followed by stirring overnight at room temperature. The reaction mixture was purified by reversed-phase high performance liquid chromatography to give the title compound as a pale yellow solid (5.20 mg, yield: 26%).
¹H-NMR (400 MHz, CD₃OD) δ: 1.30 (d, J = 6.0 Hz, 6H), 1.40 (t, J = 7.2 Hz, 3H), 4.10 (q, J = 7.2 Hz, 2H), 4.53 (sept, J = 6.0 Hz, 1H), 5.15 (s, 1H), 6.87 (d, J = 8.8 Hz, 2H), 6.96 (d, J = 8.0 Hz, 1H), 7.10 (dd, J = 8.0,2.0 Hz, 1H), 7.22 (d, J = 2.0 Hz, 1H), 7.64 (d, J = 8.0 Hz, 2H), 7.71 (t, J = 5.2 Hz, 1H), 8.52 (dd, J = 4.8, 0.8 Hz, 1H), 8.61 (d, J = 1.6 Hz, 1H); Mass spectrum (ESI) *m*/*z*: 509 (M+H)⁺

(Example 29) 4-(1-(3-Ethoxy-4-isopropoxyphenyl)-2-oxo-2-(N'-(1-oxypyridine-3-carbonyl)hydrazino)ethylamino)benzamidine trifluoroacetate 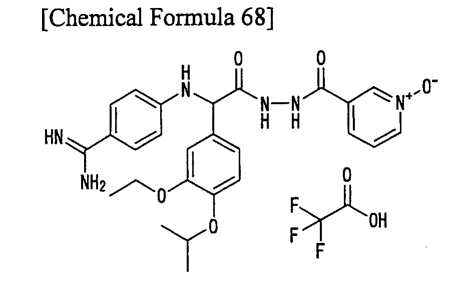 A mixture of 1-oxynicotinic acid (4.5 mg, 0.0323 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (1 ml) was stirred at 0°C for 1 hour. To the reaction mixture was added 4-(((3-ethoxy-4-isopropoxyphenyl)hydrazinocarbonylmethyl)amino)benzamidine dihydrochloride (15 mg, 0.0327 mmol) prepared in Example 4b, followed by stirring overnight at room temperature. The reaction mixture was purified by reversed-phase high performance liquid chromatography to give the title compound as a pale yellow solid (9.11 mg, yield: 45%).
¹H-NMR (400 MHz, CD₃OD) δ: 1.30 (d, J = 6.0 Hz, 6H), 1.41 (t, J = 7.2 Hz, 3H), 4.10 (q, J = 7.2 Hz, 2H), 4.53 (sept, J = 6.0 Hz, 1H), 5.15 (s, 1H), 6.87 (d, J = 8.8 Hz, 2H), 6.96 (d, J = 8.0 Hz, 1H), 7.10 (dd, J = 8.0, 2.0 Hz, 1H), 7.22 (d, J = 2.0 Hz, 1H), 7.61-7.60 (m, 3H), (d, J = 8.8 Hz, 2H), 7.94-7.97 (m, 1H), 8.44-8.47 (m, 1H), 8.68-8.70 (m, 1H); Mass spectrum (ESI) *m*/*z*: 507 (M+H)⁺

(Example 30) 4-(1-(3-Ethoxy-4-isopropoxyphenyl)-2-(N'-(4-hydroxybenzoyl)hydrazino)-2-oxoethylamino)benzamidine trifluoroacetate 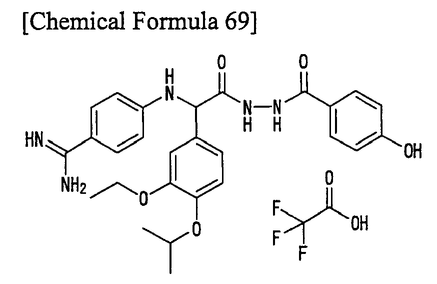 A mixture of (4-carbamimidoylphenylamino)-(3-ethoxy-4-isopropoxyphenyl)acetic acid hydrochloride (20 mg, 0.0490 mmol) prepared in Example 1b, 1-ethyl-3-(3-dimethylaminopropyl)carbodiimide hydrochloride (28 mg, 0.145 mmol), 1-hydroxybenzotriazole monohydrate (22 mg, 0.145 mmol) and N,N-dimethylformamide (1 ml) was stirred at 0°C for 1 hour. To the reaction mixture was added 4-hydroxybenzoic acid hydrazide (22 mg, 0.145 mmol), followed by stirring overnight at room temperature. The reaction mixture was purified by reversed-phase high performance liquid chromatography to give the title compound as a colorless solid (15.79 mg, yield: 52%).
¹H-NMR (400 MHz, CD₃OD) δ: 1.30 (d, J = 6.0 Hz, 6H), 1.40 (t, J = 7.2 Hz, 3H), 4.09 (q, J = 7.2 Hz, 2H), 4.52 (sept, J = 6.0 Hz, 1H), 5.15 (s, 1H), 6.82 (d, J = 8.8 Hz, 2H), 6.87 (d, J = 8.8 Hz, 2H), 6.95 (d, J = 8.4 Hz, 1H), 7.11 (dd, J = 8.4, 2.0 Hz, 1H), 7.21 (d, J = 2.0 Hz, 1H), 7.63 (d, J = 8.8 Hz, 2H), 7.72 (d, J = 8.8 Hz, 2H); Mass spectrum (ESI) *m*/*z:* 506 (M+H)⁺

(Example 31) 4-(2-(N'-(2-Cyanobenzoyl)hydrazino)-1-(3-ethoxy-4-isopropoxyphenyl)-2-oxoethylamino)benzamidine trifluoroacetate 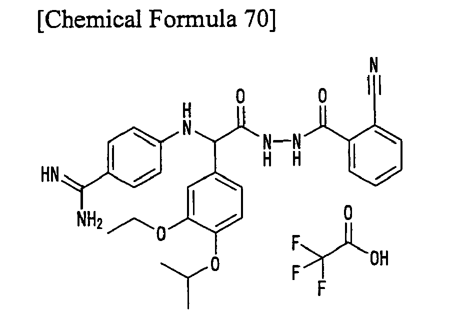 A mixture of 2-cyanobenzoic acid (4.7 mg, 0.0319 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (0.6 ml) was stirred at 0°C for 1 hour. To the reaction mixture was added 4-(((3-ethoxy-4-isopropoxyphenyl)hydrazinocarbonylmethyl)amino)benzamidine dihydrochloride (15 mg, 0.0327 mmol) prepared in Example 4b, followed by stirring overnight at room temperature. The reaction mixture was purified by reversed-phase high performance liquid chromatography to give the title compound as a colorless solid (2.27 mg, yield: 11 %).
¹H-NMR (400 MHz, CD₃OD) δ: 1.33 (d, J = 6.0 Hz, 6H), 1.40 (t, J = 7.2 Hz, 3H), 4.17 (qd, J = 7.2, 2.4 Hz, 2H), 4.57 (sept, J = 6.0 Hz, 1H), 5.23 (s, 1H), 6.91 (d, J = 8.8 Hz, 2H), 7.01 (d, J = 8.4 Hz, 1H), 7.12 (dd, J = 8.4,2.0 Hz, 1H), 7.26 (d, J = 2.0 Hz, 1H), 7.68 (d, J = 8.8 Hz, 2H), 7.86-7.93 (m, 4H); Mass spectrum (ESI) *m*/*z:* 515 (M+H)⁺

(Example 32) 4-(1-(3-Ethoxy-4-isopropoxyphenyl)-2-(N'-(2-nitrobenzoyl)hydrazino)-2-oxoethylamino)benzamidine trifluoroacetate 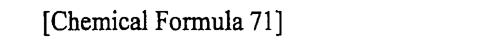 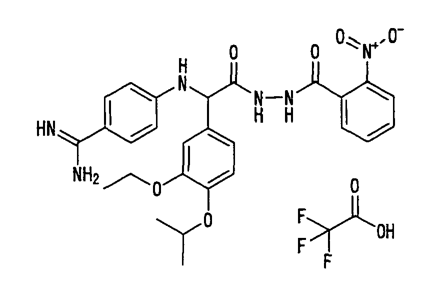 A mixture of 2-nitrobenzoic acid (5.3 mg, 0.0317 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (0.6 ml) was stirred at 0°C for 1 hour. To the reaction mixture was added 4-(((3-ethoxy-4-isopropoxyphenyl)hydrazinocarbonylmethyl)amino)benzamidine dihydrochloride (15 mg, 0.0327 mmol) prepared in Example 4b, followed by stirring overnight at room temperature. The reaction mixture was purified by reversed-phase high performance liquid chromatography to give the title compound as a pale yellow solid (1.95 mg, yield: 9%).
¹H-NMR (400 MHz, CD₃OD) δ: 1.30 (d, J = 6.0 Hz, 6H), 1.40 (t, J = 6.8 Hz, 3H), 4.09 (q, J = 6.8 Hz, 2H), 4.52 (sept, J = 6.0 Hz, 1H), 5.16 (s, 1H), 6.88 (d, J = 8.8 Hz, 2H), 6.96 (d, J = 8.0 Hz, 1H), 7.10 (dd, J = 8.0, 2.0 Hz, 1H), 7.21 (d, J = 2.0 Hz, 1H), 7.63 (d, J = 8.8 Hz, 2H), 7.69-7.81 (m, 3H), 8.12 (dd, J = 8.0, 0.8 Hz, 1H); Mass spectrum (ESI) *m*/*z:* 535 (M+H)⁺

(Example 33) 4-(2-(N'-(3-Chloropyridine-4-carbonyl)hydrazino)-1-(3-ethoxy-4-isopropoxyphenyl)-2-oxoethylamino)benzamidine trifluoroacetate 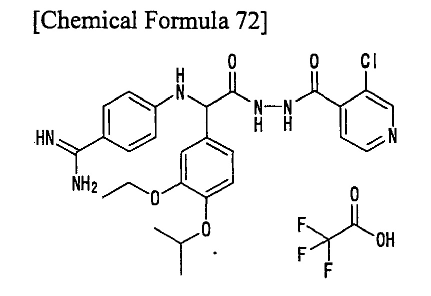 A mixture of 3-chloro-4-pyridinecarboxylic acid (5.0 mg, 0.0317 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (0.6 ml) was stirred at 0°C for 1 hour. To the reaction mixture was added 4-(((3-ethoxy-4-isopropoxyphenyl)hydrazinocarbonylmethyl)amino)benzamidine dihydrochloride (15 mg, 0.0327 mmol) prepared in Example 4b, followed by stirring overnight at room temperature. The reaction mixture was purified by reversed-phase high performance liquid chromatography to give the title compound as a pale yellow solid (3.91 mg, yield: 19%).
¹H-NMR (400 MHz, CD₃OD) δ: 1.30 (d, J = 6.0 Hz, 6H), 1.40 (t, J = 7.2 Hz, 3H), 4.10 (q, J = 7.2 Hz, 2H), 4.53 (sept, J = 6.0 Hz, 1H), 5.14 (s, 1H), 6.88 (d, J = 8.8 Hz, 2H), 6.96 (d, J = 8.4 Hz, 1H), 7.09 (dd, J = 8.4, 2.0 Hz, 1H), 7.21 (d, J = 2.0 Hz, 1H), 7.58 (dd, J = 4.8, 0.8 Hz, 1H), 7.64 (d, J = 8.8 Hz, 2H), 8.57 (d, J = 4.8 Hz, 1H), 8.68 (d, J = 0.8 Hz, 1H); Mass spectrum (ESI) *m*/*z*: 525 (M+H)⁺

(Example 34) 4-(2-(N'-(3-Bromopyridine-4-carbonyl)hydrazino)-1-(3-ethoxy-4-isopropoxyphenyl)-2-oxoethylamino)benzamidine trifluoroacetate 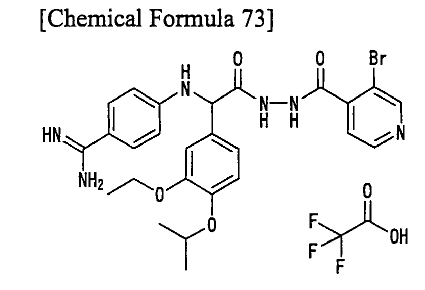 A mixture of 3-bromo-4-pyridinecarboxylic acid (6.5 mg, 0.0321 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (0.6 ml) was stirred at 0°C for 1 hour. To the reaction mixture was added 4-(((3-ethoxy-4-isopropoxyphenyl)hydrazinocarbonylmethyl)amino)benzamidine dihydrochloride (15 mg, 0.0327 mmol) prepared in Example 4b, followed by stirring overnight at room temperature. The reaction mixture was purified by reversed-phase high performance liquid chromatography to give the title compound as a pale yellow solid (4.30 mg, yield: 19%).
¹H-NMR (400 MHz, CD₃OD) δ: 1.30 (d, J = 6.4 Hz, 6H), 1.40 (t, J = 6.8 Hz, 3H), 4.10 (q, J = 6.8 Hz, 2H), 4.53 (sept, J = 6.4 Hz, 1H), 5.15 (s, 1H), 6.88 (d, J = 8.8 Hz, 2H), 6.96 (d, J = 8.4 Hz, 1H), 7.09 (dd, J = 8.4,2.0 Hz, 1H), 7.21 (d, J = 2.0 Hz, 1H), 7.57 (d, J = 4.8 Hz, 1H), 7.64 (d, J = 8.8 Hz, 2H), 8.60 (d, J = 4.8 Hz, 1H), 8.79 (s, 1H); Mass spectrum (ESI) *m*/*z*: 569 (M+H)⁺

(Example 35) 4-(2-(N'-(2-Chloropyridine-3-carbonyl)hydrazino)-1-(3-ethoxy-4-isopropoxyphenyl)-2-oxoethylamino)benzamidine trifluoroacetate 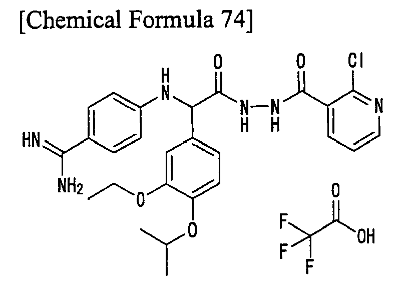 A mixture of 2-chloronicotinic acid (5.0 mg, 0.0317 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (0.6 ml) was stirred at 0°C for 1 hour. To the reaction mixture was added 4-(((3-ethoxy-4-isopropoxyphenyl)hydrazinocarbonylmethyl)amino)benzamidine dihydrochloride (15 mg, 0.0327 mmol) prepared in Example 4b, followed by stirring overnight at room temperature. The reaction mixture was purified by reversed-phase high performance liquid chromatography to give the title compound as a pale yellow solid (1.93 mg, yield: 9%).
¹H-NMR (400 MHz, CD₃OD) δ: 1.30 (d, J = 6.0 Hz, 6H), 1.40 (t, J = 7.2 Hz, 3H), 4.10 (q, J = 7.2 Hz, 2H), 4.53 (sept, J = 6.0 Hz, 1H), 5.14 (s, 1H), 6.88 (d, J = 9.2 Hz, 2H), 6.96 (d, J = 8.4 Hz, 1H), 7.09 (dd, J = 8.4,2.0 Hz, 1H), 7.21 (d, J = 2.0 Hz, 1H), 7.47 (dd, J = 7.6, 4.8 Hz, 1H), 7.64 (d, J = 9.2 Hz, 2H), 8.02 (dd, J = 7.6, 2.0 Hz, 1H), 8.47 (dd, J = 4.8, 2.0 Hz, 1H); Mass spectrum (ESI) *m*/*z:* 525 (M+H)⁺

(Example 36) 4-(1-(3-Ethoxy-4-isopropoxyphenyl)-2-(N'-(3-methylpyridine-2-carbonyl)hydrazino)-2-oxoethylamino)benzamidine trifluoroacetate 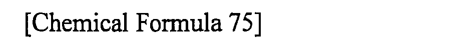 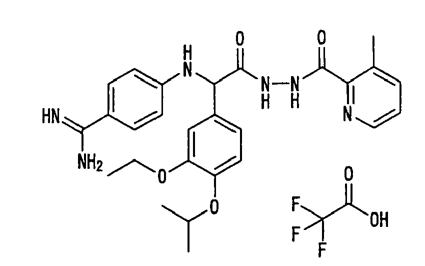 A mixture of 3-methylpicolinic acid (4.4 mg, 0.0320 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (0.6 ml) was stirred at 0°C for 1 hour. To the reaction mixture was added 4-(((3-ethoxy-4-isopropoxyphenyl)hydrazinocarbonylmethyl)amino)benzamidine dihydrochloride (15 mg, 0.0327 mmol) prepared in Example 4b, followed by stirring overnight at room temperature. The reaction mixture was purified by reversed-phase high performance liquid chromatography to give the title compound as a pale yellow solid (5.89 mg, yield: 29%).
¹H-NMR (400 MHz, CD₃OD) δ: 1.30 (d, J = 6.0 Hz, 6H), 1.40 (t, J = 7.2 Hz, 3H), 2.61 (s, 3H), 4.08-4.14 (m, 2H), 4.53 (sept, J = 6.0 Hz, 1H), 5.17 (s, 1H), 6.87 (d, J = 8.8 Hz, 2H), 6.96 (d, J = 8.4 Hz, 1H), 7.11 (dd, J = 8.4, 2.0 Hz, 1H), 7.24 (d, J = 2.0 Hz, 1H), 7.44 (dd, J = 7.6, 4.8 Hz, 1H), 7.63 (d, J = 8.4 Hz, 2H), 7.74 (dd, J = 7.6, 0.8 Hz, 1H), 8.42 (dd, J = 4.8, 0.8 Hz, 1H); Mass spectrum (ESI) *m*/*z*: 505 (M+H)⁺

(Example 37) 4-(2-(N'-(2-Bromobenzoyl)hydrazino)-1-(3-ethoxy-4-isopropoxyphenyl)-2-oxoethylamino)benzamidine trifluoroacetate 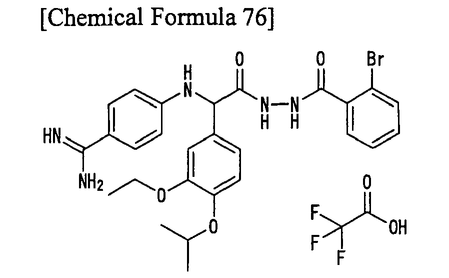 To a mixture of 4-(((3-ethoxy-4-isopropoxyphenyl)hydrazinocarbonylmethyl)amino)benzamidine dihydrochloride (15 mg, 0.0327 mmol) prepared in Example 4b, acetonitrile (1 ml) and dimethylsulfoxide (0.1 ml) were added 2-bromobenzoic acid chloride (10 mg, 0.0456 mmol) and triethylamine (2 drops) in that order, and the mixture was stirred overnight at room temperature. The reaction mixture was purified by reversed-phase high performance liquid chromatography to give the title compound as a pale yellow solid (2.08 mg, yield: 9%).
¹H-NMR (400 MHz, CD₃OD) δ: 1.30 (d, J = 6.0 Hz, 6H), 1.40 (t, J = 6.8 Hz, 3H), 4.10 (q, J = 6.8 Hz, 2H), 4.52 (sept, J = 6.0 Hz, 1H), 5.14 (s, 1H), 6.88 (d, J = 9.2 Hz, 2H), 6.96 (d, J = 8.4 Hz, 1H), 7.09 (dd, J = 8.4,2.0 Hz, 1H), 7.21 (d, J = 2.0 Hz, 1H), 7.37 (ddd, J = 7.6, 7.6, 1.6 Hz, 1H), 7.43 (ddd, J = 7.6, 7.6, 1.2 Hz, 1H), 7.56 (dd, J = 7.6, 1.6 Hz, 1H), 7.63 (d, J = 9.2 Hz, 2H), 7.65 (dd, J = 7.6, 1.2 Hz, 1H); Mass spectrum (ESI) *m*/*z*: 568 (M+H)⁺

(Example 38) N-(4-(N'-(2-(4-Carbamimidoylphenylamino)-2-(3-ethoxy-4-isopropoxyphenyl)acetyl)hydrazinocarbonyl)phenyl)acetamide trifluoroacetate 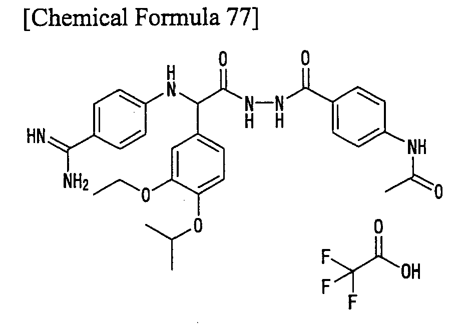 To a mixture of 4-(((3-ethoxy-4-isopropoxyphenyl)hydrazinocarbonylmethyl)amino)benzamidine dihydrochloride (15 mg, 0.0327 mmol) prepared in Example 4b, acetonitrile (1 ml) and dimethylsulfoxide (0.1 ml) were added 4-acetylaminobenzoic acid chloride (10 mg, 0.0506 mmol) and triethylamine (2 drops) in that order, and the mixture was stirred overnight at room temperature. The reaction mixture was purified by reversed-phase high performance liquid chromatography to give the title compound as a pale yellow solid (2.40 mg, yield: 11%).
¹H-NMR (400 MHz, CD₃OD) δ: 1.30 (d, J = 6.0 Hz, 6H), 1.40 (t, J = 6.8 Hz, 3H), 2.14 (s, 3H), 4.10 (q, J = 6.8 Hz, 2H), 4.52 (sept, J = 6.0 Hz, 1H), 5.15 (s, 1H), 6.88 (d, J = 8.8 Hz, 2H), 6.96 (d, J = 8.0 Hz, 1H), 7.11 (dd, J = 8.0, 2.0 Hz, 1H), 7.21 (d, J = 2.0 Hz, 1H), 7.63 (d, J = 8.8 Hz, 2H), 7.67 (d, J = 8.8 Hz, 2H), 7.81 (d, J = 8.8 Hz, 2H); Mass spectrum (ESI) *m*/*z*: 547 (M+H)⁺

(Example 39) 4-(((3-Ethoxy-4-isoprophenyl)-(N'-pydmidin-2-ylhydrazinocarbonyl)methyl)amino)benzamidine trifluoroacetate 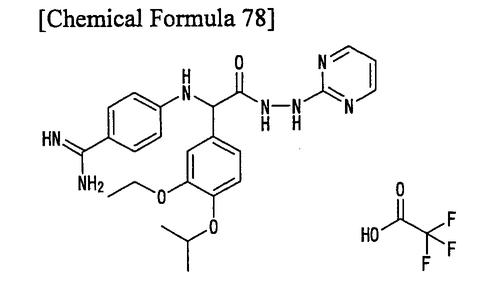 A mixture of (4-carbamimidoylphenylamino)-(3-ethoxy-4-isopropoxyphenyl)acetic acid hydrochloride (20 mg, 0.0490 mmol) prepared in Example 1b, 1-ethyl-3-(3-dimethylaminopropyl)carbodiimide hydrochloride (28 mg, 0.145 mmol), 1-hydroxybenzotriazole monohydrate (23 mg, 0.150 mmol) and N,N-dimethylformamide (1 ml) was stirred at 0°C for 1 hour. To the reaction mixture was added pyrimidin-2-ylhydrazine (16 mg, 0.145 mmol), followed by stirring overnight at room temperature. The reaction mixture was purified by reversed-phase high performance liquid chromatography to give the title compound as a colorless solid (20.15 mg, yield: 71%).
¹H-NMR (400 MHz, CD₃OD) δ: 1.30 (d, J = 6.0 Hz, 6H), 1.40 (t, J = 7.2 Hz, 3H), 4.08 (q, J = 7.2 Hz, 2H), 4.52 (sept, J = 6.0 Hz, 1H), 5.16 (s, 1H), 6.83 (t, J = 4.8 Hz, 1H), 6.87 (d, J = 9.2 Hz, 2H), 6.95 (d, J = 8.0 Hz, 1H), 7.10 (dd, J = 8.0, 2.0 Hz, 1H), 7.20 (d, J = 2.0 Hz, 1H), 7.62 (d, J = 9.2 Hz, 2H), 8.38 (d, J = 4.8 Hz, 2H); Mass spectrum (ESI) *m*/*z*: 464 (M+H)⁺

(Example 40) 4-(1-(3-Ethoxy-4-isopropoxyphenyl)-2-oxo-2-(N'-(2-thiophen-3-ylacetyl)hydrazino)ethylamino)benzamidine trifluoroacetate 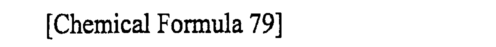 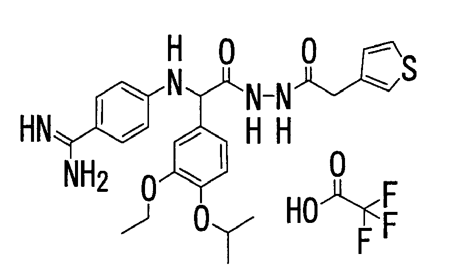 A mixture of (4-carbamimidoylphenylamino)-(3-ethoxy-4-isopropoxyphenyl)acetic acid hydrochloride (20 mg, 0.0490 mmol) prepared in Example 1b, 1-ethyl-3-(3-dimethylaminopropyl)carbodiimide hydrochloride (28.2 mg, 0.147 mmol), 1-hydroxybenzotriazole (22.5 mg, 0.147 mmol) and N,N-dimethylformamide (1 ml) was stirred at 0°C for 1 hour. To the reaction mixture was added thiophen-3-ylacetic acid hydrazide (23 mg, 0.147 mmol), followed by stirring overnight at room temperature. The reaction mixture was purified by reversed-phase high performance liquid chromatography to give the title compound as a colorless solid (18.02 mg, yield: 59%).
¹H-NMR (400 MHz, CD₃OD) δ: 1.29 (d, J = 6.0 Hz, 6H), 1.39 (t, J = 7.2 Hz, 3H), 3.59 (s, 2H), 4.07 (q, J = 7.2 Hz, 2H), 4.51 (sept, J = 6.0 Hz, 1H), 5.07 (s, 1H), 6.83 (d, J = 9.2 Hz, 2H), 6.94 (d, J = 8.0 Hz, 1H), 7.04-7.07 (m, 2H), 7.15 (d, J = 2.0 Hz, 1H), 7.24-7.25 (m, 1H), 7.33 (dd, J = 4.8, 3.2 Hz, 1H), 7.60 (d, J = 9.2 Hz, 2H); Mass spectrum (ESI) *m*/*z:* 510 (M+H)⁺

(Example 41) 4-(1-(3-Ethoxy-4-isopropoxyphenyl)-2-oxo-2-(N'-(2-pyridin-3-ylacetyl)hydrazino)ethylamino)benzamidine trifluoroacetate 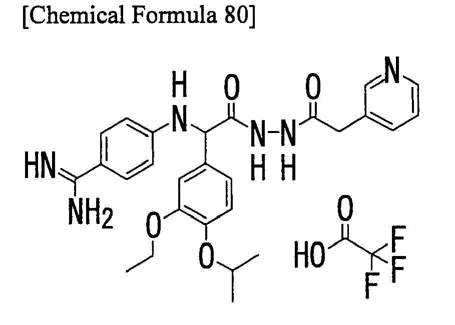 A mixture of (4-carbamimidoylphenylamino)-(3-ethoxy-4-isopropoxyphenyl)acetic acid hydrochloride (20 mg, 0.0490 mmol) prepared in Example 1b, 1-ethyl-3-(3-dimethylaminopropyl)carbodiimide hydrochloride (28.2 mg, 0.147 mmol), 1-hydroxybenzotriazole monohydrate (22.5 mg, 0.147 mmol) and N,N-dimethylformamide (1 ml) was stirred at 0°C for 1 hour. To the reaction mixture was added pyridin-3-ylacetic acid hydrazide (Australian Journal of Chemistry, 1985, 38(10), 1491) (22.2 mg, 0.147 mmol), followed by stirring overnight at room temperature. The reaction mixture was purified by reversed-phase high performance liquid chromatography to give the title compound as a colorless solid (20.20 mg, yield: 67%).
¹H-NMR (400 MHz, CD₃OD) δ: 1.29 (d, J = 6.0 Hz, 6H), 1.38 (t, J = 7.2 Hz, 3H), 3.85 (s; 2H), 4.06 (q, J = 7.2 Hz, 2H), 4.51 (sept, J = 6.0 Hz, 1H), 5.09 (s, 1H), 6.82 (d, J = 9.2 Hz, 2H), 6.93 (d, J = 8.4 Hz, 1H), 7.05 (dd, J = 8.4, 2.0 Hz, 1H), 7.16 (d, J = 2.0 Hz, 1H), 7.61 (d, J = 9.2 Hz, 2H), 7.90-7.94 (m, 1H), 8.43-8.46 (m, 1H), 8.69-8.73 (m, 2H); Mass spectrum (ESI) *m*/*z*: 505 (M+H)⁺

(Example 42) 4-(1-(3-Ethoxy-4-isopropoxyphenyl)-2-oxo-2-(N'-(pyridine-4-carbonyl)hydrazino)ethylamino)benzamidine trifluoroacetate 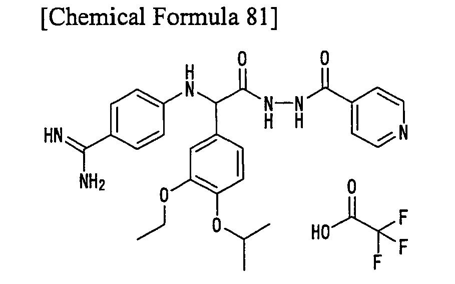 A mixture of (4-carbamimidoylphenylamino)-(3-ethoxy-4-isopropoxyphenyl)acetic acid hydrochloride (20 mg, 0.0490 mmol) prepared in Example 1b, 1-ethyl-3-(3-dimethylaminopropyl)carbodiimide hydrochloride (28.2 mg, 0.147 mmol), 1-hydroxybenzotriazole monohydrate (22.5 mg, 0.147 mmol) and N,N-dimethylformamide (1 ml) was stirred at 0°C for 1 hour. To the reaction mixture was added isonicotinic acid hydrazide (20.2 mg, 0.147 mmol), followed by stirring overnight at room temperature. The reaction mixture was purified by reversed-phase high performance liquid chromatography to give the title compound as a yellow solid (23.44 mg, yield: 79%).
¹H-NMR (400 MHz, CD₃OD) δ: 1.30 (d, J = 6.4 Hz, 6H), 1.41 (t, J = 6.8 Hz, 3H), 4.10 (q, J = 6.8 Hz, 2H), 4.53 (sept, J = 6.4 Hz, 1H), 5.17 (s, 1H), 6.88 (d, J = 8.8 Hz, 2H), 6.97 (d, J = 8.0 Hz, 1H), 7.12 (dd, J = 8.0,2.0 Hz, 1H), 7.23 (d, J = 2.0 Hz, 1H), 7.63 (d, J = 8.8 Hz, 2H), 7.94-7.96 (m, 2H), 8.79 (d, J = 5.2 Hz, 2H); Mass spectrum (ESI) *m*/*z:* 491 (M+H)⁺

(Example 43) 4-(1-(3-Ethoxy-4-isopropoxyphenyl)-2-oxo-2-(N'-(pyridine-3-carbonyl)hydrazino)ethylamino)benzamidine trifluoroacetate 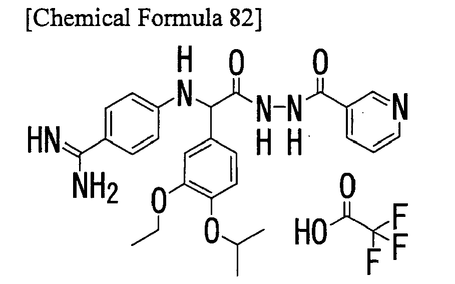 A mixture of (4-carbamimidoylphenylamino)-(3-ethoxy-4-isopropoxyphenyl)acetic acid hydrochloride (20 mg, 0.0490 mmol) prepared in Example 1b, 1-ethyl-3-(3-dimethylaminopropyl)carbodiimide hydrochloride (28.2 mg, 0.147 mmol), 1-hydroxybenzotriazole (22.5 mg, 0.147 mmol) and N,N-dimethylformamide (1 ml) was stirred at 0°C for 1 hour. To the reaction mixture was added nicotinic acid hydrazide (20.2 mg, 0.147 mmol), followed by stirring overnight at room temperature. The reaction mixture was purified by reversed-phase high performance liquid chromatography to give the title compound as a pale yellow solid (21.48 mg, yield: 73%).
¹H-NMR (400 MHz, CD₃OD) δ: 1.30 (d, J = 6.0 Hz, 6H), 1.41 (t, J = 6.8 Hz, 3H), 4.10 (q, J = 6.8 Hz, 2H), 4.53 (sept, J = 6.0 Hz, 1H), 5.17 (s, 1H), 6.88 (d, J = 8.8 Hz, 2H), 6.97 (d, J = 8.4 Hz, 1H), 7.12 (dd, J = 8.4, 2.4 Hz, 1H), 7.23 (d, J = 2.4 Hz, 1H), 7.64 (d, J = 8.8 Hz, 2H), 8.33-8.39 (m, 1H), 8.72-8.79 (m, 1H), 9.03 (brs, 1H);
Mass spectrum (ESI) *m*/*z*: 491 (M+H)⁺

(Example 44) 2-(N'-(2-(4-Carbamimidoylphenylamino)-2-(3-ethoxy-4-isopropoxyphenyl)acetyl)hydrazino)nicotinic acid trifluoroacetate 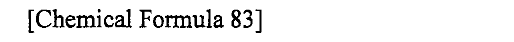 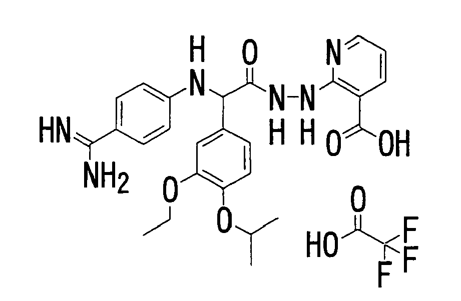 A mixture of (4-carbamimidoylphenylamino)-(3-ethoxy-4-isopropoxyphenyl)acetic acid hydrochloride (20 mg, 0.0490 mmol) prepared in Example 1b, 1-ethyl-3-(3-dimethylaminopropyl)carbodiimide hydrochloride (28.2 mg, 0.147 mmol), 1-hydroxybenzotriazole monohydrate (22.5 mg, 0.147 mmol) and N,N-dimethylformamide (1.25 ml) was stirred at 0°C for 1 hour. To the reaction mixture was added 2-hydrazinonicotinic acid (9 mg, 0.0588 mmol), followed by stirring overnight at room temperature. The reaction mixture was purified by reversed-phase high performance liquid chromatography to give the title compound as a pale yellow solid (3.11 mg, yield: 10%).
¹H-NMR (400 MHz, CD₃OD) δ: 1.30 (d, J = 6.0 Hz, 6H), 1.39 (t, J = 6.8 Hz, 3H), 4.08 (q, J = 6.8 Hz, 2H), 4.53 (sept, J = 6.0 Hz, 1H), 5.24 (s, 1H), 6.87 (d, J = 8.8 Hz, 2H), 6.92 (dd, J = 8.0, 4.8 Hz, 1H), 6.96 (d, J = 8.0 Hz, 1H), 7.12 (dd, J = 8.0, 2.0 Hz, 1H), 7.20 (d, J = 2.0 Hz, 1H), 7.61 (d, J = 8.8 Hz, 2H), 8.26 (dd, J = 4.8, 2.0 Hz, 1H), 8.37 (dd, J = 8.0, 2.0 Hz, 1H); Mass spectrum (ESI) *m*/*z*: 507 (M+H)⁺

(Example 45) 4-(2,2,2-Trifluoro-1-(4-methoxy-3,5-dimethylphenyl)-1-(N'-phenylhydrazinocarbonyl)ethyl)amino)benzamidine trifluoroacetate

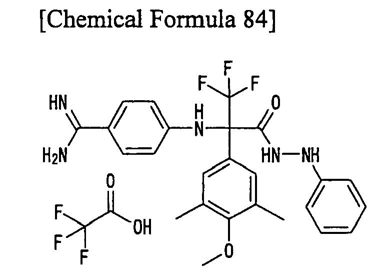

(45a) 3,3,3-Trifluoro-2-hydroxy-2-(4-hydroxy-3,5-dimethylphenyl)propionic acid ethyl ester 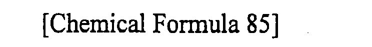 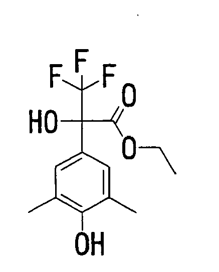 To a solution of 2,6-dimethylphenol (3.96 g, 32.4 mmol) and trifluoropyruvic acid ethyl ester (5.00 g, 29.4 mmol) in carbon tetrachloride (30 ml) was added triethylamine (0.148 g, 1.46 mmol) under a nitrogen atmosphere, followed by stirring at room temperature for 20 hours and concentration under reduced pressure. The obtained residue was purified by silica gel column chromatography (Merck, Ltd., ethyl acetate-heptane) to give the title compound as a white solid (7.96 g, yield: 84%).
¹H-NMR (400 MHz, CDCl₃) δ: 1.39 (t, J = 7.3Hz, 3H), 2.27 (s, 6H), 4.20 (s, 1H), 4.36-4.50 (m, 2H), 4.76 (s, 1H), 7.37 (s, 2H)

(45b) 2-(3,5-Dimethyl-4-oxocyclohexa-2,5-dienylidene)-3,3,3-trifluoropropionic acid ethyl ester 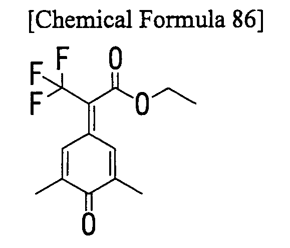 To a solution of 3,3,3-trifluoro-2-hydroxy-2-(4-hydroxy-3,5-dimethylphenyl)propionic acid ethyl ester (2.92 g, 10 mmol) prepared in Example 45a in thionyl chloride (30 ml) was added pyridine (2.37 g, 30 mmol) under a nitrogen atmosphere, followed by stirring for 6 hours. The reaction mixture was then cooled to room temperature and concentrated under reduced pressure. The obtained concentrate was dissolved in a mixed solvent (300 ml) of ethyl acetate:t-butyl methyl ether 1:1 and a 5% sulfuric acid aqueous solution (100 ml), and the organic layer was separated. The organic layer was dried over anhydrous magnesium sulfate. The desiccant was removed by filtration, and the filtrate was concentrated under reduced pressure to give the title compound as a crude product (2.80 g, yield: 102%).
¹H-NMR (400 MHz, CDCl₃) δ: 1.39 (t, J = 7.3 Hz, 3H), 2.26 (s, 6H), 4.33-4.41 (m, 2H), 7.16 (s, 2H)

(45c) 2-(4-Cyanophenylamino)-3,3,3-triffuoro-2-(4-hydroxy-3,5-dimethylphenyl)propionic acid ethyl ester 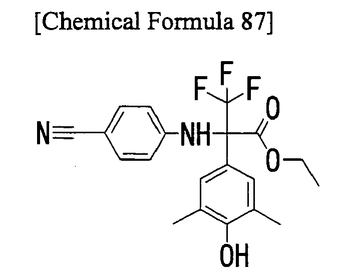 A mixture of 2-(3,5-dimethyl-4-oxocyclohexa-2,5-dienylidene)-3,3,3-trifluoropropionic acid ethyl ester (2.80 g, 10.2 mmol) prepared in Example 45b, 4-aminobenzonitrile (2.41 g, 20.4 mmol) and toluene (20 ml) was stirred under a nitrogen atmosphere at 80°C for 16 hours and then at 100°C for 24 hours. The reaction mixture was then cooled to room temperature, ethyl acetate (800 ml) and 0.5N hydrochloric acid (200 ml) were added, and the organic layer was separated. The organic layer was dried over anhydrous magnesium sulfate. The desiccant was removed by filtration and the filtrate was concentrated under reduced pressure. The obtained residue was purified by silica gel column chromatography (Merck, Ltd., ethyl acetate-heptane) to give the title compound as a colorless sticky solid (2.84 g, yield: 71 %).
¹H-NMR (400 MHz, CDCl₃) δ: 1.18 (t, J = 7.2 Hz, 3H), 2.22 (s, 6H), 4.15-4.23 (m, 1H), 4.29-4.37 (m, 1H), 4.83 (s, 1H), 5.72 (s, 1H), 6.45 (d, J = 8.8 Hz, 2H), 7.07 (s, 2H), 7.27 (d, J = 8.8Hz, 2H)

(45d) 2-(4-Cyanophenylamino)-3,3,3-trifluoro-2-(4-methoxy-3,5-dimethylphenyl)propionic acid ethyl ester 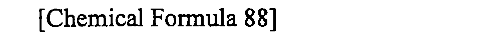 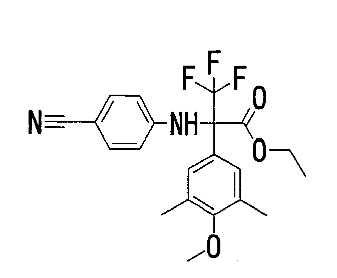 To a solution of 2-(4-cyanophenylamino)-3,3,3-trifluoro-2-(4-hydroxy-3,5-dimethylphenyl)propionic acid ethyl ester (0.786 g, 2.0 mmol) prepared in Example 45c in N,N-dimethylformamide (20 ml) were added potassium carbonate (0.332 g, 2.4 mmol) and methyl iodide (0.426 g, 3.0 mmol) under a nitrogen atmosphere, followed by stirring at room temperature for 18 hours. Next, ethyl acetate (100 ml) and water (100 ml) were added and the organic layer was separated. The organic layer was washed twice with water (50 ml) and dried over anhydrous magnesium sulfate. The desiccant was removed by filtration and the filtrate was concentrated under reduced pressure. The obtained residue was purified by silica gel column chromatography (Merck, Ltd., ethyl acetate-heptane) to give the title compound as a colorless sticky solid (0.748 g, yield: 92%).
¹H-NMR (400 MHz, CDCl₃) δ: 1.18 (t, J = 7.2Hz, 3H), 2.26 (s, 6H), 3.73 (s, 3H), 4.16-4.23 (m, 1H), 4.30-4.38 (m, 1H), 5.71 (s, 1H), 6.45 (d, J = 8.9Hz, 2H), 7.11 (s, 2H), 7.27 (d, J = 8.9Hz, 2H)

(45e) 3,3,3-Trifluoro-2-(4-(N-hydroxycarbamimidoyl)phenylamino)-2-(4-methoxy-3,5-dimethylphenyl)propionic acid ethyl ester 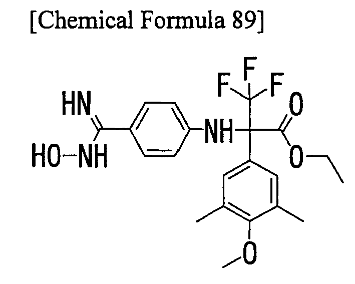 To a solution of 2-(4-cyanophenylamino)-3,3,3-trifluoro-2-(4-methoxy-3,5-dimethylphenyl)propionic acid ethyl ester (0.748 g, 1.84 mmol) prepared in Example 45d in methanol (20 ml) were added hydroxyl ammonium chloride (0.511 g, 7.36 mmol) and triethylamine (1.28 ml, 9.2 mmol) under a nitrogen atmosphere, followed by stirring at 60°C for 18 hours. Next, ethyl acetate (300 ml) and water (100 ml) were added and the organic layer was separated. The organic layer was dried over anhydrous magnesium sulfate. The desiccant was removed by filtration and the filtrate was concentrated under reduced pressure. The obtained residue was purified by silica gel column chromatography (Merck, Ltd., ethyl acetate-heptane) to give the title compound as a white solid (0.671 g, yield: 83%).
¹H-NMR (400 MHz, CDCl₃) δ: 1.18 (t, J = 7.2Hz, 3H), 2.26 (s, 6H), 3.72 (s, 3H), 4.16-4.23 (m, 1H), 4.26-4.34 (m, 1H), 4.71 (s, 2H), 5.35 (s, 1H), 6.47 (d, J = 9.0Hz, 2H), 7.16 (s, 2H), 7.28 (d, J = 9.0Hz, 2H)

(45f) 2-(4-Carbamimidoylphenylamino)-3,3,3-trifluoro-2-(4-methoxy-3,5-dimethylphenyl)propionic acid ethyl ester acetate 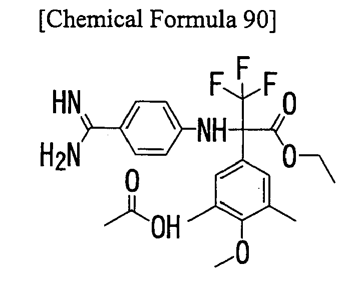 To a solution of 3,3,3-trifluoro-2-(4-(N-hydroxycarbamimidoyl)phenylamino)-2-(4-methoxy-3,5-dimethylphenyl)propionic acid ethyl ester (0.671 g, 1.53 mmol) prepared in Example 45e in acetic acid (20 ml) were added acetic anhydride (3 ml) and palladium-carbon powder (10%, 0.069 g), followed by stirring at room temperature for 13 hours under a hydrogen atmosphere. The catalyst was removed by filtration and the filtrate was concentrated under reduced pressure to give the title compound as a crude product (0.848 g, yield: 112%).
¹H-NMR (400 MHz, CD₃OD) δ: 1.15 (t, J = 7.2Hz, 3H), 2.25 (s, 6H), 3.73 (s, 3H), 4.19-4.35 (m, 2H), 6.70 (d, J = 9.0Hz, 2H), 7.26 (s, 2H), 7.50 (d, J = 9.0Hz, 2H)

(45 g) 2-(4-Carbamimidoylphenylamino)-3,3,3-trifluoro-2-(4-methoxy-3,5-dimethylphenyl)propionic acid trifluoroacetate 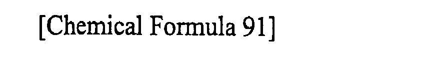 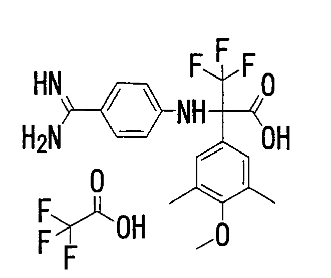 To a solution of 2-(4-carbamimidoylphenylamino)-3,3,3-trifluoro-2-(4-methoxy-3,5-dimethylphenyl)propionic acid ethyl ester acetate (0.109 g, 0.226 mmol) prepared in Example 45f in methanol (4 ml) was added a 5N sodium hydroxide aqueous solution (0.50 ml). After stirring at room temperature for 3 hours, the mixture was purified by reversed-phase high performance liquid chromatography to give the title compound as a white solid (0.058 g, yield: 50%).
¹H-NMR (400 MHz, CD₃OD) δ: 2.24 (s, 6H), 3.73 (s, 3H), 6.77 (d, J = 9.0Hz, 2H), 7.25 (s, 2H), 7.48 (d, J = 9.0Hz, 2H)

(45h) 4-(2,2,2-Trifluoro-1-(4-methoxy-3,5-dimethylphenyl)-1-(N'-phenylhydrazinocarbonyl)ethyl)amino)benzamidine trifluoroacetate 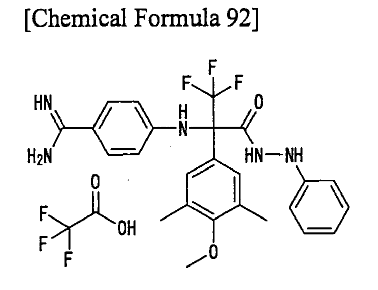 Reaction was carried out in the same manner as Example 1c using 2-(4-carbamimidoylphenylamino)-3,3,3-trifluoro-2-(4-methoxy-3,5-dimethylphenyl)propionic acid trifluoroacetate (58 mg, 0.146 mmol) prepared in Example 45g instead of (4-carbamimidoylphenylamino)-(3-ethoxy-4-isopropoxyphenyl)acetic acid, to give the title compound as a colorless solid (29 mg, yield: 41%).
¹H-NMR (400 MHz, CD₃OD) δ: 2.28 (s, 6H), 3.38 (s, 3H), 6.54 (d, J = 7.9Hz, 2H), 6.75 (d, J = 9Hz, 2H), 6.77 (t, J = 7.9Hz, 1H), 7.08 (d, J = 7.9Hz, 2H), 7.32 (s, 2H), 7.53 (d, J = 9.0Hz, 2H), 8.23 (s, 2H), 8.80 (s, 2H), 9.85 (s, 1H); Mass spectrum (ESI) *m*/*z*: 486 (M+H)⁺

(Example 46) 4-(((4-Hydroxy-3,5-dimethoxyphenyl)-(N'-phenylhydrazinocarbonyl)methyl)amino)benzamidine trifluoroacetate

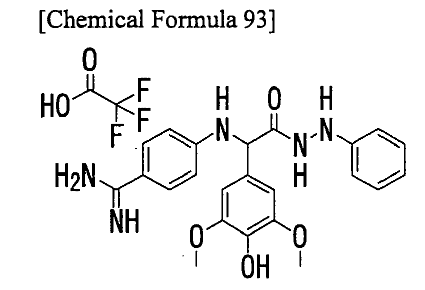

(46a) (4-Carbamimidoylphenylamino)-(4-hydroxy-3,5-dimethoxyphenyl)acetic acid trifluoroacetate 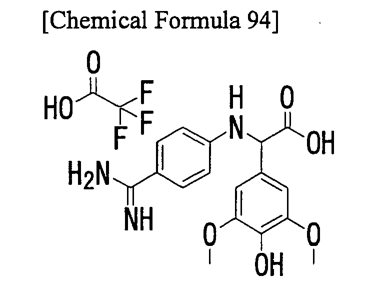 A mixture of 4-(t-butyldimethylsilanyloxy)-3,5-dimethoxybenzaldehyde (100 mg, 0.33 mmol), 4-aminobenzamidine dihydrochloride (50 mg, 0.24 mmol) and methanol (1.5 ml) was stirred at 60°C for 4 hours. The reaction mixture was cooled to room temperature, and then para-toluenesulfonylmethyl isocyanide (50 mg, 0.25 mmol) was added. After then cooling the reaction mixture to 0°C, boron trifluoride/diethyl ether complex (0.1 ml, 0.79 mmol) was added. The reaction mixture was stirred overnight at room temperature, and then heptane was added and the methanol layer was separated. Ethyl acetate and saturated aqueous sodium hydrogencarbonate were added to the obtained methanol layer, extraction was performed with ethyl acetate, and the obtained organic layer was concentrated. The obtained crude product was dissolved in methanol (1.5 ml), and a 5N sodium hydroxide aqueous solution (0.25 ml) was added. After stirring at room temperature for 3 hours, the reaction mixture was neutralized with 5N hydrochloric acid and purified by reversed-phase high performance liquid chromatography to give the title compound.
Mass spectrum (ESI) *m*/*z*: 346 (M+H)⁺

(46b) 4-(((4-Hydroxy-3,5-dimethoxyphenyl)-(N'-phenylhydrazinocarbonyl)methyl)amino)benzamidine trifluoroacetate 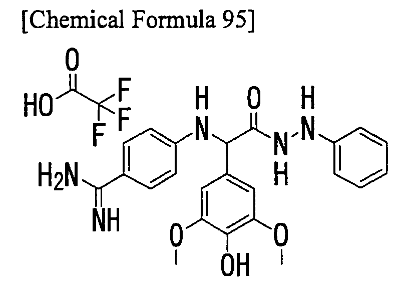 (4-Carbamimidoylphenylamino)-(4-hydroxy-3,5-dimethoxyphenyl)acetic acid trifluoroacetate (8 mg, 0.017 mmol) prepared in Example 46a was dissolved in N,N-dimethylformamide (0.5 ml) and cooled to -5°C. To the reaction mixture were added 1-hydroxybenzotriazole (8 mg, 0.052 mmol), and 1-ethyl-3-(3-dimethylaminopropyl)carbodiimide hydrochloride (10 mg, 0.052 mmol), followed by stirring for 30 minutes and addition of a solution of phenylhydrazine (10 mg, 0.092 mmol) in N,N-dimethylformamide (0.2 ml). The reaction mixture was stirred overnight at room temperature and then directly purified by reversed-phase high performance liquid chromatography to give the title compound (2.36 mg, yield: 25%).
¹H-NMR (400 MHz, CD₃OD) δ: 3.89 (s, 6H), 5.13 (s, 1H), 6.63 (m, J = 7.2 Hz, 2H), 6.72-7.24 (m, 3H), 6.88 (d, J = 8.8 Hz, 2H), 6.92 (s, 2H), 7.68 (d, J = 8.8 Hz, 2H); Mass spectrum (ESI) *m*/*z*: 436 (M+H)⁺

(Example 47) 4-(((3,4-Bisallyloxyphenyl)-(N'-pyridin-2-ylhydrazinocarbonyl)methyl)amino)benzamidine trifluoroacetate

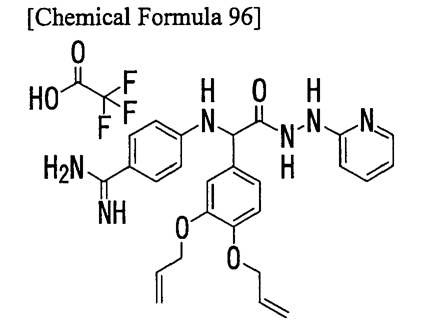

(47a) (3,4-Bisallyloxyphenyl)-(4-carbamimidoylphenylamino)acetic acid trifluoroacetate 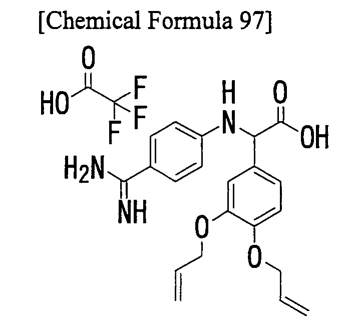 3,4-Dihydroxybenzaldehyde (100 mg, 0.72 mmol) was dissolved in N,N-dimethylformamide (2.0 ml), then allyl bromide (0.157 ml, 1.8 mmol) and potassium carbonate (250 mg, 1.8 mmol) were added thereto. The reaction mixture was stirred overnight at room temperature, 1N hydrochloric acid was added, and extraction was performed with ethyl acetate. The obtained organic layer was concentrated to give 3,4-bisallyloxybenzaldehyde as a crude product. This was dissolved in methanol (1.5 ml), and then 4-aminobenzamidine dihydrochloride (150 mg, 0.72 mmol) was added and the mixture was stirred at 60°C for 5 hours. The reaction mixture was cooled to room temperature, and then para-toluenesulfonylmethyl isocyanide (180 g, 0.92 mmol) was added. After then cooling the reaction mixture to 0°C, boron trifluoride/diethyl ether complex (0.28 ml, 2.2 mmol) was added. The reaction mixture was stirred overnight at room temperature, and then heptane was added and the methanol layer was separated. Ethyl acetate and saturated aqueous sodium hydrogencarbonate were added to the obtained methanol layer, extraction was performed with ethyl acetate, and the obtained organic layer was concentrated. The obtained crude product was dissolved in methanol (1.5 ml), and a 5N sodium hydroxide aqueous solution (0.25 ml) was added. After stirring for 3 hours at room temperature, the reaction mixture was neutralized with 5N hydrochloric acid. It was then purified by reversed-phase high performance liquid chromatography to give the title compound (45 mg, 13%).
Mass spectrum (ESI) *m*/*z:* 382 (M+H)⁺

(47b) 4-(((3,4-Bisallyloxyphenyl)-(N'-pyridin-2-yl-hydrazinocarbonyl)methyl)amino)benzamidine trifluoroacetate 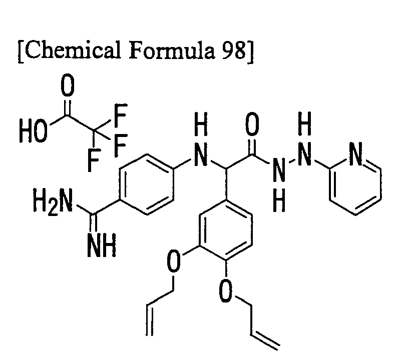 (3,4-Bisallyloxyphenyl)-(4-carbamimidoylphenylamino)acetic acid trifluoroacetate (20 mg, 0.040 mmol) prepared in Example 47a was dissolved in N,N-dimethylformamide (1.0 ml) and cooled to 0°C. To the reaction mixture were added 1-hydroxybenzotriazole monohydrate (20 mg, 0.130 mmol) and 1-ethyl-3-(3-dimethylaminopropyl)carbodiimide hydrochloride (26 mg, 0.135 mmol), followed by stirring at 0°C for 1 hour. A solution of pyridin-2-yl-hydrazine (15 mg, 0.138 mmol) in N,N-dimethylformamide (1 ml) was added, and the mixture was stirred overnight at room temperature and then directly purified by reversed-phase high performance liquid chromatography to give the title compound (19.74 mg, yield: 85%).
¹H-NMR (400 MHz, CD₃OD) δ: 4.61 (m, 4H), 5.24-5.46 (m, 4H), 5.34 (s, 1H), 6.05-6.14 (m, 2H), 6.87 (d, J = 8.8 Hz, 2H), 6.96-7.24 (m, 5H), 7.65 (d, J = 8.8 Hz, 2H), 8.02-8.08 (m, 2H); Mass spectrum (ESI) *m*/*z:* 473 (M+H)⁺

(Example 48) 4-(((3,4-Diethoxyphenyl)-(N'-pyridin-2-ylhydrazinocarbonyl)methyl)amino)benzamidine trifluoroacetate

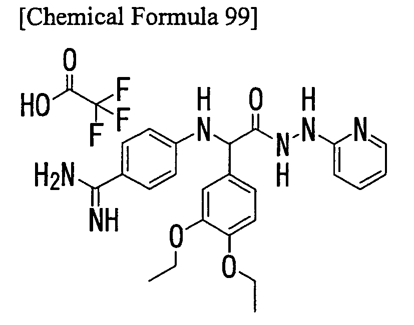

(48a) (4-Carbamimidoylphenylamino)-(3,4-diethoxyphenyl)acetic acid trifluoroacetate 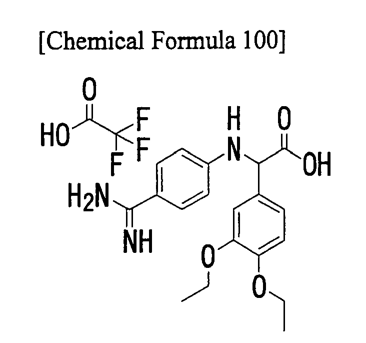 3,4-Dihydroxybenzaldehyde (100 mg, 0.72 mmol) was dissolved in N,N-dimethylformamide (2.0 ml), and ethyl iodide (0.135 ml, 1.8 mmol) and potassium carbonate (250 mg, 1.8 mmol) were added thereto. The reaction mixture was stirred overnight at room temperature, 1N hydrochloric acid was added, and extraction was performed with ethyl acetate. The obtained organic layer was concentrated to give 3,4-diethoxybenzaldehyde as a crude product. This was dissolved in methanol (1.5 ml), and then 4-aminobenzamidine dihydrochloride (150 mg, 0.72 mmol) was added and the mixture was stirred at 60°C for 5 hours. The reaction mixture was cooled to room temperature, and then para-toluenesulfonylmethyl isocyanide (180 mg, 0.92 mmol) was added. After then cooling the reaction mixture to 0°C, boron trifluoride/diethyl ether complex (0.28 ml, 2.2 mmol) was added. The reaction mixture was stirred overnight at room temperature, and then heptane was added and the methanol layer was separated. Ethyl acetate and saturated aqueous sodium hydrogencarbonate were added to the obtained methanol layer, extraction was performed with ethyl acetate, and the obtained organic layer was concentrated. The obtained crude product was dissolved in methanol (1.5 ml), and a 5N sodium hydroxide aqueous solution (0.25 ml) was added. After stirring for 3 hours at room temperature, the reaction mixture was neutralized with 5N hydrochloric acid. It was then purified by reversed-phase high performance liquid chromatography to give the title compound (65 mg, 19%).
Mass spectrum (ESI) *m*/*z*: 358 (M+H)⁺

(48b) 4-(((3,4-Diethoxyphenyl)-(N'-pyridin-2-yl-hydrazinocarbonyl)methyl)amino)benzamidine trifluoroacetate 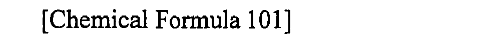 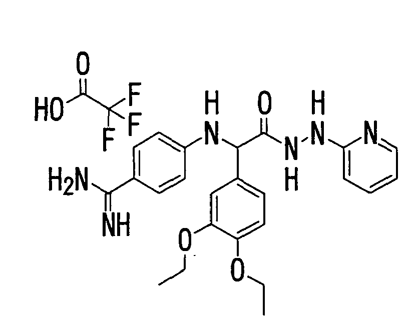 (3,4-Diethoxyphenyl)-(4-carbamimidoylphenylamino)acetic acid trifluoroacetate (30 mg, 0.063 mmol) prepared in Example 48a was dissolved in N,N-dimethylformamide (1.0 ml) and cooled to 0°C. To the reaction mixture were added 1-hydroxybenzotriazole monohydrate (28 mg, 0.183 mmol) and 1-ethyl-3-(3-dimethylaminopropyl)carbodiimide hydrochloride (40 mg, 0.208 mmol), followed by stirring for 1 hour and addition of a solution of pyridin-2-ylhydrazine (23 mg, 0.211 mmol) in N,N-dimethylformamide (1 ml). The reaction mixture was stirred overnight at room temperature and then directly purified by reversed-phase high performance liquid chromatography to give the title compound (19.32 mg, yield: 54%).
¹H-NMR (400 MHz, CD₃OD) δ: 1.43 (m, 6H), 4.10 (m, 4H), 5.34 (s, 1H), 6.87 (d, J = 8.8 Hz, 2H), 6.98-7.22 (m, 5H), 7.65 (d, J = 8.8 Hz, 2H), 8.06 (d, J = 6.8 Hz, 2H); Mass spectrum (ESI) *m*/*z:* 449 (M+H)⁺

(Example 49) 4-(1-(4-(2-Dimethylaminoethoxy)-3-ethoxyphenyl)-2-oxo-2-(N'-(pyridine-2-carbonyl)hydrazino)ethylamino)benzamidine trifluoroacetate

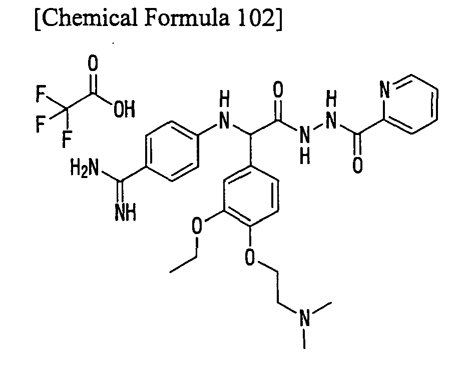

(49a) (4-Carbamimidoylphenylamino)-(4-(2-dimethylaminoethoxy)-3-ethoxyphenyl)acetic acid methyl ester 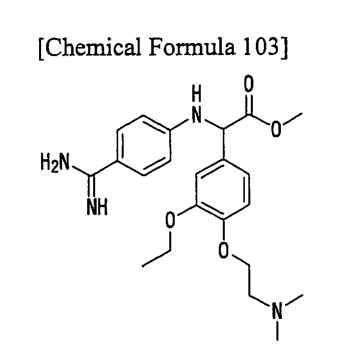 A mixture of 3-ethoxy-4-(2-dimethylaminoethoxy)benzaldehyde [CAS.No.86759-23-1] (3.3 g, 13.906 mmol), 4-aminobenzamidine dihydrochloride (2.9 g, 13.923 mmol) and methanol (50 ml) was stirred at 60°C for 30 minutes. The reaction mixture was cooled to room temperature, and then para-toluenesulfonylmethyl isocyanide (3.3 g, 16.687 mmol) was added. After then cooling the reaction mixture to 0°C, boron trifluoride/diethyl ether complex (5.3 ml, 41.718 mmol) was added. The reaction mixture was stirred at room temperature for 2 days and then filtered, and the filtrate was concentrated. The obtained residue was crudely purified by silica gel column chromatography (NH silica gel (Fuji Silysia Chemical, Ltd.), ethyl acetate-methanol-aqueous ammonia) to give the title compound as a yellow solid (4.76 g, yield: 83%).

(49b) (4-Carbamimidoylphenylamino)-(4-(2-dimethylaminoethoxy)-3-ethoxyphenyl)acetic acid trifluoroacetate 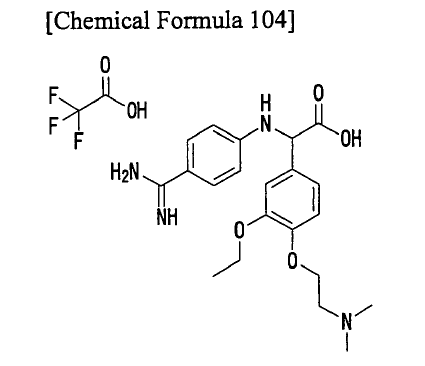 (4-Carbamimidoylphenylamino)-(4-(2-dimethylaminoethoxy)-3-ethoxyphenyl)acetic acid methyl ester (4.76 g, 11.483 mmol) prepared in Example 49a was dissolved in methanol (20 ml), and a 5N sodium hydroxide aqueous solution (2.5 ml) was added. The reaction mixture was stirred overnight at room temperature, 5N hydrochloric acid was added to neutralize the mixture, and then it was concentrated to give a crude product of the title compound (5.25 g). A portion of the crude product was purified by reversed-phase high performance liquid chromatography to give the title compound as a pale brown solid.
¹H-NMR (400 MHz, CD₃OD) δ: 1.42 (t, J = 7.2 Hz, 3H), 3.03 (s, 6H), 3.57 (t, J = 5.2 Hz, 2H), 4.08-4.15 (m, 2H), 4.32 (t, J = 5.2 Hz, 2H), 5.18 (s, 1H), 6.76 (d, J = 9.2 Hz, 2H), 7.03-7.10 (m, 2H), 7.18 (d, J = 1.6 Hz, 1H),7.58 (d, J = 8.8 Hz, 2H)

(49c) 4-(1-(4-(2-Dimethylaminoethoxy)-3-ethoxyphenyl)-2-oxo-2-(N'-(pyridine-2-carbonyl)hydrazino)ethylamino)benzamidine trifluoroacetate 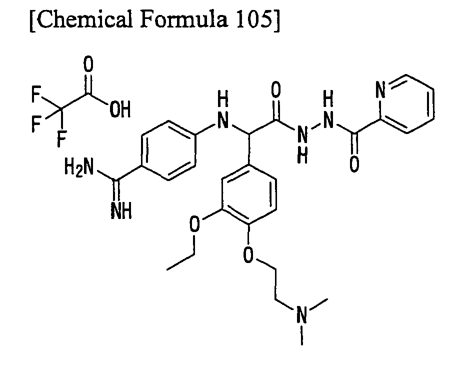 (4-Carbamimidoylphenylamino)-(4-(2-dimethylaminoethoxy)-3-ethoxyphenyl)acetic acid trifluoroacetate (10 mg, 0.017 mmol) prepared in Example 49b was dissolved in N,N-dimethylformamide (1 ml) and cooled to 0°C. To the reaction mixture were added 1-hydroxybenzotriazole monohydrate (10 mg, 0.065 mmol) and 1-ethyl-3-(3-dimethylaminopropyl)carbodiimide hydrochloride (13 mg, 0.067 mmol), followed by stirring for 1 hour and addition of pyridine-2-carboxylic acid hydrazide (10 mg, 0.073 mmol). The reaction mixture was stirred overnight at room temperature and then directly purified by reversed-phase high performance liquid chromatography to give the title compound as a colorless solid (6.13 mg, yield: 57%).
¹H-NMR (400 MHz, CD₃OD) δ: 1.43 (t, J = 6.8 Hz, 3H), 3.04 (s, 6H), 3.58 (t, J = 5.2 Hz, 2H), 4.15-4.23 (m, 2H), 4.33 (t, J = 4.8 Hz, 2H), 5.22 (s, 1H), 6.88 (d, J = 8.8 Hz, 2H), 7.08 (d, J = 8.0 Hz, 1H), 7.17 (dd, J = 1.6, 8.8 Hz, 1H), 7.33 (d, J = 1.6 Hz, 1H), 7.55-7.59 (m, 1H), 7.65 (d, J = 8.8 Hz, 2H), 7.96 (dt, J = 2.0, 8.0 Hz, 1H), 8.07 (dt, J = 0.8, 8.4 Hz, 1H), 8.63 (dt, J = 2.0, 4.8 Hz, 1H); Mass spectrum (ESI) *m*/*z:* 520 (M+H)⁺

(Example 50) 4-(1-(4-(2-Dimethylaminoethoxy)-3-ethoxyphenyl)-2-oxo-2-(N'-(pyridine-3-carbonyl)hydrazino)ethylamino)benzamidine trifluoroacetate 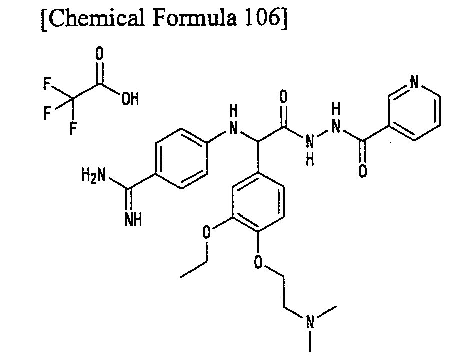 (4-Carbamimidoylphenylamino)-(4-(2-dimethylaminoethoxy)-3-ethoxyphenyl)acetic acid trifluoroacetate (10 mg, 0.017 mmol) prepared in Example 49b was dissolved in N,N-dimethylformamide (1 ml) and cooled to 0°C. To the reaction mixture were added 1-hydroxybenzotriazole monohydrate (10 mg, 0.065 mmol) and 1-ethyl-3-(3-dimethylaminopropyl)carbodiimide hydrochloride (13 mg, 0.067 mmol), followed by stirring for 1 hour and addition of pyridine-3-carboxylic acid hydrazide (10 mg, 0.073 mmol). The reaction mixture was stirred overnight at room temperature and then directly purified by reversed-phase high performance liquid chromatography to give the title compound as a colorless solid (7.55 mg, yield: 70%).
¹H-NMR (400 MHz, CD₃OD) δ: 1.43 (t, J = 7.2 Hz, 3H), 3.04 (s, 6H), 3.59 (t, J = 5.2 Hz, 2H), 4.15-4.21 (m, 2H), 4.34 (t, J = 5.2 Hz, 2H), 5.21 (s, 1H), 6.89 (d, J = 9.2 Hz, 2H), 7.09 (d, J = 8.0 Hz, 1H), 7.17 (dd, J = 1.6, 8.4 Hz, 1H), 7.33 (d, J = 1.6 Hz, 1H), 7.59-7.64 (m, 1H), 7.66 (d, J = 8.8 Hz, 2H), 8.07 (dt, J = 1.2, 8.0 Hz, 1H), 8.74 (dd, J = 1.6, 4.8 Hz, 1H), 9.00 (dd, J = 0.8, 2.0 Hz, 1H); Mass spectrum (ESI) *m*/*z:* 520 (M+H)⁺

(Example 51) 4-(1-(4-(2-Dimethylaminoethoxy)-3-ethoxyphenyl)-2-oxo-2-(N'-(pyridine-4-carbonyl)hydrazino)ethylamino)benzamidine trifluoroacetate 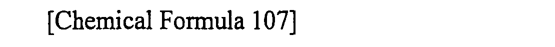 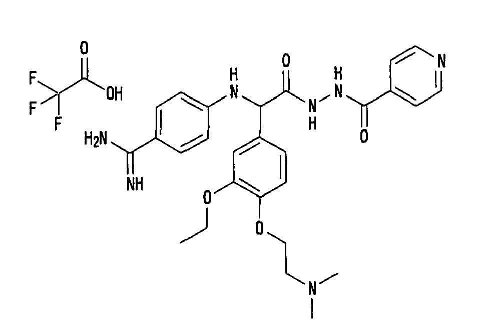 (4-Carbamimidoylphenylamino)-(4-(2-dimethylaminoethoxy)-3-ethoxyphenyl)acetic acid trifluoroacetate (10 mg, 0.017 mmol) prepared in Example 49b was dissolved in N,N-dimethylformamide (1 ml) and cooled to 0°C. To the reaction mixture were added 1-hydroxybenzotriazole monohydrate (10 mg, 0.065 mmol) and 1-ethyl-3-(3-dimethylaminopropyl)carbodiimide hydrochloride (13 mg, 0.067 mmol), followed by stirring for 1 hour and addition of pyridine-4-carboxylic acid hydrazide (10 mg, 0.073 mmol). The reaction mixture was stirred overnight at room temperature and then directly purified by reversed-phase high performance liquid chromatography to give the title compound as a colorless solid (7.91 mg, yield: 74%).
¹H-NMR (400 MHz, CD₃OD) δ: 1.43 (t, J = 7.2 Hz, 3H), 3.04 (s, 6H), 3.59 (t, J = 4.8 Hz, 2H), 4.15-4.21 (m, 2H), 4.33 (t, J = 4.8 Hz, 2H), 5.21 (s, 1H), 6.88 (d, J = 9.2 Hz, 2H), 7.09 (d, J = 8.4 Hz, 1H), 7.17 (dd, J = 1.6, 8.4 Hz, 1H), 7.33 (d, J = 2.0 Hz, 1H), 7.66 (d, J = 8.8 Hz, 2H), 7.85 (d, J = 6.0 Hz, 2H), 8.74 (d, J = 6.0 Hz, 2H); Mass spectrum (ESI) *m*/*z*: 520 (M+H)⁺

(Example 52) 4-(1-(4-(2-Dimethylaminoethoxy)-3-ethoxyphenyl)-2-oxo-2-(N'-(4-methylpyridine-3-carbonyl)hydrazino)ethylamino)benzamidine trifluoroacetate

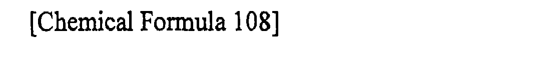

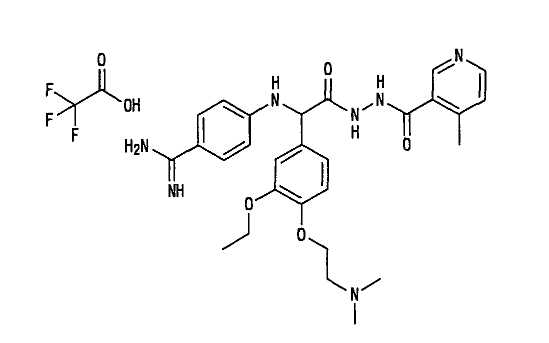

(52a) N'-(2-(4-Carbamimidoylphenylamino)-2-(4-(2-dimethylaminoethoxy)-3-ethoxyphenyl)acetyl)hydrazinecarboxylic acid t-butyl ester 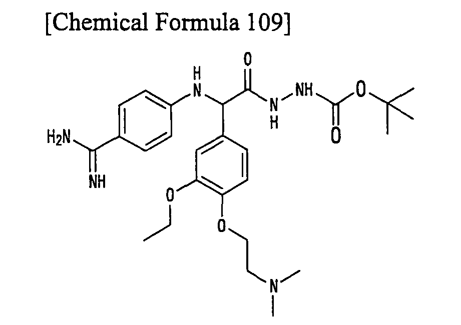 (4-Carbamimidoylphenylamino)-(4-(2-dimethylaminoethoxy)-3-ethoxyphenyl)acetic acid trifluoroacetate (318 mg, 0.506 mmol) prepared in Example 49b was dissolved in N,N-dimethylformamide (2.5 ml) and cooled to 0°C. To the reaction mixture were added 1-hydroxybenzotriazole monohydrate (310 mg, 2.02 mmol) and 1-ethyl-3-(3-dimethylaminopropyl)carbodiimide hydrochloride (390 mg, 2.03 mmol), followed by stirring for 1 hour and addition of hydrazinecarboxylic acid t-butyl ester (270 mg, 2.02 mmol). After stirring the reaction mixture at room temperature for 3 days, it was crudely purified by silica gel column chromatography (NH silica gel (Fuji Silysia Chemical, Ltd.), dichloromethane-methanol) to give the title compound as a yellow solid (370 mg, yield: 142%).

(52b) 4-(((4-(2-Dimethylaminoethoxy)-3-ethoxyphenyl)hydrazinocarbonylmethyl)amino)benzamidine bistrifluoroacetate 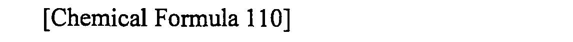 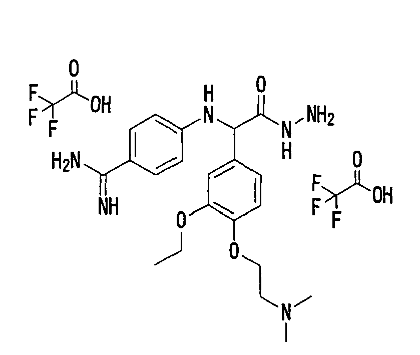 N'-(2-(4-Carbamimidoylphenylamino)-2-(4-(2-dimethylaminoethoxy)-3-ethoxyphenyl)acetyl)hydrazinecarboxylic acid t-butyl ester (370 mg, 0.719 mmol) prepared in Example 52a was dissolved in a 40% solution of hydrochloric acid in ethanol (30 ml). The reaction mixture was stirred at room temperature for 1 hour and then concentrated, and the residue was purified by reversed-phase high performance liquid chromatography to give the title compound as a colorless solid (86 mg, yield: 19%).

(52c) 4-(1-(4-(2-Dimethylaminoethoxy)-3-ethoxyphenyl)-2-oxo-2-(N'-(4-methylpyridine-3-carbonyl)hydrazino)ethylamino)benzamidine trifluoroacetate 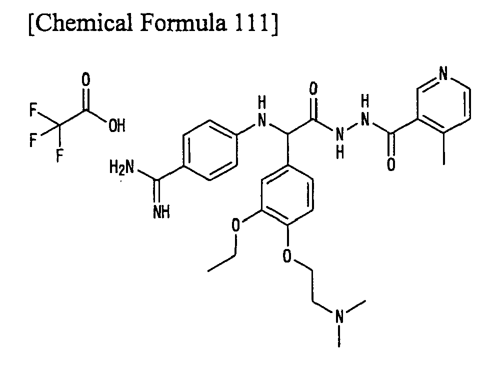 4-Methylnicotinic acid hydrochloride (4 mg, 0.023 mmol) was dissolved in N,N-dimethylformamide (0.25 ml) and cooled to 0°C. To the reaction mixture were added 1-ethyl-3-(3-dimethylaminopropyl)carbodiimide hydrochloride (4 mg, 0.0209 mmol) and 1-hydroxybenzotriazole monohydrate (5 mg, 0.0326 mmol), followed by stirring for 1 hour and addition of a solution of 4-(((4-(2-dimethylaminoethoxy)-3-ethoxyphenyl)hydrazinocarbonylmethyl)amino)benzamidine bistrifluoroacetate (10 mg, 0.0155 mmol) prepared in Example 52b in N,N-dimethylformamide (0.1 ml). The reaction mixture was stirred overnight at room temperature, and then directly purified by reversed-phase high performance liquid chromatography to give the title compound as a colorless solid (3.72 mg, yield: 37%).
¹H-NMR (400 MHz, CD₃OD) δ: 1.43 (t, J = 6.8 Hz, 3H), 2.54 (s, 3H), 3.03 (s, 6H), 3.58 (t, J = 4.8 Hz, 2H), 4.12-4.21 (m, 2H), 4.33 (t, J = 4.8 Hz, 2H), 5.20 (s, 1H), 6.88 (d, J = 8.8 Hz, 2H), 7.09 (d, J = 8.0 Hz, 1H), 7.17 (dd, J = 2.0, 8.4 Hz, 1H), 7.33 (d, J = 2.0 Hz, 1H), 7.49 (d, J = 5.6 Hz, 1H), 7.65 (d, J = 8.8 Hz, 2H), 8.53 (d, J = 5.2 Hz, 1H), 8.65 (s, 1H); Mass spectrum (ESI) *m*/*z:* 534 (M+H)⁺

(Example 53) 4-(1-(4-(2-Dimethylaminoethoxy)-3-ethoxyphenyl)-2-oxo-2-(N'-(3-fluoropyridine-4-carbonyl)hydrazino)ethylamino)benzamidine trifluoroacetate 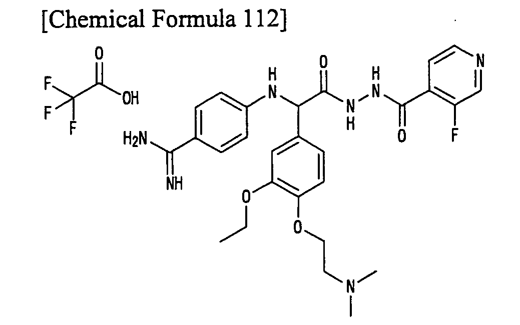 3-Fluoroisonicotinic acid (4 mg, 0.029 mmol) was dissolved in N,N-dimethylformamide (0.25 ml) and cooled to 0°C. To the reaction mixture were added 1-ethyl-3-(3-dimethylaminopropyl)carbodiimide hydrochloride (4 mg, 0.0209 mmol) and 1-hydroxybenzotriazole monohydrate (5 mg, 0.0326 mmol), followed by stirring for 1 hour and addition of a solution of 4-(((4-(2-dimethylaminoethoxy)-3-ethoxyphenyl)hydrazinocarbonylmethyl)amino)benzamidine bistrifluoroacetate (10 mg, 0.0155 mmol) prepared in Example 52b in N,N-dimethylformamide (0.1 ml). The reaction mixture was stirred overnight at room temperature and then directly purified by reversed-phase high performance liquid chromatography to give the title compound as a colorless solid (3.60 mg, yield: 36%).
¹H-NMR (400 MHz, CD₃OD) δ: 1.43 (t, J = 7.2 Hz, 3H), 3.04 (s, 6H), 3.58 (t, J = 4.8 Hz, 2H), 4.15-4.21 (m, 2H), 4.33 (t, J = 5.2 Hz, 2H), 5.19 (s, 1H), 6.88 (d, J = 9.2 Hz, 2H), 7.08 (d, J = 8.4 Hz, 1H), 7.16 (dd, J = 1.6, 8.0 Hz, 1H), 7.32 (d, J = 2.4 Hz, 1H), 7.65 (d, J = 9.2 Hz, 2H), 7.70 (t, J = 5.2 Hz, 1H), 8.53 (dd, J = 1.2, 5.2 Hz, 1H), 8.61 (d, J = 1.6 Hz, 1H); Mass spectrum (ESI) *m*/*z*: 538 (M+H)⁺

(Example 54) 4-(1-(4-(2-Dimethylaminoethoxy)-3-ethoxyphenyl)-2-oxo-2-(N'-(3-methylpyridine-4-carbonyl)hydrazino)ethylamino)benzamidine trifluoroacetate 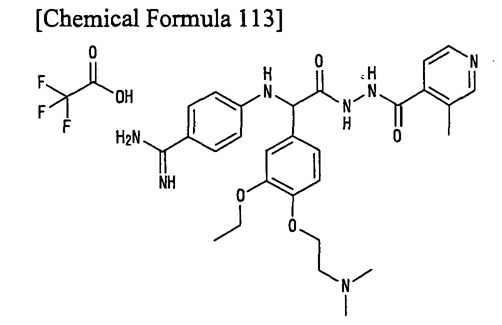 3-Methylisonicotinic acid (4 mg, 0.029 mmol) was dissolved in N,N-dimethylformamide (0.25 ml) and cooled to 0°C. To the reaction mixture were added 1-ethyl-3-(3-dimethylaminopropyl)carbodiimide hydrochloride (4 mg, 0.0209 mmol) and 1-hydroxybenzotriazole monohydrate (5 mg, 0.0326 mmol), followed by stirring for 1 hour and addition of a solution of 4-(((4-(2-dimethylaminoethoxy)-3-ethoxyphenyl)hydrazinocarbonylmethyl)amino)benzamidine bistrifluoroacetate (10 mg, 0.0155 mmol) prepared in Example 52b in N,N-dimethylformamide (0.1 ml). The reaction mixture was stirred overnight at room temperature and then directly purified by reversed-phase high performance liquid chromatography to give the title compound as a colorless solid (3.41 mg, yield: 34%).
¹H-NMR (400 MHz, CD₃OD) δ: 1.43 (t, J = 6.8 Hz, 3H), 2.48 (s, 3H), 3.03 (s, 6H), 3.58 (t, J = 4.8 Hz, 2H), 4.18 (q, J = 6.8 Hz, 2H), 4.33 (t, J = 4.8 Hz, 2H), 5.19 (s, 1H), 6.88 (d, J = 8.8 Hz, 2H), 7.09 (d, J = 8.4 Hz, 1H), 7.17 (dd, J = 2.0, 8.4 Hz, 1H), 7.33 (d, J = 2.4 Hz, 1H), 7.56 (d, J = 5.6 Hz, 1H), 7.65 (d, J = 8.8 Hz, 2H), 8.52 (d, 5.2 Hz, 1H), 8.57 (s, 1H); Mass spectrum (ESI) *m*/*z*: 534 (M+H)⁺

(Example 55) 4-(1-(4-(2-Dimethylaminoethoxy)-3-ethoxyphenyl)-2-oxo-2-(N'-(3-chloropyridine-4-carbonyl)hydrazino)ethylamino)benzamidine trifluoroacetate 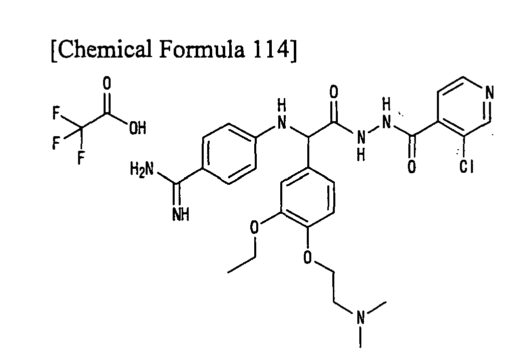 3-Chloroisonicotinic acid (4 mg, 0.025 mmol) was dissolved in N,N-dimethylformamide (0.25 ml) and cooled to 0°C. To the reaction mixture were added 1-ethyl-3-(3-dimethylaminopropyl)carbodiimide hydrochloride (4 mg, 0.0209 mmol) and 1-hydroxybenzotriazole monohydrate (5 mg, 0.0326 mmol), followed by stirring for 1 hour and addition of a solution of 4-(((4-(2-dimethylaminoethoxy)-3-ethoxyphenyl)hydrazinocarbonylmethyl)amino)benzamidine bistrifluoroacetate (10 mg, 0.0155 mmol) prepared in Example 52b in N,N-dimethylformamide (0.1 ml). The reaction mixture was stirred overnight at room temperature and then directly purified by reversed-phase high performance liquid chromatography to give the title compound as a colorless solid (3.22 mg, yield: 32%).
¹H-NMR (400 MHz, CD₃OD) δ: 1.43 (t, J = 6.8 Hz, 3H), 3.03 (s, 6H), 3.58 (t, J = 5.2 Hz, 2H), 4.18 (q, J = 6.8 Hz, 2H), 4.3 (t, J = 4.4 Hz, 2H), 5.19 (s, 1H), 6.88 (d, J = 8.8 Hz, 2H), 7.08 (d, J = 8.0 Hz, 1H), 7.16 (dd, J = 2.0, 8.4 Hz, 1H), 7.32 (d, J = 2.0 Hz, 1H), 7.58 (dd, J = 0.4, 4.4 Hz, 1H), 7.65 (d, J = 9.2 Hz, 2H), 8.56 (d, 4.8 Hz, 1H), 8.68 (s, 1H); Mass spectrum (ESI) *m*/*z:* 554 (M+H)⁺

(Example 56) 4-(((4-(2-Dimethylaminoethoxy)-3-ethoxyphenyl)-(N'-(2-methoxyphenyl)hydrazinocarbonyl)methyl)amino)benzamidine trifluoroacetate 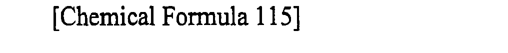 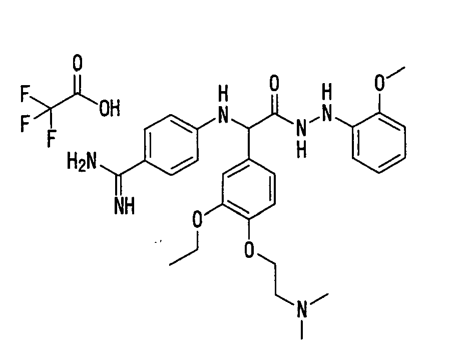 (4-Carbamimidoylphenylamino)-(4-(2-dimethylaminoethoxy)-3-ethoxyphenyl)acetic acid trifluoroacetate (10 mg, 0.017 mmol) prepared in Example 49b was dissolved in N,N-dimethylformamide (0.35 ml) and cooled to 0°C. To the reaction mixture were added 1-hydroxybenzotriazole monohydrate (10 mg, 0.065 mmol) and 1-ethyl-3-(3-dimethylaminopropyl)carbodiimide hydrochloride (13 mg, 0.067 mmol), followed by stirring for 1 hour and addition of 2-methoxyphenylhydrazine hydrochloride (10 mg, 0.057 mmol) and triethylamine (8 µl, 0.057 mmol). The reaction mixture was stirred overnight at room temperature and then directly purified by reversed-phase high performance liquid chromatography to give the title compound as a colorless solid (5.04 mg, yield: 50%).
¹H-NMR (400 MHz, CD₃OD) δ: 1.41 (t, J = 6.8 Hz, 3H), 3.04 (s, 6H), 3.59 (t, J = 4.8 Hz, 2H), 3.83 (s, 3H), 4.12 (q, J = 7.2 Hz, 2H), 4.34 (t, J = 5.2 Hz, 2H), 5.16 (s, 1H), 6.37 (dd, J = 1.6, 8.0 Hz, 1H), 6.61 (dt, J = 1.6, 7.6 Hz, 1H), 6.76 (dq, J = 1.6, 8.4 Hz, 1H), 6.83 (d, J = 9.2 Hz, 2H), 6.82-6.87 (m, 1H), 7.10 (d, J = 8.0 Hz, 1H), 7.16 (dd, J = 2.4, 8.4 Hz, 1H), 7.24 (d, J = 2.0 Hz, 1H), 7.63 (d, J = 9.2 Hz, 2H); Mass spectrum (ESI) *m*/*z:* 521 (M+H)⁺

(Example 57) 4-(((4-(2-Dimethylaminoethoxy)-3-ethoxyphenyl)-(N'-pyridin-2-ylhydrazinocarbonyl)methyl)amino)benzamidine trifluoroacetate 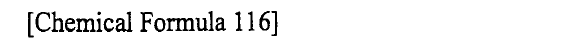 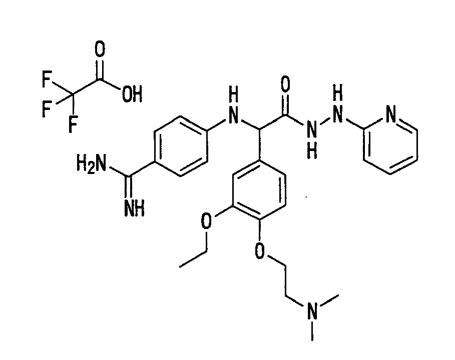 (4-Carbamimidoylphenylamino)-(4-(2-dimethylaminoethoxy)-3-ethoxyphenyl)acetic acid trifluoroacetate (10 mg, 0.017 mmol) prepared in Example 49b was dissolved in N,N-dimethylformamide (0.35 ml) and was cooled to 0°C. To the reaction mixture were added 1-hydroxybenzotriazole monohydrate (10 mg, 0.065 mmol) and 1-ethyl-3-(3-dimethylaminopropyl)carbodiimide hydrochloride (13 mg, 0.067 mmol), followed by stirring for 1 hour and addition of pyridin-2-yl-hydrazine (10 mg, 0.092 mmol). The reaction mixture was stirred overnight at room temperature and then directly purified by reversed-phase high performance liquid chromatography to give the title compound as a colorless solid (2.58 mg, yield: 26%).
¹H-NMR (400 MHz, CD₃OD) δ: 1.41 (t, J = 7.2 Hz, 3H), 3.03 (s, 6H), 3.58 (t, J = 4.8 Hz, 2H), 4.13 (q, J = 7.2 Hz, 2H), 4.33 (t, J = 5.2 Hz, 2H), 5.31 (s, 1H), 6.84 (d, J = 8.0 Hz, 1H), 6.85 (d, J = 8.8 Hz, 2H), 7.00 (t, J = 6.0 Hz, 1H), 7.09 (d, J = 8.4 Hz, 1H), 7.16 (dd, J = 1.6, 8.4 Hz, 1H), 7.25 (d, J = 2.0 Hz, 1H), 7.62 (d, J = 8.8 Hz, 2H), 7.87 (dt, J = 1.6, 6.8 Hz, 1H), 8.03 (d, J = 5.2 Hz, 1H); Mass spectrum (ESI) *m*/*z*: 492 (M+H)⁺

(Example 58) 2-(4-(1-(4-Carbamimidoylphenylamino)-2-(N'-(2-methoxypyridine-3-carbonyl)hydrazino)-2-oxoethyl)-2-ethoxyphenoxy)-N,N-dimethylacetamide trifluoroacetate

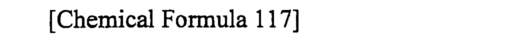

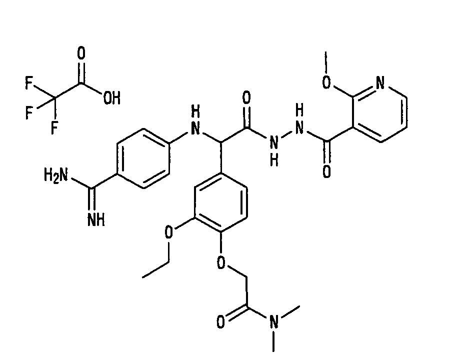

(58a) N'-(2-(4-Carbamimidoylphenylamino)-2-(4-dimethylcarbamoylmethoxy-3-ethoxyphenyl)acetyl)hydrazinocarboxylic acid t-butyl ester 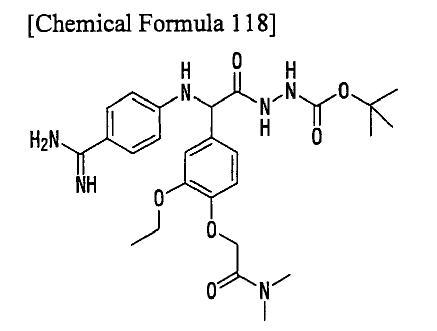 (4-Carbamimidoyl-phenylamino)-(4-dimethylcarbamoylmethoxy-3-ethoxyphenyl)acetic acid hydrochloride (397 mg, 0.885 mmol) prepared in Example 6b was dissolved in N,N-dimethylformamide (3 ml) and cooled to 0°C. To the reaction mixture were added 1-hydroxybenzotriazole monohydrate (406 mg, 2.655 mmol) and 1-ethyl-3-(3-dimethylaminopropyl)carbodiimide hydrochloride (510 mg, 2.655 mmol), followed by stirring for 1 hour and addition of hydrazinecarboxylic acid t-butyl ester (351 mg, 2.655 mmol). After stirring the reaction mixture at room temperature for 3 days, it was crudely purified by silica gel column chromatography (NH silica gel (Fuji Silysia Chemical, Ltd.), dichloromethane-methanol) to give the title compound as a yellow solid (378 mg, yield: 81%).

(58b) 2-(4-((4-Carbamimidoylphenylamino)hydrazinocarbonylmethyl)-2-ethoxyphenoxy)-N,N-dimethylacetamide dihydrochloride 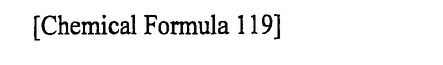 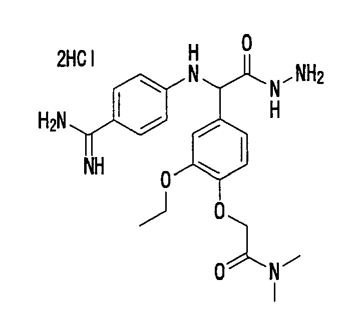 N'-(2-(4-Carbamimidoylphenylamino)-2-(4-dimethylcarbamoylmethoxy-3-ethoxyphenyl)acetyl)hydrazinocarboxylic acid t-butyl ester (378 mg, 0.715 mmol) prepared in Example 58a was dissolved in a mixed solvent of a 4N solution of hydrogen chloride in dioxane (5 ml) and methanol (1 ml). The reaction mixture was stirred at room temperature for 1 hour and then concentrated to give the title compound as a yellow solid (450 mg, yield: 126%).

(58c) 2-(4-(1-(4-Carbamimidoylphenylamino)-2-(N'-(2-methoxypyridine-3-carbonyl)hydrazino)-2-oxoethyl)-2-ethoxyphenoxy)-N,N-dimethylacetamide trifluoroacetate 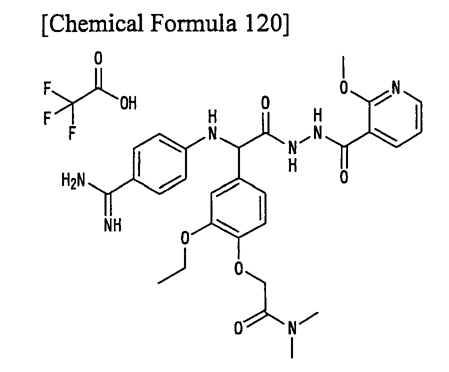 2-Methoxynicotinic acid (6 mg, 0.039 mmol) was dissolved in N,N-dimethylformamide (0.28 ml) and cooled to 0°C. To the reaction mixture were added 1-ethyl-3-(3-dimethylaminopropyl)carbodiimide hydrochloride (4 mg, 0.0209 mmol) and 1-hydroxybenzotriazole monohydrate (5 mg, 0.0326 mmol), followed by stirring for 1 hour and addition of a solution of 2-(4-((4-carbamimidoylphenylamino)hydrazinocarbonylmethyl)-2-ethoxyphenoxy)-N,N-dimethylacetamide dihydrochloride (10 mg, 0.015 mmol) prepared in Example 58b in N,N-dimethylformamide (0.1 ml). The reaction mixture was stirred overnight at room temperature and then directly purified by reversed-phase high performance liquid chromatography to give the title compound as a colorless solid (2.30 mg, yield: 17%).
¹H-NMR (400 MHz, CD₃OD) δ: 1.41 (t, J = 7.2 Hz, 3H), 2.96 (s, 3H), 3.11(s, 3H), 4.05 (s, 3H), 4.10-4.15 (m, 2H), 4.80 (s, 2H), 5.18 (s, 1H), 6.86 (d, J = 9.2 Hz, 2H), 6.93 (d, J = 8.4 Hz, 1H), 7.08-7.14 (m, 2H), 7.23 (d, J = 2.0 Hz, 1H), 7.62 (d, J = 9.2 Hz, 2H), 8.27-8.34 (m, 2H); Mass spectrum (ESI) *m*/*z*: 564 (M+H)⁺

(Example 59) 2-(4-(1-(4-Carbamimidoylphenylamino)-2-(N'-(4-methylpyridine-3-carbonyl)hydrazino)-2-oxoethyl)-2-ethoxyphenoxy)-N,N-dimethylacetamide trifluoroacetate 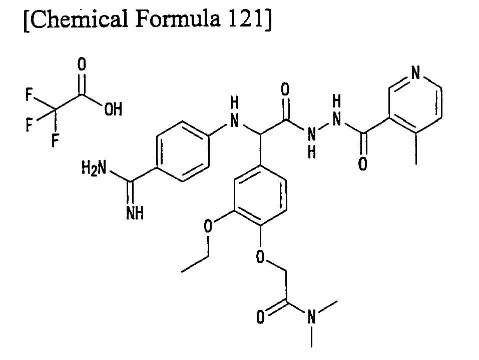 4-Methylnicotinic acid (6 mg, 0.044 mmol) was dissolved in N,N-dimethylformamide (0.28 ml) and cooled to 0°C. To the reaction mixture were added 1-ethyl-3-(3-dimethylaminopropyl)carbodiimide hydrochloride (4 mg, 0.0209 mmol) and 1-hydroxybenzotriazole monohydrate (5 mg, 0.0326 mmol), followed by stirring for 1 hour and addition of a solution of 2-(4-((4-carbamimidoylphenylamino)hydrazinocarbonylmethyl)-2-ethoxyphenoxy)-N,N-dimethylacetamide dihydrochloride (10 mg, 0.015 mmol) prepared in Example 58b in N,N-dimethylformamide (0.1 ml). The reaction mixture was stirred overnight at room temperature and then directly purified by reversed-phase high performance liquid chromatography to give the title compound as a colorless solid (1.38 mg, yield: 10%).
¹H-NMR (400 MHz, CD₃OD) δ: 1.41 (t, J = 7.2 Hz, 3H), 2.55 (s, 3H), 2.96 (s, 3H), 3.11 (s, 3H), 4.13 (q, J = 7.2 Hz, 2H), 4.82 (s, 2H), 5.16 (s, 1H), 6.87 (d, J = 8.8 Hz, 2H), 6.93 (d, J = 8.4 Hz, 1H), 7.09 (dd, J = 2.0, 8.4 Hz, 1H), 7.24 (d, J = 2.4 Hz, 1H), 7.48 (d, J = 5.2 Hz, 1H), 7.63 (d, J = 8.8 Hz, 2H), 8.52 (d, J = 5.2 Hz, 1H), 8.64 (s, 1H); Mass spectrum (ESI) *m*/*z:* 548 (M+H)⁺

(Example 60) 2-(4-(1-(4-Carbamimidoylphenylamino)-2-(N'-(3-fluoropyrridine-4-carbonyl)-hydrazino)-2-oxoethyl)-2-ethoxyphenoxy)-N,N-dimethyl-acetamide trifluoroacetate 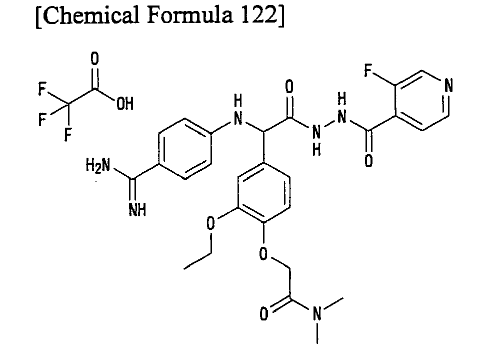 3-Fluoroisonicotinic acid (6 mg, 0.044 mmol) was dissolved in N,N-dimethylformamide (0.28 ml) and cooled to 0°C. To the reaction mixture were added 1-ethyl-3-(3-dimethylaminopropyl)carbodiimide hydrochloride (4 mg, 0.0209 mmol) and 1-hydroxybenzotriazole monohydrate (5 mg, 0.0326 mmol), followed by stirring for 1 hour and addition of a solution of 2-(4-((4-carbamimidoylphenylamino)hydrazinocarbonylmethyl)-2-ethoxyphenoxy)-N,N-dimethylacetamide dihydrochloride (10 mg, 0.015 mmol) prepared in Example 58b in N,N-dimethylformamide (0.1 ml). The reaction mixture was stirred overnight at room temperature and then directly purified by reversed-phase high performance liquid chromatography to give the title compound as a colorless solid (1.20 mg, yield: 9%).
¹H-NMR (400 MHz, CD₃OD) δ: 1.41 (t, J = 6.8 Hz, 3H), 2.96 (s, 3H), 3.11 (s, 3H), 4.13 (q, J = 7.2 Hz, 2H), 4.80 (s, 2H), 5.15 (s, 1H), 6.87 (d, J = 8.8 Hz, 2H), 6.93 (d, J = 8.4 Hz, 1H), 7.09 (d, J = 7.6 Hz, 1H), 7.23 (s, 1H), 7.63 (d, J = 8.8 Hz, 2H), 7.71 (t, J = 5.2 Hz, 1H), 8.52 (d, J = 4.8 Hz, 1H), 8.61 (s, 1H); Mass spectrum (ESI) *m*/*z:* 552 (M+H)⁺

(Example 61) 2-(4-(1-(4-Carbamimidoylphenylamino)-2-(N'-(3-chloropyridine-4-carbonyl)hydrazino)-2-oxoethyl)-2-ethoxyphenoxy)-N,N-dimethylacetamide trifluoroacetate 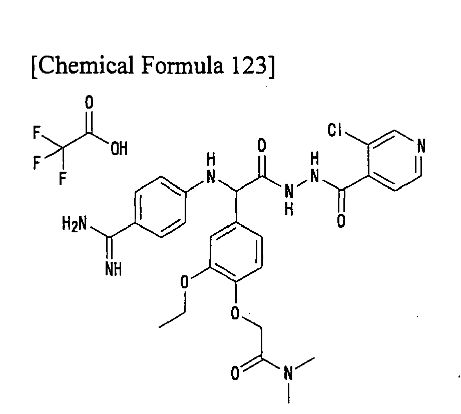 3-Chloroisonicotinic acid (6 mg, 0.038 mmol) was dissolved in N,N-dimethylformamide (0.28 ml) and cooled to 0°C. To the reaction mixture were added 1-ethyl-3-(3-dimethylaminopropyl)carbodiimide hydrochloride (4 mg, 0.0209 mmol) and 1-hydroxybenzotriazole monohydrate (5 mg, 0.0326 mmol), followed by stirring for 1 hour and addition of a solution of 2-(4-((4-carbamimidoylphenylamino)hydrazinocarbonylmethyl)-2-ethoxyphenoxy)-N,N-dimethylacetamide dihydrochloride (10 mg, 0.015 mmol) prepared in Example 58b in N,N-dimethylformamide (0.1 ml). The reaction mixture was stirred overnight at room temperature and then directly purified by reversed-phase high performance liquid chromatography to give the title compound as a colorless solid (1.80 mg, yield: 13%).
¹H-NMR (400 MHz, CD₃OD) δ: 1.41 (t, J = 6.8 Hz, 3H), 2.96 (s, 3H), 3.11 (s, 3H), 4.12 (q, J = 7.6 Hz, 2H), 4.80 (s, 2H), 5.15 (s, 1H), 6.87 (d, J = 8.8 Hz, 2H), 6.93 (d, J = 8.4 Hz, 1H), 7.08 (dd, J = 2.0, 8.4 Hz, 1H), 7.23 (d, J = 2.0 Hz, 1H), 7.58 (d, J = 4.8 Hz, 1H), 7.63 (d, J = 8.8 Hz, 2H), 8.57 (d, J = 5.2 Hz, 1H), 8.68 (s, 1H); Mass spectrum (ESI) *m*/*z:* 568 (M+H)⁺

(Example 62) 2-(4-(1-(4-Carbamimidoylphenylamino)-2-(N'-(3-methylpyridine-2-carbonyl)hydrazino)-2-oxoethyl)-2-ethoxyphenoxy)-N,N-dimethylacetamide trifluoroacetate 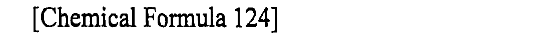 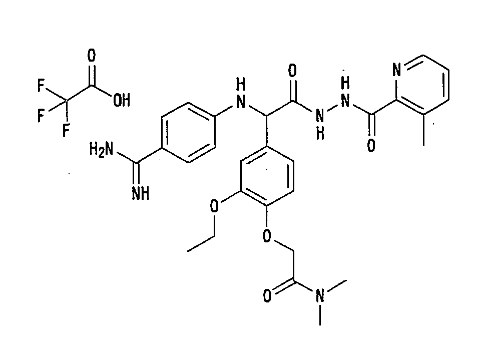 3-Methylpicolinic acid (6 mg, 0.044 mmol) was dissolved in N,N-dimethylformamide (0.28 ml) and cooled to 0°C. To the reaction mixture were added 1-ethyl-3-(3-dimethylaminopropyl)carbodiimide hydrochloride (4 mg, 0.0209 mmol) and 1-hydroxybenzotriazole monohydrate (5 mg, 0.0326 mmol), followed by stirring for 1 hour and addition of a solution of 2-(4-((4-carbamimidoylphenylamino)hydrazinocarbonylmethyl)-2-ethoxyphenoxy)-N,N-dimethylacetamide dihydrochloride (10 mg, 0.015 mmol) prepared in Example 58b in N,N-dimethylformamide (0.1 ml). The reaction mixture was stirred overnight at room temperature and then directly purified by reversed-phase high performance liquid chromatography to give the title compound as a colorless solid (2.12 mg, yield: 16%).
¹H-NMR (400 MHz, CD₃OD) δ: 1.41 (t, J = 7.2 Hz, 3H), 2.60 (s, 3H), 2.96 (s, 3H), 3.11 (s, 3H), 4.14 (dq, J = 2.4, 6.8 Hz, 2H), 4.80 (s, 2H), 5.18 (s, 1H), 6.86 (d, J = 9.2 Hz, 2H), 6.93 (d, J = 8.4 Hz, 1H), 7.08 (dd, J = 2.0, 8.4 Hz, 1H), 7.26 (d, J = 2.0 Hz, 1H), 7.43 (dd, J = 4.8, 8.0 Hz, 1H), 7.63 (d, J = 9.2 Hz, 2H), 7.73 (d, J = 7.6 Hz, 1H), 8.42 (d, J = 4.4 Hz, 1H); Mass spectrum (ESI) *m*/*z*: 548 (M+H)⁺

(Example 63) 2-(4-(1-(4-Carbamimidoylphenylamino)-2-(N'-(3-methylpyridine-4-carbonyl)hydrazino)-2-oxoethyl)-2-ethoxyphenoxy)-N,N-dimethylacetamide trifluoroacetate 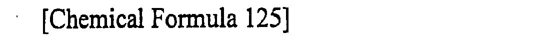 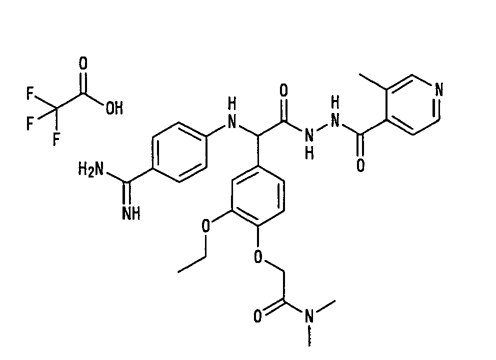 3-Methylisonicotinic acid (6 mg, 0.044 mmol) was dissolved in N,N-dimethylformamide (0.28 ml) and cooled to 0°C. To the reaction mixture were added 1-ethyl-3-(3-dimethylaminopropyl)carbodiimide hydrochloride (4 mg, 0.0209 mmol) and 1-hydroxybenzotriazole monohydrate (5 mg, 0.0326 mmol), followed by stirring for 1 hour and addition of a solution of 2-(4-((4-carbamimidoylphenylamino)hydrazinocarbonylmethyl)-2-ethoxyphenoxy)-N,N-dimethylacetamide dihydrochloride (10 mg, 0.015 mmol) prepared in Example 58b in N,N-dimethylformamide (0.1 ml). The reaction mixture was stirred overnight at room temperature and then directly purified by reversed-phase high performance liquid chromatography to give the title compound as a colorless solid (1.67 mg, yield: 12%).
¹H-NMR (400 MHz, CD₃OD) δ: 1.41 (t, J = 6.8 Hz, 3H), 2.48 (s, 3H), 2.96 (s, 3H), 3.11 (s, 3H), 4.12 (q, J = 7.2 Hz, 2H), 4.80 (s, 2H), 5.16 (s, 1H), 6.87 (d, J = 8.8 Hz, 2H), 6.93 (d, J = 8.4 Hz, 1H), 7.09 (dd, J = 2.0, 8.4 Hz, 1H), 7.24 (d, J = 2.4 Hz, 1H), 7.55 (d, J = 5.2 Hz, 1H), 7.63 (d, J = 8.8 Hz, 2H), 8.51 (d, J = 4.8 Hz, 1H), 8.56 (s, 1H); Mass spectrum (ESI) *m*/*z*: 548 (M+H)⁺

(Example 64) 4-(2-(N'-(3-Chloropyridine-4-carbonyl)hydrazino)-1-(5-ethoxy-2-fluoro-4-isopropoxyphenyl)-2-oxoethylamino)benzamidine trifluoroacetate

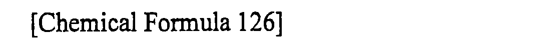

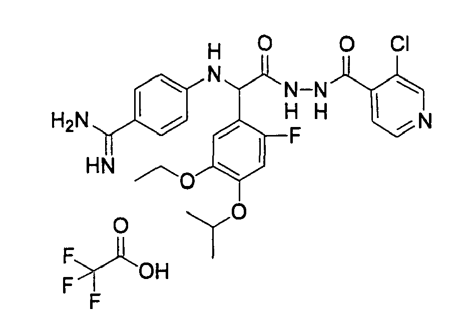

(64a) 5-Ethoxy-2-fluoro-4-isopropoxybenzaldehyde 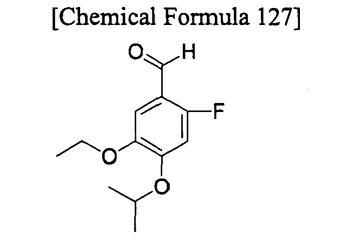 3-Ethoxy-4-isopropoxybenzaldehyde (440 mg, 2.11 mmol) was dissolved in acetonitrile (6.1 ml), SELECTFLUOR^{™} (Air Products and Chemicals, Inc.; 787 mg, 2.22 mmol) was added thereto, and the reaction mixture was stirred at 70°C for 3 hours. The reaction mixture was cooled to room temperature, water was added, and extraction was performed with ethyl acetate. The obtained organic layer was concentrated, and the crude product was purified by silica gel chromatography to give the title compound (88 mg, 18%).
¹H-NMR (400 MHz, CD₃OD) δ: 1.43 (d, J = 6.0 Hz, 6H), 1.45 (t, J = 6.8 Hz, 3H), 4.08 (q, J = 6.8 Hz, 2H), 4.60 (sept, J = 6.0 Hz, 1H), 6.62 (d, J = 12 Hz, 1H), 7.27 (d, J = 8.8 Hz, 1H), 10.19 (s, 1H)

(64b) 4-(((5-Ethoxy-2-fluoro-4-isopropoxyphenyl)hydrazinocarbonylmethyl)amino)benzamidine dihydrochloride 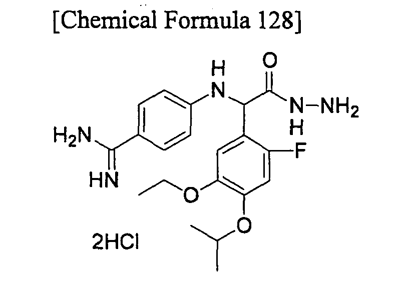 5-Ethoxy-2-fluoro-4-isopropoxybenzaldehyde (1.34 g, 5.92 mmol) prepared in Example 64a was dissolved in methanol (50 ml), 4-aminobenzamidine dihydrochloride (1.54 g, 7.4 mmol) was added thereto, and the mixture was stirred at 80°C for 6 hours. The reaction mixture was cooled to room temperature, and then para-toluenesulfonylmethyl isocyanide (1.44 g, 7.4 mmol) was added. After then cooling the reaction mixture to 0°C, boron trifluoride/diethyl ether complex (2.03 ml, 17.8 mmol) was added. The reaction mixture was stirred overnight at room temperature, and then saturated aqueous sodium hydrogencarbonate was added. Heptane was added, and the methanol layer was separated. Ethyl acetate and water were added to the obtained methanol layer, and extraction was performed twice with ethyl acetate. The aqueous layer was concentrated, and ethyl acetate was added thereto for re-extraction. The combined organic layer was dried over magnesium sulfate and, after removal of the desiccant by filtration, was concentrated. The obtained residue was crudely purified by column chromatography (NH silica gel (Fuji Silysia Chemical, Ltd.), chloroform-methanol). The obtained crude product was dissolved in methanol (5 ml), and a 1N sodium hydroxide aqueous solution (3 ml) was added. After stirring for 16 hours at room temperature, the reaction mixture was neutralized with 1N hydrochloric acid. Diethyl ether was added thereto, and the precipitated solid was collected by filtration. The obtained solid was dried under aeration and used without purification for the following reaction (291 mg).
The obtained crude product (200 mg of the 291 mg) was dissolved in N,N-dimethylformamide (2 ml) and cooled to 0°C. To the reaction mixture were added 1-hydroxybenzotriazole monohydrate (208 mg, 1.54 mmol) and 1-ethyl-3-(3-dimethylaminopropyl)carbodiimide hydrochloride (300 mg, 1.56 mmol), followed by stirring for 30 minutes and addition of hydrazinecarboxylic acid t-butyl ester (203 mg, 1.54 mmol). The reaction mixture was stirred overnight at room temperature and then crudely purified by column chromatography (NH silica gel (Fuji Silysia Chemical, Ltd.), chloroform-methanol).
The obtained crude product was dissolved in methanol (3 ml), and a 4N solution of hydrogen chloride in dioxane (1 ml) was added dropwise at room temperature. After stirring at the same temperature for 5 hours, the reaction mixture was concentrated under reduced pressure. Tetrahydrofuran was added to the residue and the mixture was triturated by sonication. The mixture was allowed to stand for a while, and the supernatant was removed. The residual solvent was distilled off under reduced pressure to give the title compound as a yellow solid (178 mg).
Mass spectrum (ESI) *m*/*z:* 404 (M+H)⁺

(64c) 4-(2-(N'-(3-Chloropyridine-4-carbonyl)hydrazino)-1-(5-ethoxy-2-fluoro-4-isopropoxyphenyl)-2-oxoethylamino)benzamidine trifluoroacetate 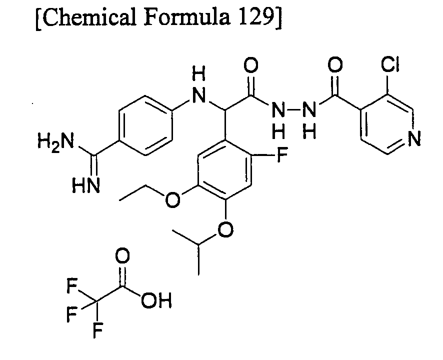 A mixture of 3-chloroisonicotinic acid (5.0 mg, 0.0314 mmol), 1-ethyl-3-(3-dimethylaminopropyl)carbodiimide hydrochloride (6.6 mg, 0.0346 mmol), 1-hydroxybenzotriazole monohydrate (5.3 mg, 0.0346 mmol) and N,N-dimethylformamide (0.8 ml) was stirred at 0°C for 30 minutes. To the reaction mixture was added 4-(((5-ethoxy-2-fluoro-4-isopropoxyphenyl)hydrazinocarbonylmethyl)amino)benzamidine dihydrochloride (15 mg, 0.0314 mmol) prepared in Example 64b, followed by stirring overnight at room temperature. The reaction mixture was purified by reversed-phase high performance liquid chromatography to give the title compound (2.55 mg, yield: 15%).
¹H-NMR (400 MHz, CD₃OD) δ: 1.21 (d, J = 6.4 Hz, 6H), 1.35 (t, J = 6.8 Hz, 3H), 4.01-4.08 (m, 2H), 4.57 (sept, J = 6.4 Hz, 1H), 5.46 (s, 1H), 6.84 (d, J = 11.6 Hz, 1H), 6.87-6.90 (m, 2H), 7.20 (d, J = 7.2 Hz, 1H), 7.59 (d, J = 5.2 Hz, 1H), 7.64-7.67 (m, 2H), 8.58 (d, J = 5.2 Hz, 1H), 8.69 (s, 1H); Mass spectrum (ESI) *m*/*z:* 543 (M+H)⁺

(Example 65) 2-(4-(1-(4-Carbamimidoylphenylamino)-2-(N'-(2-fluorobenzoyl)hydrazino)-2-oxoethyl)-2-ethoxyphenoxy)-N,N-dimethylacetamide trifluoroacetate 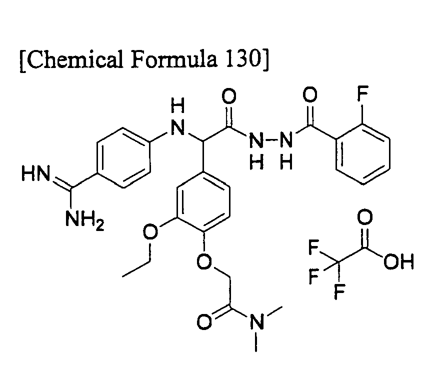 A mixture of 2-fluorobenzoic acid (2.6 mg, 0.0186 mmol), 1-ethyl-3-(3-dimethylaminopropyl)carbodiimide hydrochloride (3.5 mg, 0.0183 mmol), 1-hydroxybenzotriazole monohydrate (2.8 mg, 0.0183 mmol) and N,N-dimethylformamide (0.8 ml) was stirred at 0°C for 1 hour. To the reaction mixture was added 2-(4-((4-carbamimidoylphenylamino)hydrazinocarbonylmethyl)-2-ethoxyphenoxy)-N,N-dimethylacetamide dihydrochloride (10 mg, 0.0199 mmol) prepared in Example 58b, followed by stirring overnight at room temperature. The reaction mixture was purified by reversed-phase high performance liquid chromatography to give the title compound as a colorless solid (3.05 mg, yield: 23%).
¹H-NMR (400 MHz, CD₃OD) δ: 1.41 (t, J = 7.2 Hz, 3H), 2.96 (s, 3H), 3.30 (s, 3H), 4.12 (q, J = 7.2 Hz, 2H), 4.80 (s, 2H), 5.16 (s, 1H), 6.87 (d, J = 9.2 Hz, 2H), 6.93 (d, J = 8.4 Hz, 1H), 7.09 (dd, J = 8.4, 2.0 Hz, 1H), 7.19-7.24 (m, 2H), 7.28 (td, J = 7.6, 1.2 Hz, 1H), 7.53-7.59 (m, 1H), 7.63 (d, J = 9.2 Hz, 2H), 7.76 (td, J = 7.6, 2.0 Hz, 1H); Mass spectrum (ESI) *m*/*z:* 551 (M+H)⁺

(Example 66) 4-(2-(N'-(2-Chlorobenzoyl)hydrazino)-2-oxo-1-(3,4,5-trimethoxyphenyl)ethylamino)benzamidine trifluoroacetate

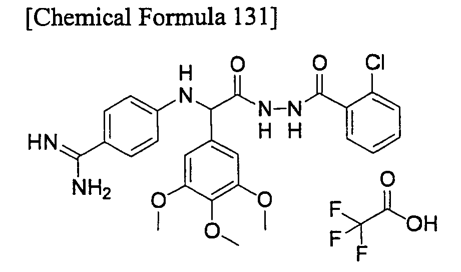

(66a) (4-Carbamimidoylphenylamino)-(3,4,5-trimethoxyphenyl)acetic acid methyl ester 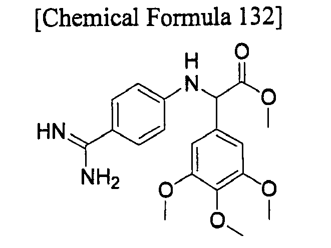 A mixture of 3,4,5-trimethoxybenzaldehyde (3.0 g, 15.3 mmol), 4-aminobenzamidine dihydrochloride (3.34 g, 16.1 mmol) and methanol (50 ml) was stirred at 70°C for 2 hours. The reaction mixture was cooled to room temperature, and then para-toluenesulfonylmethyl isocyanide (3.73 g, 19.1 mmol) was added. After then cooling the reaction mixture to 0°C, boron trifluoride/diethyl ether complex (5.81 ml, 45.9 mmol) was added. The reaction mixture was stirred overnight at room temperature, and then heptane was added and the methanol layer was separated. Saturated aqueous sodium hydrogencarbonate was added to the obtained methanol layer, and extraction was performed with ethyl acetate. The separated organic layer was washed with water and brine in that order and dried over anhydrous sodium sulfate. The desiccant was removed by filtration and the filtrate was concentrated under reduced pressure. The obtained residue was crudely purified by silica gel column chromatography (NH silica gel (Fuji Silysia Chemical, Ltd.), ethyl acetate-chloroform-methanol) to obtain the title compound as a yellow solid (874 mg).

(66b) (4-Carbamimidoylphenylamino)-(3,4,5-trimethoxyphenyl)acetic acid hydrochloride 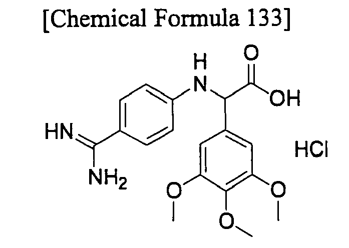 The crude product of (4-carbamimidoylphenylamino)-(3,4,5-trimethoxyphenyl)acetic acid methyl ester prepared in Example 66a (871 mg) was dissolved in methanol (10 ml), and a 2N sodium hydroxide aqueous solution (1.28 ml, 2.56 mmol) was added. The reaction mixture was stirred overnight at room temperature and then neutralized with 5N hydrochloric acid. Upon adding diethyl ether and tetrahydrofuran to the mixture, the precipitated solid was collected by filtration. The obtained solid was suspended in tetrahydrofuran and 5N hydrochloric acid was added. It was then concentrated under reduced pressure to give the title compound as a pale yellow solid (552 mg, 2-stage yield: 9.1%).
¹H-NMR (400 MHz, CD₃OD) δ: 3.75 (s, 3H), 3.83 (s, 6H), 5.17 (s, 1H), 6.78 (d, J = 8.8 Hz, 2H), 6.84 (s, 2H), 7.59 (d, J = 8.8 Hz, 2H)

(66c) 4-(2-(N'-(2-Chlorobenzoyl)hydrazino)-2-oxo-1-(3,4,5-trimethoxyphenyl)ethylamino)benzamidine trifluoroacetate 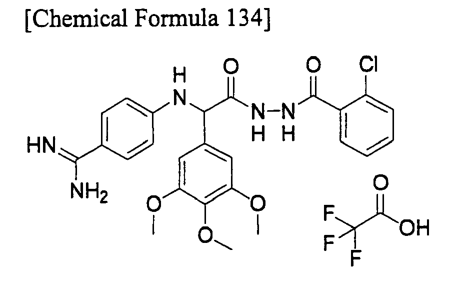 A mixture of (4-carbamimidoylphenylamino)-(3,4,5-trimethoxyphenyl)acetic acid hydrochloride (10 mg, 0.0253 mmol) prepared in Example 66b, 1-ethyl-3-(3-dimethylaminopropyl)carbodiimide hydrochloride (14.5 mg, 0.0758 mmol), 1-hydroxybenzotriazole monohydrate (11.6 mg, 0.0758 mmol) and N,N-dimethylformamide (1 ml) was stirred at 0°C for 1 hour. To the reaction mixture was added 2-chlorobenzoic acid hydrazide (12.9 mg, 0.0756 mmol), followed by stirring overnight at room temperature. The reaction mixture was purified by reversed-phase high performance liquid chromatography to give the title compound as a colorless solid (10.49 mg, yield: 66%).
¹H-NMR (400 MHz, CD₃OD) δ: 3.74 (s, 3H), 3.86 (s, 6H), 5.17 (s, 1H), 6.89 (d, J = 8.8 Hz, 2H), 6.94 (s, 2H), 7.36-7.60(m,4H), 7.64 (d, J = 8.8 Hz, 2H); Mass spectrum (ESI) *m*/*z*: 512 (M+H)⁺

(Example 67) 4-(2-(N'-(2-Chlorobenzoyl)hydrazino)-1-(3-ethoxy-4-(2-methoxyethoxy)phenyl)-2-oxoethylamino)benzamidine trifluoroacetate

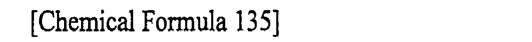

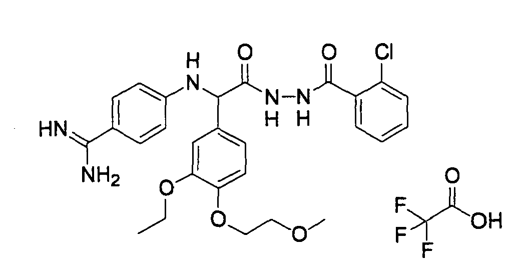

(67a) 3-Ethoxy-4-(2-methoxyethoxy)benzaldehyde 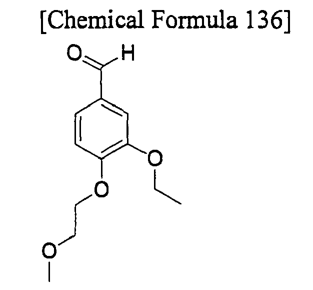 A mixture of 3-ethoxy-4-hydroxybenzaldehyde (5.32 g, 32 mmol), 2-bromoethyl methyl ether (1.5 ml, 16 mmol), potassium carbonate (4.42 g, 32 mmol), tetrabutylammonium iodide (118 mg, 0.32 mmol) and N,N-dimethylformamide (50 ml) was stirred overnight at room temperature. Water was added to the reaction mixture and extraction was performed with ethyl acetate. The separated organic layer was washed with a 0.5N sodium hydroxide aqueous solution, water and brine in that order and dried over anhydrous sodium sulfate. The desiccant was removed by filtration and the filtrate was concentrated under reduced pressure. The obtained residue was purified by silica gel column chromatography (ethyl acetate-heptane) to give the title compound as a white solid (2.79 g, yield: 78%).
¹H-NMR (400 MHz, CD₃OD) δ: 1.47 (t, J = 7.2 Hz, 3H), 3.47 (s, 3H), 3.80-3.84 (m, 2H), 4.14 (q, J = 7.2 Hz, 2H), 4.23-4.26 (m, 2H), 6.99 (d, J = 8.0 Hz, 1H), 7.38-7.42 (m,2H), 9.82 (s, 1H)

(67b) (4-Carbamimidoylphenylamino)-(3-ethoxy-4-(2-methoxyethoxy)phenyl)acetic acid methyl ester 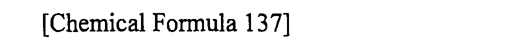 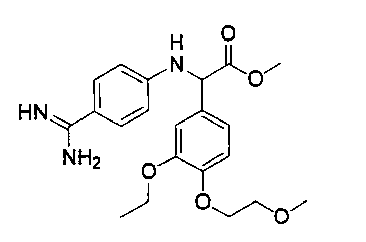 A mixture of 3-ethoxy-4-(2-methoxyethoxy)benzaldehyde (2.79 g, 12.4 mmol) prepared in Example 67a, 4-aminobenzamidine dihydrochloride (2.71 g, 13 mmol) and methanol (50 ml) was stirred at 70°C for 3 hours. The reaction mixture was cooled to room temperature, and then para-toluenesulfonylmethyl isocyanide (3.03 g, 15.5 mmol) was added. After then cooling the reaction mixture to 0°C, boron trifluoride/diethyl ether complex (4.71 ml, 37.2 mmol) was added. The reaction mixture was stirred overnight at room temperature, and then heptane was added and the methanol layer was separated. Saturated aqueous sodium hydrogencarbonate was added to the obtained methanol layer, and extraction was performed with ethyl acetate. The separated organic layer was washed with water and brine in that order and dried over anhydrous sodium sulfate. The desiccant was removed by filtration and the filtrate was concentrated under reduced pressure. The obtained residue was crudely purified by silica gel column chromatography (NH silica gel (Fuji Silysia Chemical, Ltd.), ethyl acetate-chloroform-methanol) to give the title compound as a pale yellow solid (1.396 g).

(67c) Sodium (4-carbamimidoylphenylamino)-(3-ethoxy-4-(2-methoxyethoxy)phenyl)acetate 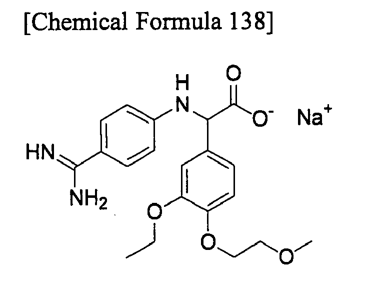 (4-Carbamimidoylphenylamino)-(3-ethoxy-4-(2-methoxyethoxy)phenyl)acetic acid methyl ester (1.396 g) prepared in Example 67b was dissolved in methanol (10 ml), and a 2N sodium hydroxide aqueous solution (1.74 ml, 3.48 mmol) was added. The reaction mixture was stirred overnight at room temperature, and then the precipitated solid was collected by filtration. The obtained solid was washed with methanol to give the title compound as a colorless solid (936 mg, two-step yield: 18%).
Mass spectrum (ESI) *m*/*z*: 388 (M+H)⁺

(67d) 4-(2-(N'-(2-Chlorobenzoyl)hydrazino)-1-(3-ethoxy-4-(2-methoxyethoxy)phenyl)-2-oxoethylamino)benzamidine trifluoroacetate 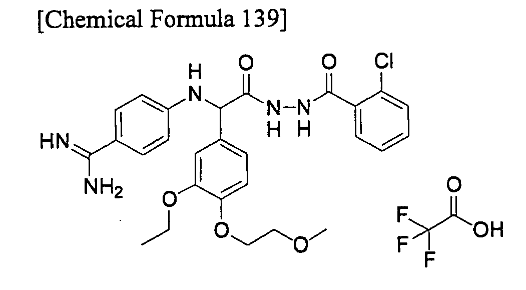 A mixture of sodium (4-carbamimidoylphenylamino)-(3-ethoxy-4-(2-methoxyethoxy)phenyl)acetate (10 mg, 0.0244 mmol) prepared in Example 67c, 1-ethyl-3-(3-dimethylaminopropyl)carbodiimide hydrochloride (14.1 mg, 0.0737 mmol), 1-hydroxybenzotriazole monohydrate (11.3 mg, 0.0737 mmol) and N,N-dimethylformamide (1 ml) was stirred at 0°C for 1 hour. To the reaction mixture was added 2-chlorobenzoic acid hydrazide (12.6 mg, 0.0737 mmol), followed by stirring overnight at room temperature. The reaction mixture was purified by reversed-phase high performance liquid chromatography to give the title compound as a colorless solid (10.76 mg, yield: 67%).
¹H-NMR (400 MHz, CD₃OD) δ: 1.38 (t, J = 7.2 Hz, 3H), 3.41 (s, 3H), 3.71-3.75 (m, 2H), 4.05-4.14 (m, 4H), 5.15 (s, 1H), 6.88 (d, J = 8.8 Hz, 2H), 6.98 (d, J = 8.8 Hz, 1H), 7.11 (dd, J = 8.8, 2.4 Hz, 1H), 7.21 (d, J = 2.4 Hz, 1H), 7.36-7.60 (m, 4H), 7.63 (d, J = 8.8 Hz, 2H); Mass spectrum (ESI) *m*/*z:* 540 (M+H)⁺

(Example 68) 4-(1-(3-Ethoxy-4-(2-methoxyethoxy)phenyl)-2-oxo-2-(N'-(pyridine-4-carbonyl)hydrazino)ethylamino)benzamidine trifluoroacetate 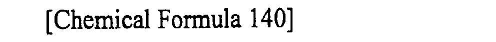 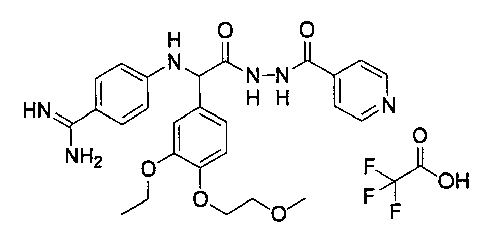 A mixture of sodium (4-carbamimidoylphenylamino)-(3-ethoxy-4-(2-methoxyethoxy)phenyl)acetate (10 mg, 0.0244 mmol) prepared in Example 67c, 1-ethyl-3-(3-dimethylaminopropyl)carbodiimide hydrochloride (14.1 mg, 0.0737 mmol), 1-hydroxybenzotriazole monohydrate (11.3 mg, 0.0737 mmol) and N,N-dimethylformamide (1 ml) was stirred at 0°C for 1 hour. To the reaction mixture was added isonicotinic acid hydrazide (10.1 mg, 0.0737 mmol), followed by stirring overnight at room temperature. The reaction mixture was purified by reversed-phase high performance liquid chromatography to give the title compound as a yellow solid (12.2 mg, yield: 81%).
¹H-NMR (400 MHz, CD₃OD) δ: 1.39 (t, J = 7.2 Hz, 3H), 3.42 (s, 3H), 3.72-3.76 (m, 2H), 4.07-4.15 (m, 4H), 5.17 (s, 1H), 6.88 (d, J = 8.8 Hz, 2H), 6.99 (d, J = 8.0 Hz, 1H), 7.14 (dd, J = 8.0, 2.4 Hz, 1H), 7.23 (d, J = 2.4 Hz, 1H), 7.64 (d, J = 8.8 Hz, 2H), 8.00 (d, J = 6.4 Hz, 2H), 8.82 (d, J = 6.4 Hz, 2H); Mass spectrum (ESI) *m*/*z*: 507 (M+H)⁺

(Example 69) 4-(1-(2-Fluoro-4,5-dimethoxyphenyl)-2-(N'-(3-methylpyridine-4-carbonyl)hydrazino)-2-oxoethylamino)benzamidine trifluoroacetate 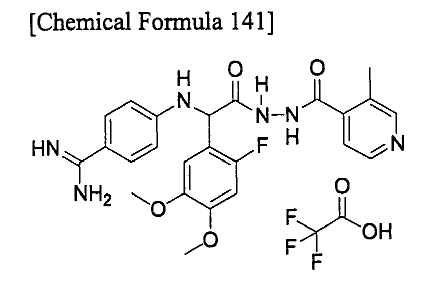 A mixture of 3-methylisonicotinic acid (3.2 mg, 0.0233 mmol), 1-ethyl-3-(3-dimethylaminopropyl)carbodiimide hydrochloride (4.4 mg, 0.0230 mmol), 1-hydroxybenzotriazole monohydrate (3.5 mg, 0.0229 mmol) and N,N-dimethylformamide (0.6 ml) was stirred at 0°C for 1 hour. To the reaction mixture was added 4-(((2-fluoro-4,5-dimethoxyphenyl)hydrazinocarbonylmethyl)amino)benzamidine dihydrochloride (10 mg, 0.0230 mmol) prepared in Example 14d, followed by stirring overnight at room temperature. The reaction mixture was purified by reversed-phase high performance liquid chromatography to give the title compound as a colorless solid (2.16 mg, yield: 16%).
¹H-NMR (400 MHz, CD₃OD) δ: 2.49 (s, 3H), 3.827 (s, 3H), 3.833 (s, 3H), 5.48 (s, 1H), 6.86 (d, J = 11.6 Hz, 1H), 6.89 (d, J = 8.8 Hz, 2H), 7.23 (d, J = 7.2 Hz, 1H), 7.57 (d, J = 5.2 Hz, 1H), 7.66 (d, J = 8.8 Hz, 2H), 8.52 (d, J = 5.2 Hz, 1H), 8.57 (s, 1H); Mass spectrum (ESI) *m*/*z:* 481 (M+H)⁺

(Example 70) 4-(1-(2-Fluoro-4,5-dimethoxyphenyl)-2-(N'-(2-methoxypyridine-3-carbonyl)hydrazino)-2-oxoethylamino)benzamidine trifluoroacetate 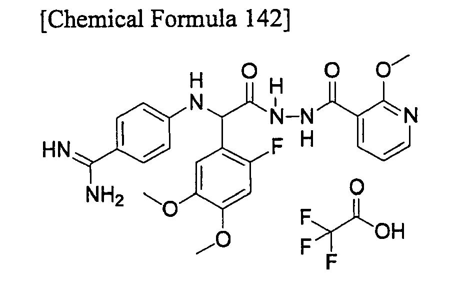 A mixture of 2-methoxynicotinic acid (3.5 mg, 0.0229 mmol), 1-ethyl-3-(3-dimethylaminopropyl)carbodiimide hydrochloride (4.4 mg, 0.0230 mmol), 1-hydroxybenzotriazole monohydrate (3.5 mg, 0.0229 mmol) and N,N-dimethylformamide (0.6 ml) was stirred at 0°C for 1 hour. To the reaction mixture was added 4-(((2-fluoro-4,5-dimethoxyphenyl)hydrazinocarbonylmethyl)amino)benzamidine dihydrochloride (10 mg, 0.0230 mmol) prepared in Example 14d, followed by stirring overnight at room temperature. The reaction mixture was purified by reversed-phase high performance liquid chromatography to give the title compound as a colorless solid (3.76 mg, yield: 27%).
¹H-NMR (400 MHz, CD₃OD) δ: 3.832 (s, 3H), 3.835 (s, 3H), 4.06 (s, 3H), 5.51 (s, 1H), 6.85 (d, J = 11.6 Hz, 1H), 6.89 (d, J = 8.8 Hz, 2H), 7.08-7.12 (m, 1H), 7.23 (d, J = 7.6 Hz, 1H), 7.65 (d, J = 8.8 Hz, 2H), 8.26-8.34 (m, 2H); Mass spectrum (ESI) *m*/*z:* 497 (M+H)⁺

(Example 71) 4-(2-(N'-(2-Dimethylaminopyridine-3-carbonyl)hydrazino)-1-(2-fluoro-4.5-dimethoxyphenyl)-2-oxoethylamino)benzamidine trifluoroacetate 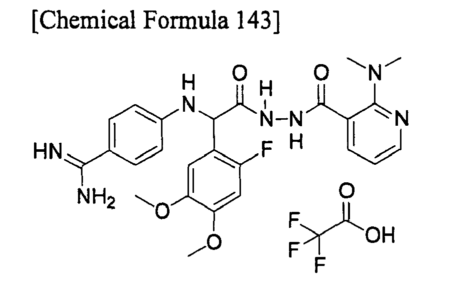 A mixture of lithium 2-dimethylaminonicotinate (7 mg, 0.0407 mmol), 1-ethyl-3-(3-dimethylaminopropyl)carbodiimide hydrochloride (4.4 mg, 0.0230 mmol), 1-hydroxybenzotriazole monohydrate (3.5 mg, 0.0229 mmol) and N,N-dimethylformamide (0.6 ml) was stirred at 0°C for 1 hour. To the reaction mixture was added 4-(((2-fluoro-4,5-dimethoxyphenyl)hydrazinocarbonylmethyl)amino)benzamidine dihydrochloride (10 mg, 0.0230 mmol) prepared in Example 14d, followed by stirring overnight at room temperature. The reaction mixture was purified by reversed-phase high performance liquid chromatography to give the title compound as a colorless solid (4.43 mg, yield: 31%).
¹H-NMR (400 MHz, CD₃OD) δ: 3.18 (s, 6H), 3.82 (s, 3H), 3.83 (s, 3H), 5.46 (s, 1H), 6.86 (d, J = 10.8 Hz, 1H), 6.88 (d, J = 8.8 Hz, 2H), 6.93 (dd, J = 7.2, 6.0 Hz, 1H), 7.21 (d, J = 6.8 Hz, 1H), 7.65 (d, J = 8.8 Hz, 2H), 8.05 (dd, J = 7.2, 2.0 Hz, 1H), 8.10 (dd, J = 6.0, 2.0 Hz, 1H); Mass spectrum (ESI) *m*/*z*: 510 (M+H)⁺

(Example 72) 4-(2-(N'-(2-Bromobenzoyl)hydrazino)-1-(2-fluoro-4,5-dimethoxyphenyl)-2-oxoethylamino)benzamidine trifluoroacetate 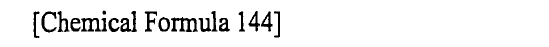 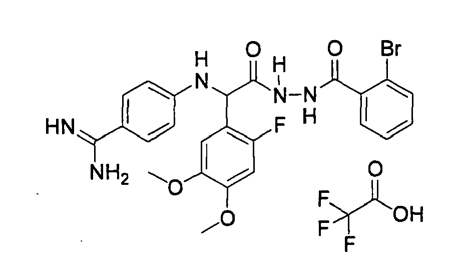 A mixture of 2-bromobenzoic acid (4.6 mg, 0.0229 mmol), 1-ethyl-3-(3-dimethylaminopropyl)carbodiimide hydrochloride (4.4 mg, 0.0230 mmol), 1-hydroxybenzotriazole monohydrate (3.5 mg, 0.0229 mmol) and N,N-dimethylformamide (0.6 ml) was stirred at 0°C for 1 hour. To the reaction mixture was added 4-(((2-fluoro-4,5-dimethoxyphenyl)hydrazinocarbonylmethyl)amino)benzamidine dihydrochloride (10 mg, 0.0230 mmol) prepared in Example 14d, followed by stirring overnight at room temperature. The reaction mixture was purified by reversed-phase high performance liquid chromatography to give the title compound as a colorless solid (1.54 mg, yield: 10%).
¹H-NMR (400 MHz, CD₃OD) δ: 3.825 (s, 3H), 3.831 (s, 3H), 5.48 (s, 1H), 6.85 (d, J = 11.6 Hz, 1H), 6.89 (d, J = 8.8 Hz, 2H), 7.21 (d, J = 6.8 Hz, 1H), 7.36-7.46 (m, 2H), 7.57 (dd, J = 7.6, 1.6 Hz, 1H), 7.63-7.68 (m, 3H); Mass spectrum (ESI) *m*/*z:* 544 (M+H)⁺

(Example 73) 4-(1-(3-Ethoxy-4-(2-methoxyethoxy)phenyl)-2-(N'-(3-methylpyridine-2-carbonyl)hydrazino)-2-oxoethylamino)benzamidine trifluoroacetate

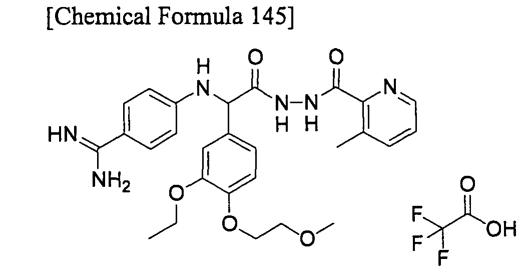

(73a) N'-(2-(4-Carbamimidoylphenylamino)-2-(3-ethoxy-4-(2-methoxyethoxy)phenyl)acetyl)hydrazinecarboxylic acid t-butyl ester 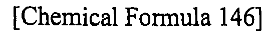 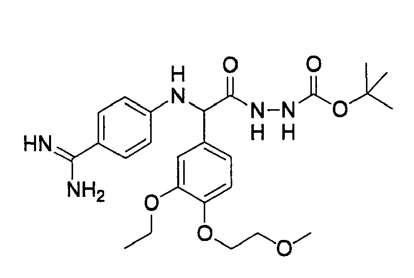 A mixture of sodium (4-carbamimidoylphenylamino)-(3-ethoxy-4-(2-methoxyethoxy)phenyl)acetate (400 mg, 0.977 mmol) prepared in Example 67c, 1-ethyl-3-(3-dimethylaminopropyl)carbodiimide hydrochloride (562 mg, 2.93 mmol), 1-hydroxybenzotriazole monohydrate (449 mg, 2.93 mmol) and N,N-dimethylformamide (5 ml) was stirred at 0°C for 1 hour and 30 minutes. To the reaction mixture was added hydrazinecarboxylic acid t-butyl ester (387 mg, 2.93 mmol), followed by stirring overnight at room temperature. The reaction mixture was crudely purified by silica gel column chromatography (NH silica gel (Fuji Silysia Chemical, Ltd.), ethyl acetate-dichloromethane-methanol) to give the title compound as a pale yellow solid (523 mg, yield: 107%).
¹H-NMR (400 MHz, CD₃OD) δ: 1.38 (t, J = 7.2 Hz, 3H), 1.46 (brs, 9H), 3.42 (s, 3H), 3.71-3.74 (m, 2H), 4.05-4.13 (m, 4H), 5.01 (s, 1H), 6.78 (d, J = 8.8 Hz, 2H), 6.94 (d, J = 8.0 Hz, 1H), 7.05 (dd, J = 8.0, 2.0 Hz, 1H), 7.15 (d, J = 2.0 Hz, 1H), 7.58 (d, J = 8.8 Hz, 2H)

(73b) 4-(((3-Ethoxy-4-(2-methoxyethoxy)phenyl)hydrazinocarbonylmethyl)amino)benzamidine dihydrochloride 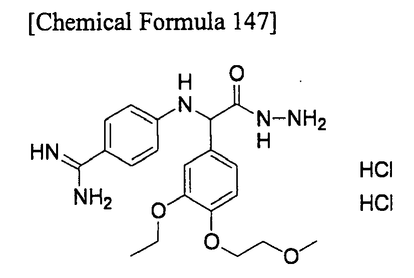 To a mixture of N'-(2-(4-carbamimidoylphenylamino)-2-(3-ethoxy-4-(2-methoxyethoxy)phenyl)acetyl)hydrazinecarboxylic acid t-butyl ester (523 mg, 1.04 mmol) prepared in Example 73a and methanol (2 ml) was added a 40% solution of hydrogen chloride in ethanol (2 ml). After stirring overnight at room temperature, the reaction mixture was concentrated under reduced pressure. The residue was washed with tetrahydrofuran to give the title compound as a yellow solid (493 mg, yield: 102%).
¹H-NMR (400 MHz, CD₃OD) δ: 1.38 (t, J = 7.2 Hz, 3H), 3.42 (s, 3H), 3.71-3.75 (m, 2H), 4.03-4.13 (m, 4H), 5.18 (s, 1H), 6.82 (d, J = 8.8 Hz, 2H), 6.98 (d, J = 8.4 Hz, 1H), 7.08 (dd, J = 8.4, 2.0 Hz, 1H), 7.13 (d, J = 2.0 Hz, 1H), 7.63 (d, J = 8.8 Hz, 2H)

(73c) 4-(1-(3-Ethoxy-4-(2-methoxyethoxy)phenyl)-2-(N'-(3-methylpyridine-2-carbonyl)hydrazino)-2-oxoethylamino)benzamidine trifluoroacetate 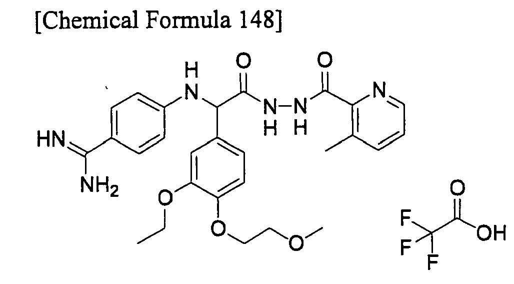 A mixture of 3-methylpyridine-2-carboxylic acid (4.5 mg, 0.0328 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (0.6 ml) was stirred at 0°C for 1 hour. To the reaction mixture was added 4-(((3-ethoxy-4-(2-methoxyethoxy)phenyl)hydrazinocarbonylmethyl)amino)benzamidine dihydrochloride (15 mg, 0.0316 mmol) prepared in Example 73b, followed by stirring overnight at room temperature. The reaction mixture was purified by reversed-phase high performance liquid chromatography to give the title compound as a colorless solid (9.33 mg, yield: 47%).
¹H-NMR (400 MHz, CD₃OD) δ: 1.39 (t, J = 7.2 Hz, 3H), 2.60 (s, 3H), 3.42 (s, 3H), 3.71-3.76 (m, 2H), 4.06-4.16 (m, 4H), 5.18 (s, 1H), 6.87 (d, J = 8.8 Hz, 2H), 6.98 (d, J = 8.4 Hz, 1H), 7.12 (dd, J = 8.4, 2.4 Hz, 1H), 7.24 (d, J = 2.4 Hz, 1H), 7.43 (dd, J = 8.0,4.4 Hz, 1H), 7.63 (d, J = 8.8 Hz, 2H), 7.72-7.77 (m, 1H), 8.40-8.44 (m, 1H); Mass spectrum (ESI) *m*/*z:* 521 (M+H)⁺

(Example 74) 4-(2-(N'-(3-Bromopyridine-2-carbonyl)hydrazino)-1-(3-ethoxy-4-(2-methoxyethoxy)phenyl)-2-oxoethylamino)benzamidine trifluoroacetate 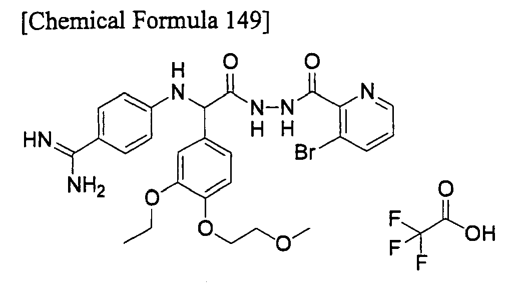 A mixture of 3-bromopyridine-2-carboxylic acid (6.5 mg, 0.0322 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (0.6 ml) was stirred at 0°C for 1 hour. To the reaction mixture was added 4-(((3-ethoxy-4-(2-methoxyethoxy)phenyl)hydrazinocarbonylmethyl)amino)benzamidine dihydrochloride (15 mg, 0.0316 mmol) prepared in Example 73b, followed by stirring overnight at room temperature. The reaction mixture was purified by reversed-phase high performance liquid chromatography to give the title compound as a colorless solid (12.31 mg, yield: 56%).
¹H-NMR (400 MHz, CD₃OD) δ: 1.39 (t, J = 7.2 Hz, 3H), 3.42 (s, 3H), 3.72-3.75 (m, 2H), 4.09-4.15 (m, 4H), 5.17 (s, 1H), 6.87 (d, J = 8.8 Hz, 2H), 6.98 (d, J = 8.4 Hz, 1H), 7.11 (dd, J = 8.4, 2.0 Hz, 1H), 7.23 (d, J = 2.0 Hz, 1H), 7.43 (dd, J = 8.0, 4.8 Hz, 1H), 7.63 (d, J = 8.8 Hz, 2H), 8.15 (dd, J = 8.0, 1.2 Hz, 1H), 8.56 (dd, J = 4.8, 1.2 Hz, 1H); Mass spectrum (ESI) *m*/*z:* 585 (M+H)⁺

(Example 75) 4-(1-(3-Ethoxy-4-(2-methoxyethoxy)phenyl)-2-(N'-(2-fluoropyridine-3-carbonyl)hydrazino)-2-oxoethylamino)benzamidine trifluoroacetate 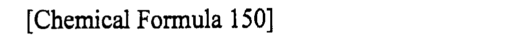 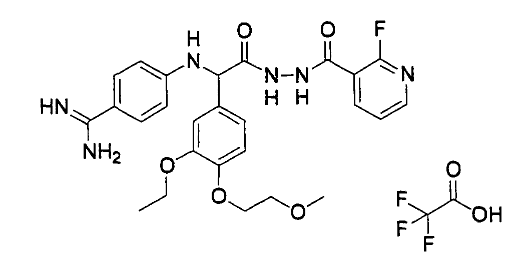 A mixture of 2-fluoronicotinic acid (4.5 mg, 0.0319 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (0.6 ml) was stirred at 0°C for 1 hour. To the reaction mixture was added 4-(((3-ethoxy-4-(2-methoxyethoxy)phenyl)hydrazinocarbonylmethyl)amino)benzamidine dihydrochloride (15 mg, 0.0316 mmol) prepared in Example 73b, followed by stirring overnight at room temperature. The reaction mixture was purified by reversed-phase high performance liquid chromatography to give the title compound as a colorless solid (10.61 mg, yield: 53%).
¹H-NMR (400 MHz, CD₃OD) δ: 1.39 (t, J = 7.2 Hz, 3H), 3.41 (s, 3H), 3.72-3.75 (m, 2H), 4.09-4.15 (m, 4H), 5.15 (s, 1H), 6.87 (d, J = 8.8 Hz, 2H), 6.98 (d, J = 8.0 Hz, 1H), 7.12 (dd, J = 8.0, 2.0 Hz, 1H), 7.22 (d, J = 2.0 Hz, 1H), 7.41-7.46 (m, 1H), 7.64 (d, J = 8.8 Hz, 2H), 8.25-8.37 (m, 2H); Mass spectrum (ESI) *m*/*z:* 525 (M+H)⁺

(Example 76) 4-(1-(3-Ethoxy-4-(2-methoxyethoxy)phenyl)-2-(N'-(4-methylpyridine-3-carbonyl)hydrazino)-2-oxoethylamino)benzamidine trifluoroacetate 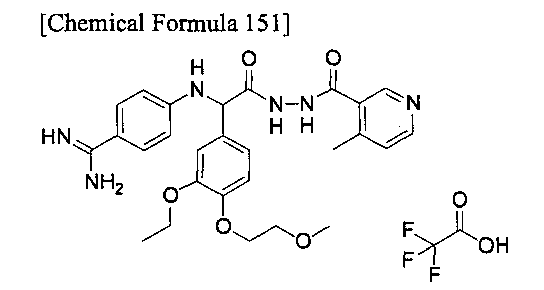 A mixture of 4-methylnicotinic acid hydrochloride (6.5 mg, 0.0374 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (0.6 ml) was stirred at 0°C for 1 hour. To the reaction mixture was added 4-(((3-ethoxy-4-(2-methoxyethoxy)phenyl)hydrazinocarbonylmethyl)amino)benzamidine dihydrochloride (15 mg, 0.0316 mmol) prepared in Example 73b, followed by stirring overnight at room temperature. The reaction mixture was purified by reversed-phase high performance liquid chromatography to give the title compound as a colorless solid (4.78 mg, yield: 24%).
¹H-NMR (400 MHz, CD₃OD) δ: 1.39 (t, J = 7.2 Hz, 3H), 2.54 (s, 3H), 3.42 (s, 3H), 3.72-3.75 (m, 2H), 4.09-4.15 (m, 4H), 5.16 (s, 1H), 6.88 (d, J = 8.8 Hz, 2H), 6.99 (d, J = 8.4 Hz, 1H), 7.12 (dd, J = 8.4, 2.4 Hz, 1H), 7.22 (d, J = 2.4 Hz, 1H), 7.47 (d, J = 5.6 Hz, 1H), 7.64 (d, J = 8.8 Hz, 2H), 8.52 (d, J = 5.6 Hz, 1H), 8.64 (s, 1H); Mass spectrum (ESI) *m*/*z*: 521 (M+H)⁺

(Example 77) 4-(1-(3-Ethoxy-4-(2-methoxyethoxy)phenyl)-2-(N'-(3-fluoropyridine-4-carbonyl)hydrazino)-2-oxoethylamino)benzamidine trifluoroacetate 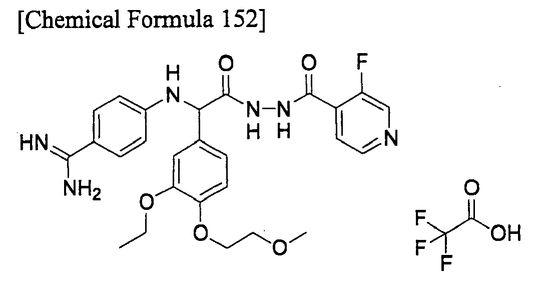 A mixture of 3-fluoroisonicotinic acid (4.5 mg, 0.0319 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (0.6 ml) was stirred at 0°C for 1 hour. To the reaction mixture was added 4-(((3-ethoxy-4-(2-methoxyethoxy)phenyl)hydrazinocarbonylmethyl)amino)benzamidine dihydrochloride (15 mg, 0.0316 mmol) prepared in Example 73b, followed by stirring overnight at room temperature. The reaction mixture was purified by reversed-phase high performance liquid chromatography to give the title compound as a colorless solid (6.40 mg, yield: 32%).
¹H-NMR (400 MHz, CD₃OD) δ: 1.39 (t, J = 7.2 Hz, 3H), 3.42 (s, 3H), 3.72-3.74
(m, 2H), 4.07-4.15 (m, 4H), 5.15 (s, 1H), 6.88 (d, J = 8.8 Hz, 2H), 6.99 (d, J = 8.4 Hz, 1H), 7.12 (dd, J = 8.4, 2.0 Hz, 1H), 7.21 (d, J = 2.0 Hz, 1H), 7.64 (d, J = 8.8 Hz, 2H), 7.71 (t, J = 5.6 Hz, 1H), 8.52 (dd, J = 4.8, 0.8 Hz, 1H), 8.61 (d, J = 2.0 Hz, 1H); Mass spectrum (ESI) *m*/*z:* 525 (M+H)⁺

(Example 78) 4-(2-(N'-(3-Chloropyridine-4-carbonyl)hydrazino)-1-(3-ethoxy-4-(2-methoxyethoxy)phenyl)-2-oxoethylamino)benzamidine trifluoroacetate 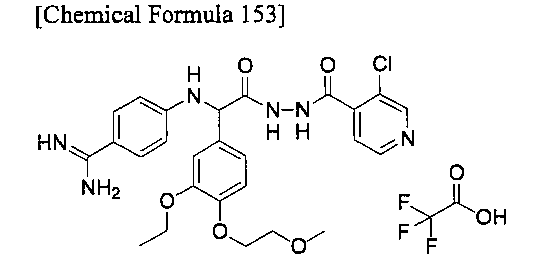 A mixture of 3-chloroisonicotinic acid (5.2 mg, 0.0330 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (0.6 ml) was stirred at 0°C for 1 hour. To the reaction mixture was added 4-(((3-ethoxy-4-(2-methoxyethoxy)phenyl)hydrazinocarbonylmethyl)amino)benzamidine dihydrochloride (15 mg, 0.0316 mmol) prepared in Example 73b, followed by stirring overnight at room temperature. The reaction mixture was purified by reversed-phase high performance liquid chromatography to give the title compound as a colorless solid (5.70 mg, yield: 28%).
¹H-NMR (400 MHz, CD₃OD) δ: 1.39 (t, J = 6.8 Hz, 3H), 3.42 (s, 3H), 3.72-3.75 (m, 2H), 4.07-4.15 (m, 4H), 5.15 (s, 1H), 6.87 (d, J = 8.8 Hz, 2H), 6.99 (d, J = 8.4 Hz, 1H), 7.11 (dd, J = 8.4, 2.0 Hz, 1H), 7.21 (d, J = 2.0 Hz, 1H), 7.58 (d, J = 4.8 Hz, 1H), 7.64 (d, J = 8.8 Hz, 2H), 8.58 (d, J = 4.8 Hz, 1H), 8.68 (s, 1H); Mass spectrum (ESI) *m*/*z:* 541 (M+H)⁺

(Example 79) 4-(2-(N'-(3-Bromopyridine-4-carbonyl)hydrazino)-1-(3-ethoxy-4-(2-methoxyethoxy)phenyl)-2-oxoethylamino)benzamidine trifluoroacetate 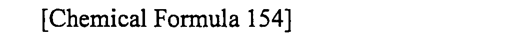 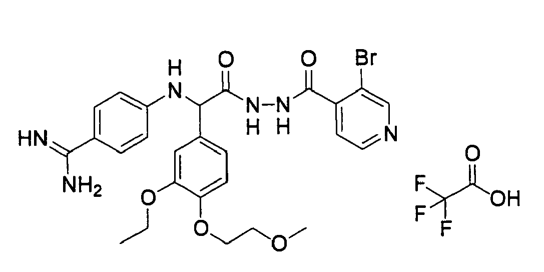 A mixture of 3-bromoisonicotinic acid (6.5 mg, 0.0322 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (0.6 ml) was stirred at 0°C for 1 hour. To the reaction mixture was added 4-(((3-ethoxy-4-(2-methoxyethoxy)phenyl)hydrazinocarbonylmethyl)amino)benzamidine dihydrochloride (15 mg, 0.0316 mmol) prepared in Example 73b, followed by stirring overnight at room temperature. The reaction mixture was purified by reversed-phase high performance liquid chromatography to give the title compound as a colorless solid (7.08 mg, yield: 32%).
¹H-NMR (400 MHz, CD₃OD) δ: 1.39 (t, J = 7.2 Hz, 3H), 3.41 (s, 3H), 3.72-3.75 (m, 2H), 4.07-4.14 (m, 4H), 5.15 (s, 1H), 6.87 (d, J = 8.8 Hz, 2H), 6.99 (d, J = 8.4 Hz, 1H), 7.11 (dd, J = 8.4, 1.6 Hz, 1H), 7.21 (d, J = 1.6 Hz, 1H), 7.56 (d, J = 4.8 Hz, 1H), 7.64 (d, J = 8.8 Hz, 2H), 8.60 (d, J = 4.8 Hz, 1H), 8.79 (s, 1H); Mass spectrum (ESI) *m*/*z*: 585 (M+H)⁺

(Example 80) 4-(1-(3-Ethoxy-4-(2-methoxyethoxy)phepyl)-2-(N'-(3-methylpyridine-4-carbonyl)hydrazino)-2-oxoethylamino)benzamidine trifluoroacetate 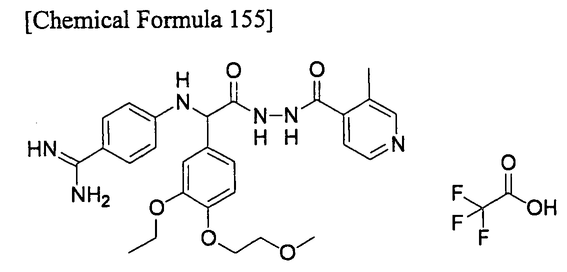 A mixture of 3-methylisonicotinic acid (4.5 mg, 0.0328 mmol), 1-ethyl-3-(3-dimethylaminopropyl)carbodiimide hydrochloride (6.1 mg, 0.0318 mmol), 1-hydroxybenzotriazole monohydrate (4.9 mg, 0.0320 mmol) and N,N-dimethylformamide (0.6 ml) was stirred at 0°C for 1 hour. To the reaction mixture was added 4-(((3-ethoxy-4-(2-methoxyethoxy)phenyl)hydrazinocarbonylmethyl)amino)benzamidine dihydrochloride (15 mg, 0.0316 mmol) prepared in Example 73b, followed by stirring overnight at room temperature. The reaction mixture was purified by reversed-phase high performance liquid chromatography to give the title compound as a colorless solid (5.06 mg, yield: 25%).
¹H-NMR (400 MHz, CD₃OD) δ: 1.39 (t, J = 6.8 Hz, 3H), 2.47 (s, 3H), 3.42 (s, 3H), 3.72-3.75 (m, 2H), 4.07-4.15 (m, 4H), 5.15 (s, 1H), 6.88 (d, J = 8.8 Hz, 2H), 6.99 (d, J = 8.4 Hz, 1H), 7.11 (dd, J = 8.4, 2.0 Hz, 1H), 7.22 (d, J = 2.0 Hz, 1H), 7.51 (d, J = 4.8 Hz, 1H), 7.64 (d, J = 8.8 Hz, 2H), 8.49 (d, J = 4.8 Hz, 1H), 8.54 (s, 1H); Mass spectrum (ESI) *m*/*z:* 521 (M+H)⁺

(Example 81) 4-{1-(2-Fluoro-5-methoxy-3-propoxyphenyl)-2-oxo-2-[N'-(pyridine-4-carbonyl)hydrazino]ethylamino}benzamidine trifluoroacetate

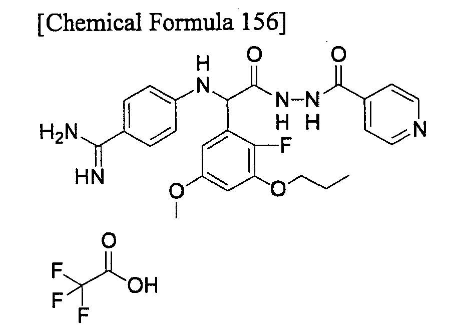

(81a) 3-Dimethoxymethyl-2-fluoro-5-methoxyphenol 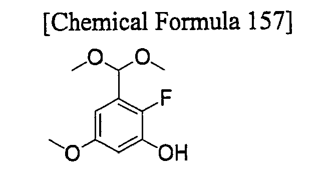 2-Fluoro-5-methoxybenzaldehyde (5 g, 32.4 mmol) was dissolved in methanol (300 ml), trimethyl orthoformate (35 mL, 320 mmol) and para-toluenesulfonic acid monohydrate (840 mg, 4.88 mmol) were added thereto and the reaction mixture was stirred overnight at 50°C. To the reaction mixture was added para-toluenesulfonic acid monohydrate (840 mg, 4.88 mmol), followed by stirring at an external temperature of 90°C for 9 hours. After cooling the reaction mixture to room temperature, triethylamine (2.3 mL) was added and the solvent was distilled off under reduced pressure. Diethyl ether and dilute hydrochloric acid were added to the obtained residue and extraction was performed with diethyl ether. The obtained organic layer was washed with saturated aqueous sodium hydrogencarbonate and brine in that order, and dried over magnesium sulfate. The desiccant was removed by filtration and the filtrate was concentrated.
The obtained crudely purified product (6.91 g) and N,N,N',N',N"-pentamethyldiethylenetriamine (6.77 mL, 32.4 mmol) were dissolved in tetrahydrofuran (200 ml), and the mixture was cooled and stirred at -78°C. To the reaction mixture was slowly added dropwise n-butyllithium (1.54 M solution in hexane, 22.1 mL, 34 mmol), followed b stirring at the same temperature for 3 hours. Trimethyl borate (7.36 mL, 64.8 mmol) was then slowly added dropwise to the reaction mixture, and the external temperature was raised to room temperature prior to stirring for 2 hours. The reaction mixture was cooled to 0°C, acetic acid (5.1 mL, 89.1 mmol) was slowly added dropwise, and the mixture was stirred at 0°C for 30 minutes. After then slowly adding 30% aqueous hydrogen peroxide (5.51 mL, 48.6 mmol) dropwise to the reaction mixture, the ice bath was removed and the mixture was stirred overnight. A saturated sodium sulfite aqueous solution (50 mL) was added to the mixture, and stirring was stopped. The solvent was distilled off under reduced pressure, water was added to the obtained residue, and extraction was performed twice with ethyl acetate. The combined organic layer was washed with brine and dried over magnesium sulfate. The desiccant was removed by filtration and the filtrate was concentrated. The obtained residue was purified by silica gel chromatography (Merck, Ltd., ethyl acetate-heptane) to give the title compound (4.13 g, 59%) as a yellow oil.
¹H-NMR (400 MHz, CDCl₃) δ: 3.38 (s, 6H), 3.76 (s, 3H), 5.23 (br, 1H), 5.55 (s, 1H), 6.55 (dd, J = 6.8, 3.2 Hz, 1H), 6.60 (dd, J = 4.8, 3.2 Hz, 1H)

(81b) 2-Fluoro-3-hydroxy-5-methoxybenzaldehyde 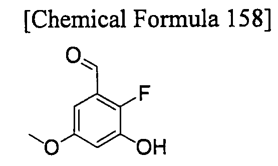 Acetic acid (10 mL) and water (5 mL) were added to 3-dimethoxymethyl-2-fluoro-5-methoxyphenol (4.13 g,19.1 mmol) prepared in Example 81a, and the mixture was stirred overnight at an external temperature of 60°C. Saturated aqueous sodium hydrogencarbonate was added to the reaction mixture, and after air cooling, the stirring was stopped. Ethyl acetate was added to the mixture and extraction was performed twice. The combined organic layer was dried over magnesium sulfate. After removing the desiccant by filtration, the solvent was distilled off under reduced pressure. The obtained residue was purified by silica gel column chromatography (Merck, Ltd., ethyl acetate-heptane) to give the title compound (2.98 g, yield: 92%) as a pale gray solid.
¹H-NMR (400 MHz, CD₃OD) δ: 3.77 (s, 3H), 6.74-6.78 (m, 2H), 10.24 (s, 1H)

(8 1 c) Ethyl (4-cyanophenylamino)-(2-fluoro-5-methoxy-3-propoxyphenyl)acetate 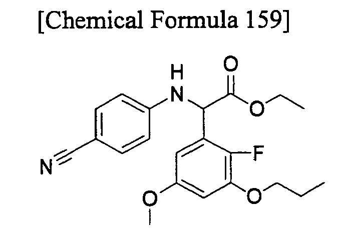 2-Fluoro-3-hydroxy-5-methoxybenzaldehyde (1.5 g, 8.82 mmol) prepared in Example 81b was dissolved in N,N-dimethylformamide (30 mL), and then potassium carbonate (2.58 g, 18.7 mmol) and 1-iodopropane (3.17 g, 18.7 mmol) were added thereto and the mixture was stirred overnight at room temperature. Water was added to the reaction mixture and extraction was performed twice with diethyl ether. The combined organic layer was washed with water and brine and then dried over magnesium sulfate. After removing the desiccant, the solvent was distilled off under reduced pressure.
The obtained crudely purified product was dissolved in ethanol (30 ml), and then 4-aminobenzonitrile (1.16 g, 9.79 mmol) and 4-(2-isocyanoethyl)morpholine (1.35 mL, 9.79 mmol) were added. After then cooling the reaction mixture to 0°C, boron trifluoride/diethyl ether complex (4.28 ml, 37.4 mmol) was added. The reaction mixture was stirred at room temperature for 3 hours, and then water was added and stirring was continued overnight at an external temperature of 50°C. After concentrating the reaction mixture, water was added to the obtained residue and extraction was performed twice with ethyl acetate. The combined organic layer was dried over magnesium sulfate and, after removal of the desiccant by filtration, was concentrated. The obtained residue was purified by silica gel column chromatography (Merck, Ltd., ethyl acetate-heptane) to give the title compound (2.32 g, yield: 64%) as a pale yellow solid.
¹H-NMR (400 MHz, CD₃OD) δ: 1.06 (t, J = 7.6 Hz, 3H), 1.21 (t, J = 6.8 Hz, 3H), 1.78-1.87 (m, 2H), 3.71 (s, 3H), 3.97 (t, J = 6.8 Hz, 2H), 4.15-4.25 (m, 2H), 5.48 (s, 1H), 6.48 (dd, J = 4.4, 2.8 Hz, 1H), 6.58 (dd, J = 7.2, 2.8 Hz, 1H), 6.69-6.72 (m, 2H), 7.38-7.41 (m, 2H)

(81d) 4-{1-(2-Fluoro-5-methoxy-3-propoxyphenyl)-2-oxo-2-[N'-(pyridine-4-carbonyl)hydrazino]ethylamino}benzamidine trifluoroacetate 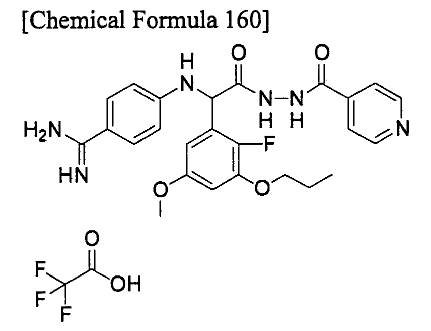 Ethyl (4-cyanophenylamino)-(2-fluoro-5-methoxy-3-propoxyphenyl)acetate (2.32 g, 5.99 mmol) prepared in Example 81 c was dissolved in ethanol (20 mL), and then hydroxylamine hydrochloride (1.66 g, 24.0 mmol) and triethylamine (2.74 g, 24.0 mmol) were added thereto and the mixture was stirred overnight at an external temperature of 70°C. After concentrating the reaction mixture, water was added and extraction was performed with ethyl acetate. The organic layer was washed with brine and dried over magnesium sulfate. After removing the desiccant by filtration, the solvent was distilled off under reduced pressure.
The obtained crudely purified product was dissolved in acetic acid (30 mL), and then acetic anhydride (3 mL) and 10% palladium-carbon (1 g) were added. The mixture was reacted for 2 hours at room temperature, ordinary pressure in a hydrogen atmosphere. The reaction mixture was filtered through celite, and the filtrate was concentrated under reduced pressure.
The crudely purified product was dissolved in methanol (30 ml), and a 5N sodium hydroxide aqueous solution (7.5 ml) was added. After stirring for 10 hours at room temperature, the reaction mixture was neutralized with hydrochloric acid. Diethyl ether was added and the precipitated solid was collected by filtration. The obtained solid was washed with a small amount of diethyl ether and a small amount of water and then dried under aeration and used without purification for the following reaction.
A portion (5 mg) of the crudely purified product was cooled to 0°C. A solution of 1-hydroxybenzotriazole monohydrate (5.6 mg, 0.0364 mmol) and 1-ethyl-3-(3-dimethylaminopropyl)carbodiimide hydrochloride (7.0 mg, 0.0364 mmol) in N,N-dimethylformamide (0.8 ml) was added, and the mixture was stirred at the same temperature for 1 hour. Isonicotinic acid hydrazide (5 mg, 0.0364 mmol) was then added to the reaction mixture. The reaction mixture was stirred overnight at room temperature and then purified by reversed-phase high performance liquid chromatography to give the title compound as a yellow oil (4.49 mg, yield: 61%). ¹H-NMR (400 MHz, CD₃OD) δ: 1.06 (t, J = 7.2 Hz, 3H), 1.78-1.87 (m, 2H), 3.78 (s, 3H), 3.99 (t, J = 6.4 Hz, 2H), 5.56 (s, 1H), 6.63 (dd, J = 7.2, 3.2 Hz, 1H), 6.74 (dd, J = 4.8, 3.2 Hz, 1H), 6.88-6.92 (m, 2H), 7.64-7.67 (m, 2H), 7.94 (dd, J = 4.8, 1.2 Hz, 2H), 8.78 (dd, J = 4.8, 1.2 Hz, 2H); Mass spectrum (ESI) *m*/*z*: 495 (M+1)⁺

The following compounds were produced similarly to the above general production processes for compounds of the invention and the above examples.

(Example X-1) 4-(2-(N'-(3-Bromopyridine-4-carbonyl)hydrazino)-1-(2-fluoro-4,5-dimethoxyphenyl)-2-oxoethylamino)benzamidine trifluoroacetate 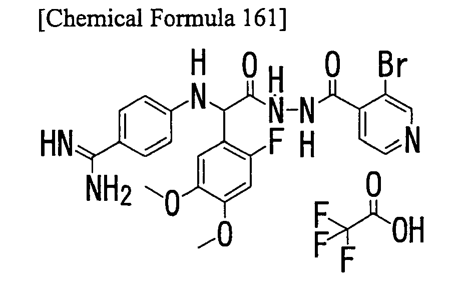 Mass spectrum (ESI) *m*/*z:* 545 (M+H)⁺

(Example X-2) 4-(2-(N'-(6-Acetylpyridine-2-carbonyl)hydrazino)-1-(3-ethoxy-4-isopropoxyphenyl)-2-oxoethylamino)benzamidine trifluoroacetate 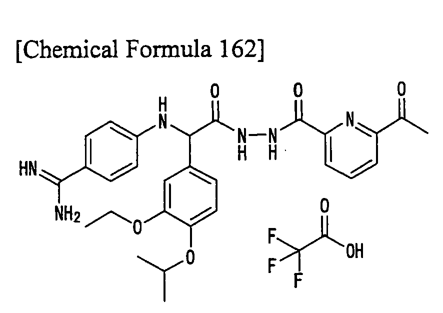 Mass spectrum (ESI) *m*/*z:* 533 (M+H)⁺

(Example X-3) 4-(2-(N'-(4-Dimethylaminopyridine-3-carbonyl)hydrazino)-1-(3-ethoxy-4-isopropoxyphenyl)-2-oxoethylamino)benzamidine trifluoroacetate 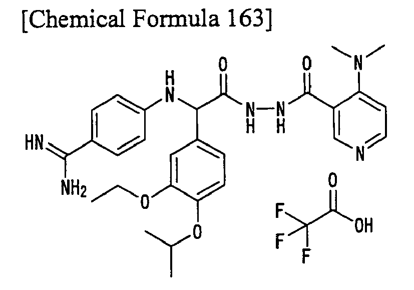 Mass spectrum (ESI) *m*/*z*: 534 (M+H)⁺

(Example X-4) 4-(2-(N'-(2-Dimethylaminopyridine-3-carbonyl)hydrazino)-1-(3-ethoxy-4-isopro-poxyphenyl)-2-oxoethylamino)benzamidine trifluoroacetate 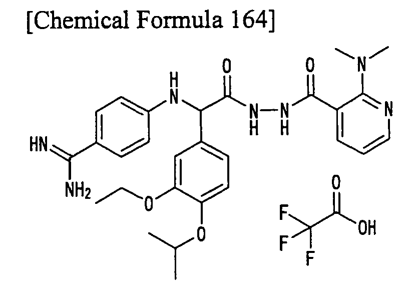 Mass spectrum (ESI) *m*/*z*: 534 (M+H)⁺

(Example X-5) 4-(2-(N'-(3-Aminopyridine-2-carbonyl)hydrazino)-1-(3-ethoxy-4-isopropoxyphenyl)-2-oxoethylamino)benzamidine trifluoroacetate 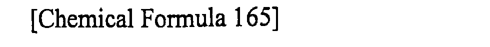 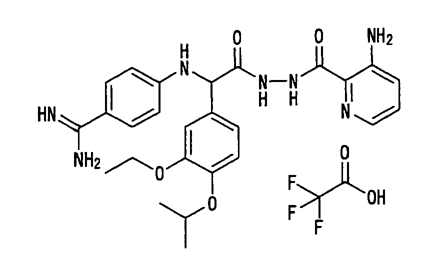 Mass spectrum (ESI) *m*/*z*: 506 (M+H)⁺

(Example X-6) 4-(2-(N'-Benzoylhydrazino)-1-(3,4-bisallyloxyphenyl)-2-oxoethylamino)benzamidine trifluoroacetate 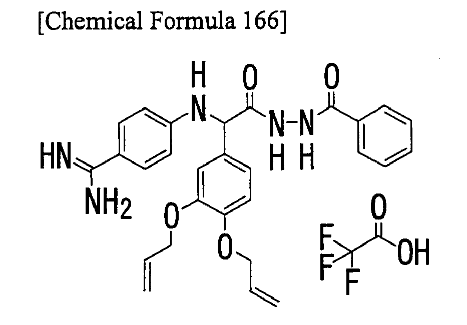 Mass spectrum (ESI) *m*/*z*: 500 (M+H)⁺

(Example X-7) 4-(1-(3,4-Bisallyloxyphenyl)-2-(N'-(furan-2-carbonyl)hydrazino)-2-oxoethylamino)benzamidine trifluoroacetate 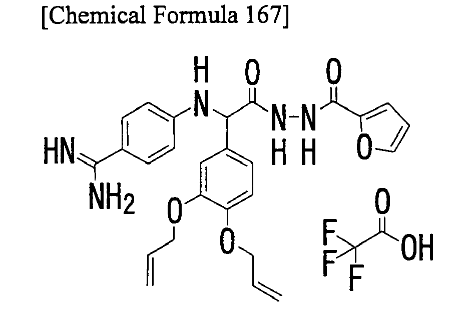 Mass spectrum (ESI) *m*/*z:* 490 (M+H)⁺

(Example X-8) 4-(1-(3,4-Bisallyloxyphenyl)-2-oxo-2-(N'-(pyridine-2-carbonyl)hydrazino)ethylamino)benzamidine trifluoroacetate 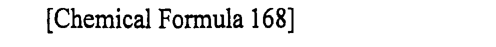 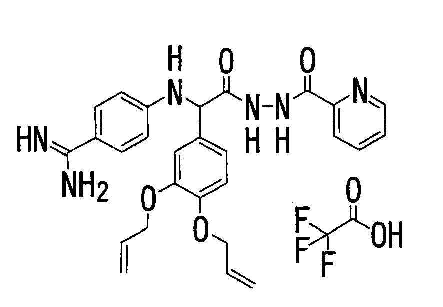 Mass spectrum (ESI) *m*/*z*: 501 (M+H)⁺

(Example X-9) 4-(1-(3,4-Bisallyloxyphenyl)-2-oxo-2-(N'-(pyridine-3-carbonyl)hydrazino)ethylamino)benzamidine trifluoroacetate 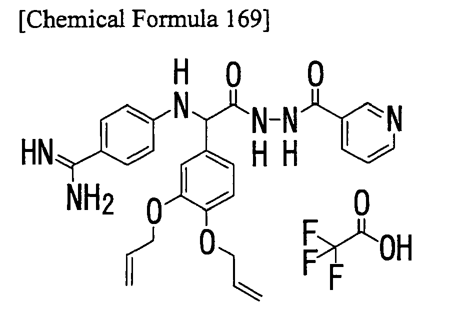 Mass spectrum (ESI) *m*/*z*: 501(M+H)⁺

(Example X-10) 4-(1-(3,4-Bisallyloxyphenyl)-2-oxo-2-(N'-(pyridine-4-carbonyl)hydrazino)ethylamino)benzamidine trifluoroacetate 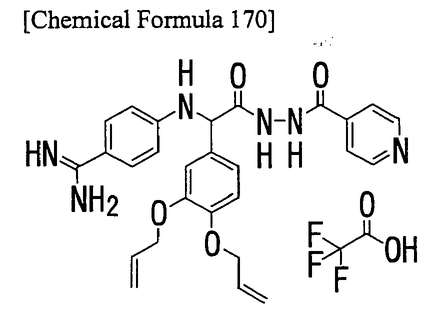 Mass spectrum (ESI) *m*/*z*: 501 (M+H)⁺

(Example X-11) 4-(1-(3,4-Bisallyloxyphenyl)-2-(N'-(3-methylpyridine-2-carbonyl)hydrazino)-2-oxoethylamino)benzamidine trifluoroacetate 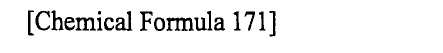 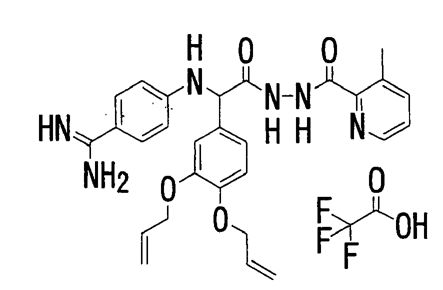 Mass spectrum (ESI) *m*/*z*: 515 (M+H)⁺

(Example X-12) 4-(1-(3,4-Bisallyloxyphenyl)-2-(N'-(3-bromopyridine-2-carbonyl)hydrazino)-2-oxoethylamino)benzamidine trifluoroacetate 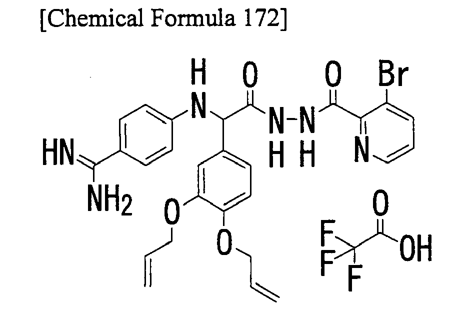 Mass spectrum (ESI) *m*/*z*: 579 (M+H)⁺

(Example X-13) 4-(1-(3,4-Bisallyloxyphenyl)-2-(N'-(2-fluoropyridine-3-carbonyl)hydrazino)-2-oxoethylamino)benzamidine trifluoroacetate 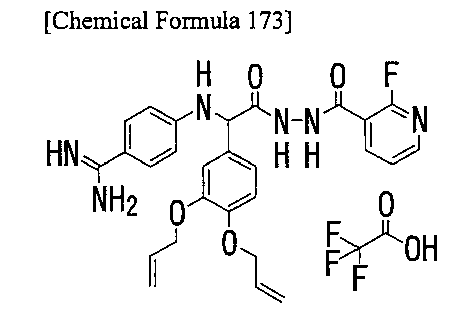 Mass spectrum (ESI) *m*/*z:* 519 (M+H)⁺

(Example X-14) 4-(1-(3,4-Bisallyloxyphenyl)-2-(N'-(4-methylpyridine-3-carbonyl)hydrazino)-2-oxoethylamino)benzamidine trifluoroacetate 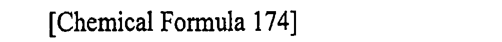 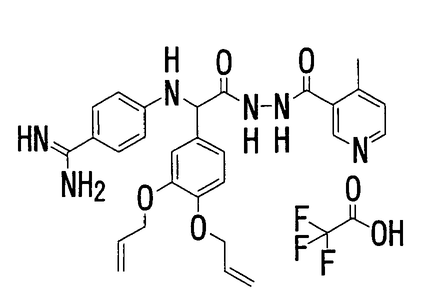 Mass spectrum (ESI) *m*/*z*: 515 (M+H)⁺

(Example X-15) 4-(1-(3,4-Bisallyloxyphenyl)-2-(N'-(4-chloropyridine-3-carbonyl)hydrazino)-2-oxoethylamino)benzamidine trifluoroacetate 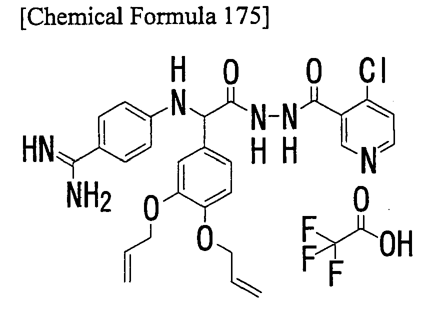 Mass spectrum (ESI) *m*/*z*: 535 (M+H)⁺

(Example X-16) 4-(1-(3,4-Bisallyloxyphenyl)-2-(N'-(3-fluoropyridine-4-carbonyl)hydrazino)-2-oxoethylamino)benzamidine trifluoroacetate 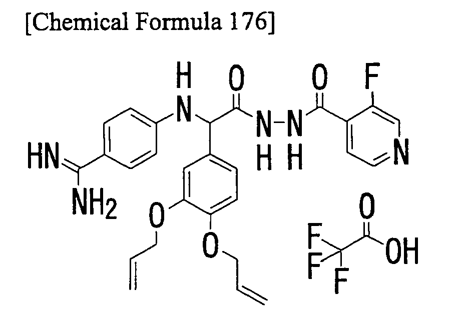 Mass spectrum (ESI) *m*/*z:* 519 (M+H)⁺

(Example X-17) 4-(1-(3,4-Bisallyloxyphenyl)-2-(N'-(3-chloropyridine-4-carbonyl)hydrazino)-2-oxoethylamino)benzamidine trifluoroacetate 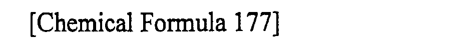 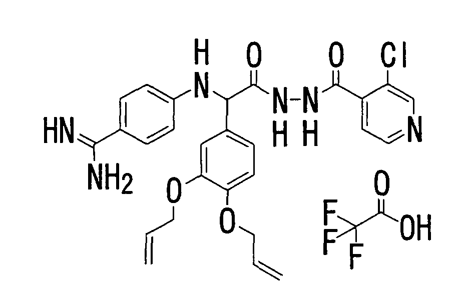 Mass spectrum (ESI) *m*/*z*: 535 (M+H)⁺

(Example X-18) 4-(1-(3,4-Bisallyloxyphenyl)-2-(N'-(3-methylpyridine-4-carbonyl)hydrazino)-2-oxoethylamino)benzamidine trifluoroacetate 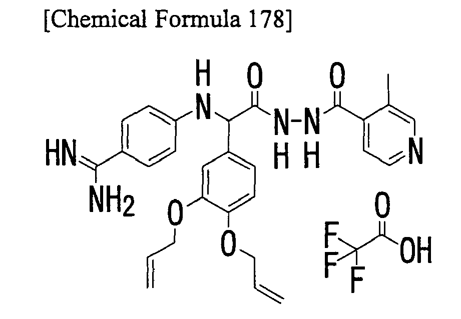 Mass spectrum (ESI) *m*/*z*: 515 (M+H)⁺

(Example X-19) 4-(((3-Ethoxy-4-isopropoxyphenyl)-(N'-(2-methoxyphenyl)hydrazinocarbonyl)methyl)amino)benzamidine trifluoroacetate 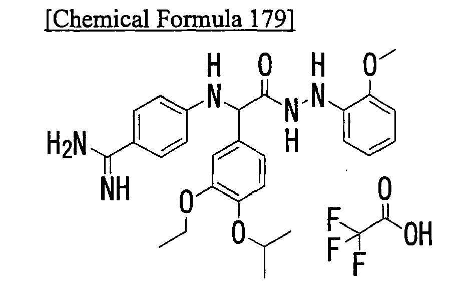 Mass spectrum (ESI) *m*/*z:* 492 (M+H)⁺

(Example X-20) 4-(((3-Ethoxy-4-isopropoxyphenyl)-(N'-(3-methoxyphenyl)hydrazinocarbonyl)methyl)amino)benzamidine trifluoroacetate 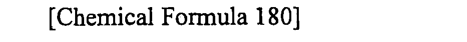 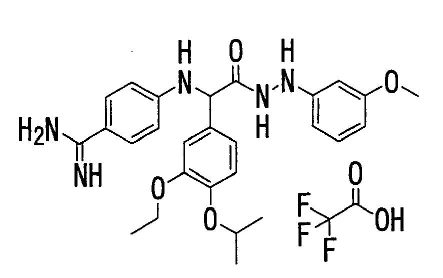 Mass spectrum (ESI) *m*/*z:* 492 (M+H)⁺

(Example X-21) 4-(((3-Ethoxy-4-isopropoxyphenyl)-(N'-(4-methoxyphenyl)hydrazinocarbonyl)methyl)amino)benzamidine trifluoroacetate 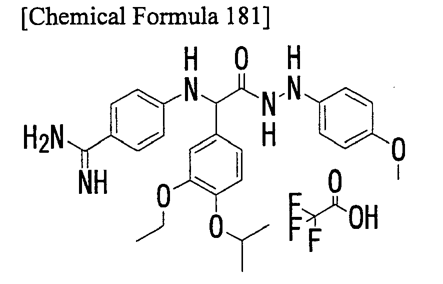 Mass spectrum (ESI) *m*/*z:* 492 (M+H)⁺

(Example X-22) 4-(((3-Ethoxy-4-isopropoxyphenyl)-(N'-(2-fluorophenyl)hydrazinocarbonyl)methyl)amino)benzamidine trifluoroacetate 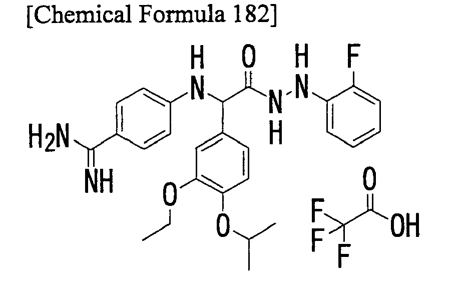 Mass spectrum (ESI) *m*/*z*: 480 (M+H)⁺

(Example X-23) 4-(((3-Ethoxy-4-isopropoxyphenyl)-(N'-(3-fluorophenyl)hydrazinocarbonyl)methyl)amino)benzamidine trifluoroacetate 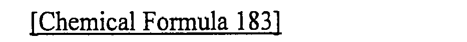 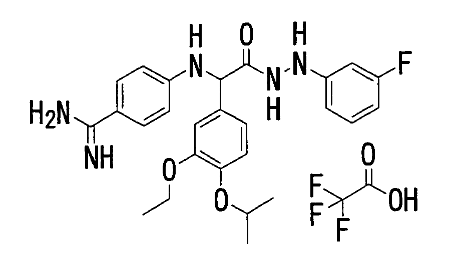 Mass spectrum (ESI) *m*/*z:* 480 (M+H)⁺

(Example X-24) 4-(((3-Ethoxy-4-isopropoxynhenyl)-(N'-(4-fluorophenyl)hydrazinocarbonyl)methyl)amino)benzamidine trifluoroacetate 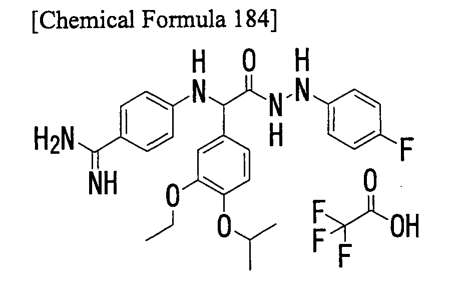 Mass spectrum (ESI) *m*/*z:* 480 (M+H)⁺

(Example X-25) 4-((1-(3-Ethoxy-4-isopropoxyphenyl)-2-oxo-2-(2-(phenylsulfonyl)hydrazino)ethyl)amino)benzamidine trifluoroacetate 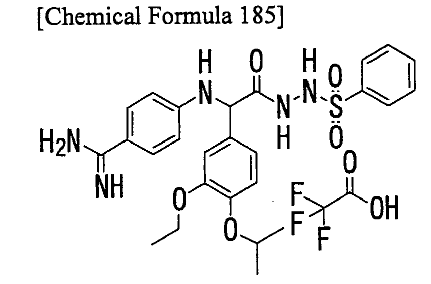 ¹H-NMR (400 MHz, CD₃OD) δ: 1.33 (d, J = 6.1Hz, 6H), 1.40 (t, J = 7.2Hz, 3H), 4.22 (q, J = 7.2Hz, 2H), 4.55 (sept, J = 6.1 Hz, 1H), 4.99 (s, 1H), 6.70 (d, J = 9.2Hz, 2H), 6.90-6.97 (m, 3H), 7.27 (t, J = 8.0Hz, 2H), 7.46-7.53 (m, 3H), 7.58 (d, J = 9.2Hz, 2H)

(Example X-26) 4-(((Benzylidenehydrazinocarbonyl)-(3-ethoxy-4-isopropoxyphenyl)methyl)amino)benzamidine trifluoroacetate 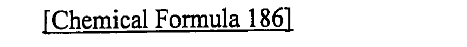 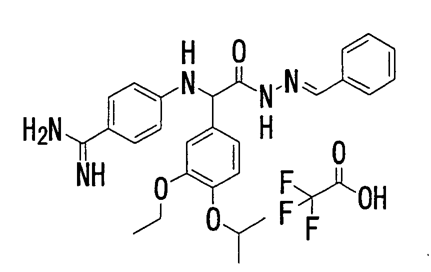 Mass spectrum (ESI) *m*/*z:* 474 (M+H)⁺

(Example X-27) 4-(((3-Ethoxy-4-isopropoxyphenyl)-(N'-methyl-N'-phenylhydrazinocarbonyl)methyl)amino)benzamidine trifluoroacetate 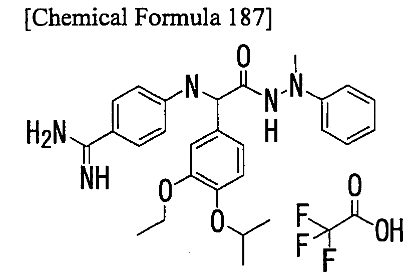 Mass spectrum (ESI) *m*/*z*: 476 (M+H)⁺

(Example X-28) 4-(((3-Ethoxy-4-isopropoxyphenyl)-(N'-quinolin-2-ylhydrazinocarbonyl)methyl)amino)benzamidine trifluoroacetate 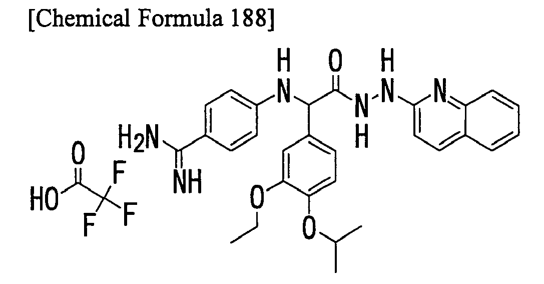 Mass spectrum (ESI) *m*/*z*: 513 (M+H)⁺

(Example X-29) 4-(((3,5-Bisallyloxyphenyl)-(N'-phenylhydrazinocarbonyl)methyl)amino)benzamidine trifluoroacetate 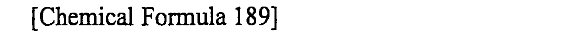 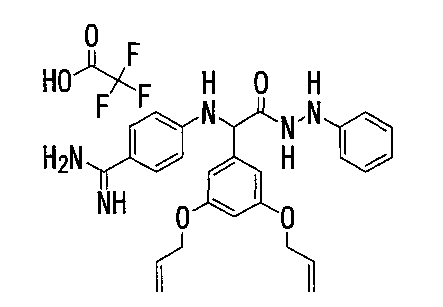 Mass spectrum (ESI) *m*/*z*: 472 (M+H)⁺

(Example X-30) 4-(((N'-Phenylhydrazinocarbonyl)-(3,4,5-trisallyloxyphenyl)methyl)amino)benzamidine trifluoroacetate 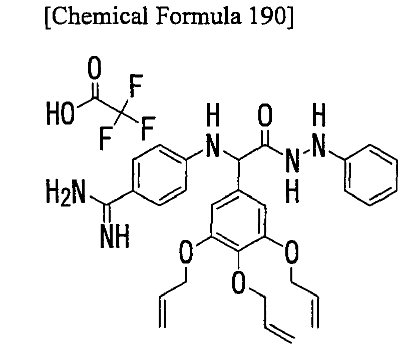 Mass spectrum (ESI) *m*/*z*: 528 (M+H)⁺

(Example X-31) 4-(((4-Allyloxy-3-bromophenyl)-(N'-phenylhydrazinocarbonyl)methyl)amino)benzamidine trifluoroacetate 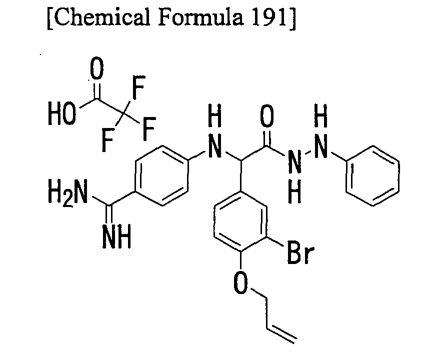 Mass spectrum (ESI) *m*/*z*: 494 (M+H)⁺

(Example X-32) 4-(((3,5-Bisallyloxyphenyl)-(N'-(2-methoxyphenyl)hydrazinocarbonyl)methyl)amino)benzamidine trifluoroacetate 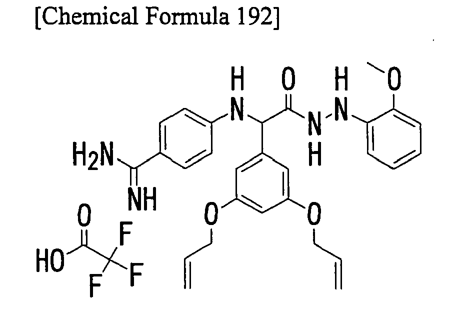 Mass spectrum (ESI) *m*/*z:* 502 (M+H)⁺

(Example X-33) 4-(((3-Ethoxy-4-hydroxyphenyl)-(N'-phenylhydrazinocarbonyl)methyl)amino)benzamidine trifluoroacetate 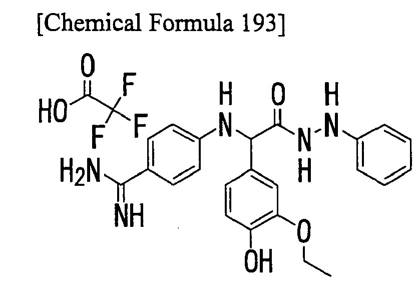 Mass spectrum (ESI) *m*/*z:* 420 (M+H)⁺

(Example X-34) 4-(((2-Benzyloxy-4,5-dimethoxyphenyl)-(N'-phenylhydrazinocarbonyl)methyl)amino)benzamidine trifluoroacetate 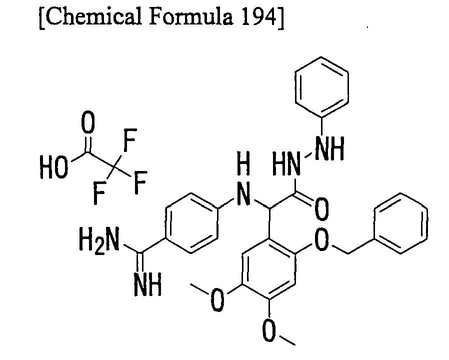 Mass spectrum (ESI) *m*/*z*: 526 (M+H)⁺

(Example X-35) 4-(((5-Ethoxy-4-isopropoxy-2-(pyridin-2-ylmethoxy)phenyl)-(N'-phenylhydrazinocarbonyl)methyl)amino)benzamidine trifluoroacetate 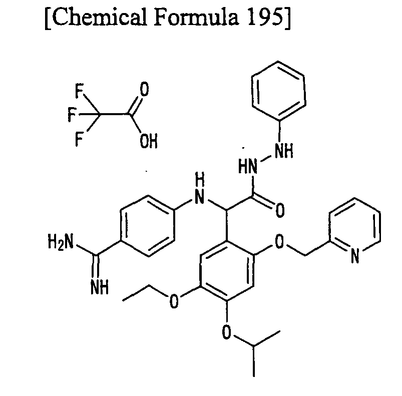 Mass spectrum (ESI) *m*/*z*: 569 (M+H)⁺

(Example X-36) 4-(((3,4-Dimethoxyphenyl)-(N'-pyridin-2-ylhydrazinocarbonyl)methyl)amino)benzamidine trifluoroacetate 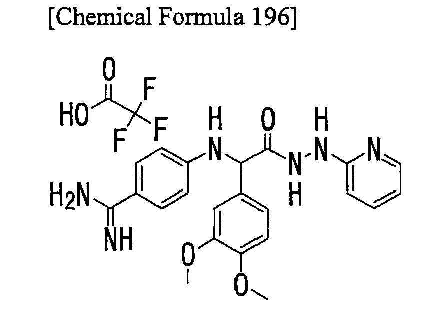 Mass spectrum (ESI) *m*/*z*: 421 (M+H)⁺

(Example X-37) 2-(N'-(2-(3,4-Bisallyloxyphenyl)-2-(4-carbamimidoylphenylamino)acetyl)hydrazino)benzoic acid trifluoroacetate 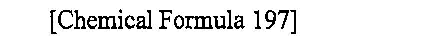 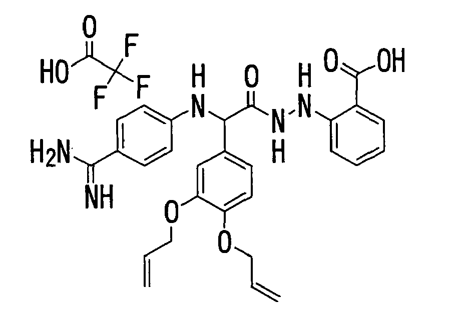 Mass spectrum (ESI) *m*/*z:* 516 (M+H)⁺

(Example X-38) 2-(N'-(2-(4-Carbamimidoylphenylamino)-2-(3,4-diethoxyphenyl)acetyl)hydrazino)benzoic acid trifluoroacetate 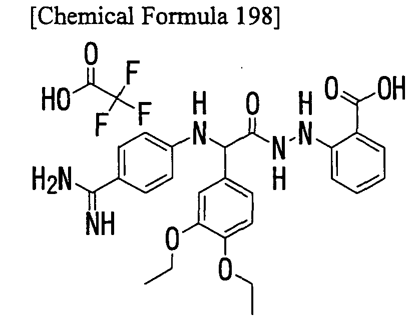 Mass spectrum (ESI) *m*/*z:* 492 (M+H)⁺

(Example X-39) 2-(N'-(2-(4-Carbamimidoylphenylamino)-2-(3,4-dimethoxyphenyl)acetyl)hydrazino)benzoic acid trifluoroacetate 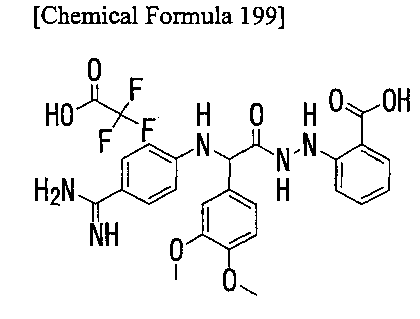 Mass spectrum (ESI) *m*/*z*: 464 (M+H)⁺

(Example X-40) 4-(((N'-Pyridin-2-ylhydrazinocarbonyl)-(3,4,5-trimethoxyphenyl)methyl)amino)benzamidine trifluoroacetate 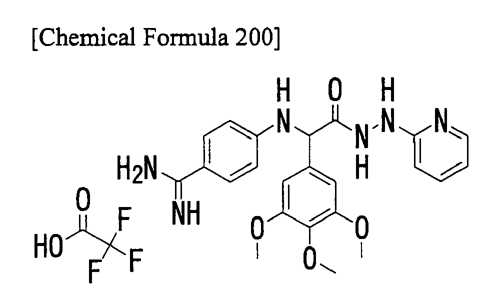 Mass spectrum (ESI) *m*/*z*: 451 (M+H)⁺

(Example X-41) 4-(N'-(2-(4-Carbamimidoylphenylamino)-2-(3-ethoxy-4-isopropoxyphenyl)acetyl)hydrazino)benzoic acid trifluoroacetate 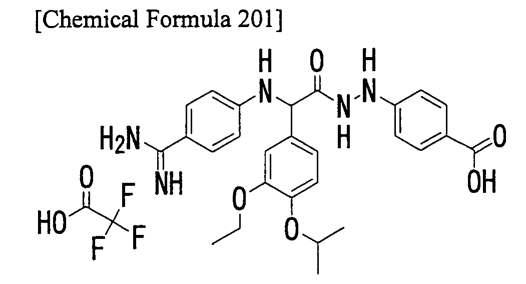 Mass spectrum (ESI) *m*/*z:* 507 (M+H)⁺

(Example X-42) 4-(((3-Ethoxy-4-isopropoxyphenyl)-(N'-(4-trifluoromethylnyrimidin-2-yl)hydrazinocarbonyl)methyl)amino)benzamidine trifluoroacetate 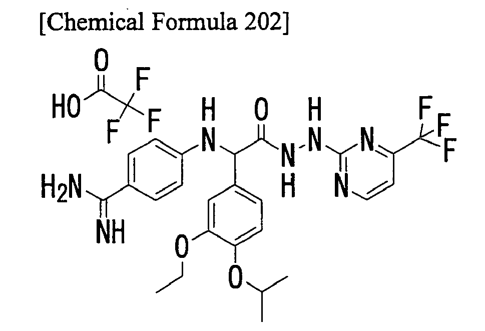 Mass spectrum (ESI) *m*/*z:* 532 (M+H)⁺

(Example X-43) 4-(((4-Allyloxy-3,5-dimethylphenyl)-(N'-phenylhydrazinocarbonyl)methyl)amino)benzamidine trifluoroacetate 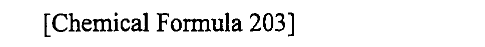 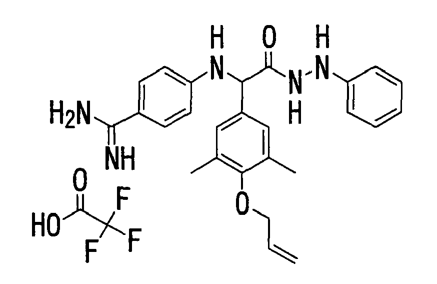 Mass spectrum (ESI) *m*/*z:* 444 (M+H)⁺

(Example X-44) 4-(((4-Hydroxy-3,5-dimethoxyphenyl)-(N'-pyridin-2-ylhydrazinocarbonyl)methyl)amino)benzamidine trifluoroacetate 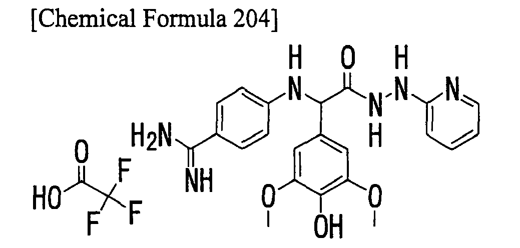 Mass spectrum (ESI) *m*/*z:* 437 (M+H)⁺

(Example X-45) 2-(N'-(2-(4-Carbamimidoylphenylamino)-2-(4-hydroxy-3,5-dimethoxyphenyl)acetyl)hydrazino)benzoic acid trifluoroacetate 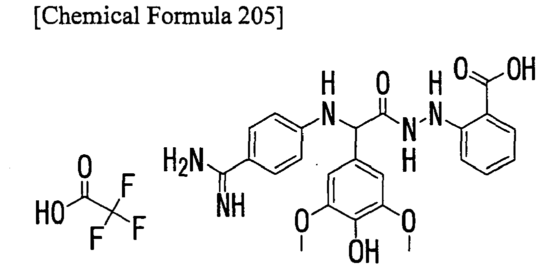 Mass spectrum (ESI) *m*/*z*: 480 (M+H)⁺

(Example X-46) 4-(((2-Ethoxy-4,5-dimethoxyphenyl)-(N'-phenylhydrazinocarbonyl)methyl)amino)benzamidine trifluoroacetate 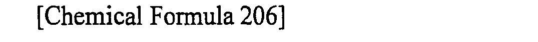 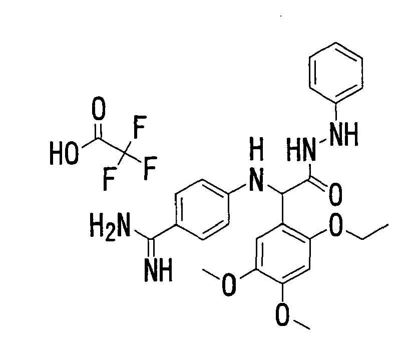 Mass spectrum (ESI) *m*/*z*: 464 (M+H)⁺

(Example X-47) 4-(((2-(2-Butynyloxy)-4,5-dimethoxyphenyl)-(N'-phenylhydrazinocarbonyl)methyl)amino)benzamidine trifluoroacetate 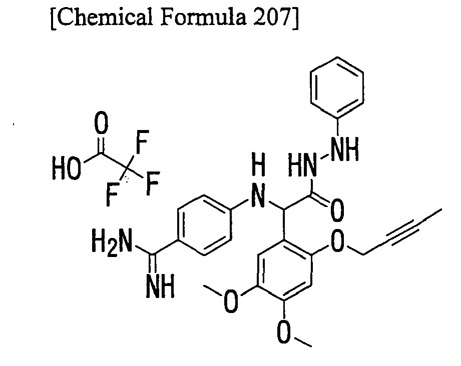 Mass spectrum (ESI) *m*/*z:* 488 (M+H)⁺

(Example X-48) 4-(((4,5-Dimethoxy-2-propoxyphenyl)-(N'-phenylhydrazinocarbonyl)methyl)amino)benzamidine trifluoroacetate 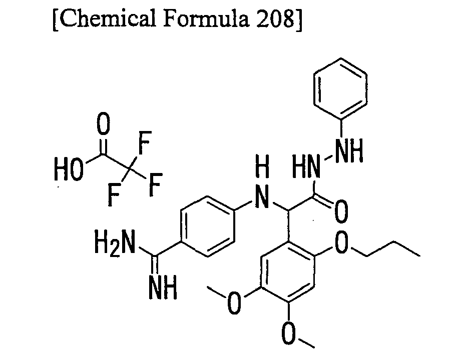 Mass spectrum (ESI) *m*/*z:* 478 (M+H)⁺

(Example X-49) 2-(4-Carbamimidoylphenylamino)-2-(3-ethoxy-4-isopropoxyphenyl)-N-(2-methyl-2,3-dihydroindol-1-yl)acetamide trifluoroacetate 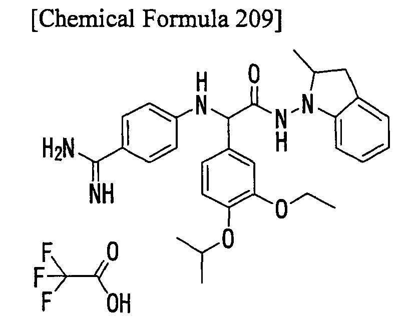 Mass spectrum (ESI) *m*/*z*: 502 (M+H)⁺

(Example X-50) 4-(((N'-(2.5-Dichlorophenyl)hydrazinocarbonyl)-(3-ethoxy-4-isopropoxyphenyl)metyl)amino)benzamidine trifluoroacetate 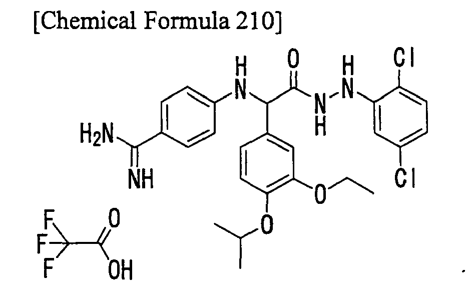 Mass spectrum (ESI) *m*/*z:* 530 (M+H)⁺

(Example X-51) 4-(((N'-Cyclohexylhydrazinocarbonyl)-(3-ethoxy-4-isopropoxyphenyl)methyl)amino)benzamidine trifluoroacetate 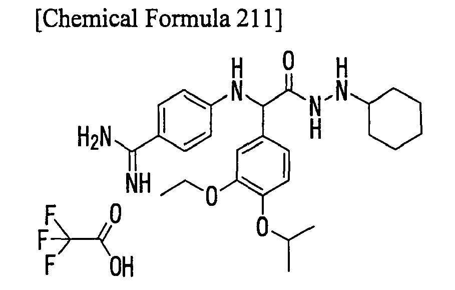 Mass spectrum (ESI) *m*/*z*: 468 (M+H)⁺

(Example X-52) 4-(((3-Ethoxy-4-isopropoxyphenyl)-(N'-methylhydrazinocarbonyl)methyl)amino)benzamidine trifluoroacetate 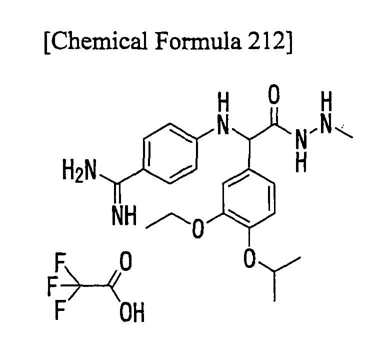 Mass spectrum (ESI) *m*/*z:* 400 (M+H)⁺

(Example X-53) 4-(((3-Ethoxy-4-isopropoxyphenyl)-N-methylhydrazinocarbonyl)methyl)amino)benzamidine trifluoroacetate 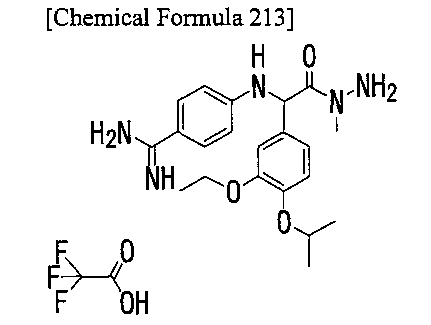 Mass spectrum (ESI) *m*/*z*: 400 (M+H)⁺

(Example X-54) 4-(((3-Ethoxy-4-isopropoxyohenyl)-(N'-ethyl-hydrazinocarbonyl)methyl)amino)benzamidine trifluoroacetate 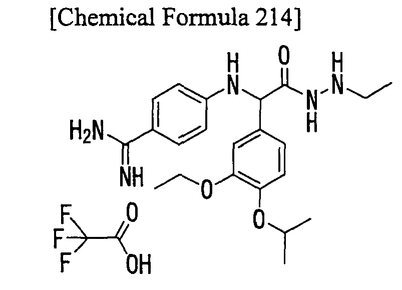 Mass spectrum (ESI) *m*/*z*: 414 (M+H)⁺

(Example X-55) 4-(((3-Ethoxy-4-isopropoxyphenyl)-(N-ethyl-hydrazinocarbonyl)methyl)amino)benzamidine trifluoroacetate 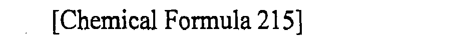 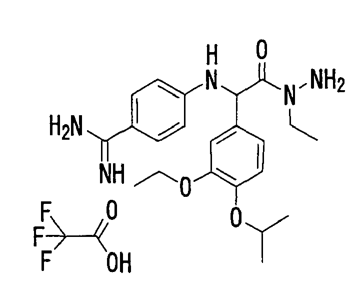 Mass spectrum (ESI) *m*/*z*: 414 (M+H)⁺

(Example X-56) 4-(2-(N'-(6-Dimethylaminopyridine-3-carbonyl)hydrazino)-1-(3-ethoxy-4-isopropoxyphenyl)-2-oxoethylamino)benzamidine trifluoroacetate 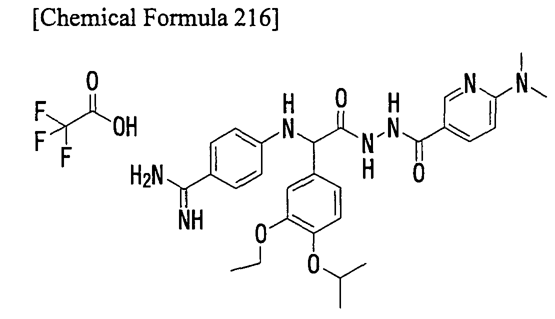 Mass spectrum (ESI) *m*/*z*: 534 (M+H)⁺

(Example X-57) 4-(1-(3-Ethoxy-4-isopropoxyphenyl)-2-(N'-(4-methoxy-2-methylpyridine-3-carbonyl)hydrazino)-2-oxoethylamino)benzamidine trifluoroacetate 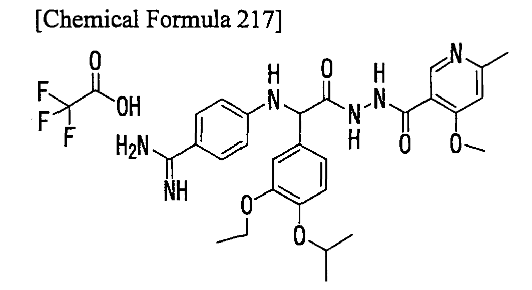 Mass spectrum (ESI) *m*/*z:* 535 (M+H)⁺

(Example X-58) N-(4-(N'-(2-(4-Carbamimidoylphenylamino)-2-(3-ethoxy-4-isopropoxyphenyl)acetyl)hydrazinocarbonyl)pyridin-3-yl)acetamide trifluoroacetate 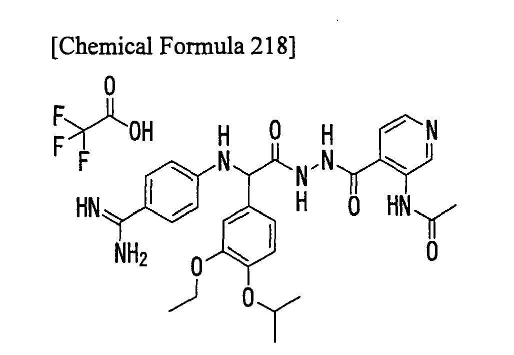 Mass spectrum (ESI) *m*/*z*: 548 (M+H)⁺

(Example X-59) 4-(1-(3-Ethoxy-4-isopropoxycarbonylphenyl)-2-(N'-(1-methyl-2-oxo-1,2-dihydropyridine-3-carbonyl)hydrazino)-2-oxoethylamino)benzamidine trifluoroacetate 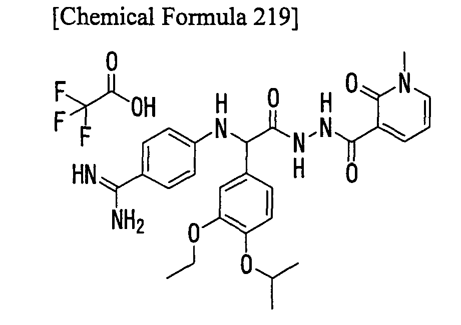 Mass spectrum (ESI) *m*/*z*: 521 (M+H)⁺

(Example X-60) 4-(2-(N'-(3-Dimethylaminopyridine-4-carbonyl)hydrazino)-1-(3-ethoxy-4-isopropoxyphenyl)-2-oxoethylamino)benzamidine trifluoroacetate 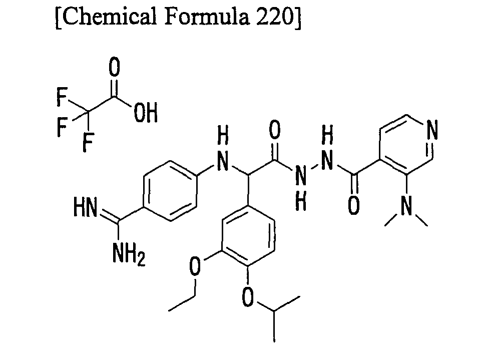 Mass spectrum (ESI) *m*/*z:* 534 (M+H)⁺

(Example X-61) 4-(2-(N'-(3-Cyanopyridine-4-carbonyl)hydrazino)-1-(3-ethoxy-4-isopropoxyphenyl)-2-oxoethylamino)benzamidine trifluoroacetate 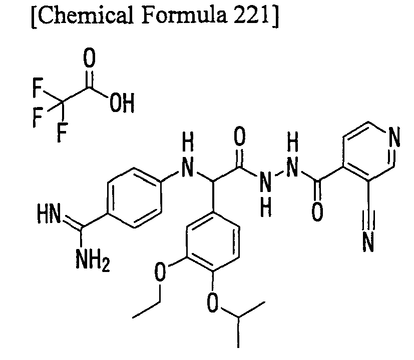 Mass spectrum (ESI) *m*/*z:* 516 (M+H)⁺

(Example X-62) 4-(1-(3-Ethoxy-4-isopropoxyphenyl)-2-(N'-(5-methylpyridine-2-carbonyl)hydrazino)-2-oxoethylamino)benzamidine trifluoroacetate 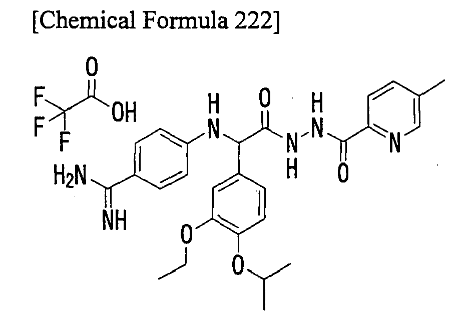 Mass spectrum (ESI) *m*/*z:* 505 (M+H)⁺

(Example X-63) 4-(1-(3-Ethoxy-4-isopropoxyphenyl)-2-(N'-(5-bromopyridine-2-carbonyl)hydrazino)-2-oxoethylamino)benzamidine trifluoroacetate 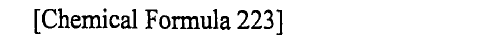 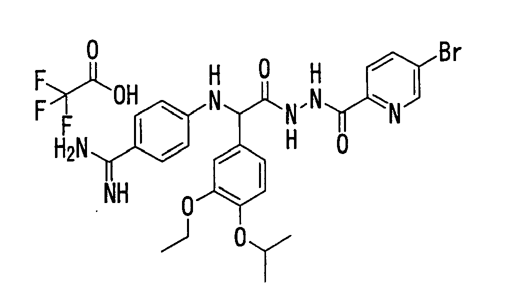 Mass spectrum (ESI) *m*/*z*: 569 (M+H)⁺

(Example X-64) 4-(((3-Ethoxy-4-isopropoxyphenyl)-(N'-methyl-N'-pyridin-2-ylhydrazinocarbonyl)methyl)amino)benzamidine trifluoroacetate 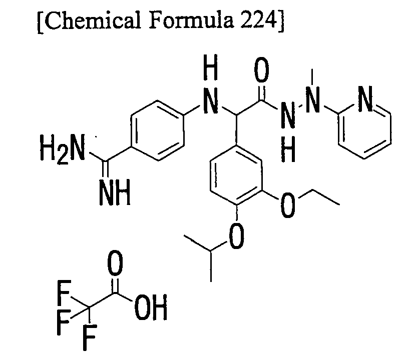 Mass spectrum (ESI) *m*/*z*: 477 (M+H)⁺

(Example X-65) 3-(N'-(2-(4-Carbamimidoylphenylamino)-2-(3-ethoxy-4-isopropoxyphenyl)acetyl)hydrazino)benzoic acid trifluoroacetate 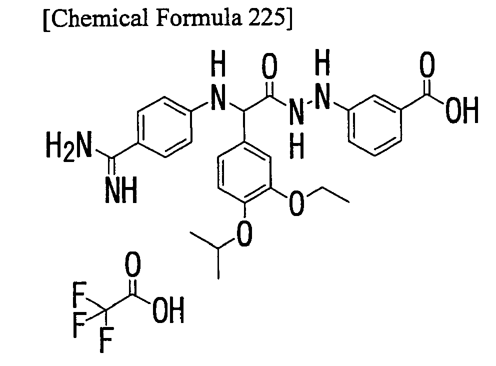 Mass spectrum (ESI) *m*/*z:* 506 (M+H)⁺

(Example X-66) 4-L2-(N'-Benzoylhydrazino)-1-(3-ethoxy-4-isopropoxyphenyl)-2-oxoethylamino)benzamidine trifluoroacetate 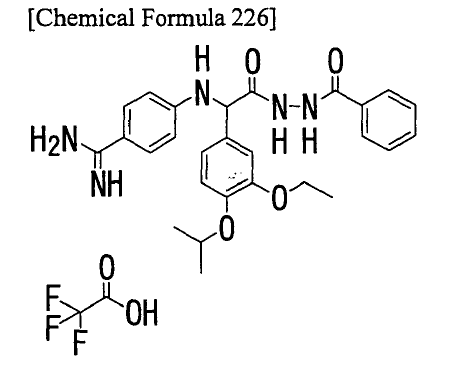 Mass spectrum (ESI) *m*/*z:* 490 (M+H)⁺

(Example X-67) 4-(1-(3-Ethoxy-4-isopropoxyphenyl)-2-oxo-2-(N'-(pyridine-2-carbonyl)hydrazino)ethylamino)benzamidine trifluoroacetate 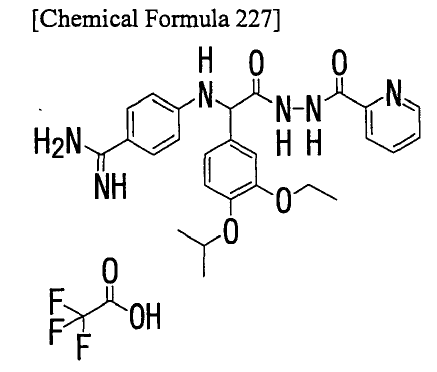 Mass spectrum (ESI) *m*/*z:* 491 (M+H)⁺

(Example X-68) 4-(((N'-(2-Bromophenyl)hydrazinocarbonyl)-(3-ethoxy-4-isopropoxyphenyl)methyl)amino)benzamidine trifluoroacetate 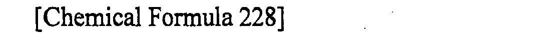 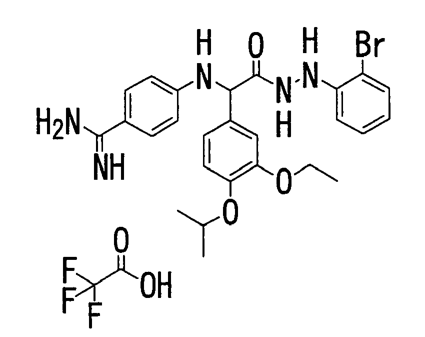 Mass spectrum (ESI) *m*/*z:* 540 (M+H)⁺

(Example X-69) 4-(((N'-(2-Chlorophenyl)hydrazinocarbonyl)-(3-ethoxy-4-isopropoxyphenyl)methyl)amino)benzamidine trifluoroacetate 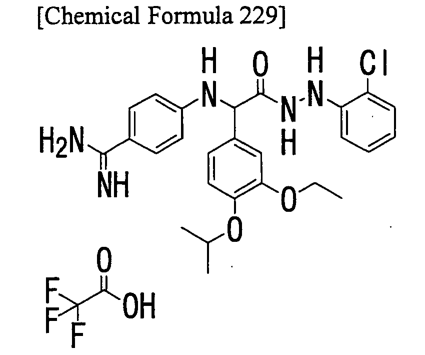 Mass spectrum (ESI) *m*/*z*: 496 (M+H)⁺

(Example X-70) 2-(4-Carbamimidoylphenylamino)-2-(3-ethoxy-4-isopropoxyphenyl)-N-morpholin-4-ylacetamide trifluoroacetate 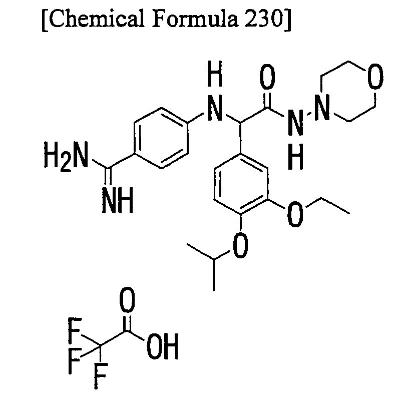 Mass spectrum (ESI) *m*/*z:* 456 (M+H)⁺

(Example X-71) 4-(((3-Ethoxy-4-isopropoxyphenyl)-(N'-(2-trifluoromethylphenyl)hydrazinocarbonyl)methyl)amino)benzamidine trifluoroacetate 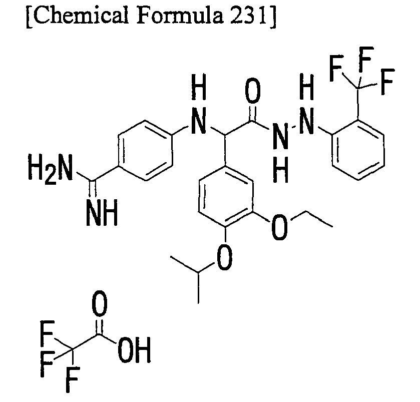 Mass spectrum (ESI) *m*/*z*: 530 (M+H)⁺

(Example X-72) 4-(((3-Ethoxy-4-isopropoxyphenyl)-(N'-(5-nitropyridin-2-yl)hydrazinocarbonyl)methyl)amino)benzamidine trifluoroacetate 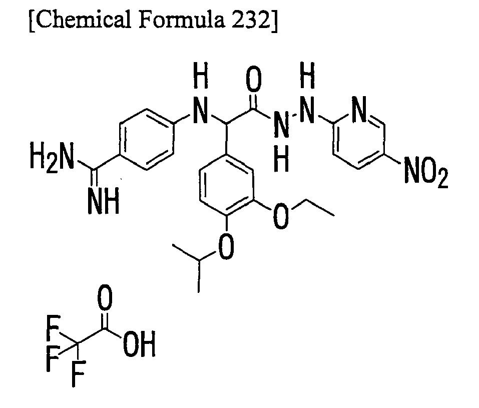 Mass spectrum (ESI) *m*/*z:* 508 (M+H)⁺

(Example X-73) 4-(((3,4-Diisopropoxyphenyl)-(N'-phenylhydrazinocarbonyl)methyl)amino)benzamidine trifluoroacetate 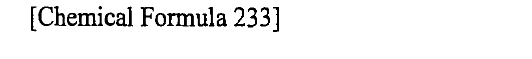 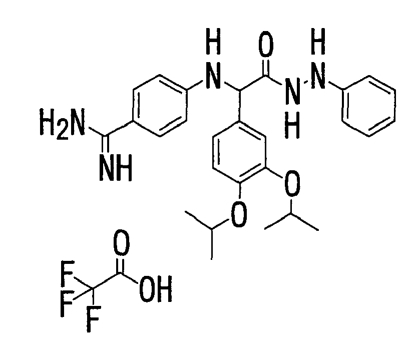 Mass spectrum (ESI) *m*/*z*: 476 (M+H)⁺

(Example X-74) 4-(((3,4-Diisopropoxyphenyl)-(N'-pyridin-2-ylhydrazinocarbonyl)methyl)amino)benzamidine trifluoroacetate 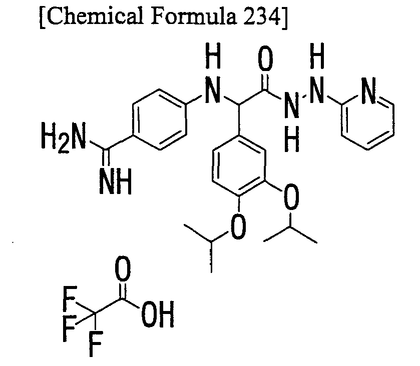 Mass spectrum (ESI) *m*/*z:* 477 (M+H)⁺

(Example X-75) 4-(((3,4-Diisopropoxyphenyl)-(N'-(2-methoxyphenyl)hydrazinocarbonyl)methyl)amino)benzamidine trifluoroacetate 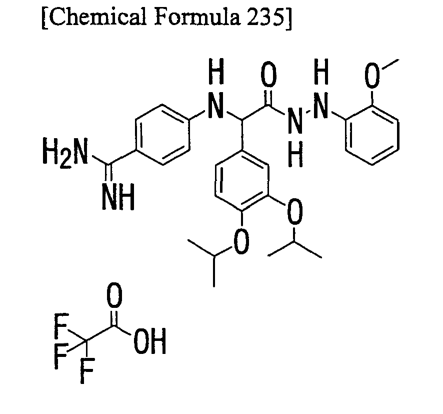 Mass spectrum (ESI) *m*/*z*: 506 (M+H)⁺

(Example X-76) 4-(1-(3,4-Diisopropoxyphenyl)-2-oxo-2-(N'-(pyridine-2-carbonyl)hydrazino)ethylamino)benzamidine trifluoroacetate 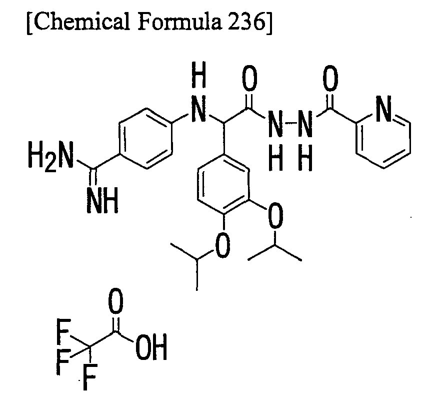 Mass spectrum (ESI) *m*/*z:* 505 (M+H)⁺

(Example X-77) 2-(N'-(2-(4-Carbamimidoylphenylamino)-2-(3,4-diisopropoxyphenyl)acetyl)hydrazino)benzoic acid trifluoroacetate 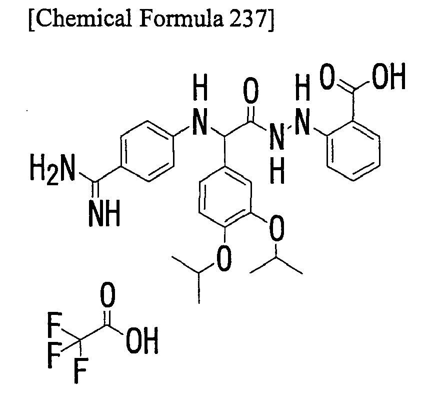 Mass spectrum (ESI) *m*/*z*: 520 (M+H)⁺

(Example X-78) 3-(N'-(2-(4-Carbamimidoylphenylamino)-2-(3-ethoxy-4-isopropoxyphenyl)acetyl)hydrazino)-2-chlorobenzoic acid trifluoroacetate 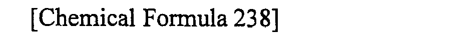 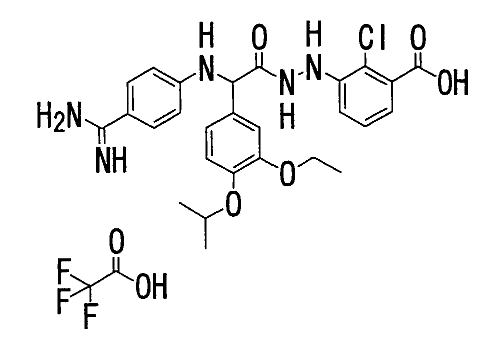 Mass spectrum (ESI) *m*/*z:* 540 (M+H)⁺

(Example X-79) 4-((2-(2-((2-Cyanophenyl)sulfonyl)hydrazinol-1-(3-ethoxy-4-isoprol)oxyphepyl)-2-oxoethyl)amino)benzamidine trifluoroacetate 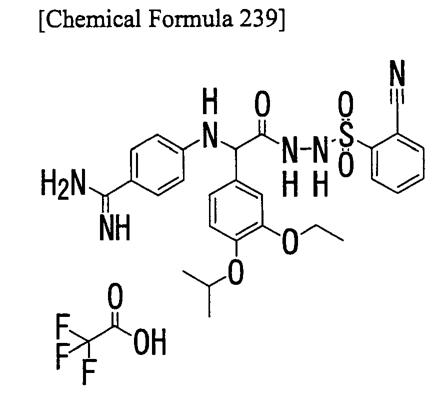 Mass spectrum (ESI) *m*/*z*: 551 (M+H)⁺

(Example X-80) 4-((2-(2-((2-Nitrophenyl)sulfonyl)hydrazino)-1-(3-ethoxy-4-isopropoxyphenyl)-2-oxoethyl)amino)benzamidine trifluoroacetate 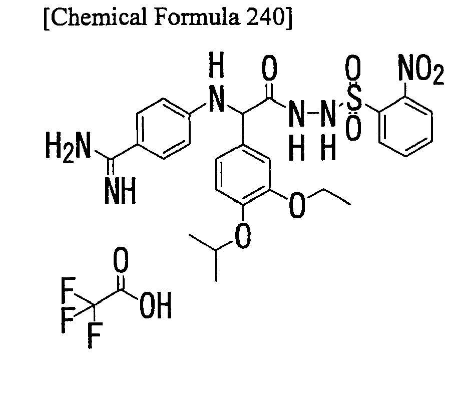 Mass spectrum (ESI) *m*/*z:* 571 (M+H)⁺

(Example X-81) 4-((2-(2-((2-Fluorophenyl)sulfonyl)hydrazino)-1-(3-ethoxy-4-isopropoxyphenyl)-2-oxoethyl)amino)benzamidine trifluoroacetate 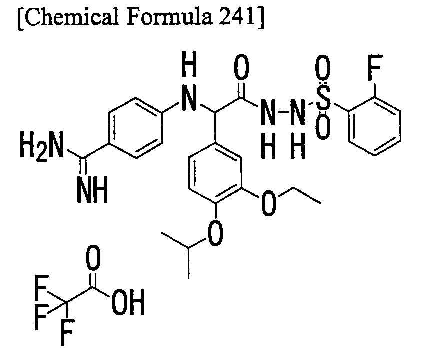 Mass spectrum (ESI) *m*/*z*: 544 (M+H)⁺

(Example X-82) 4-((2-(2-((2-Bromophenyl)sulfonyl)hydrazino)-1-(3-ethoxy-4-isopropoxyphenyl)-2-oxoethyl)amino)benzamidine trifluoroacetate 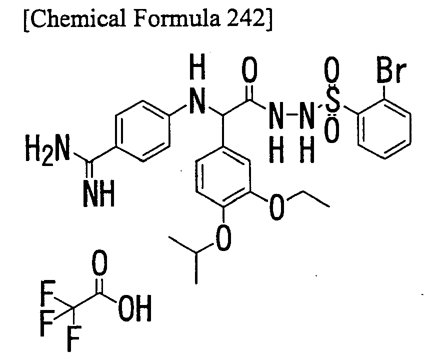 Mass spectrum (ESI) *m*/*z:* 604 (M+H)⁺

(Example X-83) 4-((2-(2-((2-Methylphenyl)sulfonyl)hydrazino)-1-(3-ethoxy-4-isopropoxyphenyl)-2-oxoethyl)amino)benzamidine trifluoroacetate 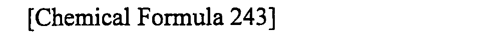 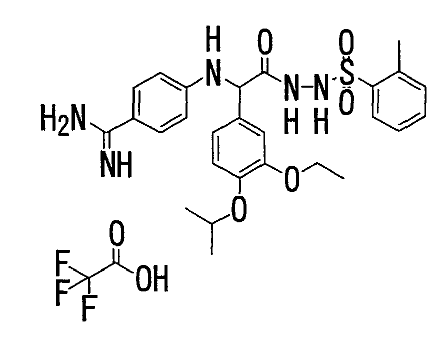 Mass spectrum (ESI) *m*/*z:* 540 (M+H)⁺

(Example X-84) N-Benzotriazol-1-yl-2-(4-carbamimidoylphenylamino)-2-(3-ethoxy-4-isopropoxyphenyl)acetamide trifluoroacetate 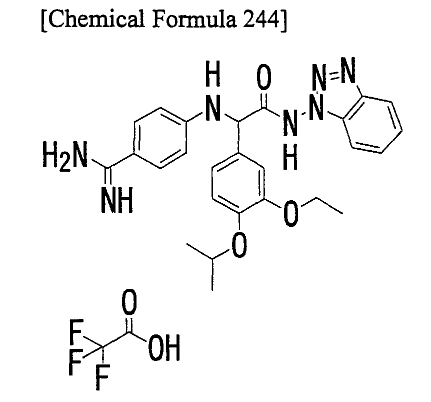 Mass spectrum (ESI) *m*/*z:* 488 (M+H)⁺

(Example X-85) 4-((2-(2-((2,4-Difluorophenyl)sulfonyl)hydrazino)-1-(3-ethoxy-4-isopropoxyphenyl)-2-oxoethyl)amino)benzamidine trifluoroacetate 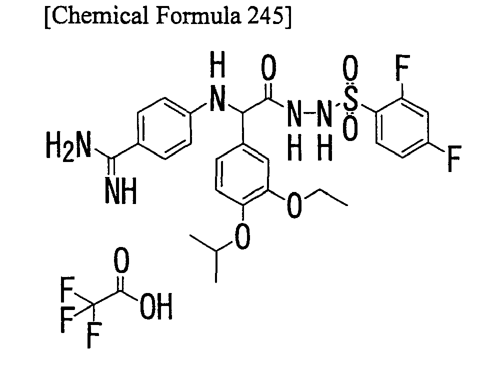 Mass spectrum (ESI) *m*/*z:* 562 (M+H)⁺

(Example X-86) 4-((2-(2-((3-Fluorophenyl)sulfonyl)hydrazino)-1-(3-ethoxy-4-isopropoxyphenyl)-2-oxoethyl)amino)benzamidine trifluoroacetate 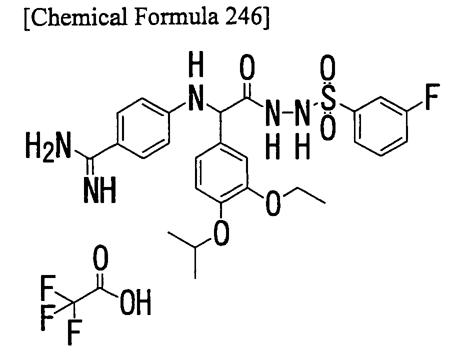 Mass spectrum (ESI) *m*/*z:* 544 (M+H)⁺

(Example X-87) 4-((2-(2-((4-Fluorophenyl)sulfonyl)hydrazino)-1-(3-ethoxy-4-isopropoxyphenyl)-2-oxoethyl)amino)benzamidine trifluoroacetate 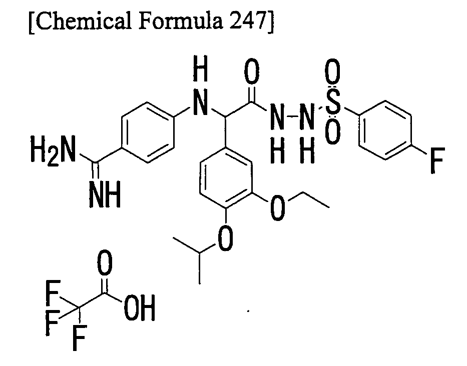 Mass spectrum (ESI) *m*/*z*: 544 (M+H)⁺

(Example X-88) 4-((2-(2-((3-Methoxyphenyl)sulfonyl)hydrazino)-1-(3-ethoxy-4-isopropoxyuhenyl)-2-oxoethyl)amino)benzamidine trifluoroacetate 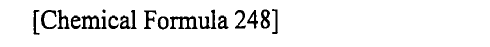 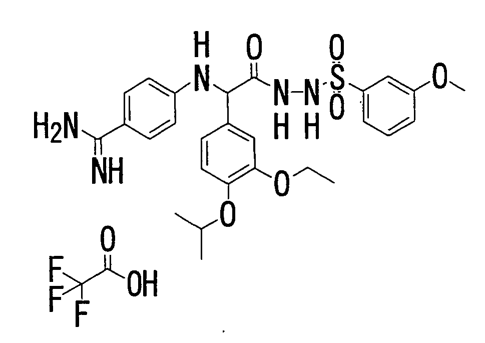 Mass spectrum (ESI) *m*/*z:* 556 (M+H)⁺

(Example X-89) 4-((2-(2-((4-Methoxyphenyl)sulfonyl)hydrazino)-1-(3-ethoxy-4-isopropoxyphenyl)-2-oxoethyl)amino)benzamidine trifluoroacetate 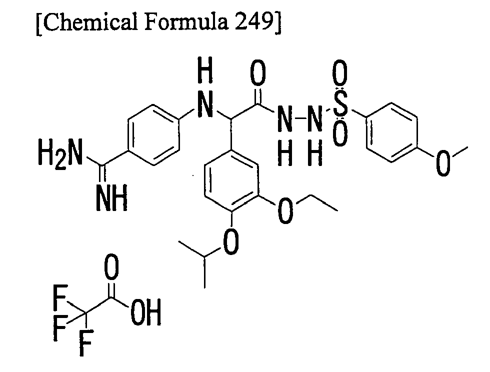 Mass spectrum (ESI) *m*/*z:* 556 (M+H)⁺

(Example X-90) 4-((2-(2-((3-Bromophenyl)sulfonyl)hydrazino)-1-(3-ethoxy-4-isopropoxyphenyl)-2-oxoethyl)amino)benzamidine trifluoroacetate 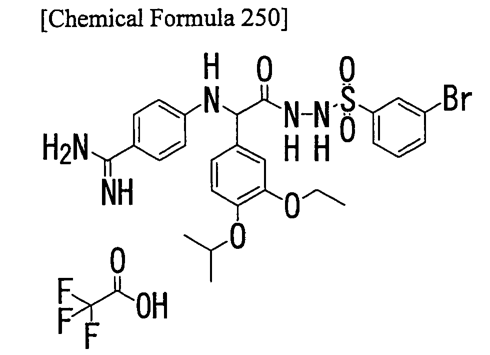 Mass spectrum (ESI) *m*/*z:* 604 (M+H)⁺

(Example X-91) 4-((2-(2-((4-Bromophenyl)sulfonyl)hydrazino)-1-(3-ethoxy-4-isopropoxyphenyl)-2-oxoethyl)amino)benzamidine trifluoroacetate 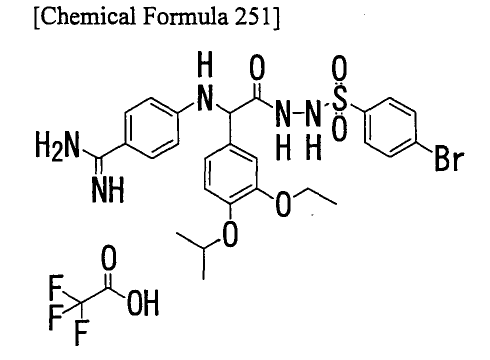 Mass spectrum (ESI) *m*/*z:* 604 (M+H)⁺

(Example X-92) 4-((2-(2-((3-Cyanophenyl)sulfonyl)hydrazino)-1-(3-ethoxy-4-isopropoxyphenyl)-2-oxoethyl)amino)benzamidine trifluoroacetate 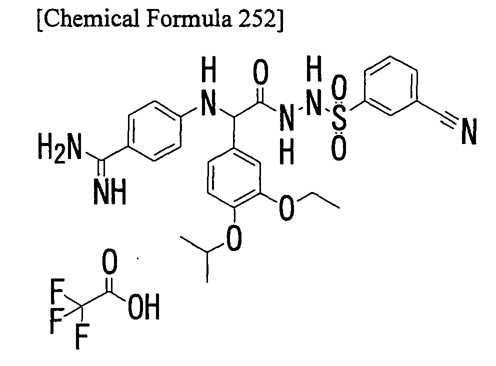 Mass spectrum (ESI) *m*/*z:* 551 (M+H)⁺

(Example X-93) 4-((2-(2-((4-Nitrophenyl)sulfonyl)hydrazino)-1-(3-ethoxy-4-isopropoxyphenyl)-2-oxoethyl)amino)benzamidine trifluoroacetate 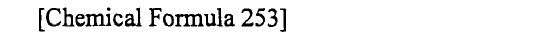 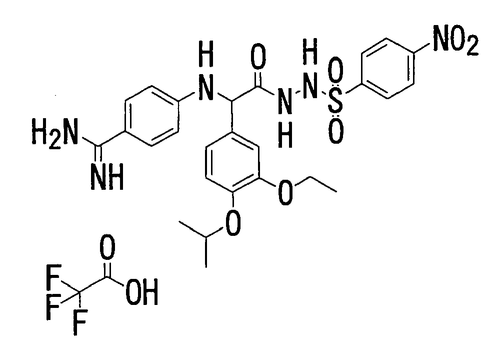 Mass spectrum (ESI) *m*/*z:* 571 (M+H)⁺

(Example X-94) 4-((2-(2-((4-Acetylphenyl)sulfonyl)hydrazino)-1-(3-ethoxy-4-isopropoxyphenyl)-2-oxoethyl)amino)benzamidine trifluoroacetate 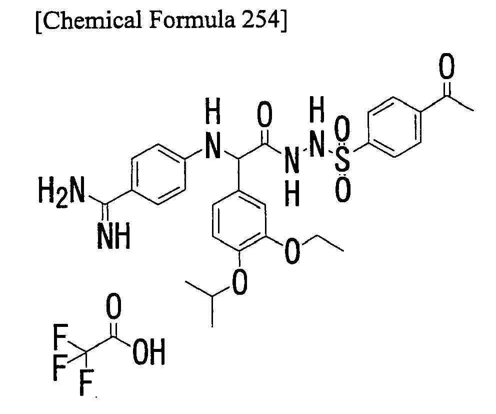 Mass spectrum (ESI) *m*/*z*: 568 (M+H)⁺

(Example X-95) 3-((2-(2-(4-Carbamimidoylphenylamino)-2-(3-ethoxy-4-isopropoxyphenyl)acetyl)hydrazino)sulfonyl)benzamide trifluoroacetate 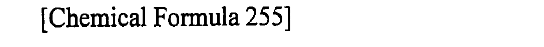 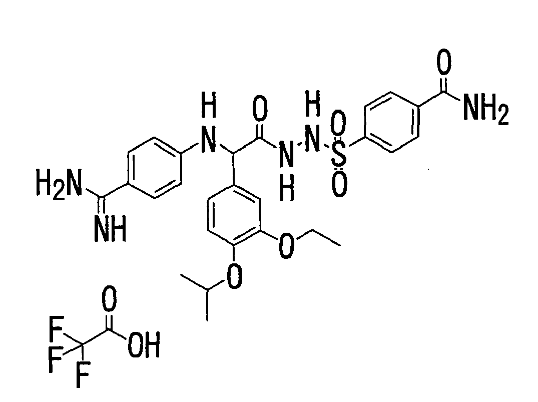 Mass spectrum (ESI) *m*/*z*: 569 (M+H)⁺

(Example X-96) 4-(1-(4-Hydroxy-3,5-dimethoxyphenyl)-2-oxo-2-(N'-(pyridine-3-carbonyl)hydrazino)ethylamino)benzamidine trifluoroacetate 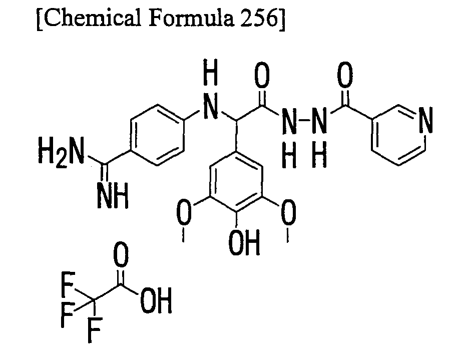 Mass spectrum (ESI) *m*/*z*: 465 (M+H)⁺

(Example X-97) 4-(1-(2-Chloro-3,4-dimethoxyphenyl)-2-oxo-2-(N'-(pyridine-2-carbonyl)hydrazino)ethylamino)benzamidine trifluoroacetate 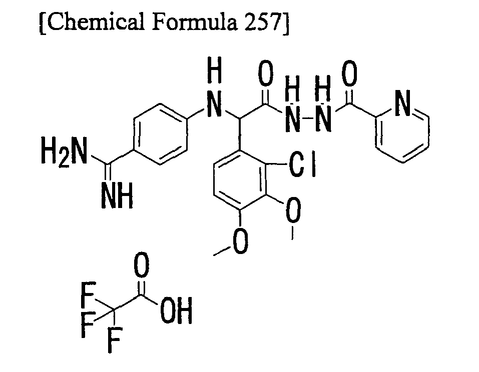 Mass spectrum (ESI) *m*/*z*: 483 (M+H)⁺

(Example X-98) 4-(1-(2-Chloro-3,4-dimethoxyphenyl)-2-oxo-2-(N'-(pyridine-3-carbonyl)hydrazino)ethylamino)benzamidine trifluoroacetate 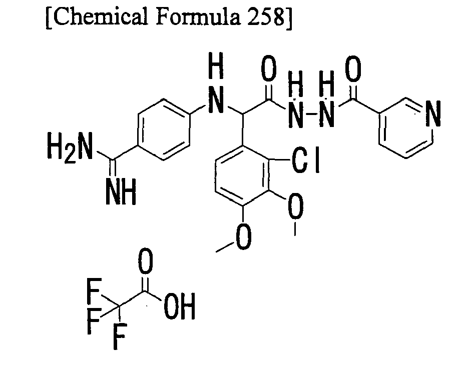 Mass spectrum (ESI) *m*/*z:* 483 (M+H)⁺

(Example X-99) 4-(1-(2-Chloro-3,4-dimethoxyphenyl)-2-oxo-2-(N'-(pyridine-4-carbonyl)hydrazino)ethylamino)benzamidine trifluoroacetate 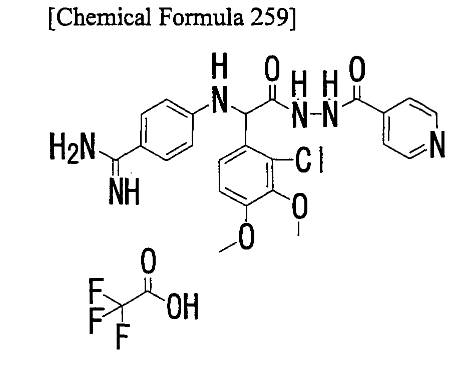 Mass spectrum (ESI) *m*/*z*: 483 (M+H)⁺

(Example X-100) 4-(2-(N'-Benzoylhydrazino)-1-(2-chloro-3,4-dimethoxyphenyl)-2-oxoethylamino)benzamidine trifluoroacetate 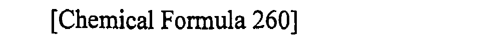 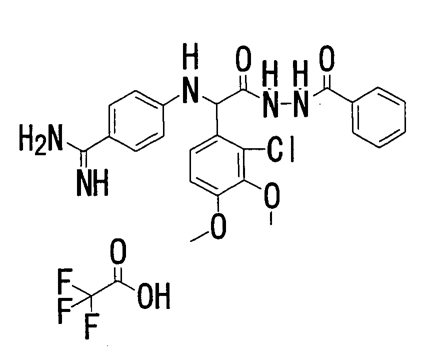 Mass spectrum (ESI) *m*/*z*: 482 (M+H)⁺

(Example X-101) 2-(N'-(2-(4-Carbamimidoylphenylamino)-2-(2-chloro-3,4-dimethoxyphenyl)acetyl)hydrazino)nicotinic acid trifluoroacetate 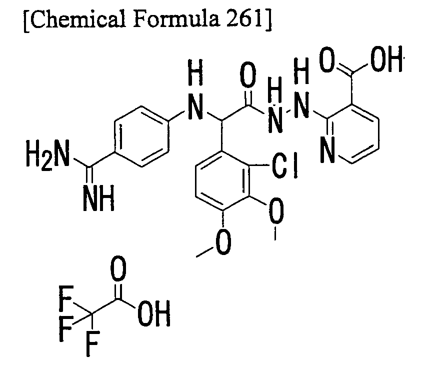 Mass spectrum (ESI) *m*/*z:* 499 (M+H)⁺

(Example X-102) 4-(1-(2-Chloro-3,4-dimethoxyphenyl)-2-(N'-(furan-2-carbonyl)hydrazino)-2-oxoethylamino)benzamidine trifluoroacetate 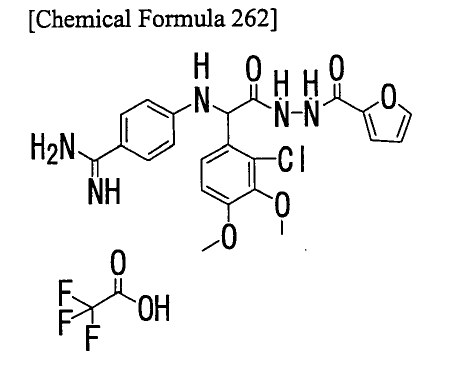 Mass spectrum (ESI) *m*/*z*: 472 (M+H)⁺

(Example X-103) 4-(((3-Ethoxy-4-isopropoxyphenyl)-(N'-thiobenzoylhydrazinocarbonyl)methyl)amino)benzamidine trifluoroacetate 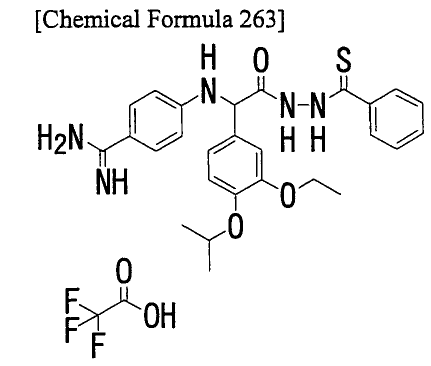 Mass spectrum (ESI) *m*/*z*: 506 (M+H)⁺

(Example X-104) 4-(1-(3-(3-Dimethylamino-2,2-dimethylpropoxy)-5-ethylphenyl)-2-oxo-2-(N'-(pyridine-3-carbonyl)hydrazino)ethylamino)benzamidine trifluoroacetate 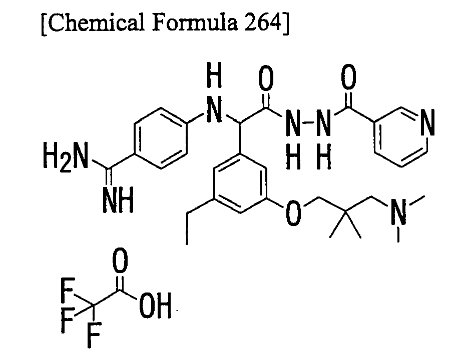 Mass spectrum (ESI) *m*/*z:* 546 (M+H)⁺

(Example X-105) 4-(1-(4-Isopropoxy-3-methoxyphenyl)-2-oxo-2-(N'-(pyridine-2-carbonyl)hydrazino)ethylamino)benzamidine trifluoroacetate 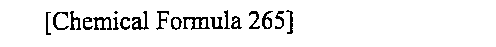 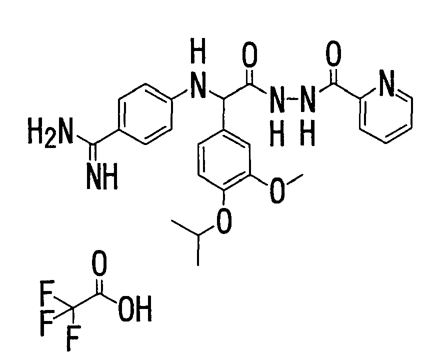 Mass spectrum (ESI) *m*/*z:* 477 (M+H)⁺

(Example X-106) 4-(1-(4-Isopropoxy-3-methoxyphenyl)-2-oxo-2-(N'-(pyridine-3-carbonyl)hydrazino)ethylamino)benzamidine trifluoroacetate 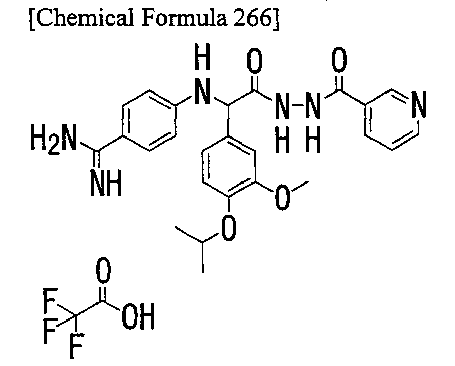 Mass spectrum (ESI) *m*/*z:* 477 (M+H)⁺

(Example X-107) 4-(1-(5-Ethoxy-2-fluoro-4-isopropoxyphenyl)-2-oxo-2-(N'-(pyridine-2-carbonyl)hydrazino)ethylamino)benzamidine trifluoroacetate 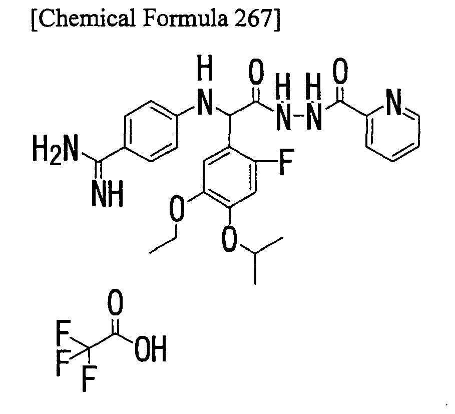 Mass spectrum (ESI) *m*/*z:* 509 (M+H)⁺

(Example X-108) 4-(1-(5-Ethoxy-2-fluoro-4-isoproxyphenyl)-2-oxo-2-(N'-(pyridine-3-carbonyl)hydrazino)ethylamino)benzamidine trifluoroacetate 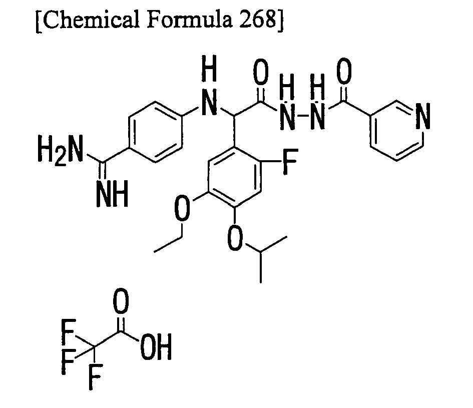 Mass spectrum (ESI) *m*/*z:* 509 (M+H)⁺

(Example X-109) 4-(1-(5-Ethoxy-2-fluoro-4-isopropoxyphenyl)-2-oxo-2-(N'-(pyridine-4-carbonyl)hydrazino)ethylamino)benzamidine trifluoroacetate 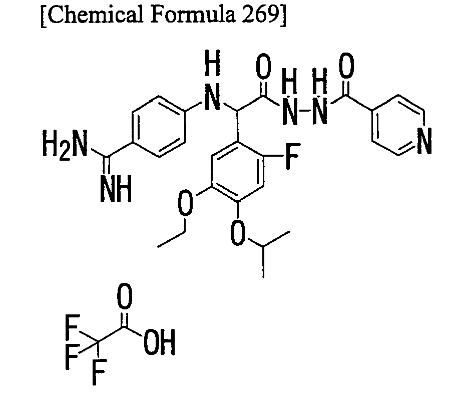 Mass spectrum (ESI) *m*/*z*: 509 (M+H)⁺

(Example X-110) 4-(2-(N'-Benzoylhydrazino)-1-(5-ethoxy-2-fluoro-4-isopropoxyphenyl)-2-oxoethylamino)benzamidine trifluoroacetate 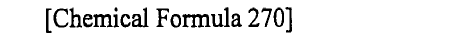 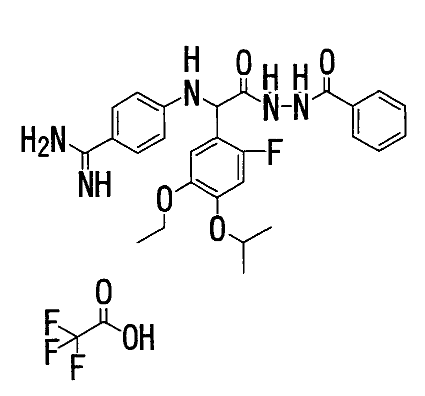 Mass spectrum (ESI) *m*/*z*: 508 (M+H)⁺

(Example X-111) 4-(2-(N'-(2-Chlorobenzoyl)hydrazino)-1-(5-ethoxy-2-fluoro-4-isopropoxyphenyl)-2-oxoethylamino)benzamidine trifluoroacetate 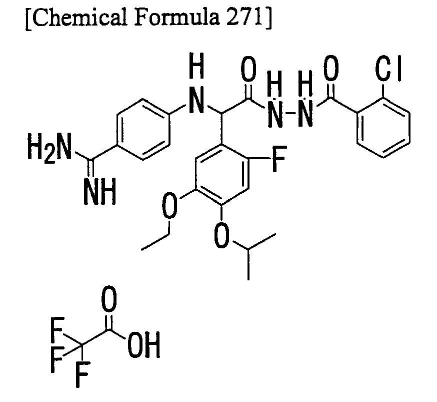 Mass spectrum (ESI) *m*/*z*: 542 (M+H)⁺

(Example X-112) 4-(1-(5-Ethoxy-2-fluoro-4-isopropoxyphenyl)-2-(N'-(furan-2-carbonyl)hydrazino)-2-oxoethylamino)benzamidine trifluoroacetate 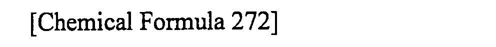 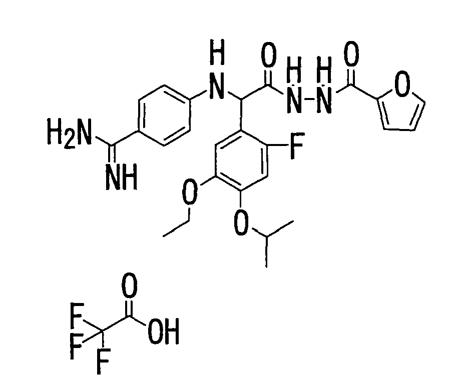 Mass spectrum (ESI) *m*/*z:* 498 (M+H)⁺

(Example X-113) 4-(1-(5-Ethoxy-2-fluoro-4-isopropoxyphenyl)-2-(N'-(4-hydroxybenzoyl)hydrazino)-2-oxoethylamino)benzamidine trifluoroacetate 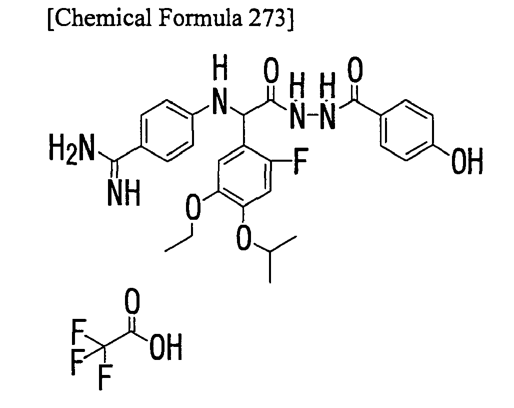 Mass spectrum (ESI) *m*/*z:* 524 (M+H)⁺

(Example X-114) 4-((2-(2-(Phenylsulfonyl)hydrazino)-1-(5-ethoxy-2-fluoro-4-isopropoxyphenyl)-2-oxoethyl)amino)benzamidine trifluoroacetate 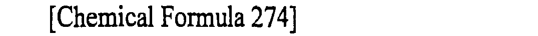 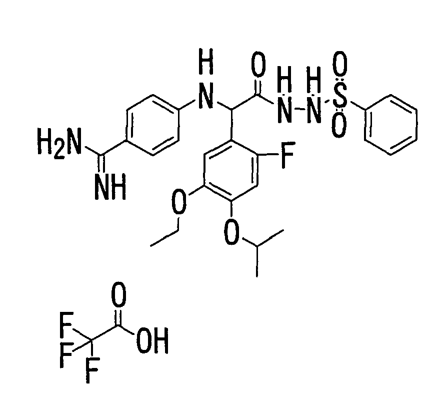 Mass spectrum (ESI) *m*/*z:* 544 (M+H)⁺

(Example X-115) 4-(((3-Ethoxy-4-isopropoxyphenyl)-(N'-o-tolylhydrazinocarbonyl)methyl)amino)benzamidine trifluoroacetate 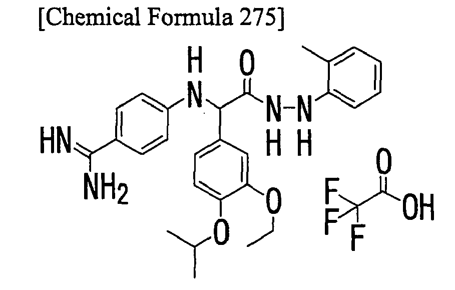 Mass spectrum (ESI) *m*/*z:* 476 (M+H)⁺

(Example X-116) 4-(((N'-(6-Chloropyridazin-3-yl)hydrazinocarbonyl)-(3-ethoxy-4-isopropoxyphenyl)methyl)amino)benzamidine trifluoroacetate 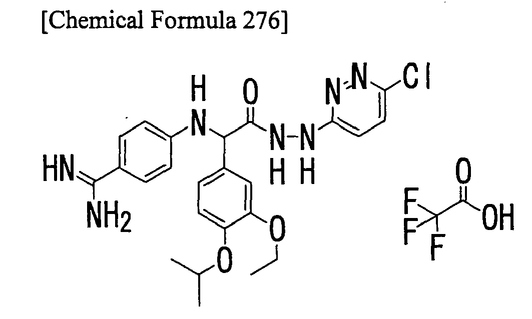 Mass spectrum (ESI) *m*/*z*: 498 (M+H)⁺

(Example X-117) 4-(((N'-(6-Chloropyridin-2-yl)hydrazinocarbonyl)-(3-ethoxy-4-isopropoxyphenyl)methyl)amino)benzamidine trifluoroacetate 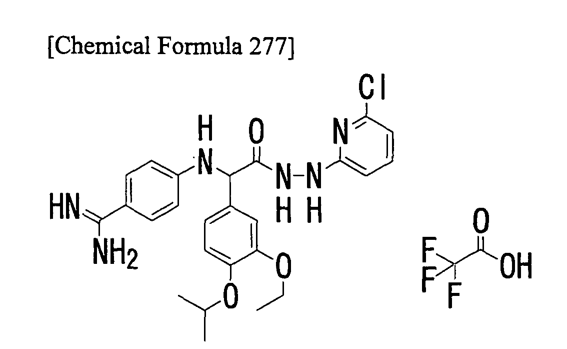 Mass spectrum (ESI) *m*/*z:* 497 (M+H)⁺

(Example X-118) 4-(((N'-(2-Cyanophenyl)hydrazinocarbonyl)-(3-ethoxy-4-isopropoxyphenyl)methyl)amino)benzamidine trifluoroacetate 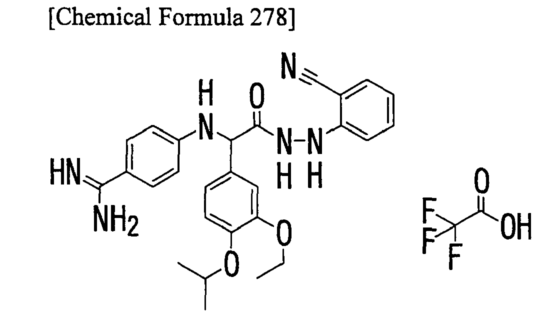 Mass spectrum (ESI) *m*/*z:* 487 (M+H)⁺

(Example X-119) 4-(((3-Ethoxy-4-isopropoxyphenyl)-(N'-(5-trifluoromethylpyridin-2-yl)hydrazinocarbonyl)metyl)amino)benzamidine trifluoroacetate 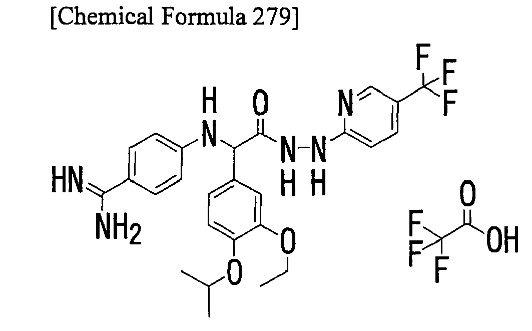 Mass spectrum (ESI) *m*/*z:* 531 (M+H)⁺

(Example X-120) 2-(4-Carbamimidoylphenylamino)-N-(1,3-dioxo-1,3-dihydroisoindol-2-yl)-2-(3-ethoxy-4-isopropoxyphenyl)acetamide trifluoroacetate 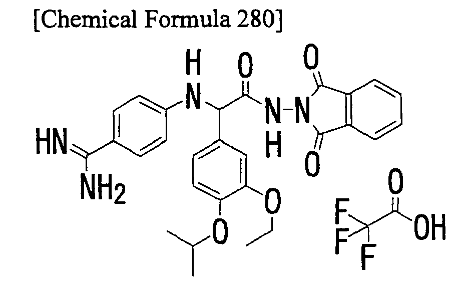 Mass spectrum (ESI) *m*/*z*: 516 (M+H)⁺

(Example X-121) 4-(1-(3-Ethoxy-4-isopropoxyphenyl)-2-(N'-(furan-2-carbonyl)hydrazino)-2-oxoethylamino)benzamidine trifluoroacetate 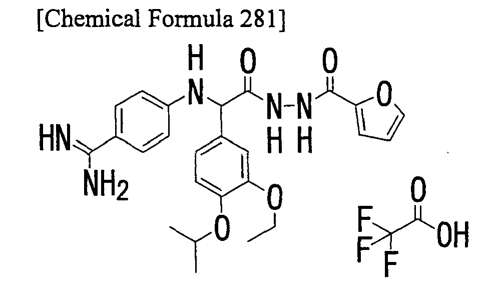 Mass spectrum (ESI) *m*/*z:* 480 (M+H)⁺

(Example X-122) 4-(((3-Ethoxy-4-iso-propoxyphenyl)-(N'-(2,4,6-trimethylphenyl)hydrazinocarbonyl)methyl)amino)benzamidine trifluoroacetate 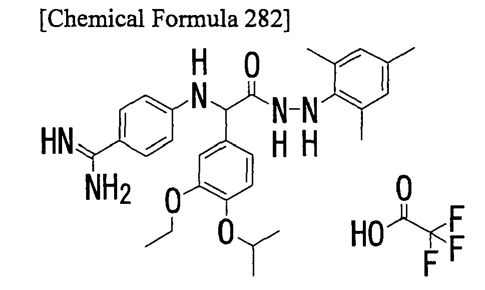 Mass spectrum (ESI) *m*/*z*: 504 (M+H)⁺

(Example X-123) 4-(1-(3-Ethoxy-4-isopropoxyphenyl)-2-(N'-(2-hydroxybenzoyl)hydrazino)-2-oxoethylamino)benzamidine trifluoroacetate 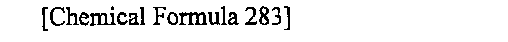 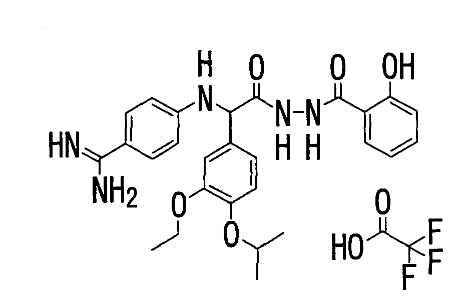 Mass spectrum (ESI) *m*/*z*: 506 (M+H)⁺

(Example X-124) 4-(1-(3-Ethoxy-4-isopropoxyphenyl-2-oxo-2-(N'-phenylacetylhydrazino)ethylamino)benzamidine trifluoroacetate 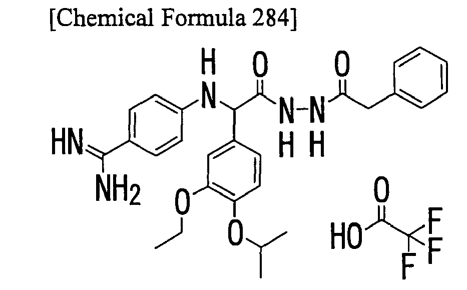 Mass spectrum (ESI) *m*/*z:* 504 (M+H)⁺

(Example X-125) 2-((4-Carbamimidoylphenylamino)-(3-ethoxy-4-isopropoxyphenyl)acetyl)-N-phenylhydrazinecarboxamide trifluoroacetate 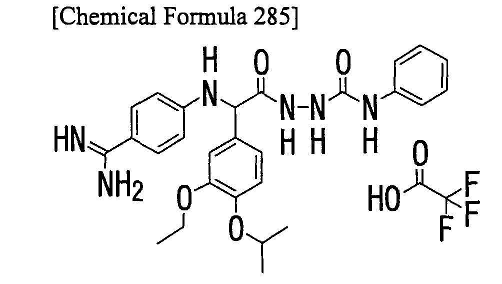 Mass spectrum (ESI) *m*/*z*: 505 (M+H)⁺

(Example X-126) 2-((4-Carbamimidoylphenylamino)-(3-ethoxy-4-isopropoxyphenyl)acetyl)-N-(4-methoxyphenyl)hydrazinecarbothioamide trifluoroacetate 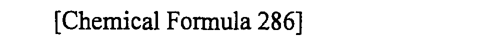 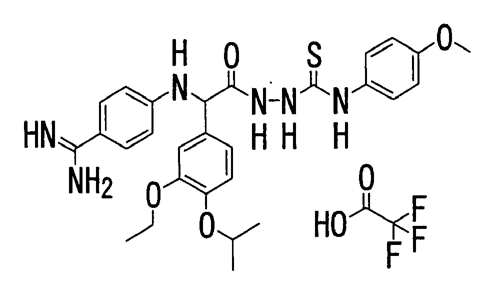 Mass spectrum (ESI) *m*/*z*: 551 (M+H)⁺

(Example X-127) 2-(N'-(2-(4-Carbamimidoylphenylamino)-2-(5-ethoxy-2-fluoro-4-isopropoxyphenyl)acetyl)hydrazino)benzoic acid trifluoroacetate 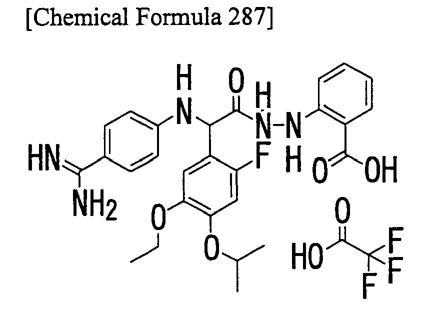 Mass spectrum (ESI) *m*/*z*: 524 (M+H)⁺

(Example X-128) 4-(((5-Ethoxy-2-fluoro-4-isopropoxyphenyl)-(N'-pyridin-2-ylhydrazinocarbonyl)methyl)amino)benzamidine trifluoroacetate 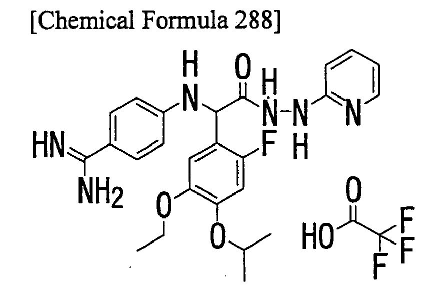 Mass spectrum (ESI) *m*/*z*: 481 (M+H)⁺

(Example X-129) 4-(((N'-(2,6-Dichlorophenyl)hydrazinocarbonyl)-(3-ethoxy-4-isopropoxyphenyl)methyl)amino)benzamidine trifluoroacetate 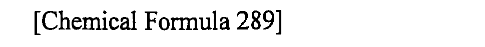 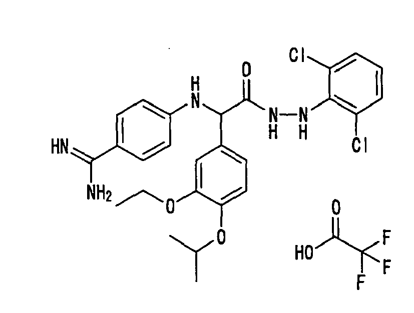 Mass spectrum (ESI) *m*/*z:* 530 (M+H)⁺

(Example X-130) 4-(((3-Ethoxy-4-isopropoxyphenyl)-(N'-(2-nitrophenyl)hydrazinocarbonyl)methyl)amino)benzamidine trifluoroacetate 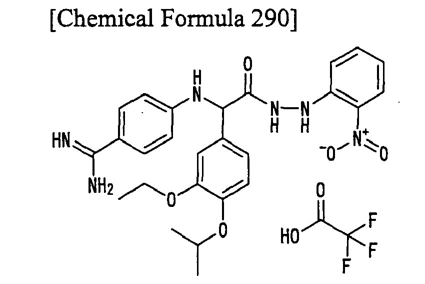 Mass spectrum (ESI) *m*/*z:* 507 (M+H)⁺

(Example X-131) 4-(((3-Ethoxy-4-isopropoxyphenyl)-(N'-pyridin-4-ylhydrazinocarbonyl)methyl)amino)benzamidine trifluoroacetate 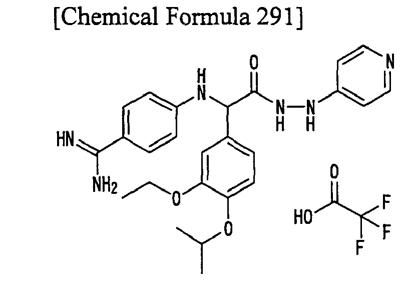 Mass spectrum (ESI) *m*/*z*: 463 (M+H)⁺

(Example X-132) 4-(((3-Ethoxy-4-isopropoxyphenyl)-hydrazinocarbonylmethyl)amino)benzamidine trifluoroacetate 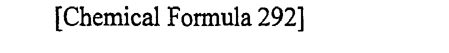 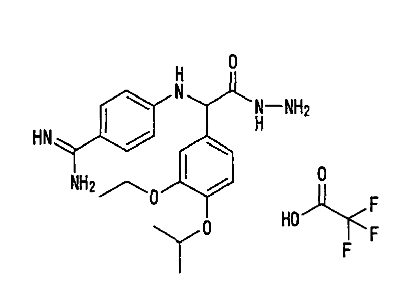 Mass spectrum (ESI) *m*/*z*: 386 (M+H)⁺

(Example X-133) 4-(1-(3-Ethoxy-4-isopropoxyphenyl)-2-(N'-(2-fluorobenzoyl)hydrazino)-2-oxoethylamino)benzamidine trifluoroacetate 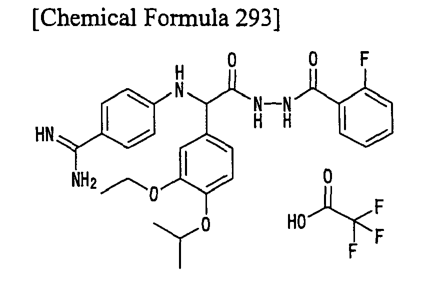 Mass spectrum (ESI) *m*/*z*: 508 (M+H)⁺

(Example X-134) 4-(((N'-Biphenyl-2-ylhydrazinocarbonyl)-(3-ethoxy-4-isopropoxyphenyl)methyl)amino)benzamidine trifluoroacetate 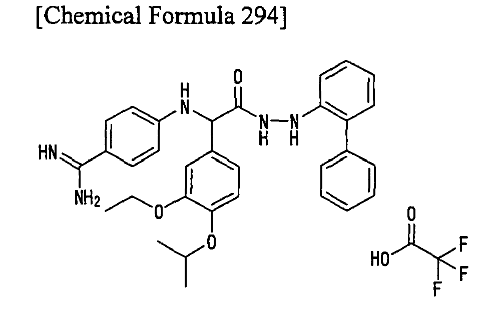 Mass spectrum (ESI) *m*/*z:* 538 (M+H)⁺

(Example X-135) 4-(((3-Ethoxy-4-isopropoxyphenyl)-(N'-(2-phenoxyphenyl)hydrazinocarbonyl)methyl)amino)benzamidine trifluoroacetate 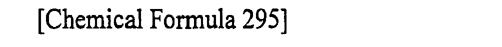 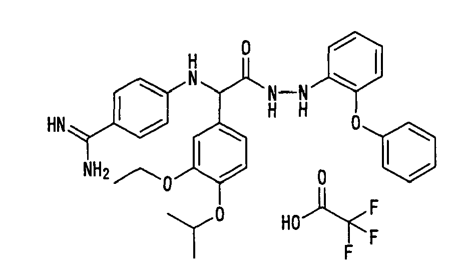 Mass spectrum (ESI) *m*/*z*: 554 (M+H)⁺

(Example X-136) 4-(1-(3-Ethoxy-4-isopropoxyphenyl)-2-(N'-(4-fluorobenzoyl)hydrazino)-2-oxoethylamino)benzamidine trifluoroacetate 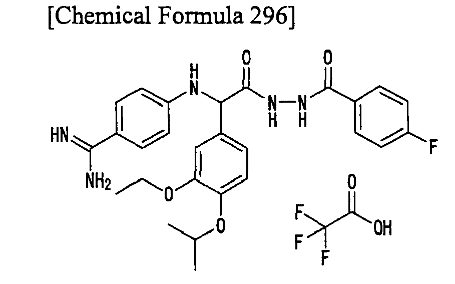 Mass spectrum (ESI) *m*/*z:* 508 (M+H)⁺

(Example X-137) 4-(1-(3-Ethoxy-4-isopropoxyphenyl)-2-(N'-(2-methoxybenzoyl)hydrazino)-2-oxoethylamino)benzamidine trifluoroacetate 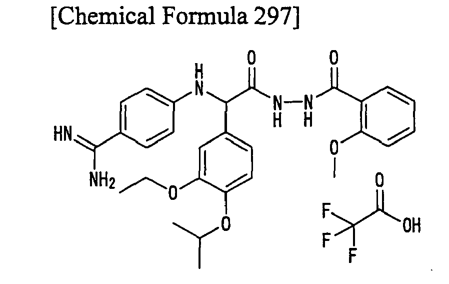 Mass spectrum (ESI) *m*/*z*: 520 (M+H)⁺

(Example X-138) 4-(1-(3-Ethoxy-4-isopropoxyphenyl)-2-(N'-(3-methoxybenzoyl)hydrazino)-2-oxoethylamino)benzamidine trifluoroacetate 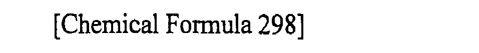 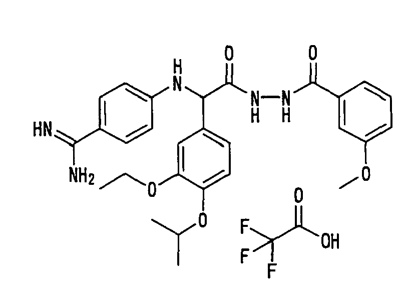 Mass spectrum (ESI) *m*/*z:* 520 (M+H)⁺

(Example X-139) 4-(1-(3-Ethoxy-4-isopropoxyphenyl)-2-(N'-(4-methoxybenzoyl)hydrazino)-2-oxoethylamino)benzamidine trifluoroacetate 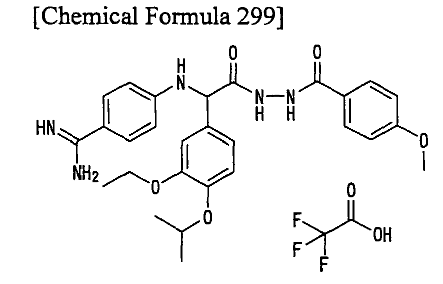 Mass spectrum (ESI) *m*/*z*: 520 (M+H)⁺

(Example X-140) 4-(1-(3-Ethoxy-4-isopropoxyphenyl)-2-(N'-(3-fluorobenzoyl)hydrazino)-2-oxoethylamino)benzamidine trifluoroacetate 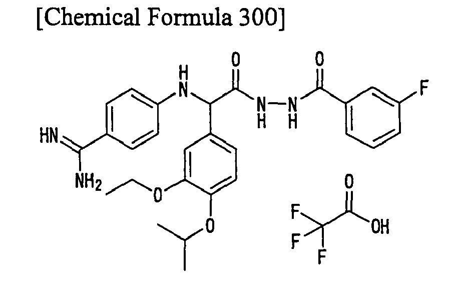 Mass spectrum (ESI) *m*/*z:* 508 (M+H)⁺

(Example X-141) 4-(1-(3-Ethoxy-4-isopropoxynhenyl)-2-(N'-(4-nitrobenzoyl)hydrazino)-2-oxoethylamino)benzamidine trifluoroacetate 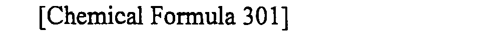 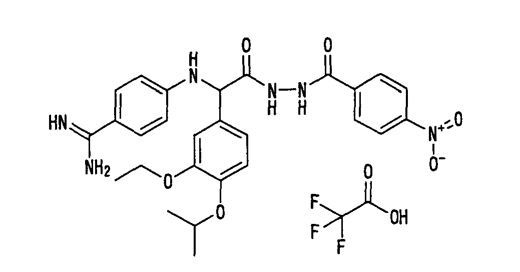 Mass spectrum (ESI) *m*/*z:* 535 (M+H)⁺

(Example X-142) 4-(2-(N'-(3-Bromobenzoyl)hydrazino)-1-(3-ethoxy-4-isopropoxyphenyl)-2-oxoethylamino)benzamidine trifluoroacetate 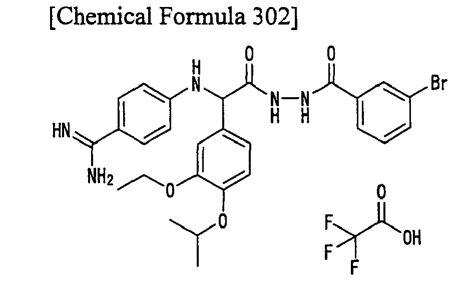 Mass spectrum (ESI) *m*/*z*: 568 (M+H)⁺

(Example X-143) 4-(2-(N'-(4-Bromobenzoyl)hydrazino)-1-(3-ethoxy-4-isopropoxyphenyl)-2-oxoethylamino)benzamidine trifluoroacetate 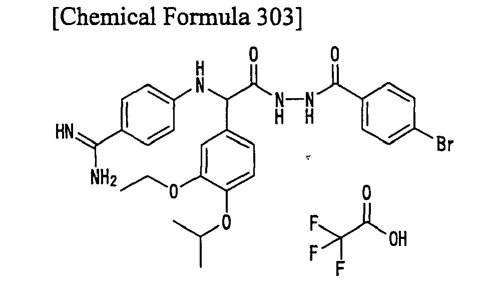 Mass spectrum (ESI) *m*/*z*: 568 (M+H)⁺

(Example X-144) 4-(1-(3-Ethoxy-4-isopropoxyohenyl)-2-(N'-(5-methyl-1H-imidazole-4-carbonyl)hydrazino)-2-oxoethylamino)benzamidine trifluoroacetate 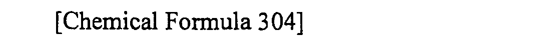 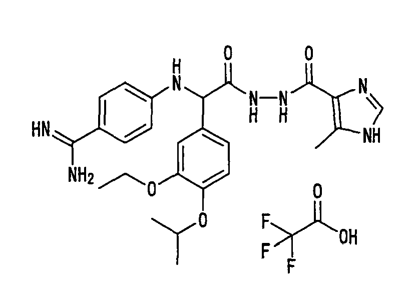 Mass spectrum (ESI) *m*/*z:* 494 (M+H)⁺

(Example X-145) 4-(1-(3-Ethoxy-4-isopropoxyphenyl)-2-oxo-2-(N'-(thiophene-3-carbonyl)hydrazino)ethylamino)benzamidine trifluoroacetate 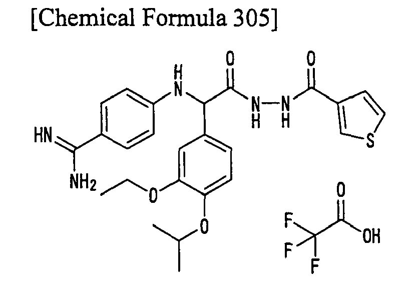 Mass spectrum (ESI) *m*/*z:* 496 (M+H)⁺

(Example X-146) 4-(2-(N'-(2-Dimethylaminobenzoyl)hydrazino)-1-(3-ethoxy-4-isopropoxyphenyl)-2-oxoethylamino)benzamidine trifluoroacetate 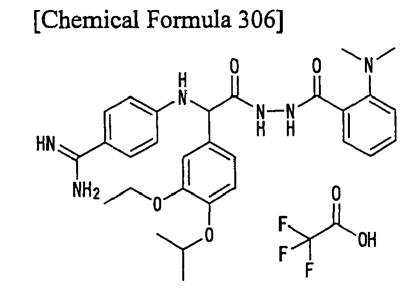 Mass spectrum (ESI) *m*/*z:* 533 (M+H)⁺

(Example X-147) 4-(1-(3-Ethoxy-4-isopropoxyphenyl)-2-oxo-2-(N'-(pyrimidine-4-carbonyl)hydrazino)ethylamino)benzamidine trifluoroacetate 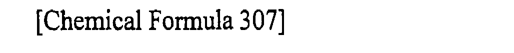 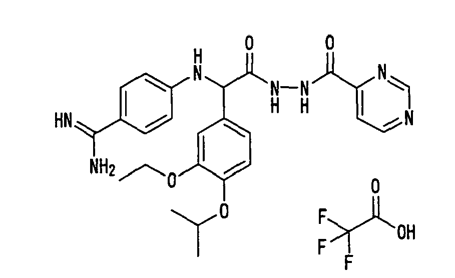 Mass spectrum (ESI) *m*/*z:* 492 (M+H)⁺

(Example X-148) 4-(1-(3-Ethoxy-4-isopropoxyphenyl)-2-oxo-2-(N'. (pyrimidine-5-carbonyl)hydrazino)ethylamino)benzamidine trifluoroacetate 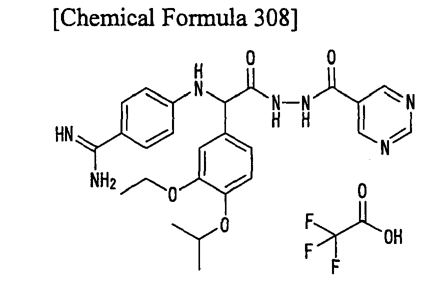 Mass spectrum (ESI) *m*/*z*: 492 (M+H)⁺

(Example X-149) 4-(1-(3-Ethoxy-4-isopropoxyphenyl)-2-oxo-2-(N'-(pyrazine-2-carbonyl)hydrazino)ethylamino)benzamidine trifluoroacetate 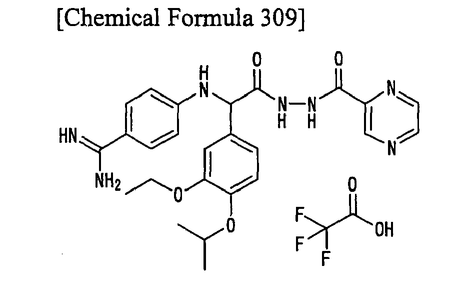 Mass spectrum (ESI) *m*/*z:* 492 (M+H)⁺

(Example X-150) 4-(2-(N'-(2-Cyanopyridine-4-carbonyl)hydrazino)-1-(3-ethoxy-4-isopropoxyphenyl)-2-oxoethylamino)benzamidine trifluoroacetate 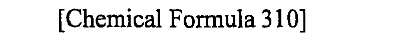 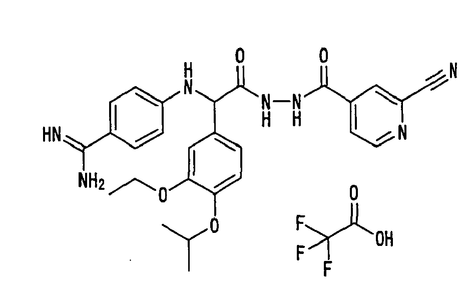 Mass spectrum (ESI) *m*/*z*: 516 (M+H)⁺

(Example X-151) 4-(2-(N'-(2-Bromopyridine-4-carbonyl)hydrazino)-1-(3-ethoxy-4-isopropoxyphenyl)-2-oxoethylamino)benzamidine trifluoroacetate 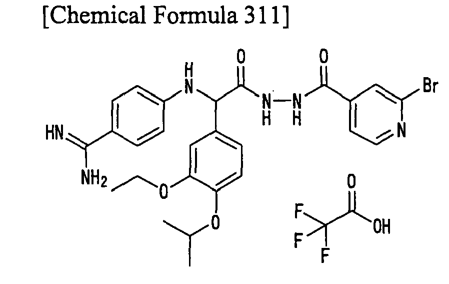 Mass spectrum (ESI) *m*/*z*: 569 (M+H)⁺

(Example X-152) 4-(2-(N'-(2-Chloropyridine-4-carbonyl)hydrazino)-1-(3-ethoxy-4-isopropoxyphenyl)-2-oxoethylamino)benzamidine trifluoroacetate 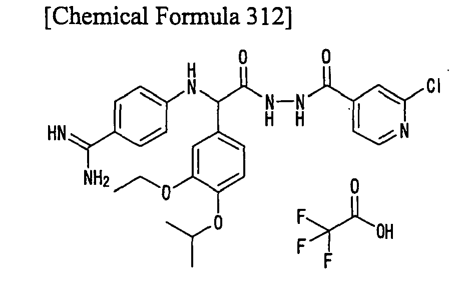 Mass spectrum (ESI) *m*/*z:* 525 (M+H)⁺

(Example X-153) 4-(1-(3-Ethoxy-4-isopropoxyphenyl)-2-(N'-(2-methoxypyridine-3-carbonyl)hydrazino)-2-oxoethylamino)benzamidine trifluoroacetate 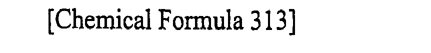 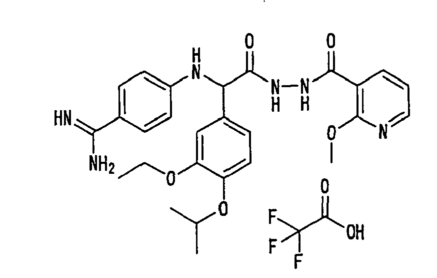 Mass spectrum (ESI) *m*/*z*: 521 (M+H)⁺

(Example X-154) 4-(1-(3-Ethoxy-4-isopropoxyphenyl)-2-oxo-2-(N'-(4-trifluoromethylpyridine-3-carbonyl)hydrazino)ethylamino)benzamidine trifluoroacetate 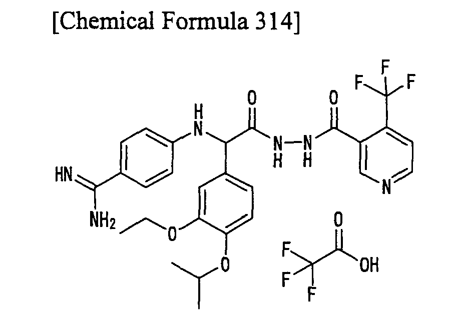 Mass spectrum (ESI) *m*/*z*: 559 (M+H)⁺

(Example X-155) 4-(1-(3-Ethoxy-4-isopropoxyphenyl)-2-oxo-2-(N'-(6-trifluoromethylpyridine-3-carbonyl)hydrazino)ethylamino)benzamidine trifluoroacetate 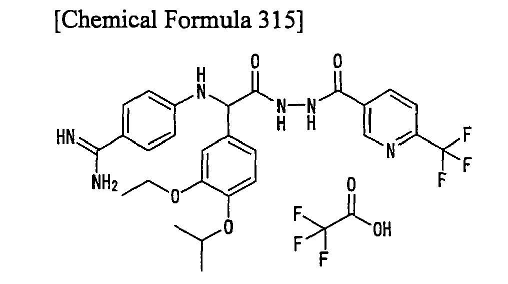 Mass spectrum (ESI) *m*/*z:* 559 (M+H)⁺

(Example X-156) 4-(1-(3-Ethoxy-4-isopropoxyphenyl)-2-(N'-(4-ethylpyridine-2-carbonyl)hydrazino)-2-oxoethylamino)benzamidine trifluoroacetate 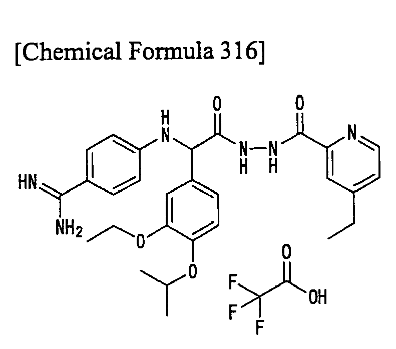 Mass spectrum (ESI) *m*/*z*: 519 (M+H)⁺

(Example X-157) 4-(2-(N'-(6-Bromopyridine-2-carbonyl)hydrazino)-1-(3-ethoxy-4-isopropoxyhenyl)-2-oxoethylamino)benzamidine trifluoroacetate 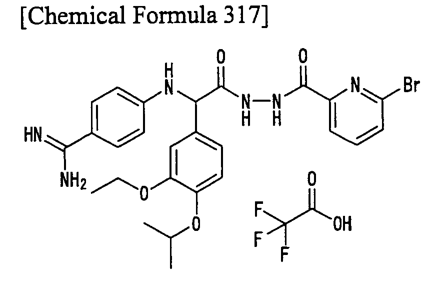 Mass spectrum (ESI) *m*/*z:* 569 (M+H)⁺

(Example X-158) 4-(1-(3-Ethoxy-4-isopropoxyphenyl)-2-(N'-(6-methylpyridine-2-carbonyl)hydrazino)-2-oxoethylamino)benzamidine trifluoroacetate 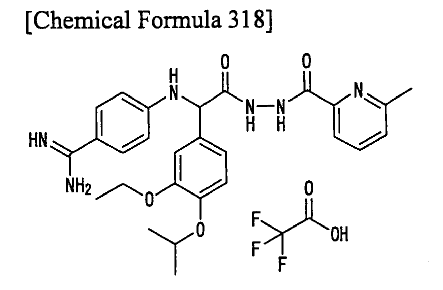 Mass spectrum (ESI) *m*/*z:* 505 (M+H)⁺

(Example X-159) 4-(1-(3-Ethoxy-4-isopropoxyphenyl)-2-oxo-2-(N'-(thiophene-2-carbonyl)hydrazino)ethylamino)benzamidine trifluoroacetate 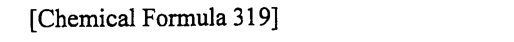 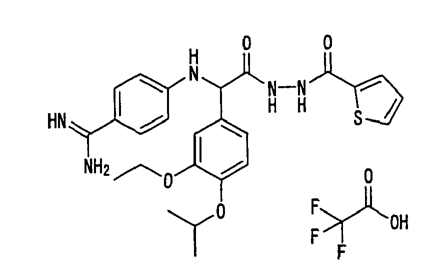 Mass spectrum (ESI) *m*/*z*: 496 (M+H)⁺

(Example X-160) 4-(1-(3-Ethoxy-4-isopropoxyphenyl)-2-(N'-(3-nitrobenzoyl)hydrazino)-2-oxoethlyamino)benzamidine trifluoroacetate 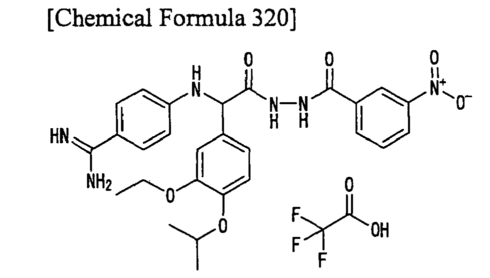 Mass spectrum (ESI) *m*/*z:* 535 (M+H)⁺

(Example X-161) 4-(1-(3-Ethoxy-4-isopropoxyphenyl)-2-oxo-2-(N'-(1-oxypyridine-2-carbonyl)hydrazino)ethylamino)benzamidine trifluoroacetate 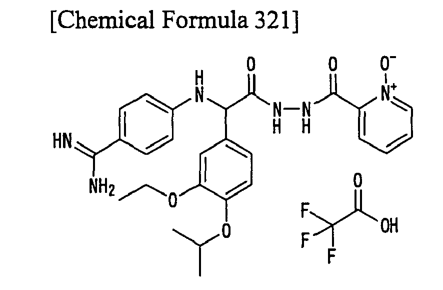 Mass spectrum (ESI) *m*/*z*: 507 (M+H)⁺

(Example X-162) 4-(1-(3-Ethoxy-4-isopropoxyphenyl)-2-oxo-2-(N'-(1-oxypyridine-4-carbonyl)hydrazino)ethylamino)benzamidine trifluoroacetate 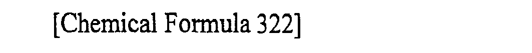 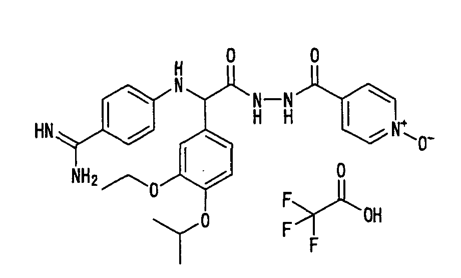 Mass spectrum (ESI) *m*/*z*: 507 (M+H)⁺

(Example X-163) 4-(1-(3-Ethoxy-4-isopropoxyphenyl)-2-(N'-(2-methanesulfonylbenzoyl)hydrazino)-2-oxoethylamino)benzamidine trifluoroacetate 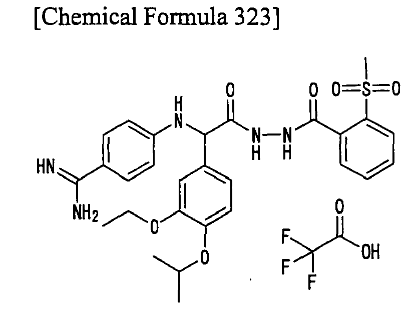 Mass spectrum (ESI) *m*/*z:* 568 (M+H)⁺

(Example X-164) 4-(1-(3-Ethoxy-4-isopropoxyphenyl)-2-oxo-2-(N'-(2-pyrrol-1-ylbenzoyl)hydrazino)ethylamino)benzamidine trifluoroacetate 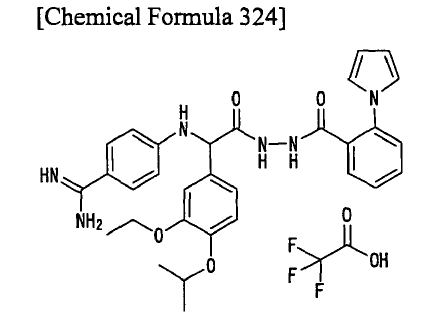 Mass spectrum (ESI) *m*/*z*: 555 (M+H)⁺

(Example X-165) 4-(1-(3-Ethoxy-4-isopropoxyphenyl)-2-oxo-2-(N'-(2-(1H-tetrazol-5-yl)benzoyl)hydrazino)ethylamino)benzamidine trifluoroacetate 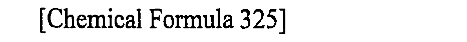 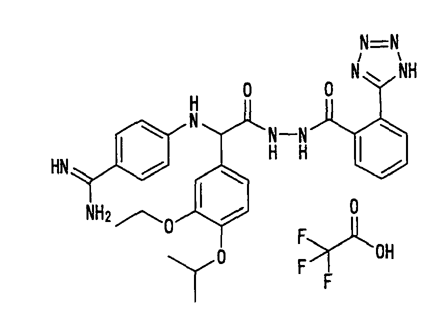 Mass spectrum (ESI) *m*/*z*: 558 (M+H)⁺

(Example X-166) N-(4-(N'-(2-(4-Carbamimidoylphenylamino)-2-(3-ethoxy-4-isopropoxyphenyl)acetyl)hydrazinocarbonyl)pyridin-2-yl)acetamide trifluoroacetate 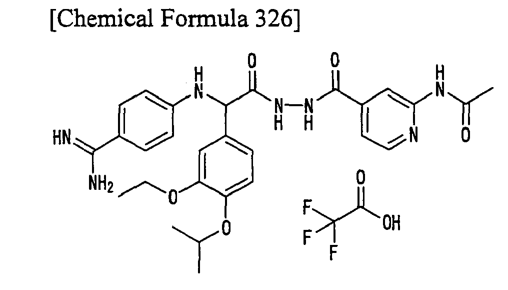 Mass spectrum (ESI) *m*/*z*: 548 (M+H)⁺

(Example X-167) 4-(1-(3-Ethoxy-4-isopropoxyphenyl)-2-(N'-(2-methylsulfanylpyridine-3-carbonyl)hydrazino)-2-oxoethylamino)benzamidine trifluoroacetate 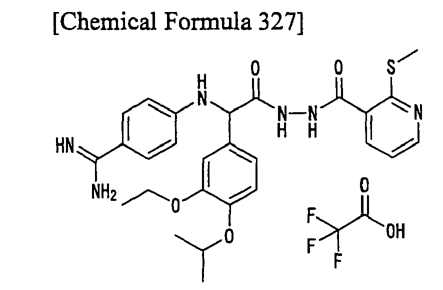 Mass spectrum (ESI) *m*/*z:* 537 (M+H)⁺

(Example X-168) 4-(2-(N'-(5-Bromopyridine-3-carbonyl)hydrazino)-1-(3-ethoxy-4-isopropoxyphenyl)-2-oxoethylamino)benzamidine trifluoroacetate 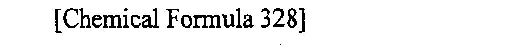 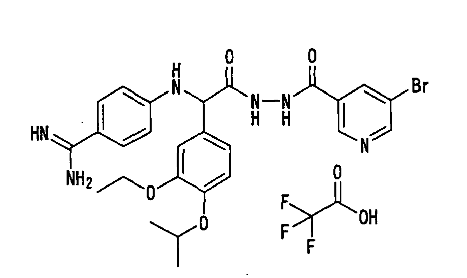 Mass spectrum (ESI) *m*/*z:* 569 (M+H)⁺

(Example X-169) 4-(1-(3-Ethoxy-4-isopropoxyphenyl)-2-(N'-(5-methylpyridine-3-carbonyl)hydrazino)-2-oxoethylamino)benzamidine trifluoroacetate 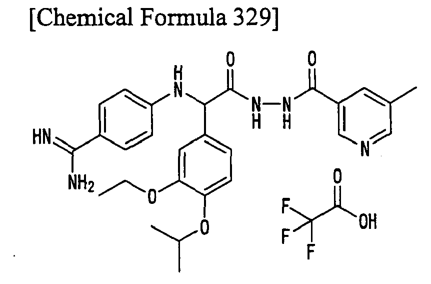 Mass spectrum (ESI) *m*/*z*: 505 (M+H)⁺

(Example X-170) 4-(1-(3-Ethoxy-4-isopropoxyphenyl)-2-(N'-(6-methylpyridine-3-carbonyl)hydrazino)-2-oxoethylamino)benzamidine trifluoroacetate 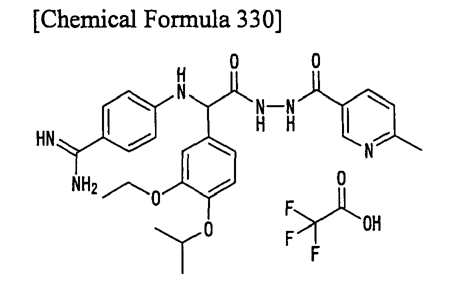 Mass spectrum (ESI) *m*/*z:* 505 (M+H)⁺

(Example X-171) 4-(2-(N'-(6-Chloropyridine-3-carbonyl)hydrazino)-1-(3-ethoxy-4-isopropoxyphenyl)-2-oxoethylamino)benzamidine trifluoroacetate 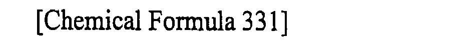 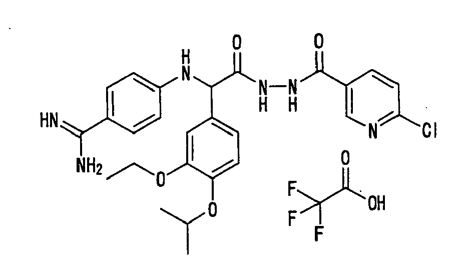 Mass spectrum (ESI) *m*/*z:* 525 (M+H)⁺

(Example X-172) 4-(1-(3-Ethoxy-4-isopropoxyphenyl)-2-(N'-(6-morpholin-4-ylpyridine-3-carbonyl)hydrazino)-2-oxoethylamino)benzamidine trifluoroacetate 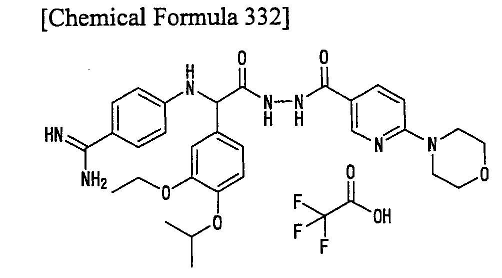 Mass spectrum (ESI) *m*/*z:* 576 (M+H)⁺

(Example X-173) 4-(1-(3-Ethoxy-4-isopropoxyphenyl)-2-oxo-2-(N'-(3-propoxypyridine-2-carbonyl)hydrazino)ethylamino)benzamidine trifluoroacetate 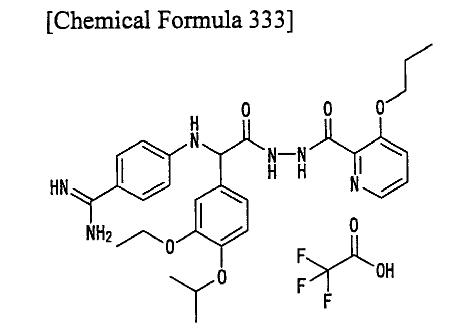 Mass spectrum (ESI) *m*/*z:* 549 (M+H)⁺

(Example X-174) 4-(2-(N'-(4-Chloropylidine-2-carbonyl)hydrazino)-1-(3-ethoxy-4-isopropoxyphenyl)-2-oxoethylamino)benzamidine trifluoroacetate 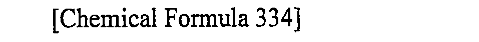 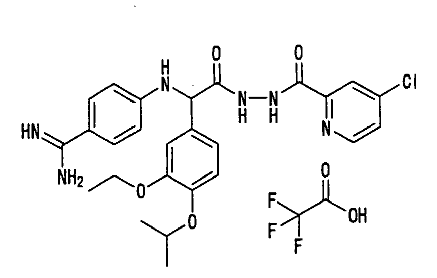 Mass spectrum (ESI) *m*/*z*: 525 (M+H)⁺

(Example X-175) 4-(1-(3-Ethoxy-4-isopropoxyphenyl)-2-oxo-2-(N'-(2-phenoxybenzoyl)hydrazino)ethylamino)benzamidine trifluoroacetate 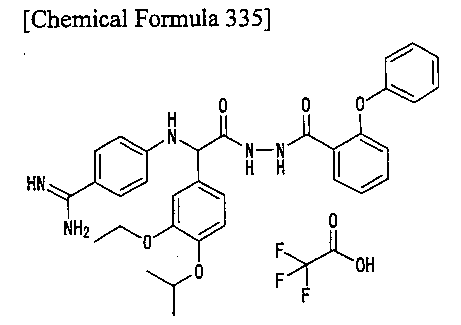 Mass spectrum (ESI) *m*/*z*: 582 (M+H)⁺

(Example X-176) 4-(2-(N'-(2-Benzyloxybenzoyl)hydrazino)-1-(3-ethoxy-4-isopropoxyphenyl)-2-oxoethylamino)benzamidine trifluoroacetate 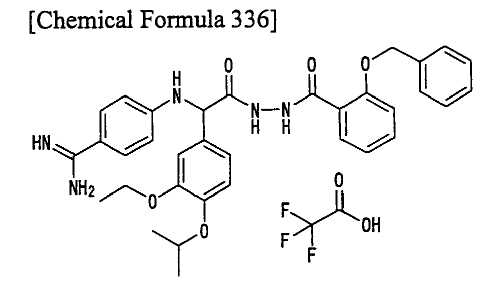 Mass spectrum (ESI) *m*/*z*: 596 (M+H)⁺

(Example X-177) 2-(N'-(2-(4-Carbamimidoylphenylamino)-2-(2-fluoro-4,5-dimethoxyphenyl)acetyl)hydrazino)benzoic acid trifluoroacetate 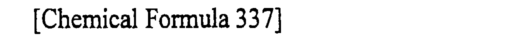 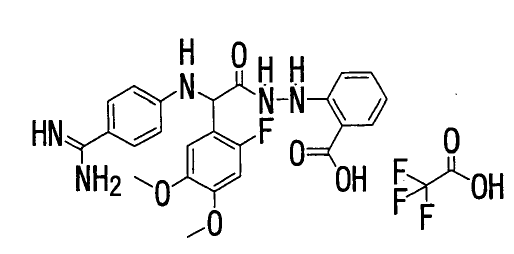 Mass spectrum (ESI) *m*/*z:* 482 (M+H)⁺

(Example X-178) 4-(2-(N'-Benzoylhydrazino)-1-(2-fluoro-4,5-dimethoxynhenyl)-2-oxoethylamino)benzamidine trifluoroacetate 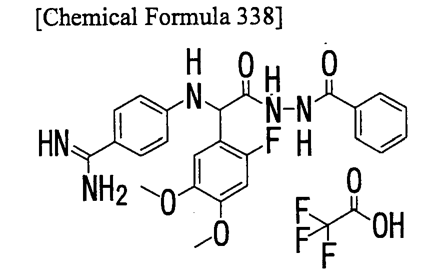 Mass spectrum (ESI) *m*/*z:* 466 (M+H)⁺

(Example X-179) 4-(1-(2-Fluoro-4,5-dimethoxyphenyl)-2-(N'-(furan-2-carbonyl)hydrazino)-2-oxoethylamino)benzamidine trifluoroacetate 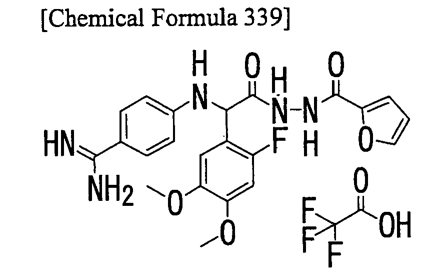 Mass spectrum (ESI) *m*/*z:* 456 (M+H)⁺

(Example X-180) 2-(4-Carbamimidoylphenylamino)-N-(1,3-dioxo-1,3-dihydroisoindol-2-yl)-2-(2-fluoro-4,5-dimethoxyphenyl)acetamide trifluoroacetate 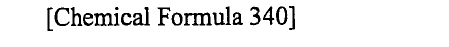 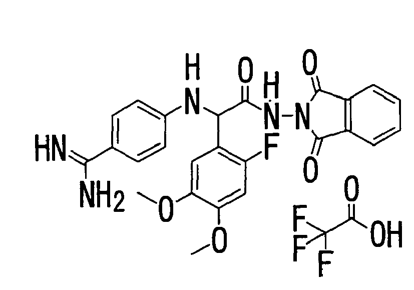 Mass spectrum (ESI) *m*/*z:* 492 (M+H)⁺

(Example X-181) 4-((2-(2-(Phenylsulfonyl)hydrazino)-1-(2-fluoro-4,5-dimethoxyphenyl)-2-oxoethyl)amino)benzamidine trifluoroacetate 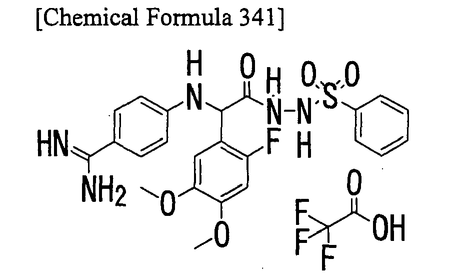 Mass spectrum (ESI) *m*/*z:* 502 (M+H)⁺

(Example X-182) 4-(1-(2-Fluoro-4,5-dimethoxyphenyl)-2-oxo-2-(N'-(pyridine-2-carbonyl)hydrazino)ethylamino)benzamidine trifluoroacetate 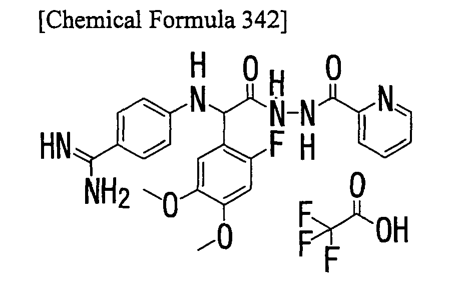 Mass spectrum (ESI) *m*/*z:* 467 (M+H)⁺

(Example X-183) 4-(1-(2-Fluoro-4,5-dimethoxyphenyl)-2-oxo-2-(N'-(pyridine-3-carbonyl)hydrazino)ethylamino)benzamidine trifluoroacetate 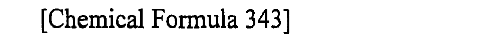 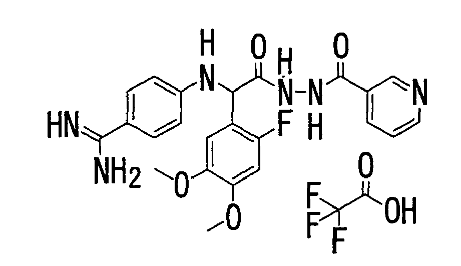 Mass spectrum (ESI) *m*/*z*: 467 (M+H)⁺

(Example X-184) 4-(2-(N'-(2-Chlorobenzoyl)hydrazino)-1-(3-ethoxy-4-isopropoxyphenyl)-2-oxoethylamino)benzamidine trifluoroacetate 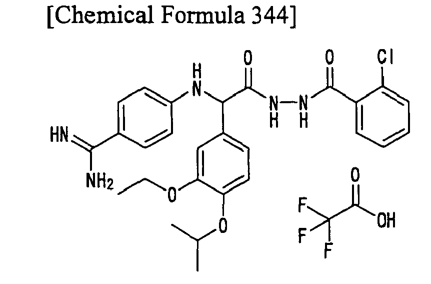 Mass spectrum (ESI) *m*/*z*: 524 (M+H)⁺

(Example X-185) 4-(1-(3-Ethoxy-4-isopropoxyphenyl)-2-(N'-(3-hydroxybenzoyl)hydrazino)-2-oxoethylamino)benzamidine trifluoroacetate 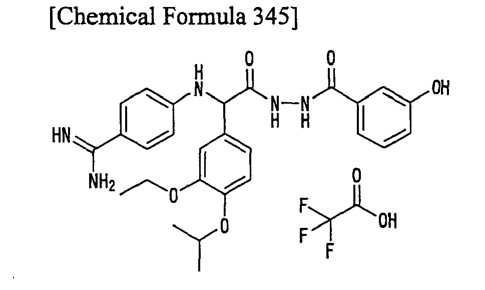 Mass spectrum (ESI) *m*/*z*: 506 (M+H)⁺

(Example X-186) 4-(2-(N'-(3,6-Dichloropyridine-2-carbonyl)hydrazino)-1-(3-ethoxy-4-isopropoxyphenyl)-2-oxoethylamino)benzamidine trifluoroacetate 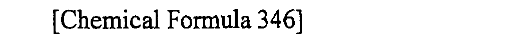 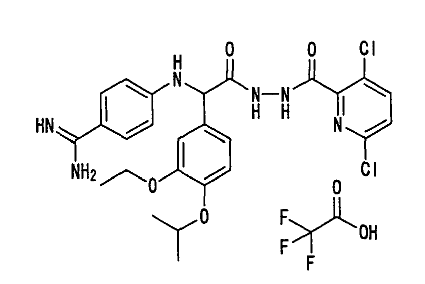 Mass spectrum (ESI) *m*/*z:* 559 (M+H)⁺

(Example X-187) 2-(4-Carbamimidoylphenylamin)-N-(1,3-dioxo-1,3-dihydropyrrolo[3,4-c]pyridin-2-yl)-2-(3-ethoxy-4-isopropoxyphenyl)acetamide trifluoroacetate 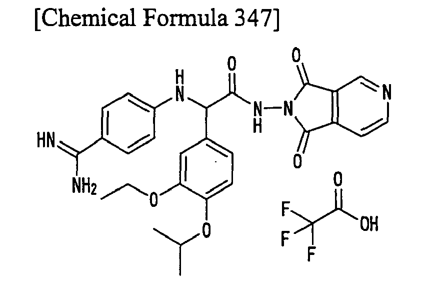 Mass spectrum (ESI) *m*/*z*: 517 (M+H)⁺

(Example X-188) 4-((2-(2-(Pyridine-2-sulfonyl)hydrazino)-1-(3-ethoxy-4-isopropoxyphenyl)-2-oxoethyl)amino)benzamidine trifluoroacetate 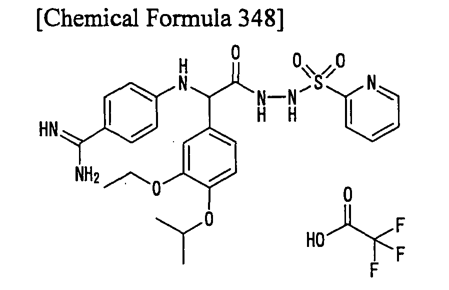 Mass spectrum (ESI) *m*/*z*: 527 (M+H)⁺

(Example X-189) 4-((2-(2-(Pyridine-3-sulfonyl)hydrazino)-1-(3-ethoxy-4-isopropoxyphenyl)-2-oxoethyl)amino)benzamidine trifluoroacetate 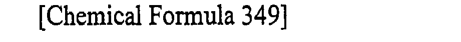 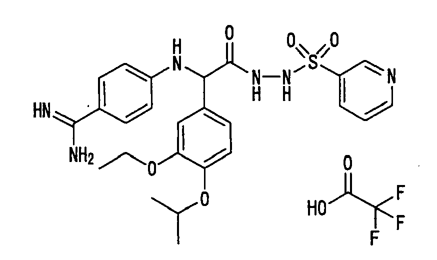 Mass spectrum (ESI) *m*/*z:* 527 (M+H)⁺

(Example X-190) 4-(2-(N'-(6-Chloropyridine-2-carbonyl)hydrazino)-1-(3-ethoxy-4-isopropoxyphenyl)-2-oxoethylamino)benzamidine trifluoroacetate 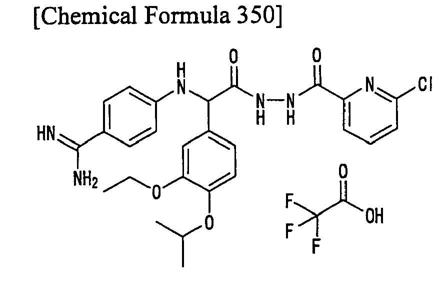 Mass spectrum (ESI) *m*/*z*: 525 (M+H)⁺

(Example X-191) 4-(1-(3-Ethoxy-4-isopropoxyphenyl)-2-(N'-(2-moroholin-4-ylpyridine-3-carbonyl)hydrazino)-2-oxoethylamino)benzamidine trifluoroacetate 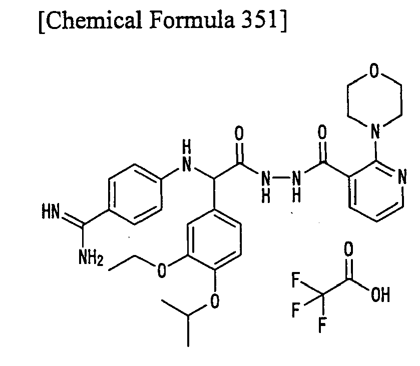 Mass spectrum (ESI) *m*/*z:* 576 (M+H)⁺

(Example X-192) 4-(1-(3-Ethoxy-4-isopropoxyphenyl)-2-(N'-(4-morpholin-4-ylpyridine-3-carbonyl)hydrazino)-2-oxoethylamino)benzamidine trifluoroacetate 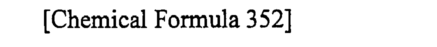 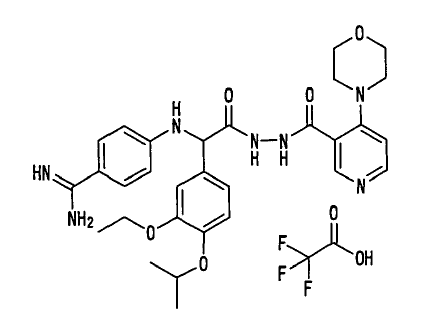 Mass spectrum (ESI) *m*/*z:* 576 (M+H)⁺

(Example X-193) 4-(1-(2-Fluoro-4,5-dimethoxyphenyl)-2-(N'-(4-methylpyridine-3-carbonyl)hydrazino)-2-oxoethylamino)benzamidine trifluoroacetate 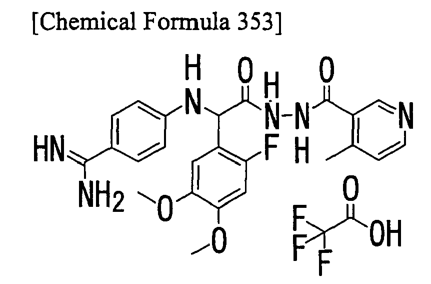 Mass spectrum (ESI) *m*/*z*: 481 (M+H)⁺

(Example X-194) 2-(4-(1-(4-Carbamimidoylphenylamino)-2-(N'-(6-methylpyridine-2-carbonyl)-hydrazino)-2-oxoethyl)-2-ethoxyphenoxy)-N,N-dimethylacetamide trifluoroacetate 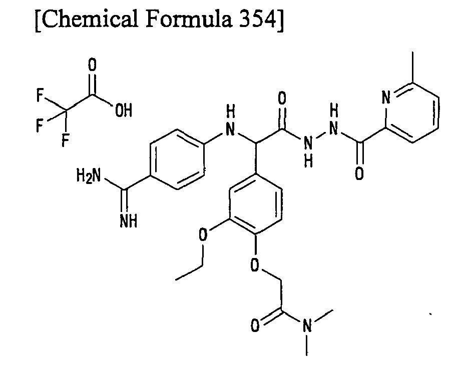 Mass spectrum (ESI) *m*/*z*: 548 (M+H)⁺

(Example X-195) 4-(2-(N'-(2-Chlorobenzoyl)hydrazino)-1-(4-(2-dimethylaminoethoxy)-3-ethoxyphenyl)-2-oxoethylamino)benzamidine trifluoroacetate 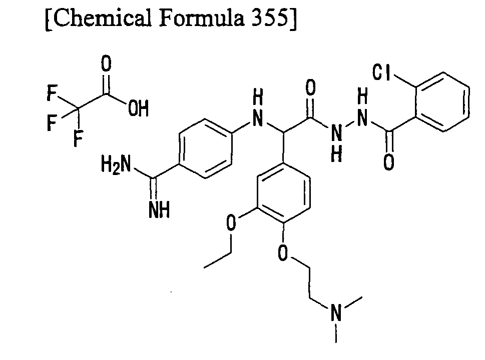 Mass spectrum (ESI) *m*/*z:* 553 (M+H)⁺

(Example X-196) 4-(1-(4-(2-Dimethylaminoethoxy)-3-ethoxyphenyl)-2-oxo-2-(N'-(2-methoxypylidine-3-carbonyl)hydrazino)ethylamino)benzarnidine trifluoroacetate 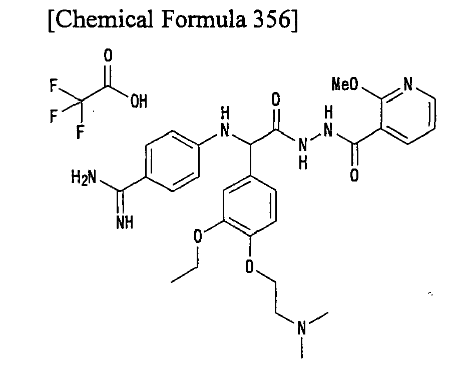 Mass spectrum (ESI) *m*/*z:* 550 (M+H)⁺

(Example X-197) 4-(1-(4-(2-Dimethylaminoethoxy)-3-ethoxyphenyl)-2-oxo-2-(N'-(3-methylpyddine-2-carbonyl)hydrazino)ethylamino)benzamidine trifluoroacetate 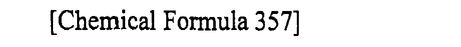 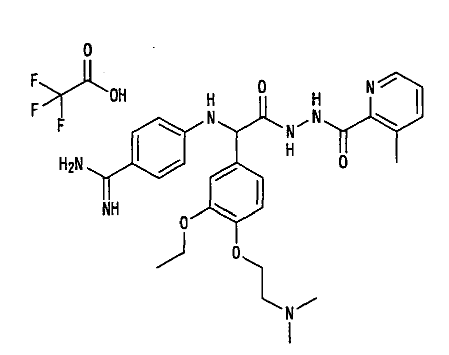 Mass spectrum (ESI) *m*/*z*: 534 (M+H)⁺

(Example X-198) 4-(((4-(2-Dimethylaminoethoxy)-3-ethoxyphenyl)-(N'-(phenylhydrazinocarboxyl)methyl)amino)benzamidine trifluoroacetate 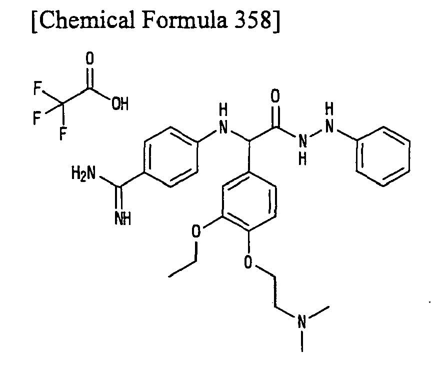 Mass spectrum (ESI) *m*/*z:* 491 (M+H)⁺

(Example X-199) 4-(((4-(2-Dimethylaminoethoxy)-3-ethoxyphenyl)-(N'-(2-fluorophenylhydrazinocarbonyl)methyl)amino)benzamidine trifluoroacetate 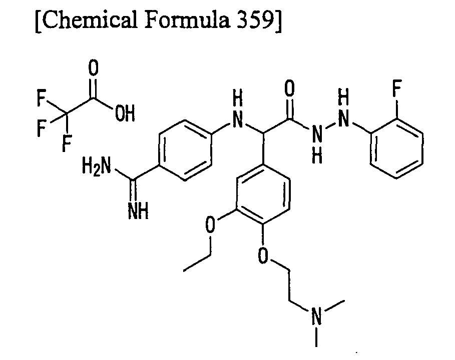 Mass spectrum (ESI) *m*/*z:* 509 (M+H)⁺

(Example X-200) 4-(((4-(2-Dimethylaminoethoxy)-3-ethoxyphenyl)-(N'-(2-chlorophenylhydrazinocarbonyl)methyl)amino)benzamidine trifluoroacetate 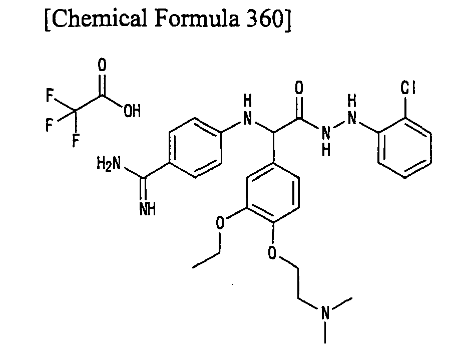 Mass spectrum (ESI) *m*/*z*: 525 (M+H)⁺

(Example X-201) 4-(((4-(2-Dimethylaminoethoxy)-3-ethoxyphenyl)-(N'-(2-bromophenylhydrazinocarbonyl)methyl)amino)benzamidine trifluoroacetate 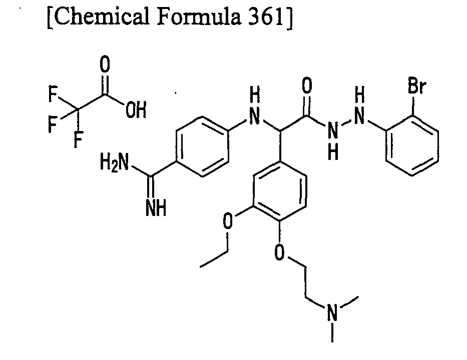 Mass spectrum (ESI) *m*/*z:* 569 (M+H)⁺

(Example X-202) 4-(((4-(2-Dimethylaminoethoxy)-3-ethoxyphenyl)-(N'-(2-trifluoromethylhydrazinocarbonyl)methyl)amino)benzamidine trifluoroacetate 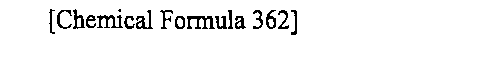 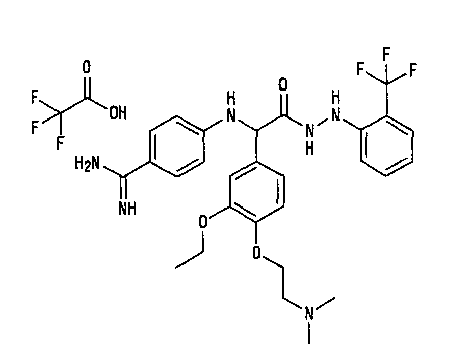 Mass spectrum (ESI) *m*/*z:* 559 (M+H)⁺

(Example X-203) 2-(3-((4-Carbamimidoylphenylamino)-(N'-(2-methoxyphenyl)hydrazinocarbonyl)methyl)-5-ethoxyphenoxy)-N,N-dimethylacetamide trifluoroacetate 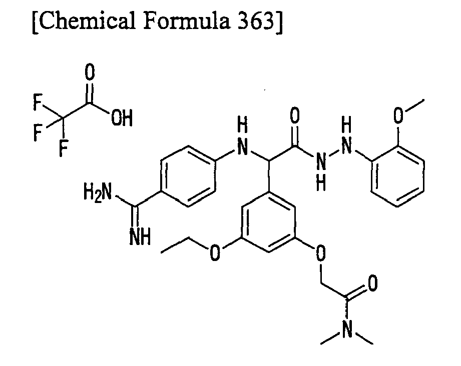 Mass spectrum (ESI) *m*/*z:* 535 (M+H)⁺

(Example X-204) 4-(((3-(2-Dimethylaminoethoxy)-5-ethoxyphenyl)-(N'-(2-methoxyphenyl)hydrazinocarbonyl)methyl)amino)benzamidine trifluoroacetate 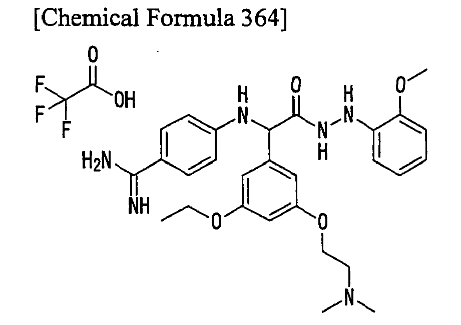 Mass spectrum (ESI) *m*/*z:* 521 (M+H)⁺

(Example X-2051 4-(1-(3-(2-Dimethylaminoethoxy)-5-ethoxyphenyl)-2-oxo-2-(N'-(pyridine-2-carbonyl)hydrazino)ethylamino)benzamidine trifluoroacetate 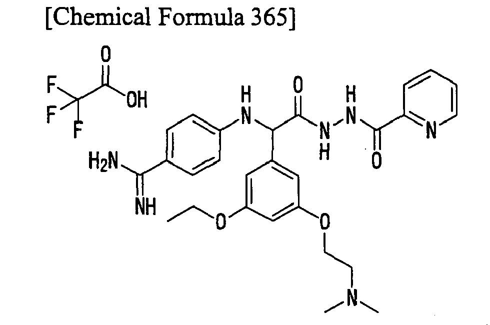 Mass spectrum (ESI) *m*/*z:* 520 (M+H)⁺

(Example X-206) 2-(4-Carbamimidoylphenylamino)-N-(1,3-dioxo-1,3-dihydroisoindol-2-yl)-2-(5-ethoxy-2-fluoro-4-isoproxyphenyl)acetamide trifluoroacetate 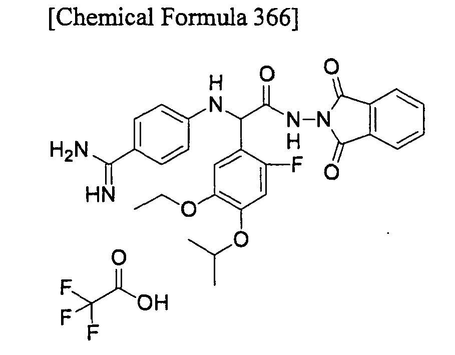 Mass spectrum (ESI) *m*/*z:* 534 (M+H)⁺

(Example X-207) 4-(1-(5-Ethoxy-2-fluoro-4-isopropoxyphenyl)-2-(N'-(3-methylpyridine-2-carbonyl)hydrazino)-2-oxoethylamino)benzamidine trifluoroacetate 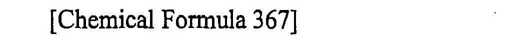 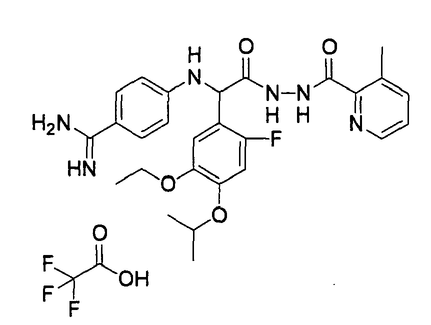 Mass spectrum (ESI) *m*/*z:* 523 (M+H)⁺

(Example X-208) 4-(2-(N'-(3-Bromopyridine-2-carbonyl)hydrazino)-1-(5-ethoxy-2-fluoro-4-isopropoxyphenyl)-2-oxoethylamino)benzamidine trifluoroacetate 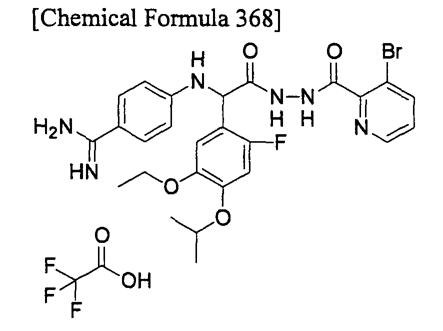 Mass spectrum (ESI) *m*/*z*: 587 (M+H)⁺

(Example X-209) 4-(1-(5-Ethoxy-2-fluoro-4-isopropoxyphenyl)-2-(N'-(4-methylpyridine-3-carbonyl)hydrazino)-2-oxoethylamino)benzamidine trifluoroacetate 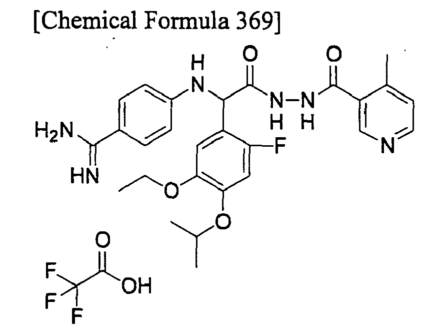 Mass spectrum (ESI) *m*/*z*: 523 (M+H)⁺

(Example X-210) 4-(1-(5-Ethoxy-2-fluoro-4-isopropoxyphenyl)-2-(N'-(2-fluoropyridine-3-carbonyl)hydrazino)-2-oxoethylamino)benzamidine trifluoroacetate 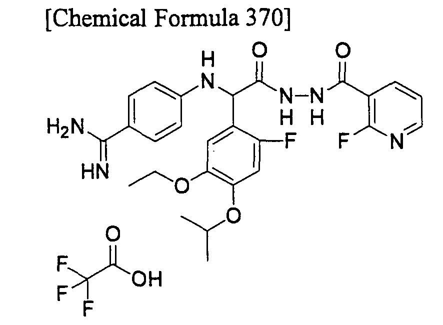 Mass spectrum (ESI) *m*/*z:* 527 (M+H)⁺

(Example X-211) 4-(1-(5-Ethoxy-2-fluoro-4-isopropoxyphenyl)-2-(N'-(3-methylpyridine-4-carbonyl)hydrazino)-2-oxoethylamino)benzamidine trifluoroacetate 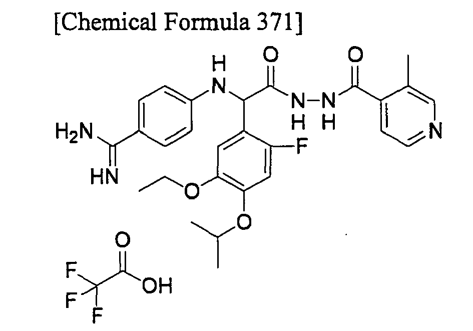 Mass spectrum (ESI) *m*/*z:* 523 (M+H)⁺

(Example X-212) 4-(1-(5-Ethoxy-2-fluoro-4-isopropoxyphenyl)-2-(N'-(3-fluoropyridine-4-carbonyl)hydrazino)-2-oxoethylamino)benzamidine trifluoroacetate 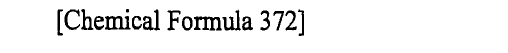 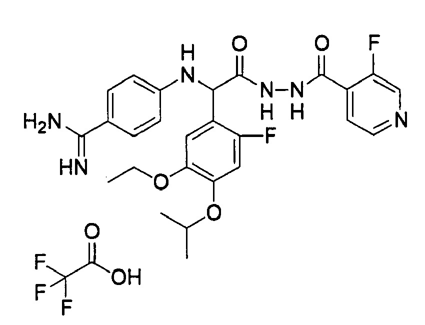 Mass spectrum (ESI) *m*/*z*: 527 (M+H)⁺

(Example X-213) 4-(2-(N'-(3-Bromopyridine-4-carbonyl)hydrazino)-1-(5-ethoxy-2-fluoro-4-isopropoxyphenyl)-2-oxoethylamino)benzamidine trifluoroacetate 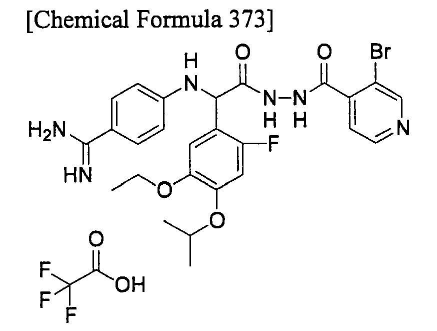 Mass spectrum (ESI) *m*/*z:* 587 (M+H)⁺

(Example X-214) 4-(2-(N'-Benzoylhydrazino)-2-oxo-1-(3,4,5-trimethoxyphenyl)ethylamino)benzamidine trifluoroacetate 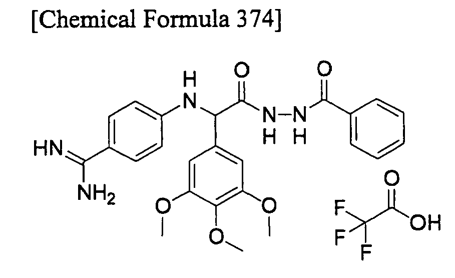 Mass spectrum (ESI) *m*/*z:* 478 (M+H)⁺

(Example X-215) 4-(2-(N'-(4-Hydroxybenzoyl)hydrazino)-2-oxo-1-(3,4,5-trimethoxyphenyl)ethylamino)benzamidine trifluoroacetate 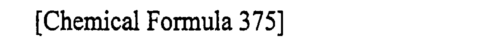 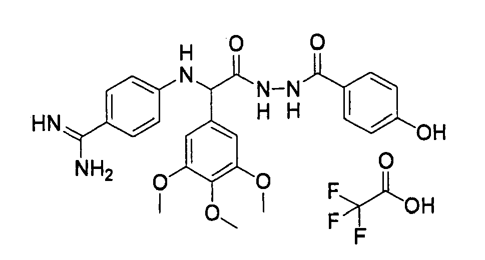 Mass spectrum (ESI) *m*/*z*: 494 (M+H)⁺

(Example X-216) 2-(4-Carbamimidoylphenylamino)-N-(1,3-dioxo-1,3-dihydroisoindol-2-yl)-2-(3,4,5-trimetboxyphenyl)acetamide trifluoroacetate 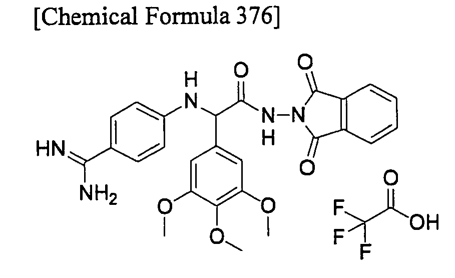 Mass spectrum (ESI) *m*/*z:* 504 (M+H)⁺

(Example X-217) 4-(2-(N'-(Furan-2-carbonyl)hydrazino)-2-oxo-1-(3,4,5-trimethoxyphenyl)ethylamino)benzamidine trifluoroacetate 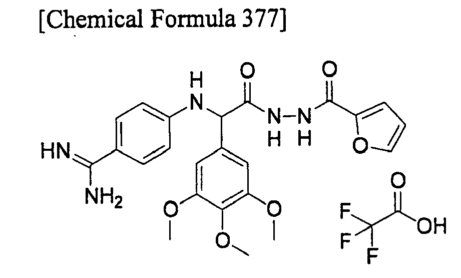 Mass spectrum (ESI) *m*/*z:* 468 (M+H)⁺

(Example X-218) 4-(2-Oxo-2-(N'-(pyridine-2-carbonyl)hydrazino)-1-(3,4,5-trimethoxyphenyl)ethylamino)benzamidine trifluoroacetate 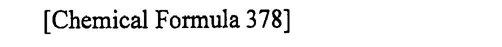 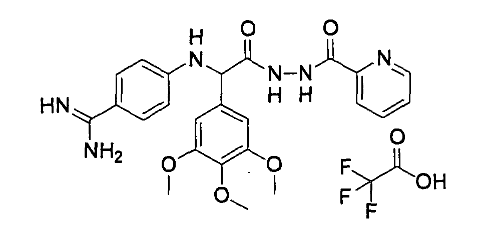 Mass spectrum (ESI) *m*/*z:* 479 (M+H)⁺

(Example X-219) 4-(2-Oxo-2-(N'-(pyridine-3-carbonyl)hydrazino)-1-(3,4,5-trimethoxyphenyl)ethylamino)benzamidine trifluoroacetate 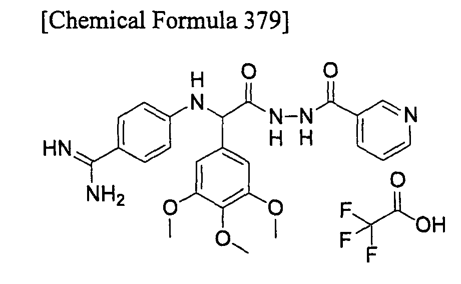 Mass spectrum (ESI) *m*/*z:* 479 (M+H)⁺

(Example X-220) 4-(2-Oxo-2-(N'-(pyridine-4-carbonyl)hydrazino)-1-(3,4,5-trimethoxyphenyl)ethylamino)benzamidine trifluoroacetate 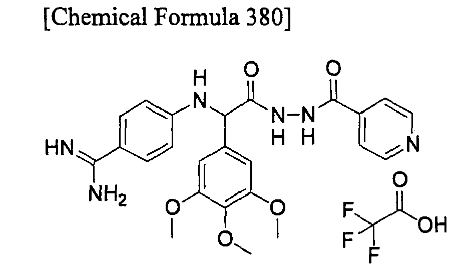 Mass spectrum (ESI) *m*/*z:* 479 (M+H)⁺

(Example X-221) 4-(2-(N'-Benzoylhydrazino)-1-(3,4-diethoxyphenyl)-2-oxoethylamino)benzamidine trifluoroacetate 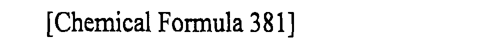 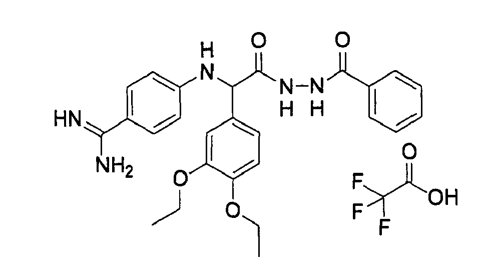 Mass spectrum (ESI) *m*/*z:* 476 (M+H)⁺

(Example X-222) 4-(2-(N'-(2-Chlorobenzoyl)hydrazino)-1-(3,4-diethoxyphenyl)-2-oxoethylamino)benzamidine trifluoroacetate 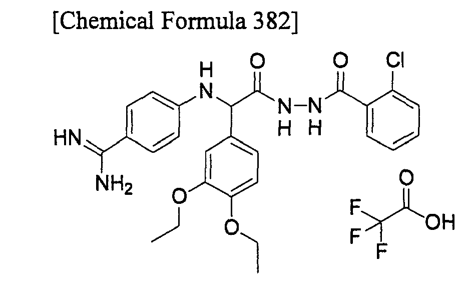 Mass spectrum (ESI) *m*/*z*: 510 (M+H)⁺

(Example X-223) 4-(1-(3,4-Diethoxyphenyl)-2-(N'-(4-hydroxybenzoyl)hydrazino)-2-oxoethylamino)benzamidine trifluoroacetate 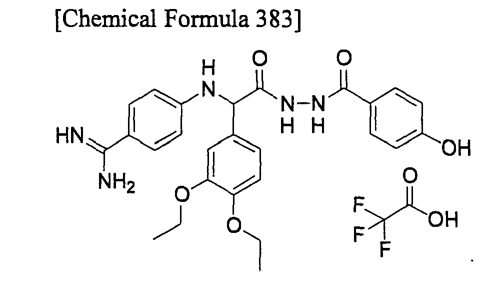 Mass spectrum (ESI) *m*/*z*: 492 (M+H)⁺

(Example X-224) 2-(4-Carbamimidoylphenylamino)-2-(3,4-diethoxyphenyl)-N-(1,3-dioxo-1,3-dihydroisoindol-2-yl)acetamide trifluoroacetate 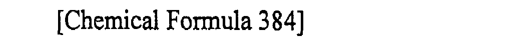 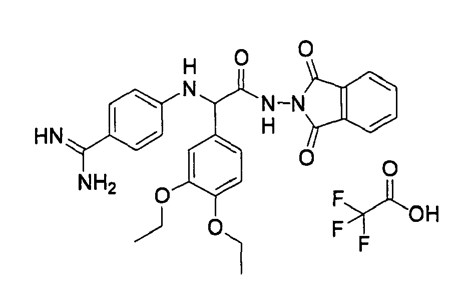 Mass spectrum (ESI) *m*/*z*: 502 (M+H)⁺

(Example X-225) 4-(1-(3,4-Diethoxyphenyl)-2-(N'-(furan-2-carbonyl)hydrazino)-2-oxo-ethylamino)benzamidine trifluoroacetate 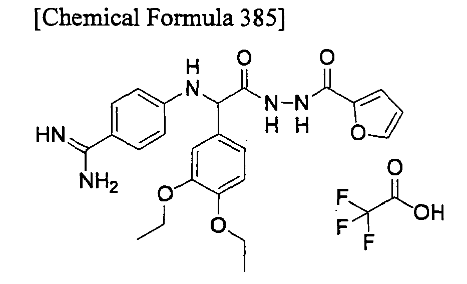 Mass spectrum (ESI) *m*/*z*: 466 (M+H)⁺

(Example X-226) 4-(1-(3,4-Diethoxyphenyl)-2-oxo-2-(N'-(pyridine-2-carbonyl)hydrazino)ethylamino)benzamidine trifluoroacetate 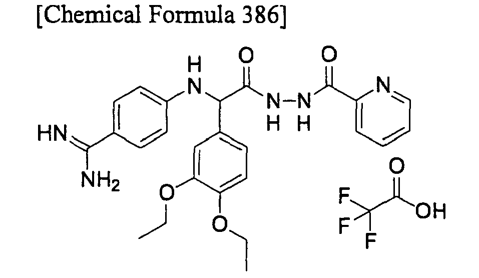 Mass spectrum (ESI) *m*/*z*: 477 (M+H)⁺

(Example X-227) 4-(1-(3,4-Diethoxyphenyl)-2-oxo-2-(N'-(pyridine-3-carbonyl)hydrazino)ethylamino)benzamidine trifluoroacetate 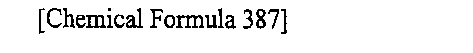 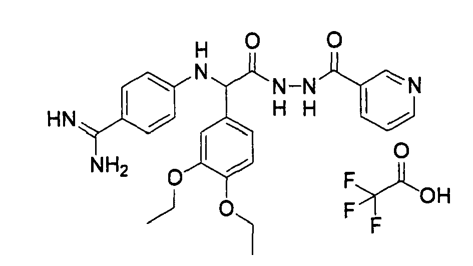 Mass spectrum (ESI) *m*/*z:* 477 (M+H)⁺

(Example X-228) 4-(1-(3,4-Diethoxyphenyl)-2-oxo-2-(N'-(pyridine-4-carbonyl)hydrazino)ethylamino)benzamidine trifluoroacetate 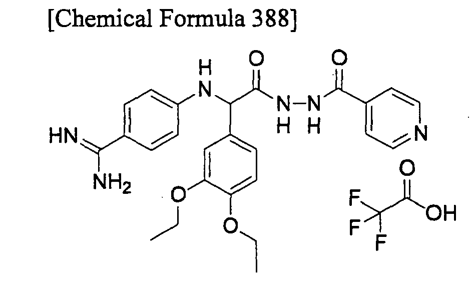 Mass spectrum (ESI) *m*/*z*: 477 (M+H)⁺

(Example X-229) 4-(2-(N'-Benzoylhydrazino)-1-(4-isopropoxy-3-methoxyphenyl)-2-oxoethylamino)benzamidine trifluoroacetate 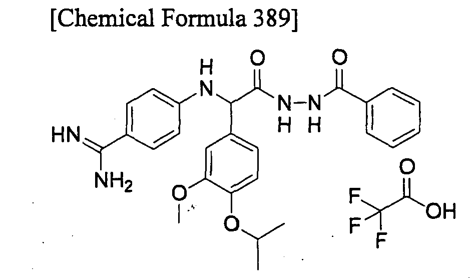 Mass spectrum (ESI) *m*/*z:* 476 (M+H)⁺

(Example X-230) 4-(2-(N'-(2-Chlorobenzoyl)hydrazino)-1-(4-isopropoxy-3-methoxyphenyl)-2-oxoethylamino)benzamidine trifluoroacetate 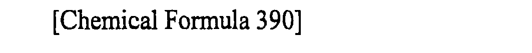 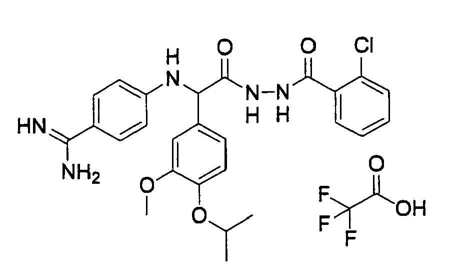 Mass spectrum (ESI) *m*/*z:* 510 (M+H)⁺

(Example X-231) 4-(2-(N'-(4-Hydroxybenzoyl)hydrazino)-1-(4-isopropoxy-3-methoxyphenyl)-2-oxoethylamino)benzamidine trifluoroacetate 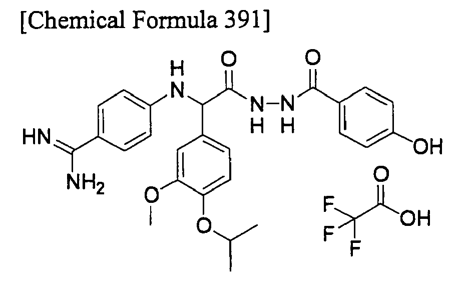 Mass spectrum (ESI) *m*/*z*: 492 (M+H)⁺

(Example X-232) 2-(4-Carbamimidoylphenylamino)-N-(1,3-dioxo-1,3-dihydroisoindol-2-yl)-2-(4-isopropoxy-3-methoxyphenyl)acetamide trifluoroacetate 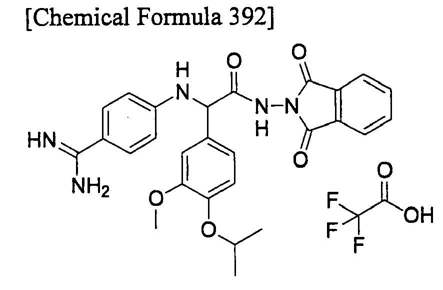 Mass spectrum (ESI) *m*/*z:* 502 (M+H)⁺

(Example X-233) 4-(2-(N'-(Furan-2-carbonyl)hydrazino)-1-(4-isopropoxy-3-methoxyphenyl)-2-oxoethylamino)benzamidine trifluoroacetate 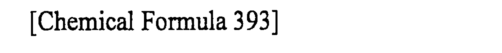 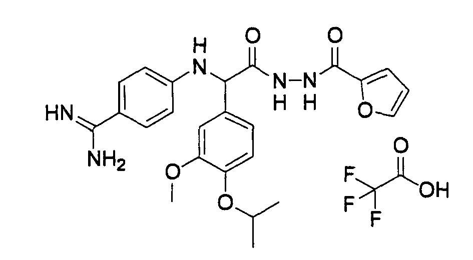 Mass spectrum (ESI) *m*/*z:* 466 (M+H)⁺

(Example X-234) 4-(2-(N'-Benzoylhydrazino)-1-(3-ethoxy-4-(2-methoxyethoxy)phenyl)-2-oxoethylamino)benzamidine trifluoroacetate 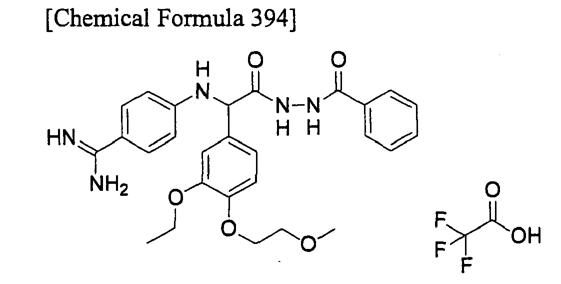 Mass spectrum (ESI) *m*/*z*: 506 (M+H)⁺

(Example X-235) 4-(1-(3-Ethoxy-4-(2-methoxyethoxy)phenyl)-2-(N'-(4-hydroxybenzoyl)hydrazino)-2-oxoethylamino)benzamidine trifluoroacetate 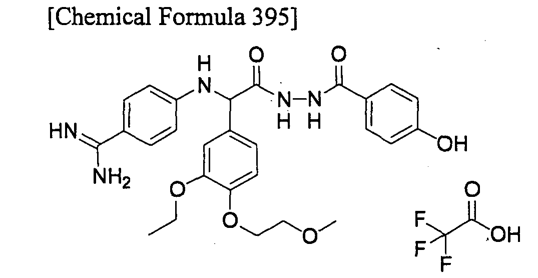 Mass spectrum (ESI) *m*/*z:* 522 (M+H)⁺

(Example X-236) 2-(4-Carbamimidoylphenylamino)-N-(1,3-dioxo-1,3-dihydroisoindol-2-yl)-2-(3-ethoxy-4-(2-methoxyethoxy)phenyl)acetamide trifluoroacetate 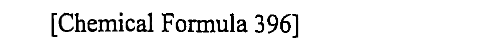 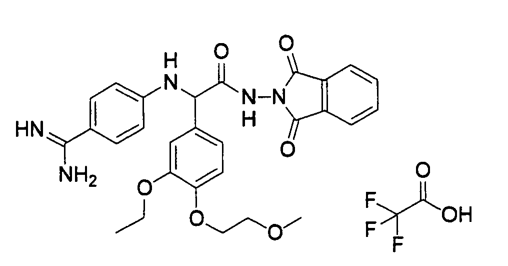 Mass spectrum (ESI) *m*/*z*: 532 (M+H)⁺

(Example X-237) 4-(1-(3-Ethoxy-4-(2-methoxyethoxy)phenyl)-2-(N'-(furan-2-carbonyl)hydrazino)-2-oxoethylamino)benzamidine trifluoroacetate 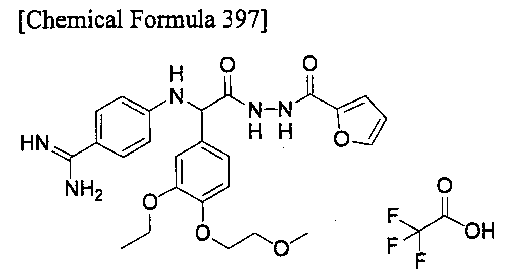 Mass spectrum (ESI) *m*/*z*: 496 (M+H)⁺

(Example X-238) 4-(1-(3-Ethoxy-4-(2-methoxyethoxy)phenyl)-2-oxo-2-(N'-(pyridine-2-carbonyl)hydrazino)ethylamino)benzamidine trifluoroacetate 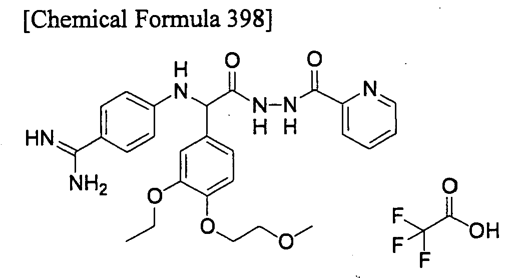 Mass spectrum (ESI) *m*/*z*: 507 (M+H)⁺

(Example X-239) 4-(1-(3-Ethoxy-4-(2-methoxyethoxy)phenyl)-2-oxo-2-(N'-(pyridine-3-carbonyl)hydrazino)ethylamino)benzamidme trifluoroacetate 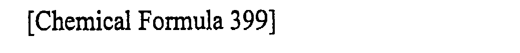 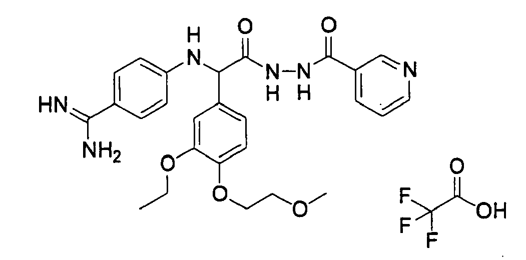 Mass spectrum (ESI) *m*/*z:* 507 (M+H)⁺

(Example X-240) 4-(1-(2-Fluoro-4,5-dimethoxyphenyl)-2-oxo-2-(N'-(1-oxypyridine-2-carbonyl)hydrazino)ethylamino)benzamidine trifluoroacetate 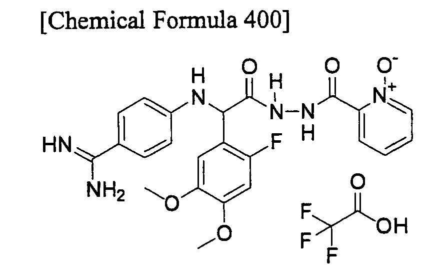 Mass spectrum (ESI) *m*/*z:* 483 (M+H)⁺

(Example X-241) 4-(2-(N'-(4-Chloropyridine-3-carbonyl)hydrazino)-1-(2-fluoro-4,5-dimethoxypheal)-2-oxoethylamino)benzamidine trifluoroacetate 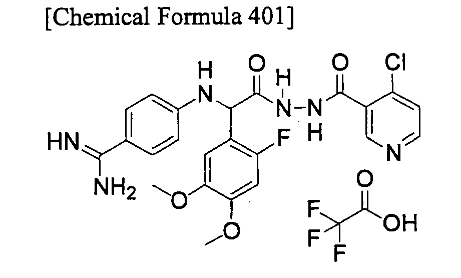 Mass spectrum (ESI) *m*/*z*: 501 (M+H)⁺

(Example X-242) 4-(1-(2-Fluoro-4,5-dimethoxyphenyl)-2-(N'-(4-morpholin-4-ylpylidine-3-carbonyl)hydrazino)-2-oxoethylamino)benzamidine trifluoroacetate 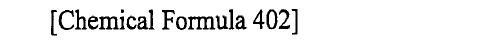 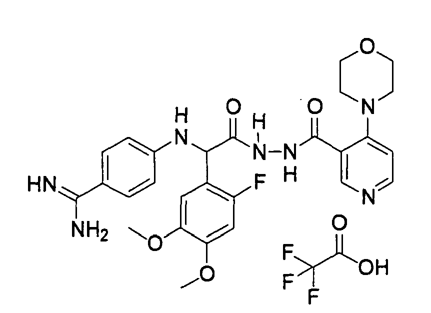 Mass spectrum (ESI) *m*/*z*: 552 (M+H)⁺

(Example X-243) 4-(2-(N'-(2-Fluorobenzoyl)hydrazino)-1-(2-fluoro-4,5-dimethoxyphenyl)-2-oxoethylamino)benzamidine trifluoroacetate 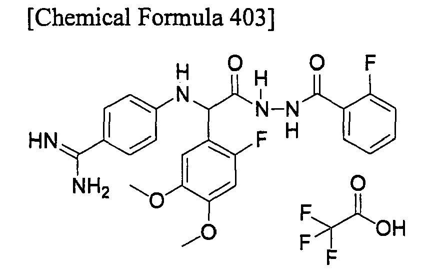 Mass spectrum (ESI) *m*/*z:* 484 (M+H)⁺

(Example X-244) 4-(1-(2-Fluoro-4.5-dimethoxyphenyl)-2-(N'-(2-nitrobenzoyl)hydrazino)-2-oxoethylamino)benzamidine trifluoroacetate 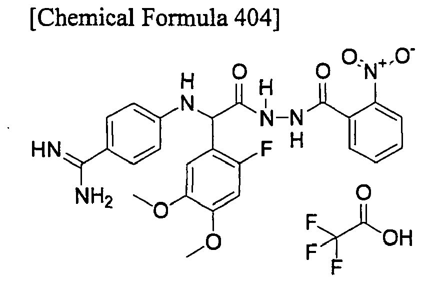 Mass spectrum (ESI) *m*/*z:* 511 (M+H)⁺

(Example X-245) 4-(2-(N'-(2-Dimethylaminobenzoyl)hydrazino)-1-(2-fluoro-4,5-dimethoxyphenyl)-2-oxoethylamino)benzamidine trifluoroacetate 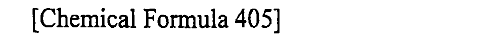 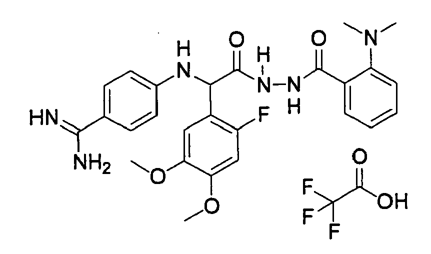 Mass spectrum (ESI) *m*/*z:* 509 (M+H)⁺

(Example X-246) 2-[N'-[2-(4-Carbamimidoylphenylamino)-2-(4-dimethylcarbamoylmethoxy-3-ethoxyphenyl)acetyl]hydrazino]benzoic acid trifluoroacetate 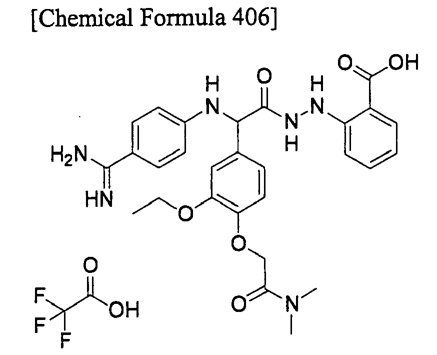 Mass spectrum (ESI) *m*/*z*: 549 [(M+1) (M+1)⁺

(Example X-247) 4-[1-[4-(2-Amino-2-methylpropoxy)-3-ethoxyphenyl]-2-[N'-(2-chlorobenzoyl)-hydrazino]-2-oxoethylamino]benzamidine trifluoroacetate 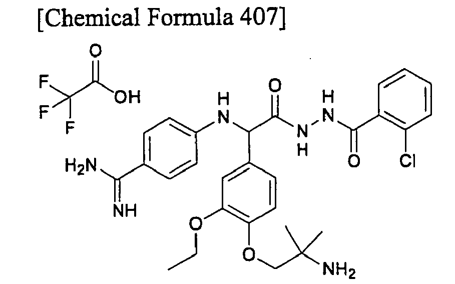 Mass spectrum (ESI) *m*/*z:* 554 (M+1)⁺

(Example X-248) 4-[1-[4-(2-Amino-2-methylpropoxy)-3-ethoxyphenyl]-2-[N'-(3-fluoropyndine-4-carbonyl)-hydrazino]-2-oxoethylamino]benzamidme trifluoroacetate 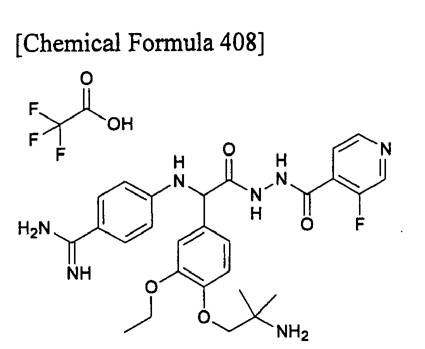 Mass spectrum (ESI) *m*/*z*: 538 (M+1)⁺

(Example X-249) 2-[N'-[2-[4-(2-Amino-2-methylpropoxy)-3-ethoxyohenyl]-2-(4-carbamimidoylphenylamino)acetyl]hydrazino]benzoic acid trifluoroacetate 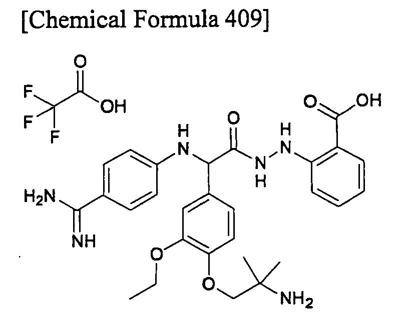 Mass spectrum (ESI) *m*/*z:* 535 (M+1)⁺

(Example X-250) 2-(N'-(2-(4-Carbamimidoylphenylamino)-2-(3-ethoxy-4-(2-methoxyethoxyl)henyl)acetyl)hydrazino)benzoic acid trifluoroacetate 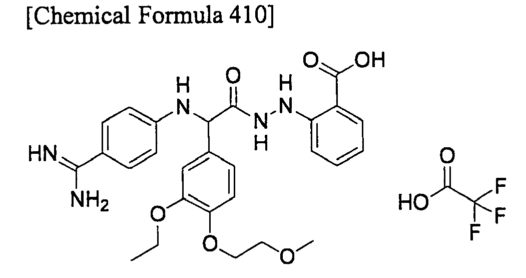 Mass spectrum (ESI) *m*/*z:* 522 (M+1)⁺

(Example X-251) 2-(N'-(2-(4-Carbamimidoylphenylamino)-2-(4-(2-dimethylamino-1-methylethoxy)-3-ethoxyphenyl)acetyl)hydrazino)benzoic acid trifluoroacetate 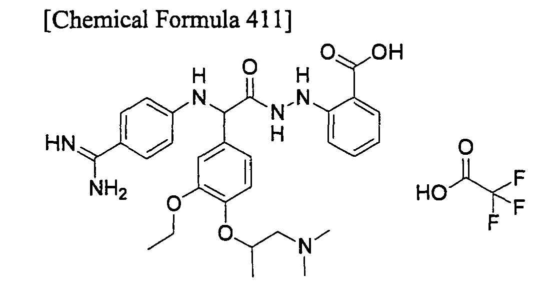 Mass spectrum (ESI) *m*/*z:* 549 (M+1)⁺

(Example X-252) 4-(2-(N'-(2-Chlorobenzoyl)hydrazino)-1-(4-(2-dimethylamino-1-methylethoxy)-3-ethoxyphenyl)-2-oxoethylamino)benzamidine trifluoroacetate 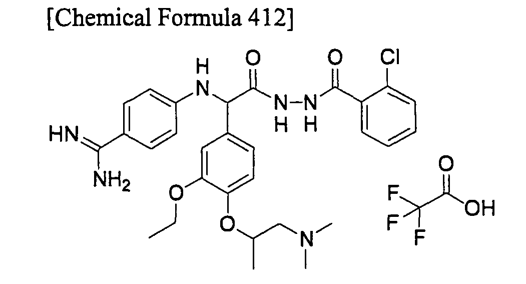 Mass spectrum (ESI) *m*/*z*: 567 (M+1)⁺

(Example X-253) 4-(1-(4-(2-Dimethylamino-1-methylethoxy)-3-ethoxyphenyl)-2-oxo-2(N'-(pyridine-2-carbonyl)hydrazino)ethylamino)benzamidine trifluoroacetate 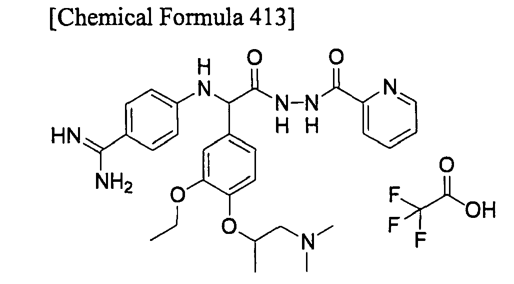 Mass spectrum (ESI) *m*/*z:* 534 (M+1)⁺

(Example X-254) 4-(1-(4-(2-Dimethylamino-1-methylethoxy)-3-ethoxyphenyl)-2-(N'-(3-fluoropyridine-4-carbonyl)hydrazino)-2-oxoethylamino)benzamidine trifluoroacetate 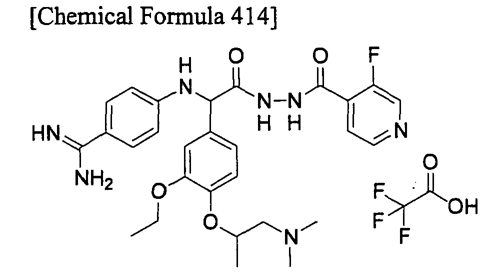 Mass spectrum (ESI) *m*/*z*: 552 (M+1)⁺

(Example X-255) 4-{1-(2-Fluoro-5-methoxy-3-propoxyphenyl)-2-oxo-2-[N'-(pyridine-2-carbonyl)hydrazino]ethylamino}benzamidine trifluoroacetate 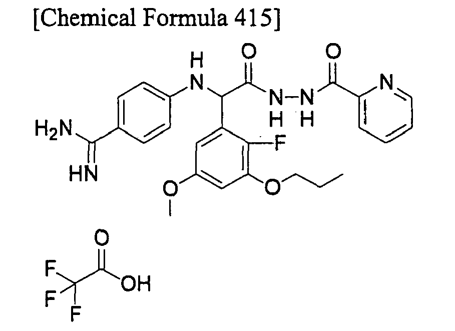 Mass spectrum (ESI) *m*/*z:* 495 (M+1)⁺

(Example X-256) 4-{1-(2-Fluoro-5-methoxy-3-propoxyphenyl)-2-oxo-2-[N'-(pyridine-3-carbonyl)hydrazino]ethylamino}benzamidine trifluoroacetate 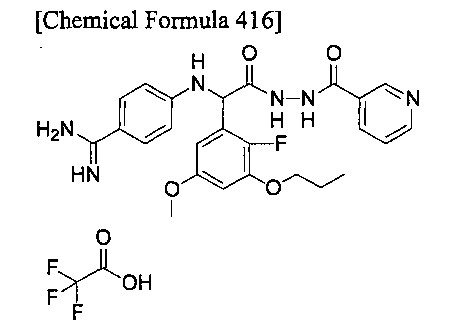 Mass spectrum (ESI) *m*/*z*: 495 (M+1)⁺

(Example X-257) 4-[2-[N'-(2-Chlorobenzoyl)hydrazino]-1-(2-fluoro-5-methoxy-3-propoxyphenyl)-2-oxoethylamino]benzamidine hydrochloride 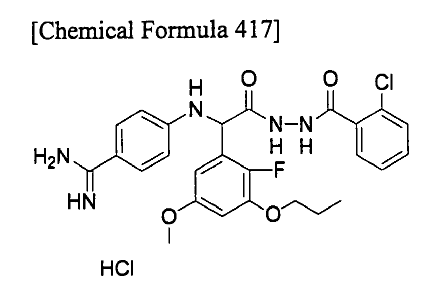 Mass spectrum (ESI) *m*/*z:* 528 (M+1)⁺

(Example X-258) 2-{N'-[2-(4-Carbamimidoylphenylamino)-2-(2-fluoro-5-methoxy-3-propoxyphenyl)acetyl]hydrazino}benzoic acid hydrochloride 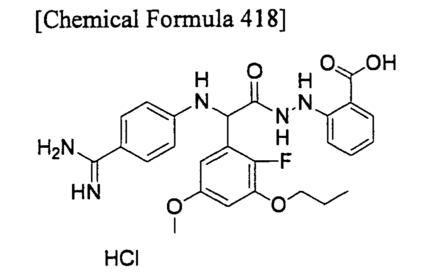 Mass spectrum (ESI) *m*/*z*: 510 (M+1)⁺

(Example X-259) 4-{1-(2-Fluoro-5-methoxy-3-propoxyphenyl)-2-[N'-(3-methylpyridine-2-carbonyl)hydrazinol]-2-oxoethylamino}benzamidine hydrochloride 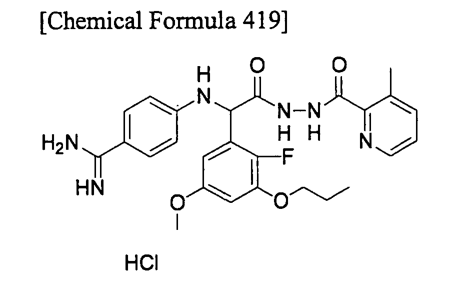 Mass spectrum (ESI) *m*/*z*: 509 (M+1)⁺

(Example X-260) 4-[2-[N'-(3-Bromopyridine-2-carbonyl)hydrazino]-1-(2-fluoro-5-methoxy-3-propoxyphenyl)-2-oxoethylamino]benzamidine hydrochloride 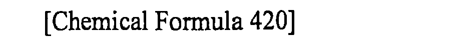 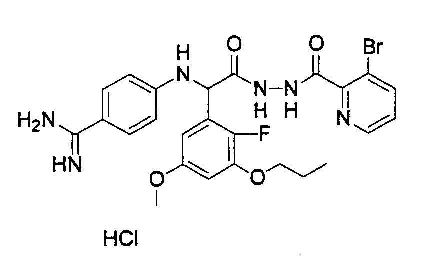 Mass spectrum (ESI) *m*/*z:* 573 (M+1)⁺

(Example X-261) 4-{1-(2-Fluoro-5-methoxy-3-propoxyphenyl)-2-[N'-(4-methylpyridine-3-carbonyl)hydrazino]-2-oxoethylamino}benzamidine hydrochloride 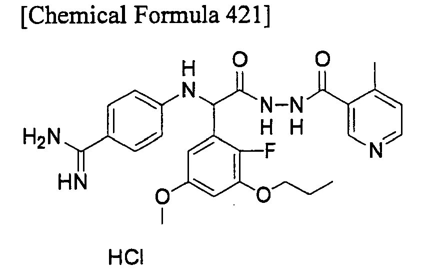 Mass spectrum (ESI) *m*/*z:* 509 (M+1)⁺

(Example X-262) 4-[2-[N'-(4-Chloropyridine-3-carbonyl)hydrazino]-1-(2-fluoro-5-methoxy-3-propoxyphenyl)-2-oxoethylamino]benzamidine hydrochloride 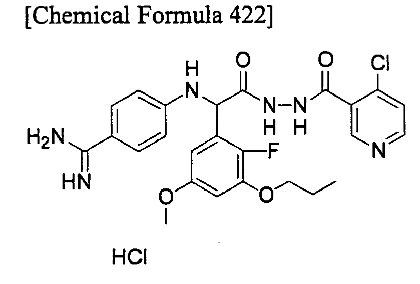 Mass spectrum (ESI) *m*/*z:* 529 (M+1)⁺

(Example X-263) 4-{1-(2-Fluoro-5-methoxy-3-propoxyphenyl)-2-[N'-(2-fluoropyridine-3-carbonyl)hydrazino]-2-oxoethylamino}benzamidine hydrochloride 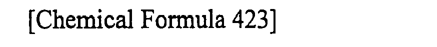 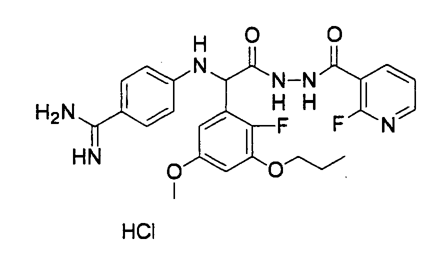 Mass spectrum (ESI) *m*/*z*: 513 (M+1)⁺

(Example X-264) 4-{1-(2-Fluoro-5-methoxy-3-propoxyphenyl)-2-[N'-(3-methylpyridine-4-carbonyl)hydrazino]-2-oxoethylamino}benzamidine hydrochloride 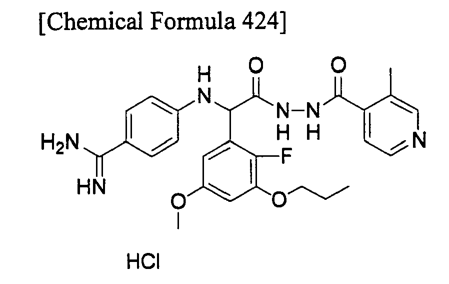 Mass spectrum (ESI) *m*/*z*: 509 (M+1)⁺

(Example X-265) 4-{1-(2-Fluoro-5-methoxy-3-propoxyphenyl)-2-[N'-(3-fluoropyridine-4-carbony)hydrazino]-2-oxoethylamino}benzamidine hydrochloride 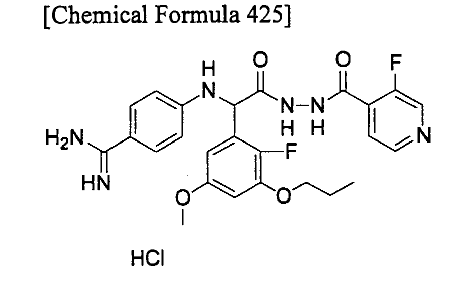 Mass spectrum (ESI) *m*/*z:* 513 (M+1)⁺

(Example X-266) 4-[2-[N'-(3-Chloropyridine-4-carbonyl)hydrazino]-1-(2-fluoro-5-methoxy-3-propoxyphenyl)-2-oxoethylamino]benzamidine hydrochloride 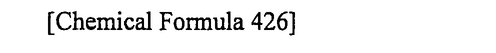 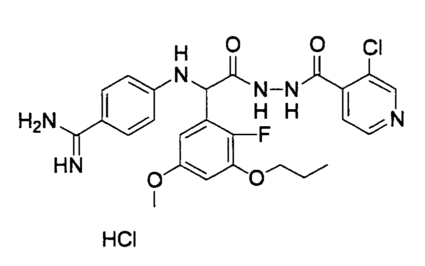 Mass spectrum (ESI) *m*/*z:* 529 (M+1)⁺

(Example X-267) 4-[2-[N'-(3-Bromopyridine-4-carbonyl)hydrazino]-1-(2-fluoro-5-methoxy-3-propoxyphenyl)-2-oxoethylamino]benzamidine hydrochloride 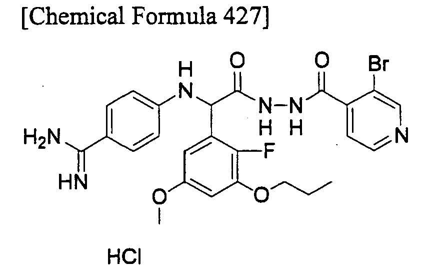 Mass spectrum (ESI) *m*/*z:* 573 (M+1)⁺

(Pharmacological Test Example 1)

### [Inhibitory activity against clotting factor VIIa]

### (1) Method

Dimethylsulfoxide (DMSO) solutions were prepared with compounds of the invention at concentration of 10 mmol/L (10 mmol/L compound solutions).
One packet of tris-hydroxymethylaminomethane-preset (Tris Preset) (product of SIGMA Corp., Catalog No. T8293), 8.8 g of sodium chloride (NaCl) and 1 g of bovine serum albumin (BSA) were dissolved in 1 L of water to prepare a Tris-BSA buffer (100 mmol/L Tris, 0.15 mol/L NaCl, 0.1% BSA, pH 7.4).
The Tris-BSA buffer (180 µL) was added to the aforementioned 10 mmol/L compound solution (20 µL). A 10-fold dilution series was prepared for this mixture using the aforementioned Tris-BSA buffer, and solutions with the compound at concentrations of 1.0 mmol/L, 100, 10, 1, 0.1, 0.01 and 0.001 µmol/L were prepared (1.0 mmol-0.001 µmol/L compound solutions).
As a control, a solution was prepared by 10-fold dilution of DMSO with the Tris-BSA buffer (control 10% solution).
After dissolving one packet of Tris preset, NaCl (8.8 g) and BSA (1 g) in water (about 900 mL), there were added 1 mol/L aqueous calcium chloride (CaCl₂) (15 mL) and 1 mg/mL aqueous cephalin (30 mL), and the total volume was brought to 1 L by adding water. To this solution was added a human tissue factor (TF) sample (product of Calbiochem, Catalog No. 612151) (450 µg) to a TF sample concentration of 10 nmol/L, and then a human clotting factor VIIa (Factor VIIa) purified sample (product of Enzyme Research Laboratories, Catalog No. HFVIIa) (250 µg) was added to a Factor VIIa purified sample concentration of 5 nmol/L, to prepare an enzyme solution (100 mmol/L Tris-HCl, 0.15 mol/L NaCl, 15 mmol/L CaCl₂, 30 µg/mL cephalin, 1 mg/mL BSA, 10 nmol/L TF, 5 nmol/L Factor VIIa).
To 110 µL of this enzyme solution was added 15 µL of each of the 1.0 mmol-0.001 µmol/L compound solutions, and then 25 µL of a 1.0 mmol/L synthetic chromogenic substrate solution (Spectrozyme FVIIa) was added and the mixture was allowed to stand at room temperature for 40 minutes. Next, the amount of 4-nitroanilide released into the solution was quantitated by spectrophotometry (405 nm).
A control measurement was conducted in the same manner, using the control 10% solution instead of the compound solution.
This measurement yielded the enzyme reaction inhibition in the presence of 100 µmol/L to 0.1 nmol/L of each compound of the invention. The enzyme reaction inhibition at each compound concentration was subjected to non-linear regression analysis, and the IC50 value for inhibitory activity of each compound against clotting factor VIIa was calculated.

### (2) Results

Table 1 shows the IC50 value for inhibitory activity of each compound against clotting factor VIIa (IC50 FVIIa (µM)).

**[Table 1]**

| | IC50 of FVIIa (mM) | | IC50 of FVIIa (mM) |
|---|---|---|---|
| Example 2 | 0.096 | Example 60 | 0.203 |
| Example 3 | 0.051 | Example 61 | 0.184 |
| Example 9 | 0.316 | Example 72 | 0.259 |
| Example 10 | 0.233 | Example 76 | 0.169 |
| Example 23 | 0.255 | Example 78 | 0.049 |
| Example 25 | 0.084 | Example 81 | 0.078 |
| Example 27 | 0.069 | Example X-246 | 0.165 |
| Example 32 | 0.110 | Example X-247 | 0.336 |
| Example 33 | 0.057 | Example X-248 | 0.173 |
| Example 34 | 0.063 | Example X-251 | 0.166 |
| Example 53 | 0.164 | Example X-252 | 0.098 |
| Example 56 | 0.198 | Example X-254 | 0.050 |

(Pharmacological Test Example 2)

### [Blood clotting time prolongation]

### (1) Method

Dimethylsulfoxide (DMSO) solutions were prepared with compounds of the invention at concentration of 10 mmol/L (10 mmol/L compound solutions).
One packet of tris-hydroxymethylaminomethane-preset (Tris Preset) (product of SIGMA Corp., Catalog No. T8293), 8.8 g of sodium chloride (NaCl) and 1 g of bovine serum albumin (BSA) were dissolved in 1 L of water to prepare a Tris-BSA buffer (100 mmol/L Tris, 0.15 mol/L NaCl, 0.1% BSA, pH 7.4). The Tris-BSA buffer (180 µL) was added to the aforementioned 10 mmol/L compound solution (20 µL). Compound solutions at concentrations of 1000, 300, 100, 30, 10, 3 and 1 µmol/L were prepared for this mixture using the aforementioned Tris-BSA buffer.
As a control, a solution was prepared by 10-fold dilution of DMSO with the Tris-BSA buffer (control 10% solution).
Measurement of the prothrombin time as an index of extrinsic clotting was accomplished by adding 10 µL of the compound solution to 90 µL of healthy human plasma and incubating at 37°C for 3 minutes. To this was added 100 µL of a 0.5 unit/mL human thromboplastin solution (THROMBOREL S, product of Dade Behring, Marburg, Catalog No. GTS-200A) heated to 37°C, for clotting of the blood. The blood clotting time was measured by the steel ball method.
The blood clotting times were measured in this manner in the presence of 100, 30, 10, 3, 1, 0.3 and 0.1 µmol/L of each compound of the invention. The blood clotting time at each compound concentration was used to calculate the compound concentration which produced 10% prolongation compared to the control clotting time.

### (2) Results

Table 2 shows the blood clotting time for each compound as the compound concentration which produced 10% prolongation compared to the control clotting time (h PT x1.1 (µM)).

**[Table 2]**

| | h PT × 1.1 (mM) | | h PT × 1.1 (mM) |
|---|---|---|---|
| Example 2 | 1.00 | Example 60 | 0.30 |
| Example 3 | 1.00 | Example 61 | 0.41 |
| Example 9 | 1.30 | Example 72 | 1.20 |
| Example 10 | 1.20 | Example 76 | 0.63 |
| Example 23 | 1.20 | Example 78 | 0.42 |
| Example 25 | 1.20 | Example 81 | 1.20 |
| Example 27 | 0.74 | Example X-246 | 0.90 |
| Example 32 | 0.80 | Example X-247 | 0.80 |
| Example 33 | 1.10 | Example X-248 | 0.83 |
| Example 34 | 1.40 | Example X-251 | 0.68 |
| Example 53 | 0.65 | Example X-252 | 0.76 |
| Example 56 | 0.41 | Example X-254 | 0.59 |

### Industrial Applicability

Since the compounds of the invention have excellent suppressing effects against blood clotting and are safe with suitable physicochemical stability, they are useful as medicaments, and especially as therapeutic or prophylactic agents for diseases associated with thrombus formation.

## Claims

1. A compound represented by the following general formula (1) or a salt thereof or a hydrate of the foregoing: 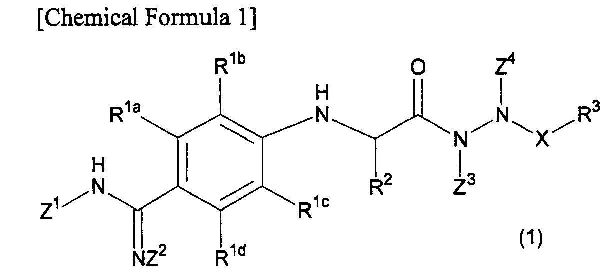 wherein R^{1a}, R^{1b}, R^{1c} and R^{1d} each independently represent hydrogen, C1-6 alkyl or halogen;
R² represents phenyl optionally having 1-5 substituents selected from Group A1 below;
R³ represents hydrogen, C1-6 alkyl, C3-8 cycloalkyl optionally having 1-5 substituents selected from Group A1 below, 5- or 6-membered non-aromatic heterocyclyl optionally having 1-5 substituents selected from Group A1 below, C6-10 aryl optionally having 1-5 substituents selected from Group A1 below, 5- to 10-membered heteroaryl optionally having 1-5 substituents selected from Group A1 below, C6-10 arylmethyl optionally having 1-5 substituents selected from Group A1 below, C6-10 arylamino optionally having 1-5 substituents selected from Group A1 below, 5- to 10-membered heteroarylmethyl optionally having 1-5 substituents selected from Group A1 below or 5- to 10-membered heteroarylamino optionally having 1-5 substituents selected from Group A1 below;
Z¹, Z² and Z³ each independently represent hydrogen or C1-6 alkyl;
Z⁴ represents hydrogen or C1-6 alkyl;
X represents a single bond or -SO₂-, -CO- or -CS-;
Group A1 consists of hydroxyl, halogen, cyano, carboxyl, carbamoyl, nitro, C1-6 alkyl optionally having 1-3 substituents selected from Group B1 below, C3-8 cycloalkyl optionally having 1-5 substituents selected from Group C1 below, C2-6 alkenyl, C2-6 alkynyl, C1-6 alkoxy optionally having 1-3 substituents selected from Group B1 below, C3-8 cycloalkyloxy optionally having 1-5 substituents selected from Group C1 below, C2-6 alkenyloxy, C2-6 alkynyloxy, C1-6 alkylthio, C1-6 alkylsulfinyl, C1-6 alkylsulfonyl, C2-7 alkylcarbonyl, C6-10 aryl optionally having 1-5 substituents selected from Group C1 below, C6-10 aryloxy optionally having 1-5 substituents selected from Group C1 below, 5- to 10-membered heteroaryl optionally having 1-5 substituents selected from Group C1 below, 5- to 10-membered heteroaryloxy optionally having 1-5 substituents selected from Group C1 below, 5- or 6-membered non-aromatic heterocyclyl optionally having 1-5 substituents selected from Group C1 below, 5- or 6-membered non-aromatic heterocyclooxy optionally having 1-5 substituents selected from Group C1 below, and -NR^{1t}-R^{2t} wherein R^{1t} and R^{2t} each independently represent hydrogen, C1-6 alkyl, C2-7 alkylcarbonyl, C6-10 aryl optionally having 1-5 substituents selected from Group C1 below or 5- to 10-membered heteroaryl optionally having 1-5 substituents selected from Group C1 below;
Group B1 consists of halogen, C1-6 alkoxy, C3-8 cycloalkyl, amino, mono(C1-6 alkyl)amino, di(C1-6 alkyl)amino, carbamoyl, mono(C1-6 alkyl)aminocarbonyl, di(C1-6 alkyl)aminocarbonyl, C6-10 aryl optionally having 1-5 substituents selected from Group C1 below and 5- to 10-membered heteroaryl optionally having 1-5 substituents selected from Group C1 below; and
Group C1 consists of halogen, C1-6 alkyl and C1-6 alkoxy.

2. A compound according to claim 1 or a salt thereof or a hydrate of the foregoing, wherein R^{1a}, R^{1b}, R^{1c} and R^{1d} are hydrogen.

3. A compound according to claim 1 or 2 or a salt thereof or a hydrate of the foregoing, wherein Z¹ is hydrogen.

4. A compound according to any one of claims 1 to 3 or a salt thereof or a hydrate of the foregoing, wherein Z² is hydrogen.

5. A compound according to any one of claims 1 to 4 or a salt thereof or a hydrate of the foregoing, wherein Z³ is hydrogen.

6. A compound according to any one of claims 1 to 5 or a salt thereof or a hydrate of the foregoing, wherein Z⁴ is hydrogen.

7. A compound according to any one of claims 1 to 6 or a salt thereof or a hydrate of the foregoing, wherein X is a single bond, -SO₂- or -CO-.

8. A compound according to any one of claims 1 to 6 or a salt thereof or a hydrate of the foregoing, wherein X is a single bond or -CO-.

9. A compound according to any one of claims 1 to 8 or a salt thereof or a hydrate of the foregoing, wherein R² is phenyl optionally having 1-3 substituents selected from Group A2 below;
Group A2 consists of C1-6 alkoxy optionally having a group selected from Group B2 below, C1-6 alkyl, C1-6 alkoxy-C1-6 alkyl, C3-8 cycloalkyloxy, C2-6 alkenyloxy, C2-6 alkynyloxy, benzyloxy, pyridylmethoxy, hydroxyl and halogen; and
Group B2 consists of fluorine, C1-6 alkoxy, amino, mono(C1-6 alkyl)amino, di(C1-6 alkyl)amino, carbamoyl, mono(C1-6 alkyl)aminocarbonyl and di(C1-6 alkyl)aminocarbonyl.

10. A compound according to any one of claims 1 to 8 or a salt thereof or a hydrate of the foregoing, wherein R² is phenyl having 2 or 3 substituents selected from Group A3 below;
Group A3 consists of C1-6 alkoxy optionally having a group selected from Group B3 below, C2-6 alkenyloxy, hydroxyl, and halogen; and
Group B3 consists of fluorine, C1-6 alkoxy, amino, mono(C1-6 alkyl)amino, di(C1-6 alkyl)amino and di(C1-6 alkyl)aminocarbonyl.

11. A compound according to any one of claims 1 to 8 or a salt thereof or a hydrate of the foregoing, wherein R² is phenyl having 2 or 3 substituents selected from Group A4 below; and
Group A4 consists of methoxy, ethoxy, isopropoxy, n-propoxy, dimethylaminocarbonylmethoxy, 2-dimethylaminoethoxy, 2-amino-2-methylpropoxy, 2-dimethylamino-1-methylethoxy, 2-methoxyethoxy, allyloxy, hydroxyl, fluorine and 2-fluoroethoxy.

12. A compound according to any one of claims 1 to 8 or a salt thereof or a hydrate of the foregoing, wherein R² is phenyl having 2 or 3 substituents represented by the following formula: 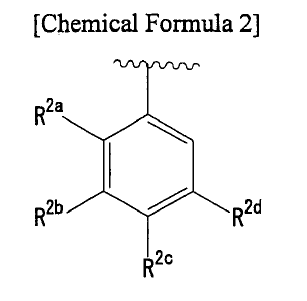 wherein R^{2a} represents hydrogen or fluorine;
R^{2b} represents hydrogen, methoxy, isopropoxy, n-propoxy, allyloxy or 2-fluoroethoxy; and
R^{2c} and R^{2d} each independently represent hydrogen, methoxy, ethoxy, isopropoxy, n-propoxy, dimethylaminocarbonylmethoxy, 2-dimethylaminoethoxy, 2-amino-2-methylpropoxy, 2-dimethylamino-1-methylethoxy, 2-methoxyethoxy, allyloxy, hydroxyl, fluorine or 2-fluoroethoxy.

13. A compound according to any one of claims 1 to 8 or a salt thereof or a hydrate of the foregoing, wherein R² is 3-ethoxy-4-dimethylaminocarbonylmethoxyphenyl, 3,4-diallyloxyphenyl, 3-ethoxy-4-(2-dimethylaminoethoxy)phenyl, 3-ethoxy-4-(2-methoxyethoxy)phenyl, 3,4-diethoxyphenyl, 3,5-dimethoxy-4-hydroxyphenyl, 3-ethoxy-6-fluoro-4-isopropoxyphenyl, 3-ethoxy-4-isopropoxyphenyl, 3-methoxy-4-isopropoxyphenyl, 3,4-dimethoxy-6-fluorophenyl, 3,4,5-trimethoxyphenyl, 3-methoxy-5-n-propoxy-6-fluorophenyl, 3-methoxy-5-allyloxy-6-fluorophenyl, 3-methoxy-5-isopropoxy-6-fluorophenyl, 3-methoxy-5-(2-fluoroethoxy)-6-fluorophenyl, 4-(2-amino-2-methylpropoxy)-3-ethoxyphenyl or 4-(2-dimethylamino-1-methylethoxy)-3-ethoxyphenyl.

14. A compound according to any one of claims 1 to 13 or a salt thereof or a hydrate of the foregoing, wherein R³ is 5- or 6-membered non-aromatic heterocyclyl optionally having 1-3 substituents selected from Group D1 below, phenyl optionally having 1-3 substituents selected from Group D1 below, 5- to 10-membered heteroaryl optionally having 1-3 substituents selected from Group D1 below, C6-10 arylmethyl optionally having 1-3 substituents selected from Group D1 below or 5- to 10-membered heteroarylmethyl optionally having 1-3 substituents selected from Group D1 below; and
Group D1 consists of hydroxyl, halogen, cyano, nitro, carboxyl, carbamoyl, C2-7 alkylcarbonyl, C1-6 alkyl, C1-6 alkyl having 1-3 halogen, C1-6 alkoxy, C1-6 alkylthio, C1-6 alkylsulfonyl, amino, mono(C1-6 alkyl)amino, di(C1-6 alkyl)amino, mono(C2-7 alkylcarbonyl)amino, di(C2-7 alkylcarbonyl)amino, phenyl, phenoxy, benzyloxy, 5-tetrazolyl, pyrrolyl and morpholino.

15. A compound according to any one of claims 1 to 13 or a salt thereof or a hydrate of the foregoing, wherein R³ is phenyl, indazolyl, furyl, pyridyl, N-oxypyridyl, pyrimidinyl, thienyl, pyrazinyl, benzotriazolyl, pyridonyl, benzyl, pyridylmethyl or thienylmethyl and R³ optionally has 1-3 substituents selected from Group D2 below; and
Group D2 consists of hydroxyl, fluorine, chlorine, bromine, cyano, nitro, carboxyl, carbamoyl, acetyl, methyl, ethyl, trifluoromethyl, methoxy, ethoxy, n-propoxy, isopropoxy, benzyloxy, phenoxy, methylthio, methylsulfonyl, amino, methylamino, dimethylamino, acetylamino, phenyl, 5-tetrazolyl, pyrrolyl and morpholino.

16. A compound according to any one of claims 1 to 13 or a salt thereof or a hydrate of the foregoing, wherein R³ is (1) phenyl optionally having a group selected from the group consisting of hydroxyl, fluorine, chlorine, bromine, cyano, nitro, carboxyl, acetylamino, methylsulfonyl and methoxy, (2) pyridyl optionally having a group selected from the group consisting of fluorine, chlorine, bromine, methyl, dimethylamino and carboxyl, (3) N-oxypyridyl, (4) pyrimidinyl, (5) N-methylpyridonyl, (6) pyridylmethyl or (7) thienylmethyl.

17. A compound according to any one of claims 1 to 13 or a salt thereof or a hydrate of the foregoing, wherein R³ is 2-pyridyl, 3-bromopyridin-2-yl, 3-carboxypyridin-2-yl, 3-methylpyridin-2-yl, 3-pyridyl, 2-chloropyridin-3-yl, 2-fluoropyridin-3-yl, 2-methylpyridin-3-yl, 4-chloropyridin-3-yl, 4-methylpyridin-3-yl, 2-(dimethylamino)pyridin-3-yl, 4-pyridyl, 3-bromopyridin-4-yl, 3-chloropyridin-4-yl, 3-fluoropyridin-4-yl, 3-methylpyridin-4-yl, 2-pyrimidinyl, (pyridine-N-oxide)-3-yl, (pyridine-N-oxide)-2-yl, 3-pyridylmethyl, 3-thienylmethyl, N-methyl-2-pyridon-5-yl, N-methyl-4-pyridon-3-yl, phenyl, 2-bromophenyl, 2-chlorophenyl, 2-fluorophenyl, 2-cyanophenyl, 2-carboxyphenyl, 2-nitrophenyl, 2-methoxyphenyl, 2-methylsulfonylphenyl, 4-acetylaminophenyl or 4-hydroxyphenyl.

18. A medicament comprising a compound according to any one of claims 1 to 17 or a salt thereof or a hydrate of the foregoing.

19. A therapeutic or prophylactic agent for a disease associated with thrombus formation, comprising a compound according to any one of claims 1 to 17 or a salt thereof or a hydrate of the foregoing.

20. A therapeutic or prophylactic agent for a disease selected from Group E1 below, comprising a compound according to any one of claims 1 to 17 or a salt thereof or a hydrate of the foregoing,
wherein Group E1 consists of thrombosis, deep vein thrombosis, pulmonary embolism, cerebral infarction, myocardial infarction, vascular restenosis, disseminated intravascular coagulation syndrome and malignant tumor.

21. A therapeutic or prophylactic agent for a disease selected from Group E2 below, comprising a compound according to any one of claims 1 to 17 or a salt thereof or a hydrate of the foregoing,
wherein Group E2 consists of thrombosis, deep vein thrombosis, pulmonary embolism, cerebral infarction, myocardial infarction, vascular restenosis and disseminated intravascular coagulation syndrome.

22. A compound represented by the following general formula (1-2) or a salt thereof or a hydrate of the foregoing: 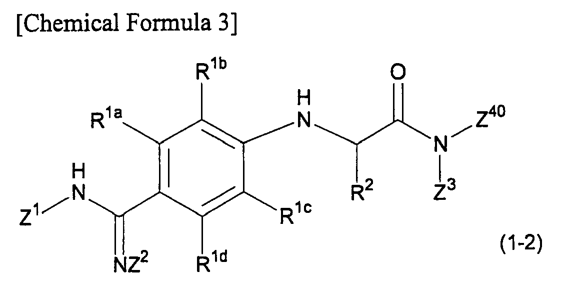 wherein Z⁴⁰ is morpholino or a group represented by the formula: 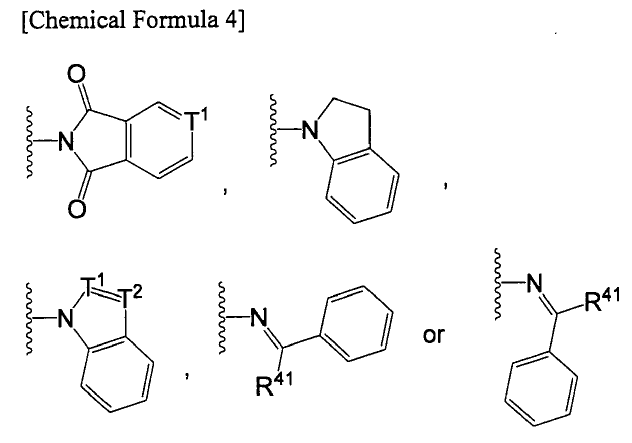 wherein R⁴¹ represents hydrogen or C1-6 alkyl and T¹ and T² each independently represent methine or nitrogen and Z⁴⁰ optionally has 1-3 substituents selected from Group A1 recited in claim 1; and
R^{1a}, R^{1b}, R^{1c}, R^{1d}, R², Z¹, Z² and Z³ have the same definitions as R^{1a}, R^{1b}, R^{1c} R^{1d}, R², Z¹, Z² and Z³ recited in claim 1.
